(19) Europäisches Patentamt
European Patent Office
Office européen des brevets

(11) EP 4 467 546 A1

(12) **EUROPEAN PATENT APPLICATION**
published in accordance with Art. 153(4) EPC

(43) Date of publication:
**27.11.2024 Bulletin 2024/48**

(21) Application number: **23742678.8**

(22) Date of filing: **04.01.2023**

(51) International Patent Classification (IPC):
***C07D 487/04*** (2006.01) ***C07D 487/08*** (2006.01)
***C07D 491/04*** (2006.01) ***A61K 31/519*** (2006.01)
***A61P 29/02*** (2006.01) ***A61P 35/00*** (2006.01)
***A61P 37/00*** (2006.01)

(52) Cooperative Patent Classification (CPC):
**A61K 31/519; A61K 31/5386; A61P 29/00; A61P 29/02; A61P 31/00; A61P 35/00; A61P 37/00; A61P 37/06; C07D 487/04; C07D 487/08; C07D 491/04; C07D 519/00;** Y02P 20/55

(86) International application number:
**PCT/CN2023/070285**

(87) International publication number:
**WO 2023/138362 (27.07.2023 Gazette 2023/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **18.01.2022 CN 202210053373**

(71) Applicant: **Jiangsu Tasly Diyi Pharmaceutical Co., Ltd.**
**Huai'an, Jiangsu 223003 (CN)**

(72) Inventors:
- **SONG, Li**
  **Jiangsu 223003 (CN)**
- **TANG, Hai**
  **Jiangsu 223003 (CN)**
- **MA, Xiaohui**
  **Jiangsu 223003 (CN)**
- **ZHOU, Shuiping**
  **Jiangsu 223003 (CN)**
- **CAI, Jinyong**
  **Jiangsu 223003 (CN)**
- **DONG, Liming**
  **Jiangsu 223003 (CN)**
- **SONG, Zhuang**
  **Jiangsu 223003 (CN)**

(74) Representative: **Gulde & Partner**
**Patent- und Rechtsanwaltskanzlei mbB**
**Wallstraße 58/59**
**10179 Berlin (DE)**

# (54) WEE1 INHIBITOR, PREPARATION THEREFOR, AND USE THEREOF

(57) The present invention relates to a compound of formula (I), or a stereoisomer thereof, or a pharmaceutically acceptable salt thereof, and a use thereof in the preparation of a drug for treating diseases related to WEE1 activity.

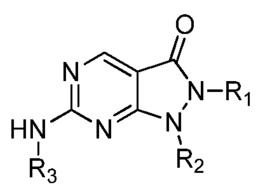

I

EP 4 467 546 A1

## Description

### TECHNICAL FIELD

The present invention relates to compounds that inhibit WEE1 kinase activity, especially to the compounds represented by Formula (I), or a stereoisomer thereof, or a pharmaceutically acceptable salt thereof. The present invention also relates to the preparation method of these compounds, pharmaceutical compositon thereof, and the use thereof in the preparation of the drug for the treatment of WEE1 activity related diseases.

### BACKGROUND ART

Wee1 tyrosine kinase is the checkpoint of G2 phase of cell cycle. Cell cycle is tightly regulated and controlled. When the cellular DNA is not damaged, the checkpoints of G1, S and G2 phases promote cells to enter division phase to ensure the successful completion of cell cycle (Clinical Cancer Research,2011,17(13):4200-4207). Cell cycle is regulated and controlled by CDKs (Cyclin-dependent kinases). CDKs family comprises 14 kinds of serine/threonine protein kinases. The activity of CDK is regulated and controlled by phosphorylation and the binding of different cyclins. The transition of cells from G2 phase to division phase is positively regulated by the phosphorylation of CDK1(also called CDC2) and its associated cyclin B. CDK1 is in an inactive state before divison and is phosphorylated by WEE1 in tyrosine 15, and then phosphorylated by myelin transcription factor (MYT1) in threonine 14. Therefore, WEE1 is a negative regulator of cell cycle that negatively regulates the passage of cells from G2 phase to division phase by preventing cyclin B and activated CDK1 complexes from entering the nucleus. The expression and activity of WEE1 are both increased in S and G2 phases and decreased in the highly phosphorylated M phase. When cells enter G2 phase and no DNA damage occurs, polo-like protein kinase 1 (PLK1) phosphorylates WEE1, which is degraded by the ubiquitin ligase complex. PLK1 also phosphorylates and activates the protein phosphatase cell division cycle 25 analog (CDC25), which activates CDK1 by dephosphorylation. Active CDK1 binds to cyclin B and promotes cell entry into division phase (Molecular&CellularBiology, 2012, 32(20):4226).

When a cell's DNA is damaged, the checkpoints of G1, S, and G2 phases delay the cell's entry into division phase, buying time to repair the damaged DNA before the cell enters division, thus ensuring the integrity of the genome. The key regulator of the G1 phase checkpoint P53 is in a mutated form in many malignant cells (Proceedings of the National Academy of Sciences of the United States of America,2007,104(10):3753-3758 ). The tumor cells with defective P53 function fail to block the cell cycle in G1 phase when DNA is damaged, and are therefore more dependent on the G2 phase checkpoint. In response to DNA damage, the G2 phase checkpoint inhibits CDK1 phosphorylation through two parallel and interconnected pathways, thereby delaying cell entry into division phase. Depending on the type of DNA damage, ataxia telangiectasia mutated (ATM) protein kinase or ataxia telangiectasia-related (ATR) protein kinase is activated. (Oncotarget, 2016, 7 (31):49902-49916)

ATM is activated by ionizing radiation, radioactive agents, and agents that cause double-stranded DNA breaks. ATM phosphorylates and activates checkpoint kinase 2 (CHK2), CHK2 phosphorylates Ser216 of cell division cycle 25C phosphatase (CDC25C). This leads to a nuclear export and cytoplasmic segregation of CDC25C, thereby inhibiting its phosphorylation activity. Inhibition of CDC25C activity leads to inhibition of CDK1/CDK2 binding cyclin B complex phosphorylation, which puts CDK1 in an inactivated form and inhibits cell entry into division (MolecularCancer, 2014, 13(1):72).

ATR is activated by a wide range of genotoxic stimuli that cause single-stranded DNA breaks. ATR is the main kinase responsible for the phosphorylation and activation of CHK1. In contrast to CHK2, which can only be activated by ATM, CHK1 can be activated by both ATM and ATR. CHK1 phosphorylates both WEE1 and CDC25C, activates WEE1 kinase activity and inhibits CDC25C phosphatase activity. WEE1 phosphorylates CDK1-binding cyclin B, leading to cell cycle arrest in G2 phase and providing time for DNA repair (Drug News&Perspectives,2010,23(7):425).

WEE1 is overexpressed in many malignant tumors, such as hepatocellular carcinoma, breast cancer, malignant glioma, melanoma, adult and pediatric brain tumors. Part of these tumor cells have abnormal G1 checkpoints, and inhibition of WEE1 activity leads to G2 phase checkpoint malfunction, at this time cells with unrepaired damaged DNA will continue to divide and eventually divide to death (Molecular Cancer Therapeutics, 2013,12(12):2675-2684). Inhibition of WEE1 activity, whether by pyrimidine derivatives (PD0166285) or small interfering RNA knockdown, will make ovarian, colon, cervical, osteosarcoma, malignant glioma, and lung cancer cells more sensitive to DNA damage produced by radiation and topoisomerase inhibition. Therefore, WEE1 inhibitors have a wide scope for development both as single drug and concomitant drugs (Cancer Biology&Therapy, 2010, 9(7):523-525).

Small molecule compounds with WEE1 kinase inhibitory activity were disclosed in the patent applications of WO2007126122, WO2008133866, WO2013012681, WO2013126656, WO2014167347, WO2015092431, WO2018011569, WO2018011570, WO2018090939, WO2018133829, WO2018171633, etc. At present, the compound with the fastest development progress is AZD1775, which has entered the phase II clinical trial and shows favorable cancer

treatment results. But, AZD1775 also has rather obvious adverse reactions, and drug activity and efficacy need to be improved. Therefore, it is necessary to develop Wee1 inhibitors with higher safety and wider range of applications.

## SUMMARY OF THE INVENTION

The present invention provides a compound represented by formula I, or a stereoisomer thereof, or a pharmaceutically acceptable salt thereof:

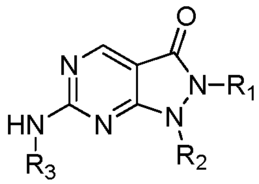

I

wherein, $R_1$ is selected from a group consisting of $-C_{1\sim6}$ alkyl, $-C_{2\sim6}$ alkenyl, $-C_{2\sim6}$ alkynyl, $-C_{0\sim2}$ alkylene-CN, $-C_{0\sim2}$ alkylene-(3-10-membered cycloalkyl), $-C_{0\sim2}$ alkylene-(3-10-membered heterocycloalkyl); wherein said $-C_{0\sim2}$ alkylene, $-C_{1\sim6}$ alkyl, $-C_{2\sim6}$ alkenyl, $-C_{2\sim6}$ alkynyl, 3~10-membered cycloalkyl, 3~10-membered heterocycloalkyl are optionally substituted by one, two, three or four independent $R^{11}$;

$R^{11}$ is selected from a group consisting of H,$-C_{1\sim6}$ alkyl, halogen substituted $-C_{1\sim6}$ alkyl, halogen;
$R_2$ is selected from a group consisting of

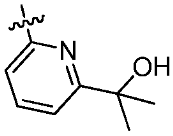 , 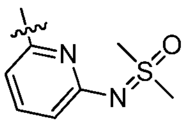 , 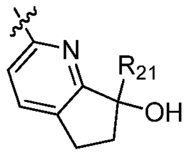 ,

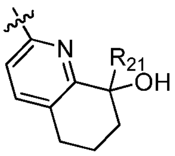 , 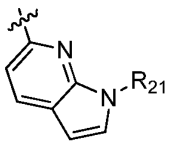 , 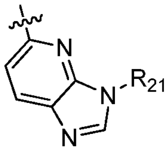 , 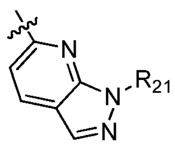 , 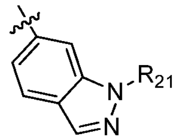 , 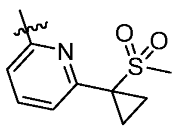 ,

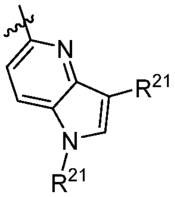 , 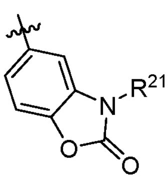 , 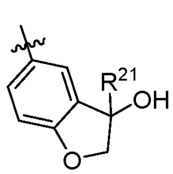 , 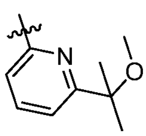 , 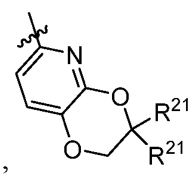 , 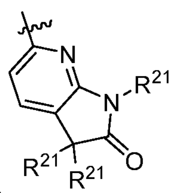 , 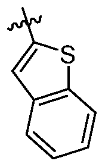 ,

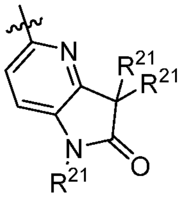 ;

$R^{21}$ is selected from a group consisting of H, $-C_{1\sim6}$ alkyl, halogen substituted $-C_{1\sim6}$ alkyl, $-C_{0\sim2}$ alkylene-$C(O)OC_{1\sim6}$ alkyl, $-C_{0\sim2}$ alkylene-COOH, $-C(O)C_{1\sim6}$ alkyl, halogen;
$R_3$ is selected from a group consisting of

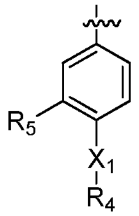, 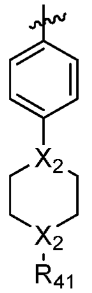, 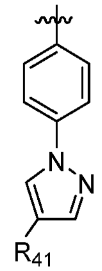, 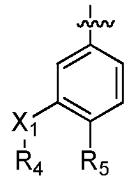,

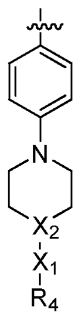, 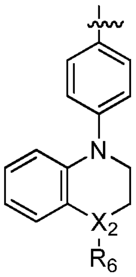, 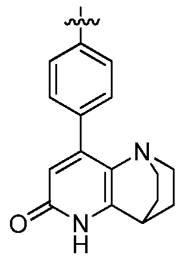, 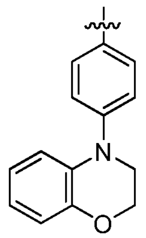, 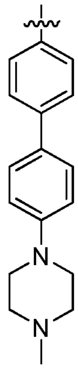, 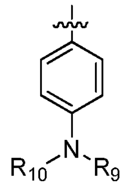,, 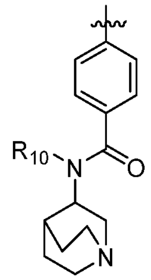, 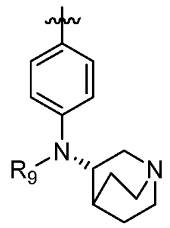,

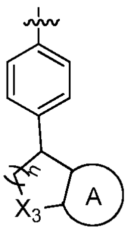, 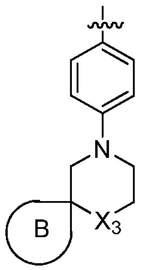, 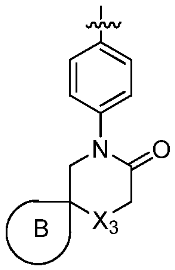, 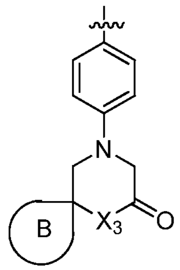, 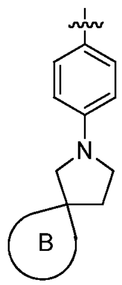, 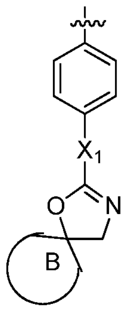, 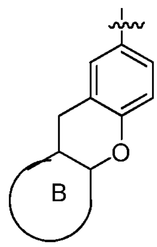,

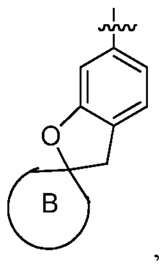, 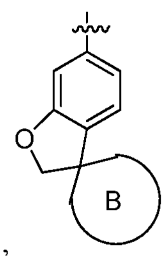, 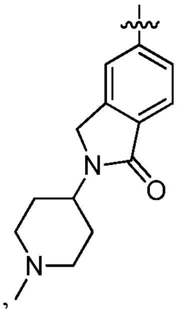, 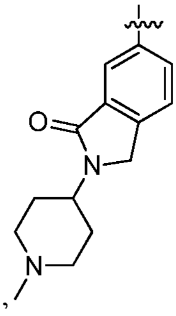, 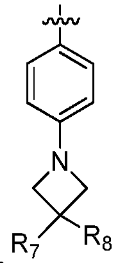, 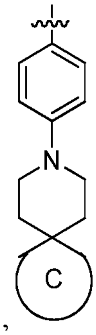, 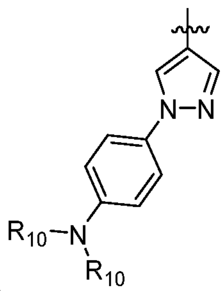;

$X_1$ is selected from a group consisting of chemical bond, O, $NR^{N1}$ or $CR^{C1}R^{C2}$; $X_2$ is selected from N or $CR^{C1}$; $X_3$ is selected from a group consisting of O, $NR^{N1}$ or $CR^{C1}R^{C2}$;

n is selected from 1, 2 or 3;

$R^{N1}$ is selected from a group consisting of H, halogen, -OH, $-C_{1\sim6}$ alkyl, halogen substituted $-C_{1\sim6}$ alkyl, $-O(C_{1\sim6}$ alkyl), -O(halogen substituted $-C_{1\sim6}$ alkyl), $-NH_2$, $-NH(C_{1\sim6}$ alkyl), $-N(C_{1\sim6}$ alkyl)( $C_{1\sim6}$ alkyl), $-C(O)(C_{1\sim6}$ alkyl);

$R^{C1}$, $R^{C2}$ are independently selected from a group consisting of H, halogen, cyano, nitro, -OH, $-C_{1\sim6}$ alkyl, halogen substituted $-C_{1\sim6}$ alkyl, $-O(C_{1\sim6}$ alkyl), -O(halogen substituted $-C_{1\sim6}$ alkyl), $-NH_2$, $-C_{0\sim2}$ alkylene-$NH(C_{1\sim6}$ alkyl), $-C_{0\sim2}$ alkylene-$N(C_{1\sim6}$ alkyl)( $C_{1\sim6}$ alkyl);

$R_4$ is selected from -(5~12-membered bridged heterocylcylalkyl); wherein said bridged heterocylcylalkyl is optionally substituted by one, two, three or four independent $R^{41}$;

$R^{41}$ is selected from a group consisting of H, oxo, $-C_{1\sim6}$ alkyl, $-C_{2\sim6}$ alkenyl, halogen substituted $-C_{1\sim6}$ alkyl, hydroxy-substituted $-C_{1\sim6}$ alkyl, halogen, cyano, nitro, -OH, $-C_{0\sim2}$ alkylene-O($C_{1\sim6}$ alkyl), -O(halogen substituted $-C_{1\sim6}$ alkyl), $-NH_2$, -NH($C_{1\sim6}$ alkyl), -N($C_{1\sim6}$ alkyl)( $C_{1\sim6}$ alkyl), $-C_{0\sim2}$ alkylene-C(O)$R^{42}$, $-C_{0\sim2}$ alkylene-C(O)N$R^{42}R^{43}$, $-C_{0\sim2}$ alkylene-C(O)O$R^{42}$, $-C_{0\sim2}$ alkylene-S(O)$R^{42}$, $-C_{0\sim2}$ alkylene-S(O)N$R^{42}R^{43}$, $-C_{0\sim2}$ alkylene-S(O)O$R^{42}$, $-C_{0\sim2}$ alkylene-$S(O)_2R^{42}$, $-C_{0\sim2}$ alkylene-$S(O)_2NR^{42}R^{43}$, $-C_{0\sim2}$ alkylene-$S(O)_2OR^{42}$, $-C_{0\sim2}$ alkylene-(3-10-membered cycloalkyl), $-C_{0\sim2}$ alkylene-(3-10-membered heterocycloalkyl), $-C_{0\sim2}$ alkylene-(5~ 10-membered aromatic cyclyl), $-C_{0\sim2}$ alkylene-(5~ 10-membered aromatic heterocyclyl); wherein said alkyl, alkylene, cycloalkyl, heterocycloalkyl, aromatic cyclyl, aromatic heterocyclyl are optionally substituted by one, two, three or four independent $R^{44}$;

$R^{42}$, $R^{43}$ are independently selected from a group consisting of H, halogen, cyano, nitro, -OH , $-C_{1\sim6}$ alkyl, halogen substituted $-C_{1\sim6}$ alkyl, -O($C_{1\sim6}$ alkyl),-O(halogen substituted $-C_{1\sim6}$ alkyl), $-NH_2$, -NH($C_{1\sim6}$ alkyl), -N($C_{1\sim6}$ alkyl)( $C_{1\sim6}$ alkyl), $-C_{0\sim2}$ alkylene-(3-10-membered cycloalkyl), $-C_{0\sim2}$ alkylene-(3-10-membered heterocycloalkyl), $-C_{0\sim2}$ alkylene-(5-10-membered aromatic cyclyl), $-C_{0\sim2}$ alkylene-(5~ 10-membered aromatic heterocyclyl);

$R^{44}$ is selected from a group consisting of H, oxo, halogen, cyano, nitro, -OH, $-C_{1\sim6}$ alkyl, halogen substituted $-C_{1\sim6}$ alkyl, -O($C_{1\sim6}$ alkyl), -O(halogen substituted $-C_{1\sim6}$ alkyl), $-NH_2$, $-C_{0\sim2}$ alkylene-NH($C_{1\sim6}$ alkyl), -N($C_{1\sim6}$ alkyl)( $C_{1\sim6}$ alkyl);

or, $R^{41}$ and $R^{C1}$ , or $R^{C2}$ together with the atom adjacent therewith form 3~10-membered carbocyclyl, 3~10-membered heterocyclyl;

$R_5$ is selected from a group consisting of H, oxo, halogen, cyano, nitro, -OH, $-NH_2$, $-C_{1\sim6}$ alkyl, halogen substituted $-C_{1\sim6}$ alkyl, hydroxy-substituted $-C_{1\sim6}$ alkyl, -O($C_{1\sim6}$ alkyl), -O(halogen substituted $-C_{1\sim6}$ alkyl), $-C_{0\sim2}$ alkylene-NH($C_{1\sim6}$ alkyl), -O-$C_{0\sim2}$ alkylene-N($C_{1\sim6}$ alkyl)( $C_{1\sim6}$ alkyl), $-C_{0\sim2}$ alkylene-N($C_{1\sim6}$ alkyl)( $C_{1\sim6}$ alkyl), $-C_{0\sim2}$ alkylene-(3-10-membered cycloalkyl), $-C_{0\sim2}$ alkylene-(3-10-membered heterocycloalkyl), -O-(3~10-membered cycloalkyl), -O-$C_{0\sim2}$ alkylene-(3~10-membered heterocycloalkyl), $-C_{0\sim2}$ alkylene-(5-10-membered aromatic cyclyl), $-C_{0\sim2}$ alkylene-(5-10-membered aromatic heterocyclyl); wherein said alkyl, alkylene, cycloalkyl, heterocycloalkyl, aromatic cyclyl, aromatic heterocyclyl are optionally substituted by one, two, three or four independent $R^{51}$;

$R^{51}$ is selected from a group consisting of H, $-C_{1\sim6}$ alkyl, halogen substituted $-C_{1\sim6}$ alkyl, oxo, halogen, cyano, nitro, -OH, -O($C_{1\sim6}$ alkyl), -O(halogen substituted $-C_{1\sim6}$ alkyl), $-NH_2$, $-C_{0\sim2}$ alkylene-NH($C_{1\sim6}$ alkyl), -N($C_{1\sim6}$ alkyl)( $C_{1\sim6}$ alkyl);

each $R_6$ is independently selected from a group consisting of H, $-C_{1\sim6}$ alkyl, halogen substituted $-C_{1\sim6}$ alkyl, halogen, cyano, nitro, -OH, -O($C_{1\sim6}$ alkyl), -O(halogen substituted $-C_{1\sim6}$ alkyl), $-NH_2$, -NH($C_{1\sim6}$ alkyl), -N($C_{1\sim6}$ alkyl)( $C_{1\sim6}$ alkyl), $-C_{0\sim2}$ alkylene-(3-10-membered cycloalkyl), $-C_{0\sim2}$ alkylene-(3-10-membered heterocycloalkyl), $-C_{0\sim2}$ alkylene-(5-10-membered aromatic cyclyl), $-C_{0\sim2}$ alkylene-(5~10-membered aromatic heterocyclyl), $-C_{0\sim2}$ alkylene-(5~ 12-membered spirocyclyl), $-C_{0\sim2}$ alkylene-(5~ 12-membered spiro heterocyclyl), $-C_{0\sim2}$ alkylene-(5~ 12-membered bridged cyclyl), $-C_{0\sim2}$ alkylene-(5~ 12-membered bridged heterocyclyl);

wherein,

A ring is selected from a group consisting of 5-12-membered spirocyclyl, 5-12-membered spiro heterocyclyl, 5-12-membered bridged cyclyl, 5~12-membered bridged heterocyclyl; wherein said spirocyclyl, spiro heterocyclyl, bridged cyclyl, bridged heterocyclyl are optionally substituted by one, two, three or four independent $R^{A1}$;

each $R^{A1}$ is independently selected from a group consisting of H, halogen, cyano, nitro, -OH, $-C_{1\sim6}$ alkyl, halogen substituted $-C_{1\sim6}$ alkyl, -O($C_{1\sim6}$ alkyl), -O(halogen substituted $-C_{1\sim6}$ alkyl), $-NH_2$, -NH($C_{1\sim6}$ alkyl), -N($C_{1\sim6}$ alkyl)( $C_{1\sim6}$ alkyl), $-C_{0\sim2}$ alkylene-(3-10-membered cycloalkyl), $-C_{0\sim2}$ alkylene-(3-10-membered heterocycloalkyl), $-C_{0\sim2}$ alkylene-(5-10-membered aromatic cyclyl), $-C_{0\sim2}$ alkylene-(5-10-membered aromatic heterocyclyl), $-C_{0\sim2}$ alkylene-(5-12-membered spirocyclyl), $-C_{0\sim2}$ alkylene-(5-12-membered spiro heterocyclyl), $-C_{0\sim2}$ alkylene-(5-12-membered bridged cyclyl), $-C_{0\sim2}$ alkylene-(5-12-membered bridged heterocyclyl);

wherein,

B ring is selected from a group consisting of 3~10-membered carbocyclyl, 3-10-membered heterocyclyl, 5~10-membered aromatic cyclyl, 5~10-membered aromatic heterocyclyl, 5-12-membered spirocyclyl, 5-12-membered spiro heterocyclyl, 5-12-membered bridged cyclyl, 5~12-membered bridged heterocyclyl, 3~10-membered fused cyclyl, 3~10-membered fused heterocyclyl ; wherein said carbocyclyl, heterocyclyl, aromatic cyclyl, aromatic heterocyclyl, spirocyclyl, spiro heterocyclyl, bridged cyclyl, bridged heterocyclyl, fused cyclyl, fused heterocyclyl are optionally substituted by one, two, three or four independent $R^{B1}$;

each $R^{B1}$ is independently selected from a group consisting of H, $-C_{1\sim6}$ alkyl, halogen substituted $-C_{1\sim6}$ alkyl, halogen, cyano, nitro, -OH, -O($C_{1\sim6}$ alkyl), -O(halogen substituted $-C_{1\sim6}$ alkyl), $-NH_2$, -NH($C_{1\sim6}$ alkyl), -N($C_{1\sim6}$ alkyl)( $C_{1\sim6}$ alkyl), $-C_{0\sim2}$ alkylene-(3-10-membered cycloalkyl), $-C_{0\sim2}$ alkylene-(3-10-membered heterocycloalkyl), $-C_{0\sim2}$ alkylene-(5-10-membered aromatic cyclyl), $-C_{0\sim2}$ alkylene-(5~ 10-membered aromatic heterocyclyl);

$R_7$, $R_8$ are independently selected from a group consisting of H, halogen, cyano, nitro, -OH, $-C_{1\sim6}$ alkyl, halogen substituted $-C_{1\sim6}$ alkyl, -O($C_{1\sim6}$ alkyl), -O(halogen substituted $-C_{1\sim6}$ alkyl), $-NH_2$, -NH($C_{1\sim6}$ alkyl), -N($C_{1\sim6}$ alkyl)( $C_{1\sim6}$ alkyl), $-C_{0\sim2}$ alkylene-(3-10-membered cycloalkyl), $-C_{0\sim2}$ alkylene-(3-10-membered heterocycloalkyl), $-C_{0\sim2}$ alkylene-(5-10-membered aromatic cyclyl), $-C_{0\sim2}$ alkylene-(5-10-membered aromatic heterocyclyl);

or $R_7$, $R_8$ together with the atom adjacent therewith form 3~10-membered fused cyclyl, 3~10-membered fused heterocyclyl, 3~10-membered spirocyclyl, 3~10-membered spiro heterocyclyl; wherein said fused cyclyl, fused heterocyclyl, spirocyclyl, spiro heterocyclyl are optionally substituted by one, two, three or four independent $R^{71}$;

each $R^{71}$ is independently selected from a group consisting of H, -$C_{1\sim6}$ alkyl, halogen substituted -$C_{1\sim6}$ alkyl, halogen, cyano, nitro, -OH, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl);

wherein,

C ring is selected from a group consisting of 5-12-membered spirocyclyl, 5-12-membered spiro heterocyclyl, 5-12-membered bridged cyclyl, 5~12-membered bridged heterocyclyl, 3-10-membered fused cyclyl, 3~10-membered fused heterocyclyl; wherein said spirocyclyl, spiro heterocyclyl, bridged cyclyl, bridged heterocyclyl, fused cyclyl, fused heterocyclyl are optionally substituted by one, two, three or four independent $R^{C1}$;

each $R^{C1}$ is independently selected from a group consisting of H, halogen, cyano, nitro, -OH, -$C_{1\sim6}$ alkyl, halogen substituted -$C_{1\sim6}$ alkyl, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl), -$C_{0\sim2}$ alkylene-(3-10-membered cycloalkyl), -$C_{0\sim2}$ alkylene-(3-10-membered heterocycloalkyl), -$C_{0\sim2}$ alkylene-(5-10-membered aromatic cyclyl), -$C_{0\sim2}$ alkylene-(5-10-membered aromatic heterocyclyl), -$C_{0\sim2}$ alkylene-(5-12-membered spirocyclyl), -$C_{0\sim2}$ alkylene-(5-12-membered spiro heterocyclyl), -$C_{0\sim2}$ alkylene-(5-12-membered bridged cyclyl), -$C_{0\sim2}$ alkylene-(5-12-membered bridged heterocyclyl);

$R_{10}$ is selected from a group consisting of H, -$C_{1\sim6}$ alkyl, halogen substituted -$C_{1\sim6}$ alkyl;

$R_9$ is selected from -$C_{0\sim2}$ alkylene-C(O)$NR^{91}R^{92}$, -C(O)$C_{1\sim6}$ alkyl; each $R^{91}$, $R^{92}$ is independently selected from a group consisting of H, -$C_{1\sim6}$ alkyl, halogen substituted -$C_{1\sim6}$ alkyl;

or $R^{91}$, $R^{92}$ together with the atom adjacent therewith form 3~10-membered heterocyclyl; wherein said heterocyclyl is optionally substituted by one, two, three or four independent $R^{93}$,

each $R^{93}$ is independently selected from a group consisting of H, -$C_{1\sim6}$ alkyl, halogen substituted -$C_{1\sim6}$ alkyl, halogen, cyano, nitro, -OH, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl);

when $R_3$ is

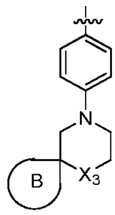
,

B ring is 3-membered carbocyclyl and $R_1$ is

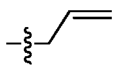
,

$R_2$ is not

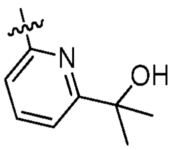
;

when $R_3$ is

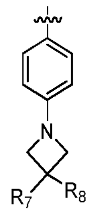

,

$R_7$, $R_8$ are both H and $R_1$ is

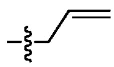

,

$R_2$ is not

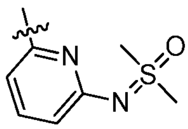

;

when $R_3$ is

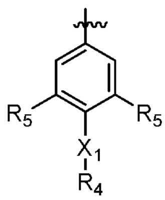

,

$X_1$ is bond, $R_4$ is

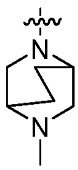

, and $R_1$ is

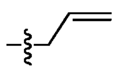

,

$R_2$ is not

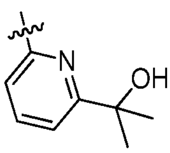

;

when $R_3$ is

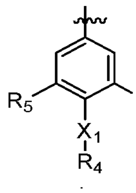,

$X_1$ is bond, $R_4$ is

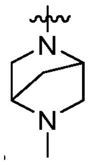

and $R_1$ is

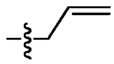,

$R_2$ is not

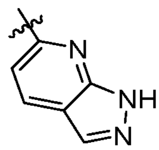;

when $R_3$ is

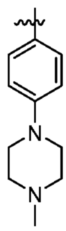

and $R_1$ is

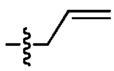,

$R_2$ is selected from a group consisting of

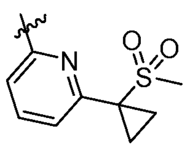, 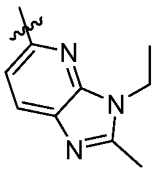, 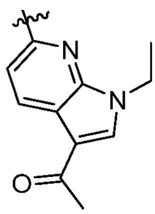, 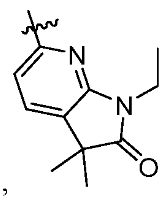, 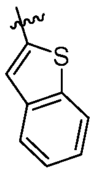, 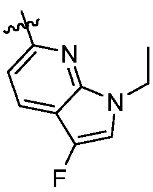 or 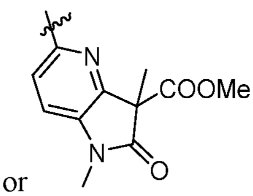,

when $R_3$ is

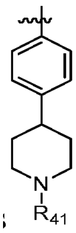

; ;

and $R_1$ is

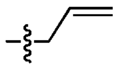

,

$R_2$ is not selected from

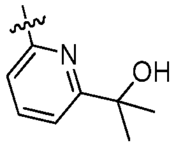

,

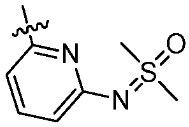

or

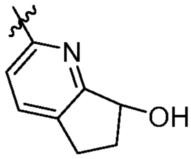

,

when $R_2$ is

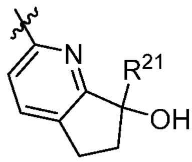

,

$R_{21}$ is methyl and $R_1$ is

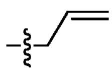

,

$R_3$ is not

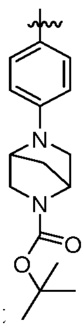

; or

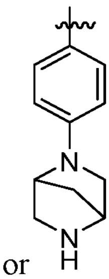

;

when $R_2$ is

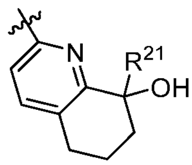

,

$R_{21}$ is H and $R_1$ is

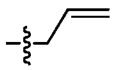

,

$R_3$ is not

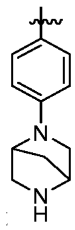

;

when $R_2$ is

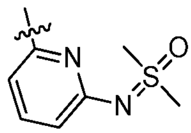

,

$R_{21}$ is H and $R_1$ is

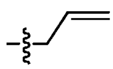

,

$R_3$ is not selected from

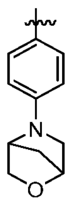

,

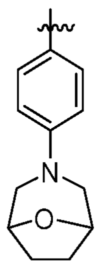

or

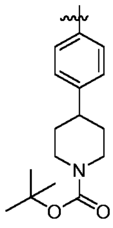

when $R_2$ is

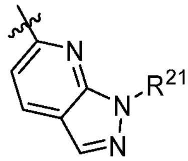

,

$R_{21}$ is H and $R_1$ is

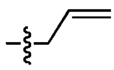

,

$R_3$ is not

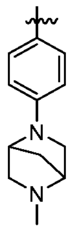

;

when $R_2$ is

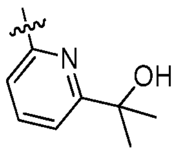

and $R_1$ is

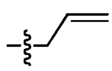

,

$R_3$ is not selected from

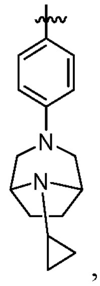 , 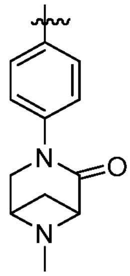 ,

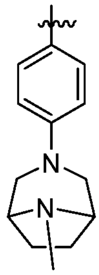 or 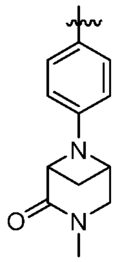 .

More preferably, in the compound of the present invention, or a stereoisomer thereof, or a pharmaceutically acceptable salt thereof, $R_1$ is selected from a group consisting of methyl, ethyl, propyl, isopropyl, cyclopropyl,

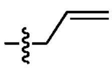 , 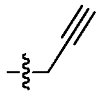 , 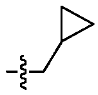 , 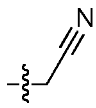 , 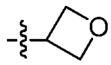 ,

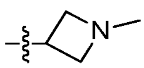 , 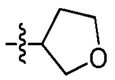 .

More preferably, in the compound of the present invention, or a stereoisomer thereof, or a pharmaceutically acceptable salt thereof, $R_2$ is selected from a group consisting of

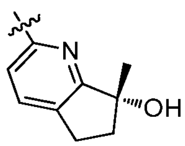 , 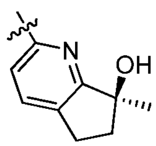 , 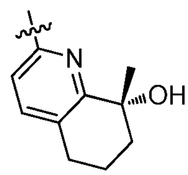 , 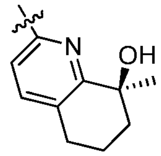 , 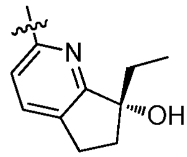 , 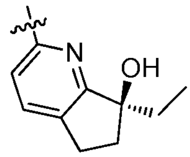 ,

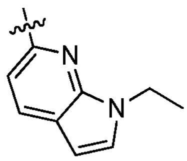 , 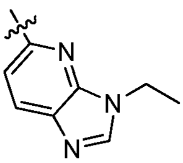 , 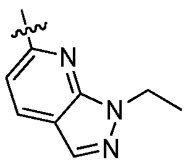 , 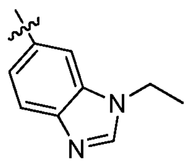 , 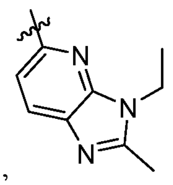 , 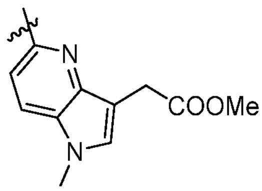 ,

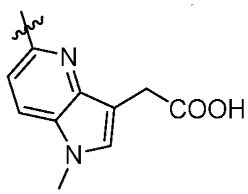 , 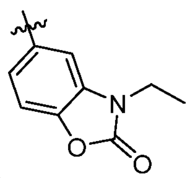 , 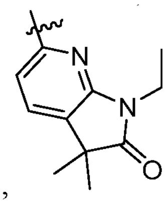 , 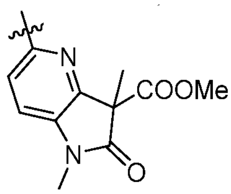 , 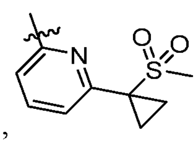 , 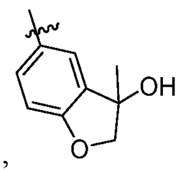 ,

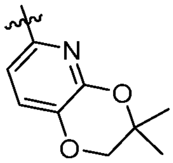, 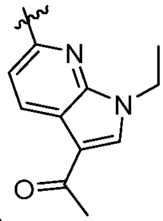, 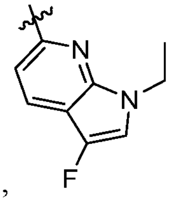

More preferably, in the compound of the present invention, or a stereoisomer thereof, or a pharmaceutically acceptable salt thereof, $X_1$ is selected from a group consisting of chemical bond, O, $NR^{N1}$ or $CR^{C1}R^{C2}$; $R^{C1}$, $R^{C2}$ are independently selected from a group consisting of H, $-C_{1\sim3}$ alkyl, halogen, halogen substituted $-C_{1\sim3}$ alkyl, $-C_{1\sim2}$ alkylene-$N(C_{1\sim6}$ alkyl)( $C_{1\sim6}$ alkyl);

$R^{N1}$ is selected from a group consisting of H, $-C_{1\sim3}$ alkyl, halogen substituted $-C_{1\sim3}$ alkyl, -(3~10-membered heterocycloalkyl). More preferably, in the compound of the present invention, or a stereoisomer thereof, or a pharmaceutically acceptable salt thereof, $R_4$ is selected from a group consisting of

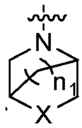, 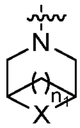, 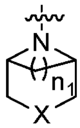, 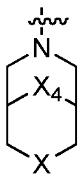, 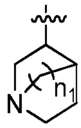, 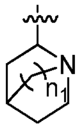, 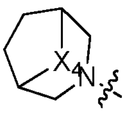;

wherein X is selected from O or $NR^{41}$; $X_4$ is selected from O, NH or $CH_2$; $n_1$ is selected from 1 or 2; wherein said $R_4$ is optionally substituted by one, two, three or four independent $R^{41}$;

$R^{41}$ is selected from a group consisting of H, oxo, $-C_{1\sim3}$ alkyl, $-C_{2\sim6}$ alkenyl, halogen substituted $-C_{1\sim3}$ alkyl, hydroxy-substituted $-C_{1\sim6}$ alkyl, $-C_{0\sim2}$ alkylene-$O(C_{1\sim3}$ alkyl), $-NH_2$, $-NH(C_{1\sim3}$ alkyl), $-N(C_{1\sim3}$ alkyl)( $C_{1\sim3}$ alkyl), $-C(O)R^{42}$, $-C(O)NR^{42}R^{43}$, $-C(O)OR^{42}$, -(3~10-membered cycloalkyl), - (3-10-membered heterocycloalkyl), $-C_{1\sim2}$ alkylene-(5-10-membered aromatic cyclyl); wherein said alkyl, alkylene, cycloalkyl, heterocycloalkyl, aromatic cyclyl are optionally substituted by one, two, three or four independent R44;

$R^{42}$, $R^{43}$ are independently selected from a group consisting of H, halogen, -OH, $-C_{1\sim3}$ alkyl, halogen substituted $-C_{1\sim3}$ alkyl;

$R^{44}$ is selected from a group consisting of H, $-C_{1\sim3}$ alkyl, halogen substituted $-C_{1\sim3}$ alkyl, $-O(C_{1\sim3}$ alkyl).

More preferably, in the compound of the present invention, or a stereoisomer thereof, or a pharmaceutically acceptable salt thereof, $R_4$ is selected specifically from a group consisting of

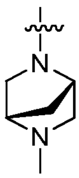, 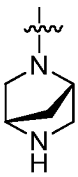, 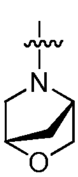, 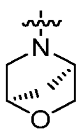, 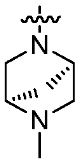, 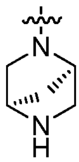, 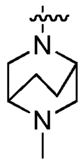, 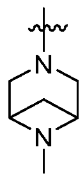, 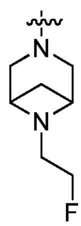, 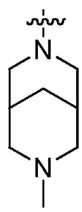, 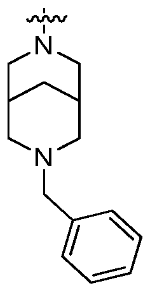, 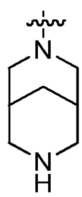,

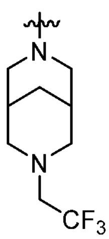, 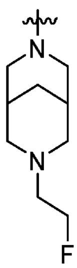, 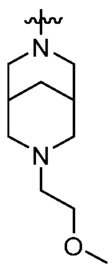, 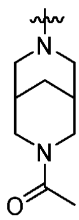, 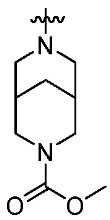, 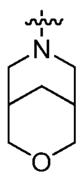, 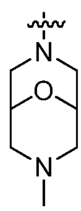, 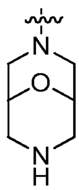, 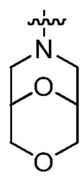, 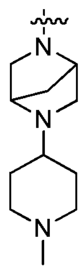, 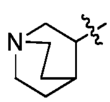,

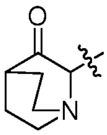, 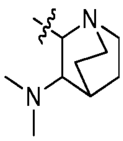, 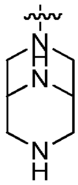, 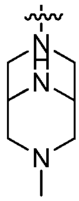, 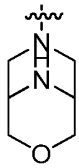, 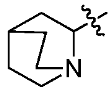, 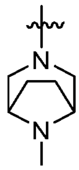, 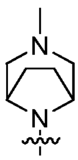, 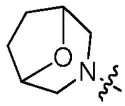, 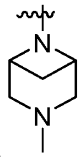, 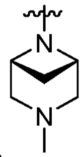, 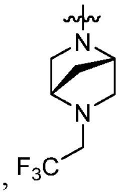,

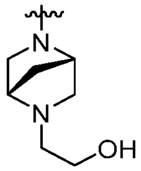, 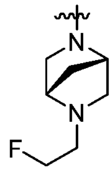, 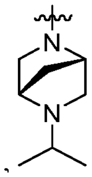, 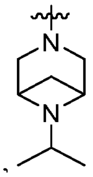, 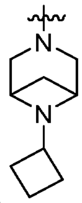, 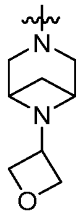, 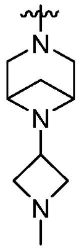, 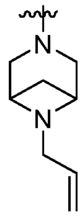, 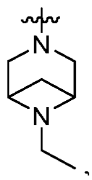, 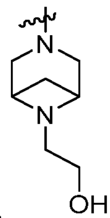, 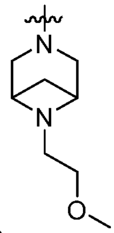, 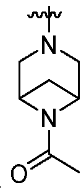,

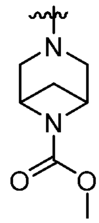.

More preferably, in the compound of the present invention, or a stereoisomer thereof, or a pharmaceutically acceptable salt thereof, $R_5$ is selected from a group consisting of H, halogen, -$C_{1\sim3}$ alkyl, halogen substituted -$C_{1\sim3}$ alkyl, hydroxy-substituted -$C_{1\sim3}$ alkyl, -O($C_{1\sim3}$ alkyl), -NH($C_{1\sim3}$ alkyl), -O-$C_2$ alkylene-N($C_{1\sim3}$ alkyl)( $C_{1\sim3}$ alkyl), -O-(3~10-membered cycloalkyl), -O-(3~10-membered heterocycloalkyl); wherein said alkyl, alkylene, cycloalkyl, heterocycloalkyl are optionally substituted by one, two, three or four independent $R^{51}$;

$R^{51}$ is selected from a group consisting of H, -$C_{1\sim3}$ alkyl, halogen substituted -$C_{1\sim3}$ alkyl.

More preferably, in the compound of the present invention, or a stereoisomer thereof, or a pharmaceutically acceptable salt thereof, $R_5$ is selected from a group consisting of methyl, ethyl, ethoxyl, F, hydroxy-substituted methyl,

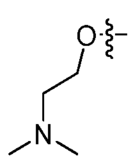, 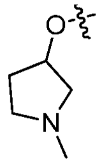

More preferably, in the compound of the present invention, or a stereoisomer thereof, or a pharmaceutically acceptable salt thereof, $R_6$ is selected from a group consisting of H, -$C_{1\sim3}$ alkyl, -NH($C_{1\sim3}$ alkyl), -N($C_{1\sim3}$ alkyl)( $C_{1\sim3}$ alkyl).

More preferably, in the compound of the present invention, or a stereoisomer thereof, or a pharmaceutically acceptable salt thereof, A ring is selected from

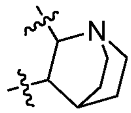

More preferably, in the compound of the present invention, or a stereoisomer thereof, or a pharmaceutically acceptable salt thereof, B ring is selected from a group consisting of 3~10-membered heterocyclyl, 5-12-membered bridged heterocyclyl, 3~10-membered fused heterocyclyl; wherein said heterocyclyl, bridged heterocyclyl, fused heterocyclyl are optionally substituted by one, two, three or four independent $R^{B1}$;

each $R^{B1}$ is independently selected from a group consisting of H, -$C_{1\sim3}$ alkyl, halogen substituted -$C_{1\sim3}$ alkyl.

More preferably, B ring is selected from a group consisting of

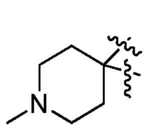, 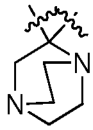, 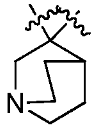,

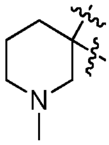, 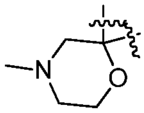, 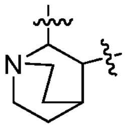, 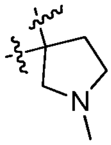, 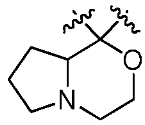, 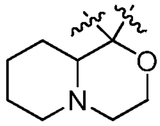

More preferably, in the compound of the present invention, or a stereoisomer thereof, or a pharmaceutically acceptable salt thereof, C ring is selected from a group consisting of

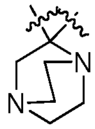, 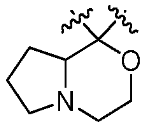, 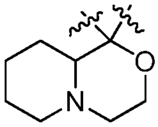

More preferably, in the compound of the present invention, or a stereoisomer thereof, or a pharmaceutically acceptable salt thereof, $R_7$, $R_8$ are independently selected from a group consisting of -OH,

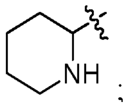;

or $R_7$, $R_8$ together with the atom adjacent therewith form

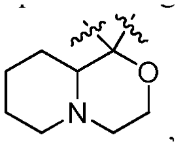,

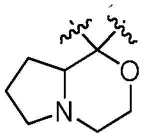.

More preferably, in the compound of the present invention, or a stereoisomer thereof, or a pharmaceutically acceptable salt thereof, $R_3$ is selected from a group consisting of

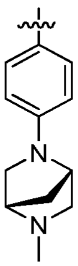, 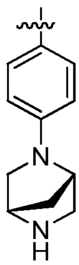, 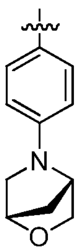, 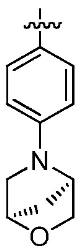, 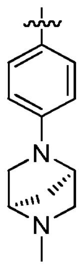, 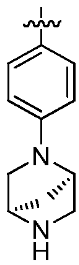, 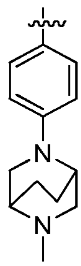, 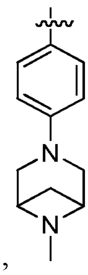, 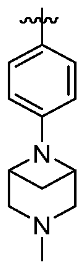, 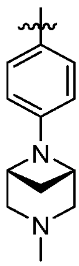, 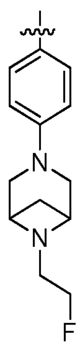, 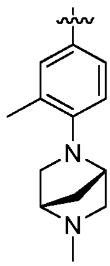, 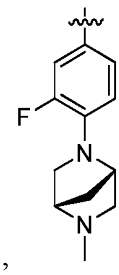,

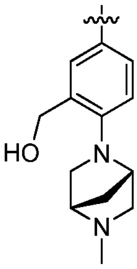, 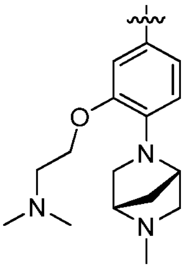, 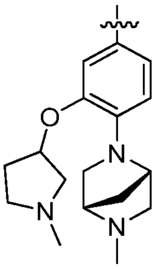, 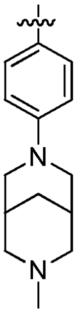, 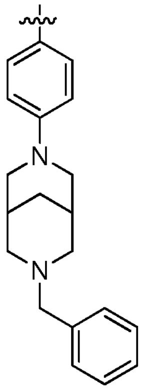, 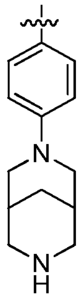, 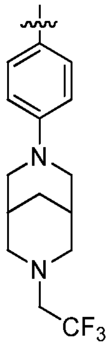, 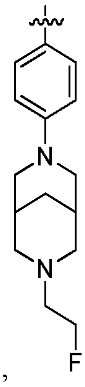, 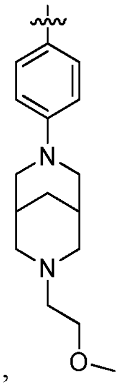, 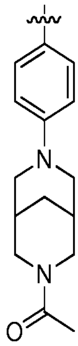,

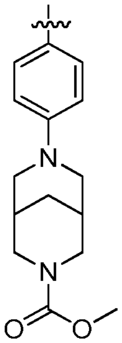, 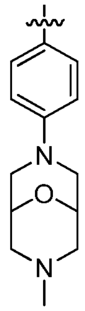, 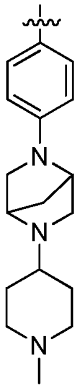, 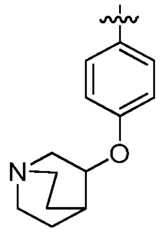, 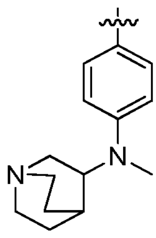, 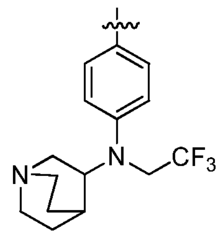, 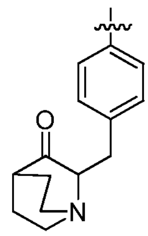, 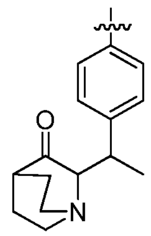,

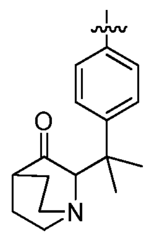, 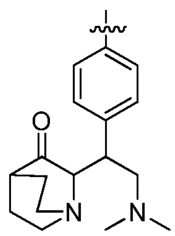, 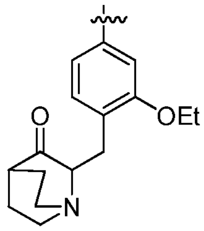, 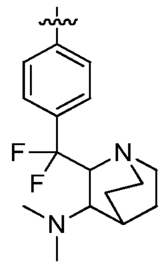, 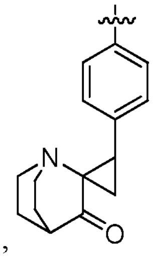, 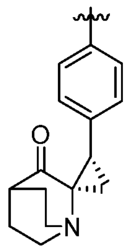, 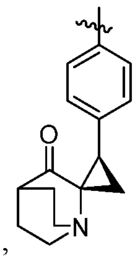, 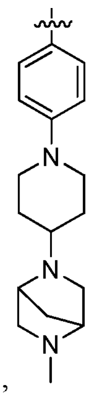,

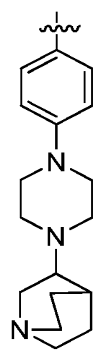, 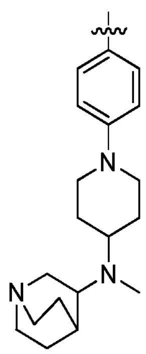, 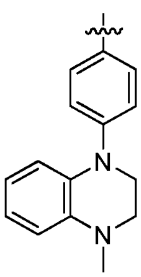, 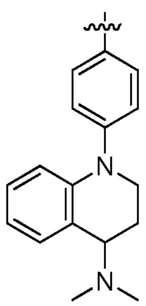, 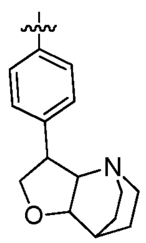, 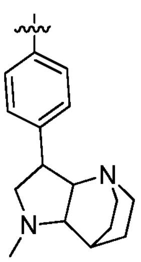, 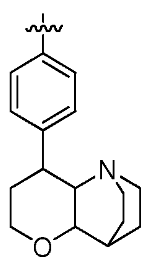, 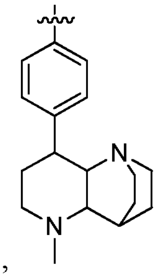,

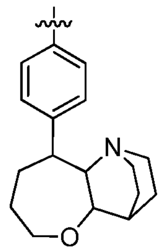 , 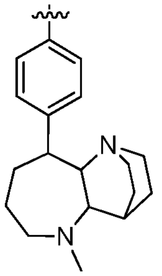 , 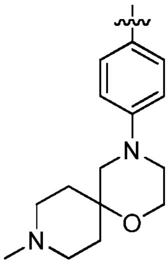 , 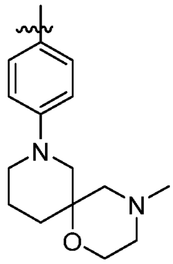 , 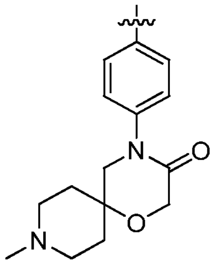 , 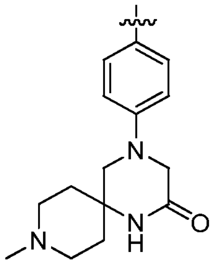 ,

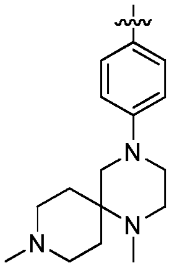 , 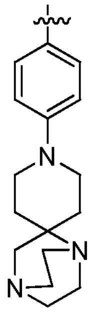 , 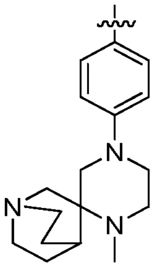 , 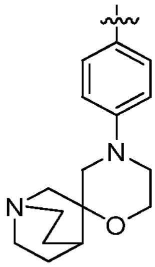 , 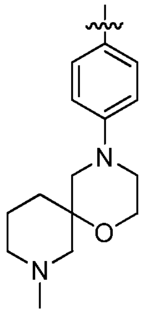 , 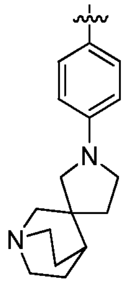 , 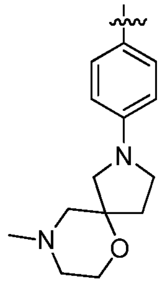 , 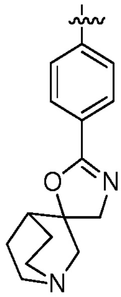 ,

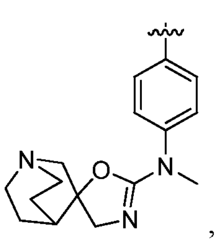 , 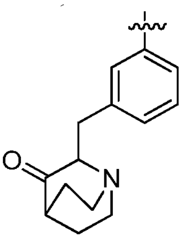 , 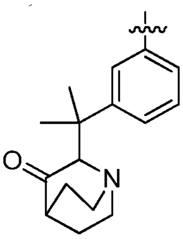 , 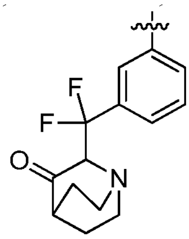 , 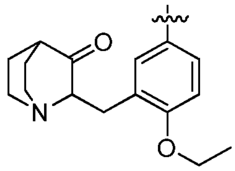 , 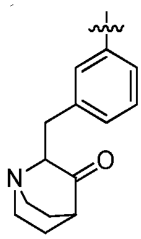 ,

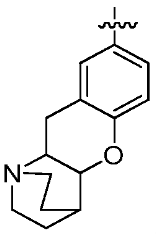 , 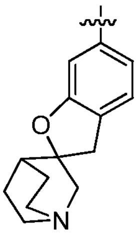 , 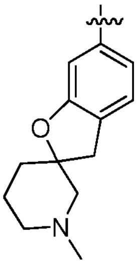 , 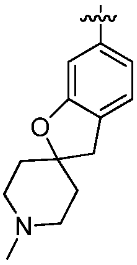 , 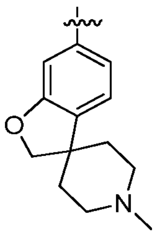 , 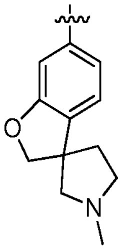 , 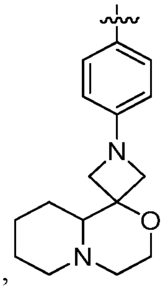 , 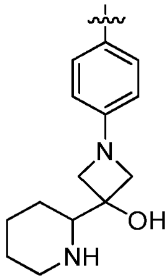 ,

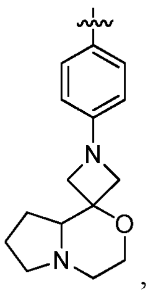 , 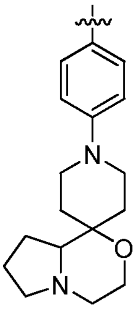 , 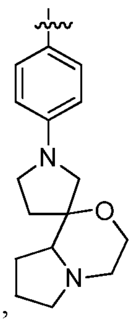 , 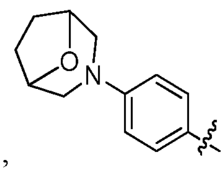 , 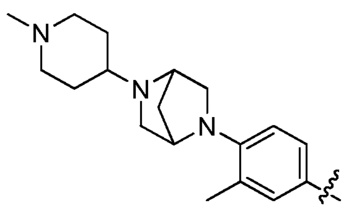 , 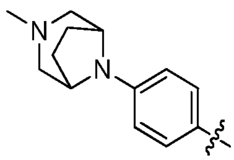 ,

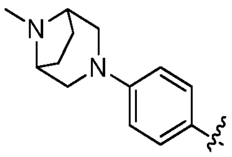, 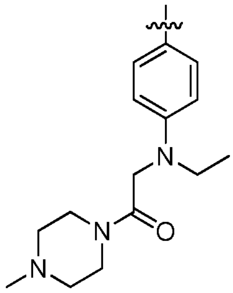, 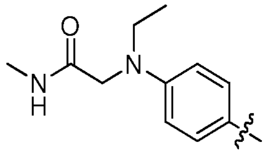, 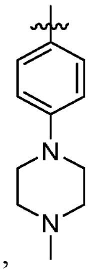, 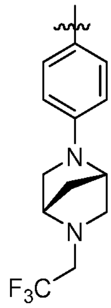, 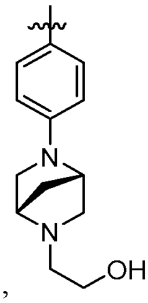, 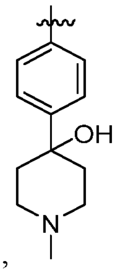,

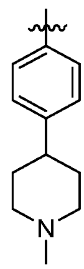, 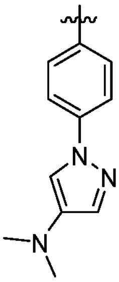, 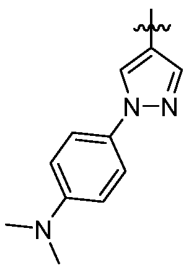, 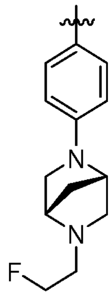, 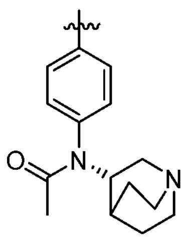, 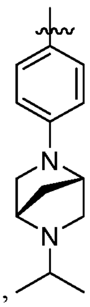, 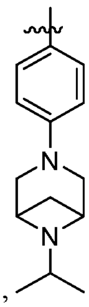, 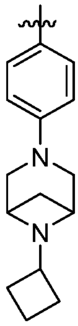, 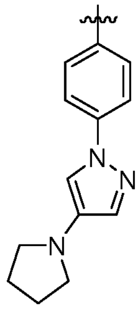, 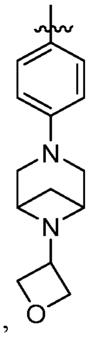,

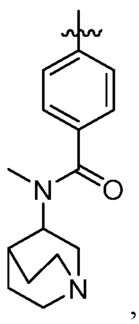, 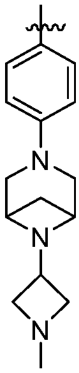, 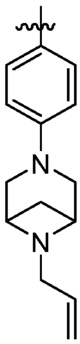, 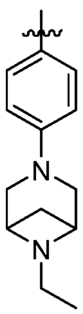, 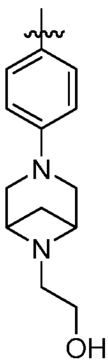, 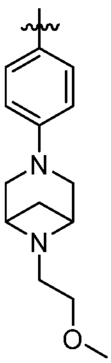, 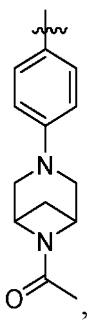, 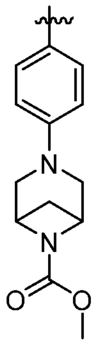, 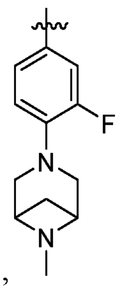, 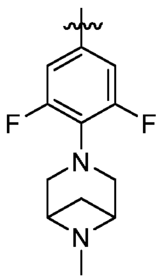

More preferably, in specific, the compound represented by Formula I of the present invention, or a stereoisomer thereof, or a pharmaceutically acceptable salt thereof, is

| No. of the compound | Chemical structure | No. of the compound | Chemical structure |
|---|---|---|---|
| WEE1-001 | 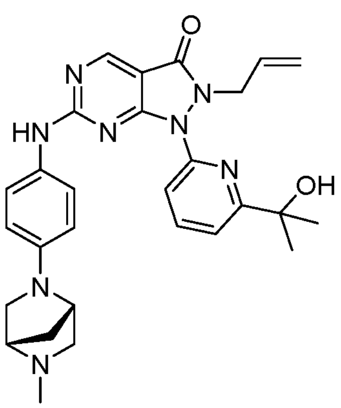 | WEE1-002 | 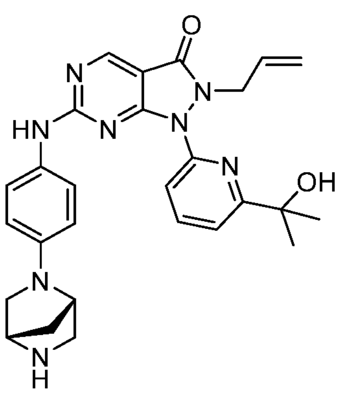 |

(continued)

| No. of the compound | Chemical structure | No. of the compound | Chemical structure |
|---|---|---|---|
| WEE1-003 | 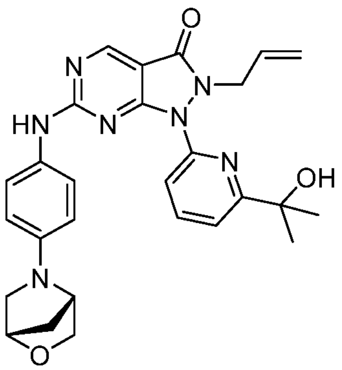 | WEE1-004 | 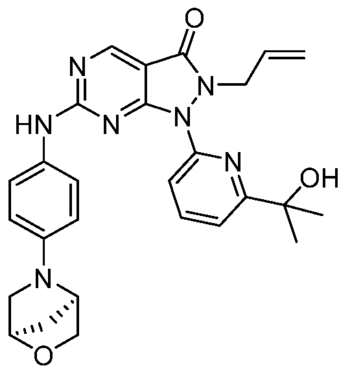 |
| WEE1-005 | 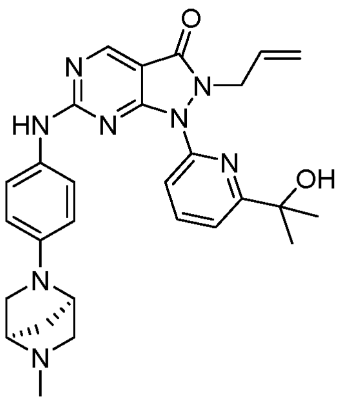 | WEE1-006 | 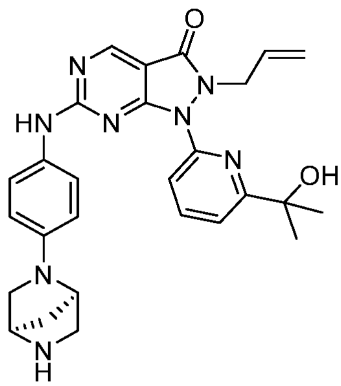 |
| WEE1-007 | 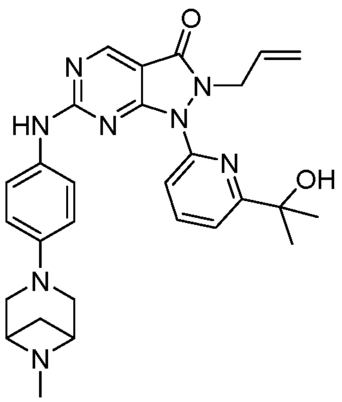 | WEE1-008 | 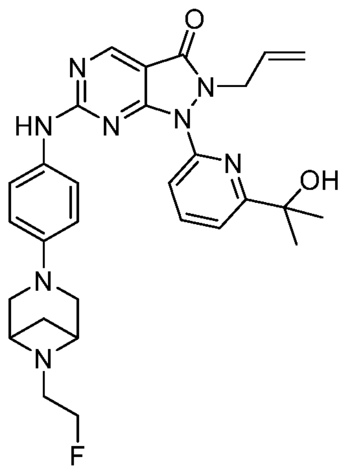 |
| WEE1-009 | 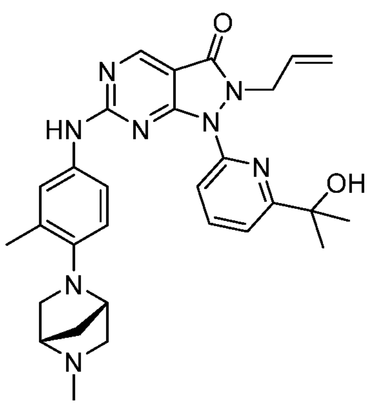 | WEE1-010 | 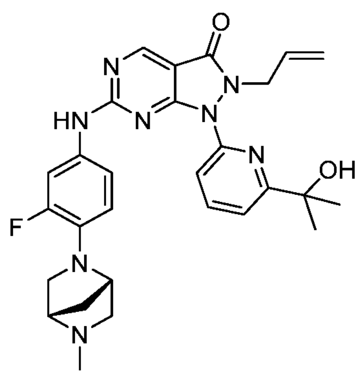 |

(continued)

| No. of the compound | Chemical structure | No. of the compound | Chemical structure |
|---|---|---|---|
| WEE1-011 | 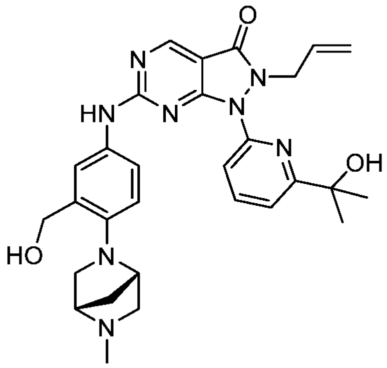 | WEE1-012 | 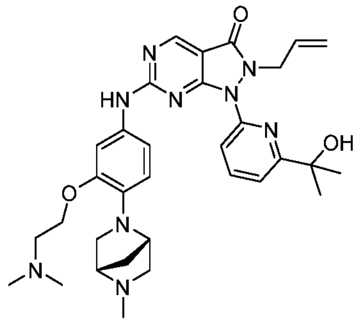 |
| WEE1-013 | 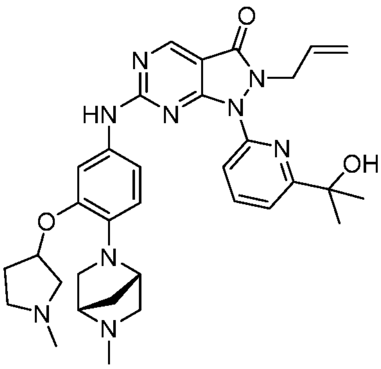 | WEE1-014 | 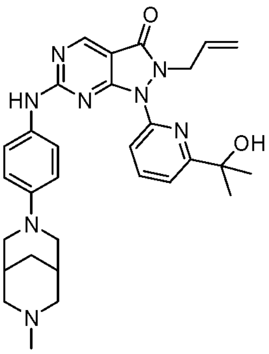 |
| WEE1-015 | 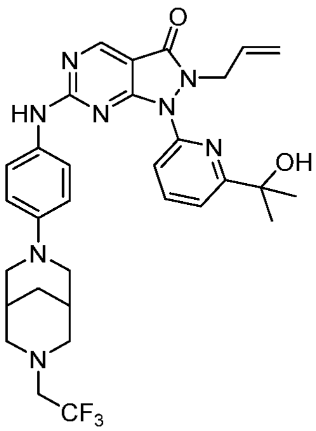 | WEE1-016 | 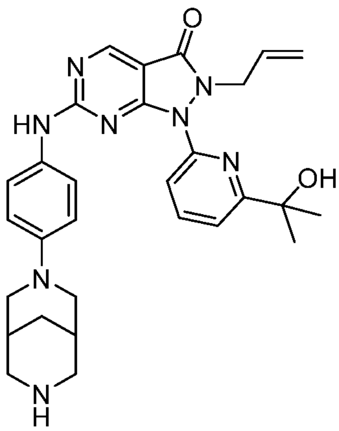 |
| WEE1-017 | 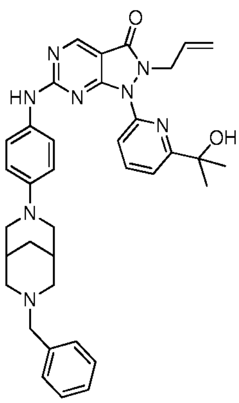 | WEE1-018 | 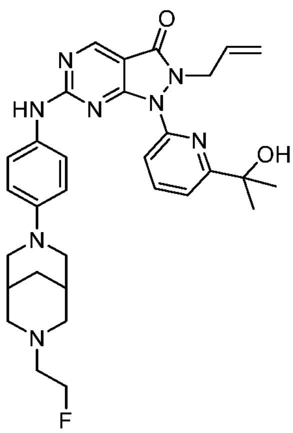 |

(continued)

| No. of the compound | Chemical structure | No. of the compound | Chemical structure |
|---|---|---|---|
| WEE1-019 | 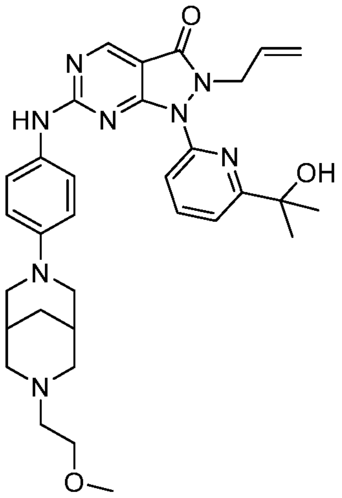 | WEE1-020 | 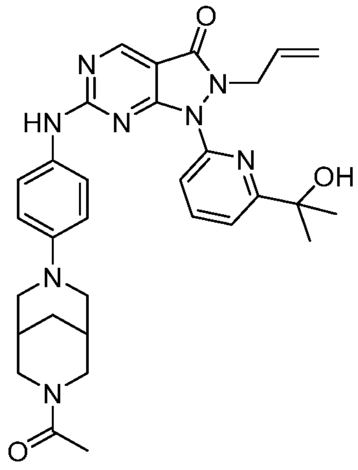 |
| WEE1-021 | 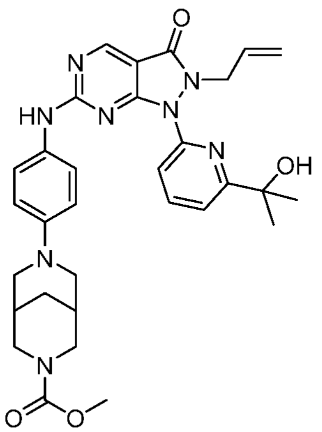 | WEE1-022 | 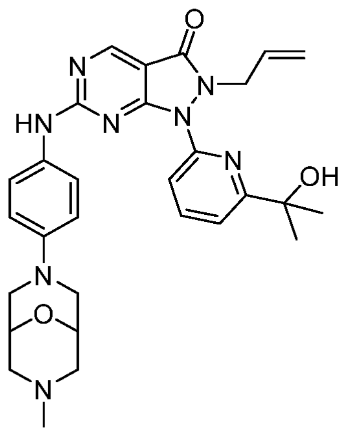 |
| WEE1-023 | 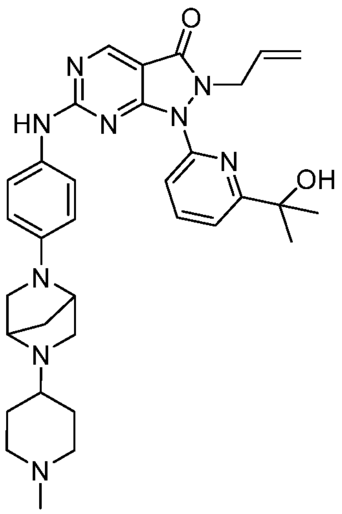 | WEE1-024 | 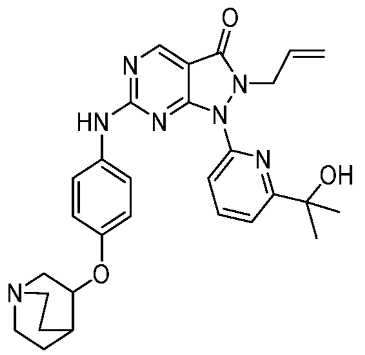 |

(continued)

| No. of the compound | Chemical structure | No. of the compound | Chemical structure |
|---|---|---|---|
| WEE1-025 | 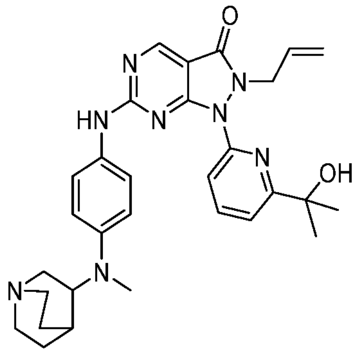 | WEE1-026 | 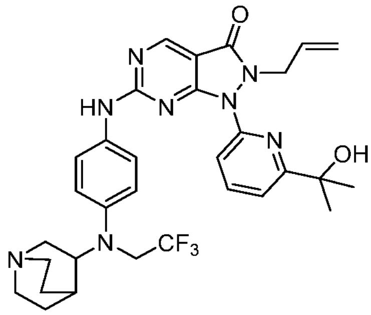 |
| WEE1-027 | 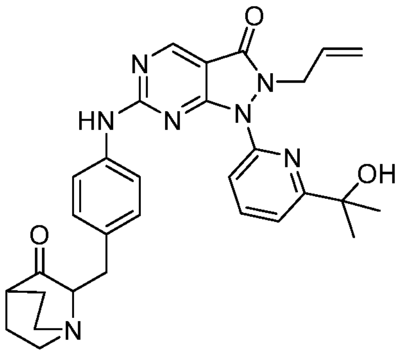 | WEE1-028 | 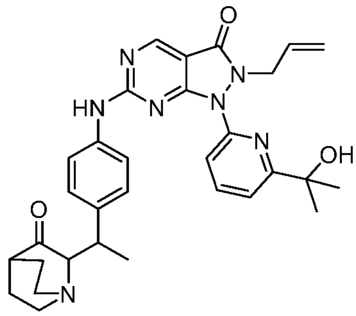 |
| WEE1-029 | 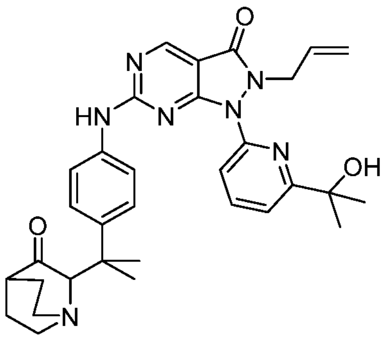 | WEE1-030 | 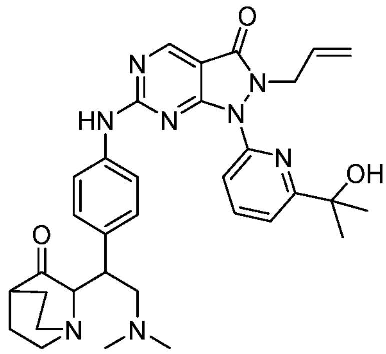 |
| WEE1-031 | 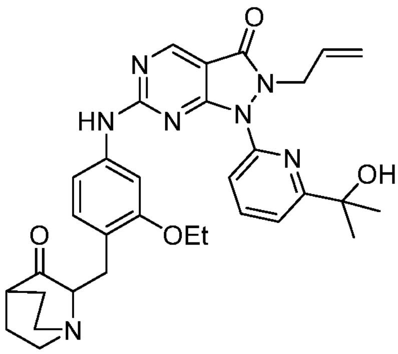 | WEE1-032 | 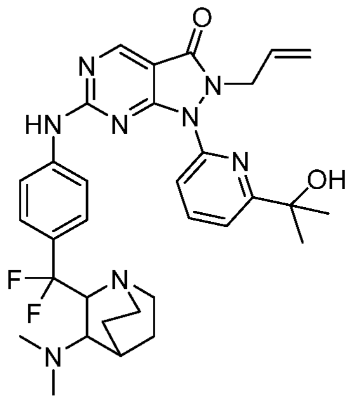 |

(continued)

| No. of the compound | Chemical structure | No. of the compound | Chemical structure |
|---|---|---|---|
| WEE1-033 | 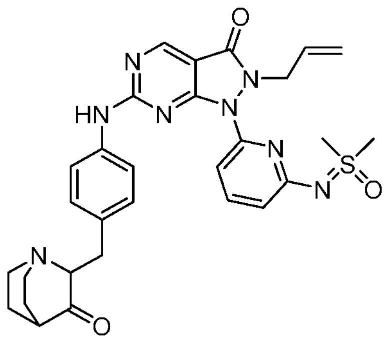 | WEE1-034 | 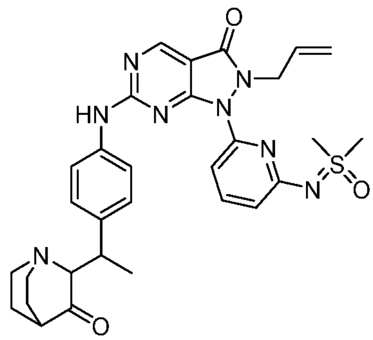 |
| WEE1-035 | 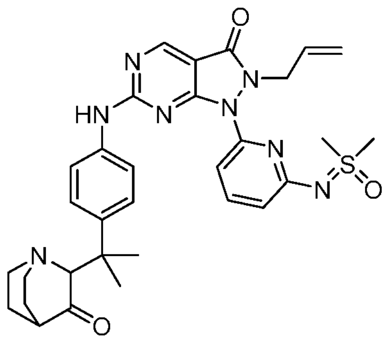 | WEE1-036 | 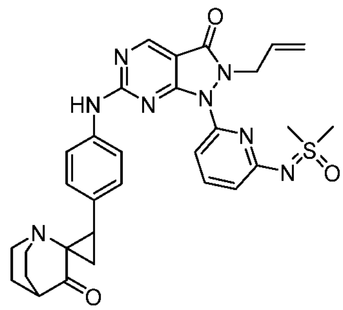 |
| WEE1-037 | 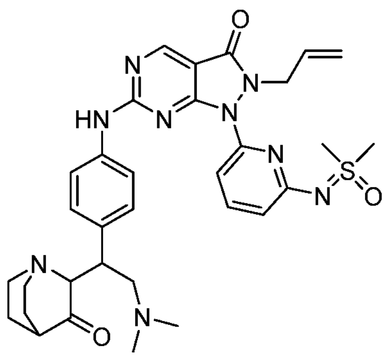 | WEE1-038 | 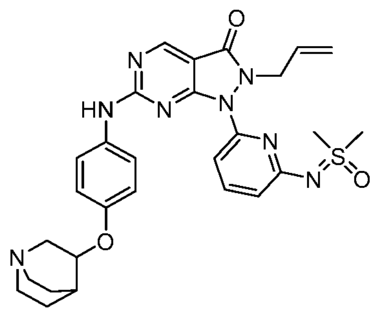 |
| WEE1-039 | 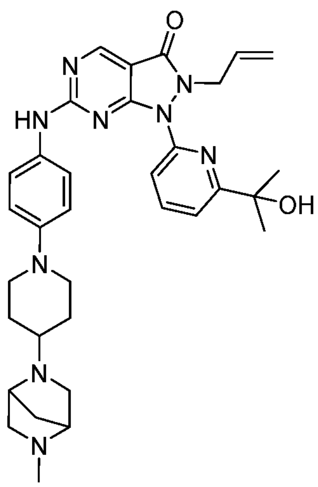 | WEE1-040 | 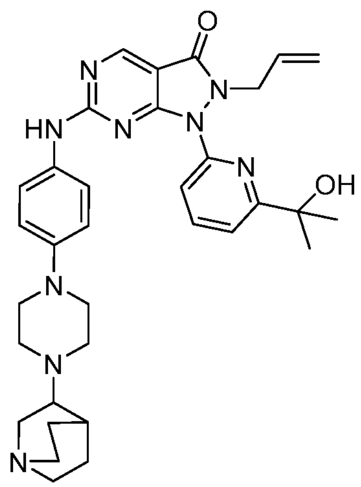 |

(continued)

| No. of the compound | Chemical structure | No. of the compound | Chemical structure |
|---|---|---|---|
| WEE1-041 | 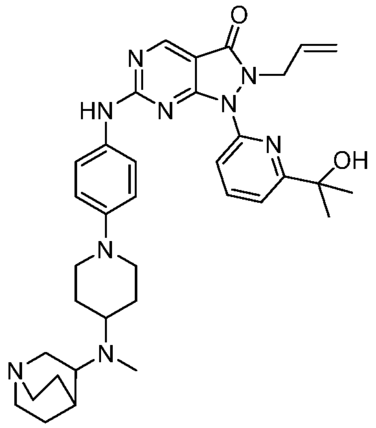 | WEE1-042 | 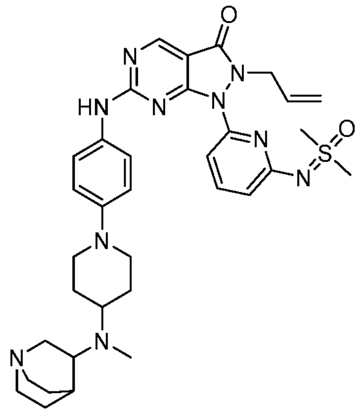 |
| WEE1-043 | 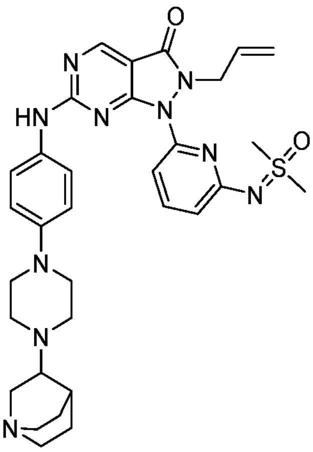 | WEE1-044 | 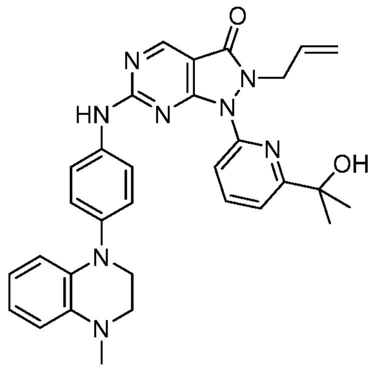 |
| WEE1-045 | 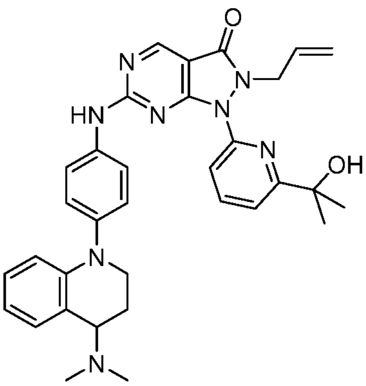 | WEE1-046 | 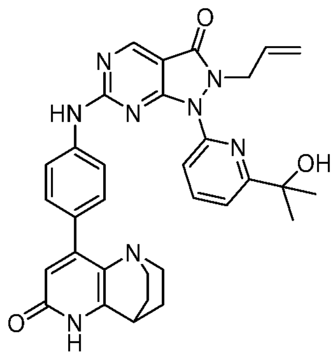 |

(continued)

| No. of the compound | Chemical structure | No. of the compound | Chemical structure |
|---|---|---|---|
| WEE1-047 | 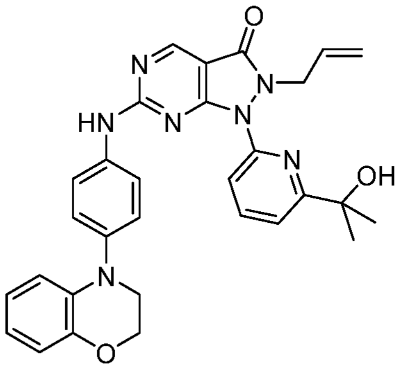 | WEE1-048 | 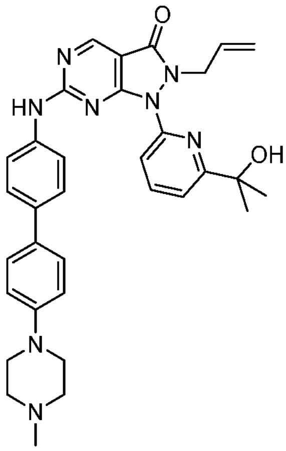 |
| WEE1-049 | 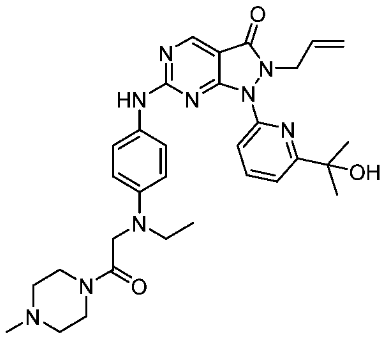 | WEE1-050 | 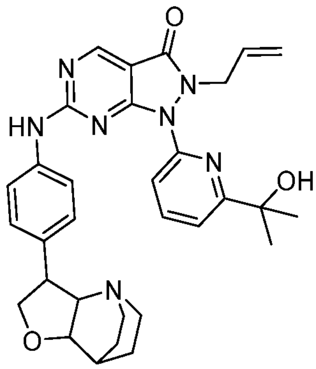 |
| WEE1-051 | 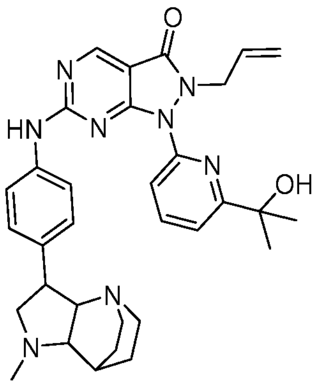 | WEE1-052 | 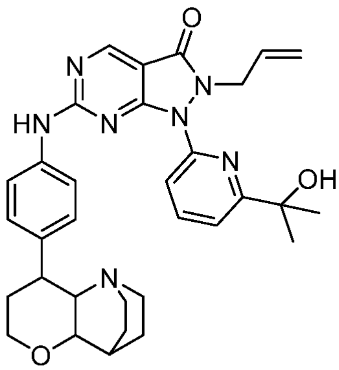 |
| WEE1-053 | 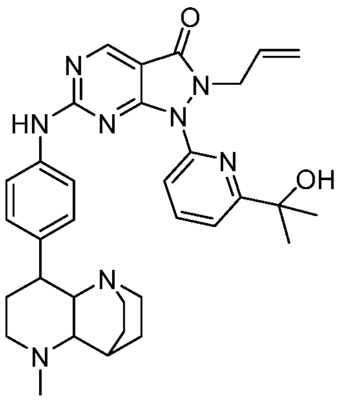 | WEE1-054 | 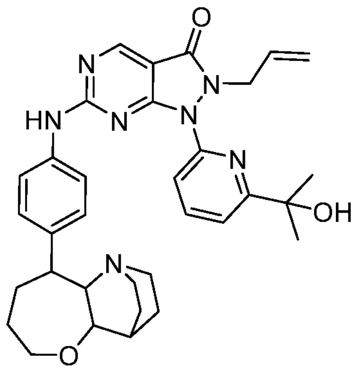 |

(continued)

| No. of the compound | Chemical structure | No. of the compound | Chemical structure |
|---|---|---|---|
| WEE1-055 | 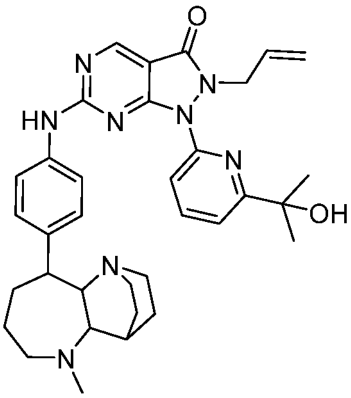 | WEE1-056 | 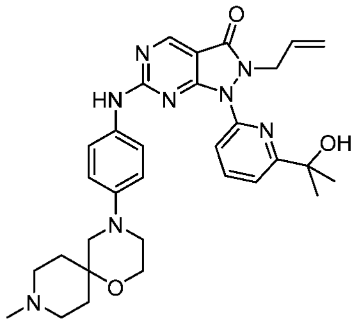 |
| WEE1-057 | 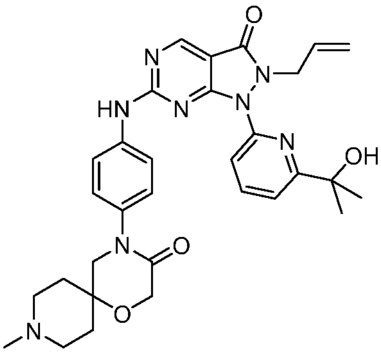 | WEE1-058 | 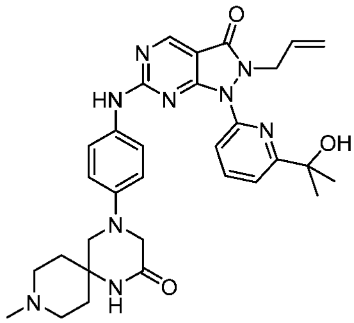 |
| WEE1-059 | 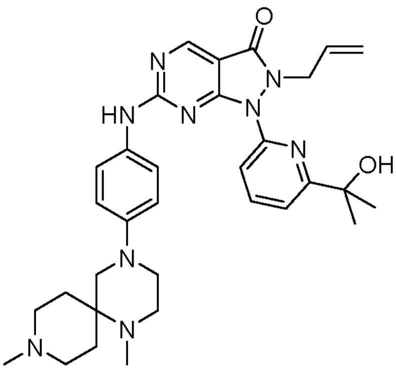 | WEE1-060 | 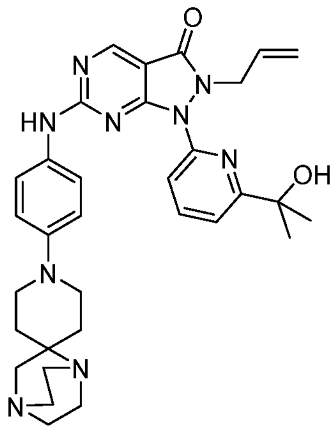 |
| WEE1-061 | 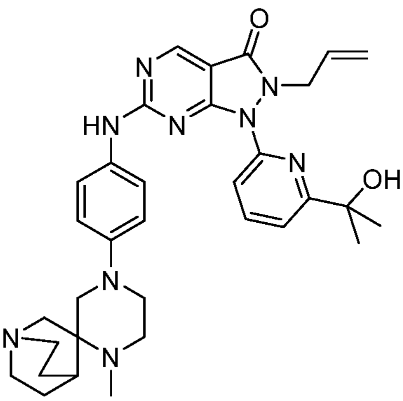 | WEE1-062 | 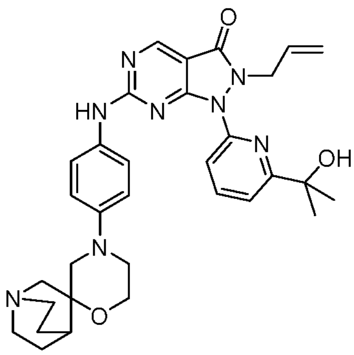 |

(continued)

| No. of the compound | Chemical structure | No. of the compound | Chemical structure |
|---|---|---|---|
| WEE1-063 | 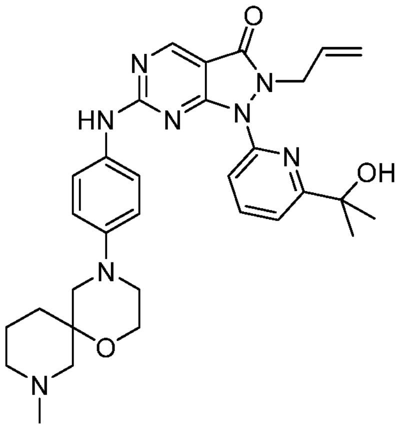 | WEE1-064 | 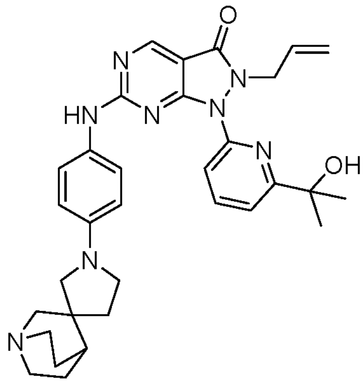 |
| WEE1-065 | 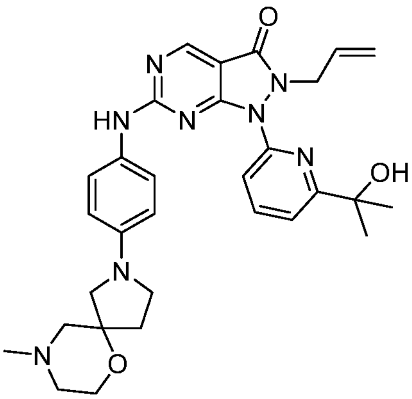 | WEE1-066 | 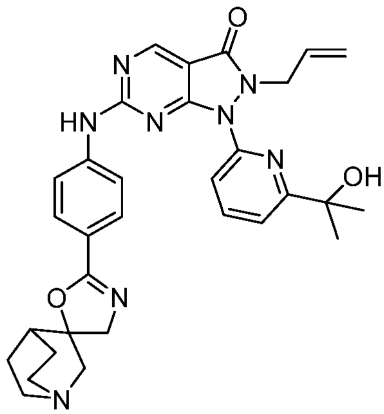 |
| WEE1-067 | 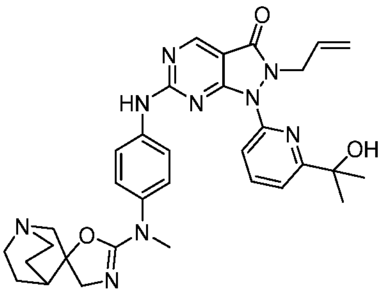 | WEE1-068 | 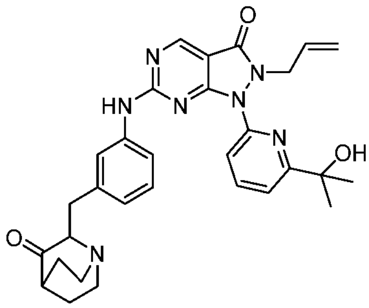 |
| WEE1-069 | 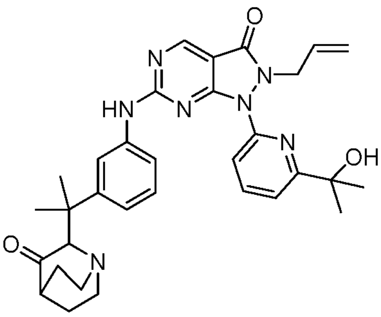 | WEE1-070 | 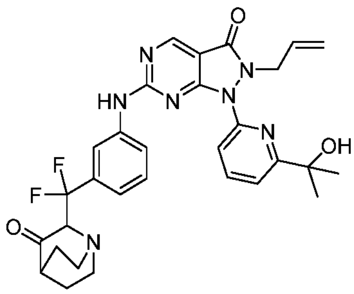 |

(continued)

| No. of the compound | Chemical structure | No. of the compound | Chemical structure |
|---|---|---|---|
| WEE1-071 | 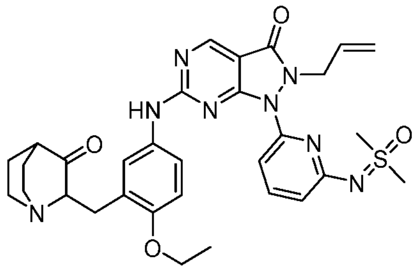 | WEE1-072 | 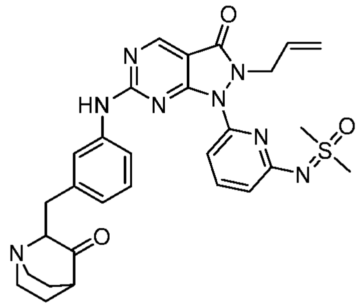 |
| WEE1-073 | 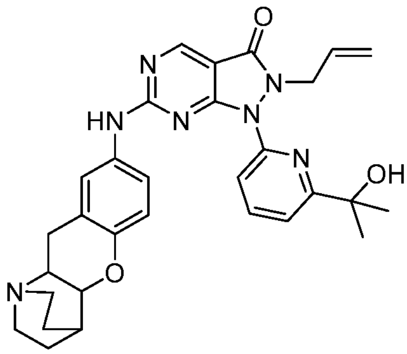 | WEE1-074 | 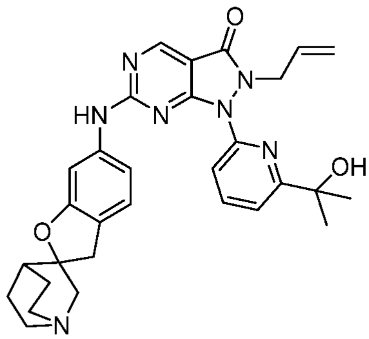 |
| WEE1-075 | 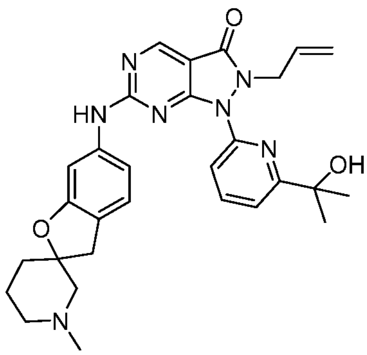 | WEE1-076 | 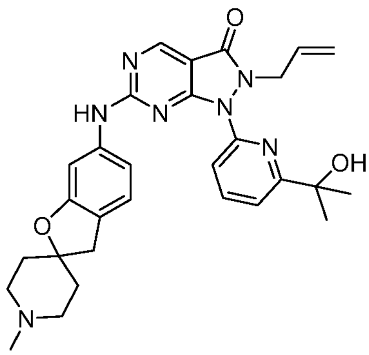 |
| WEE1-077 | 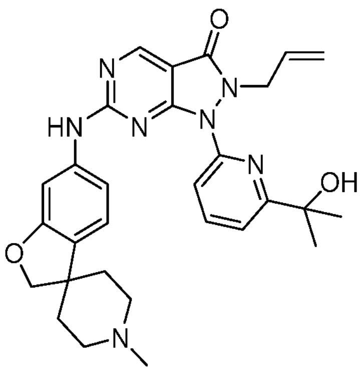 | WEE1-078 | 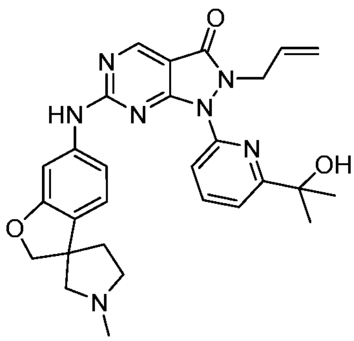 |

(continued)

| No. of the compound | Chemical structure | No. of the compound | Chemical structure |
|---|---|---|---|
| WEE1-079 | 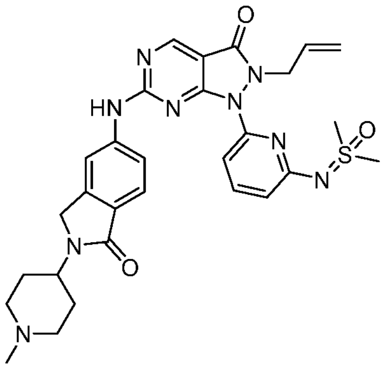 | WEE1-080 | 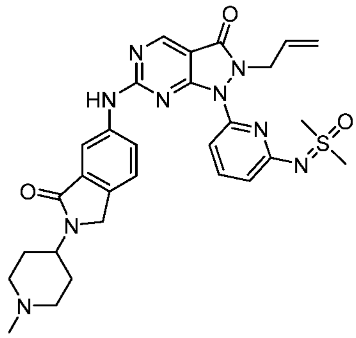 |
| WEE1-081 | 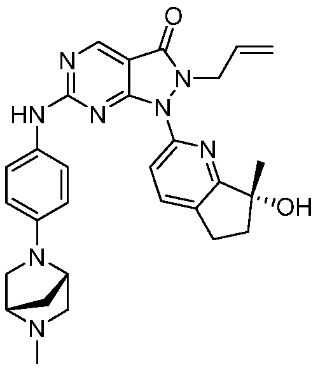 | WEE1-082 | 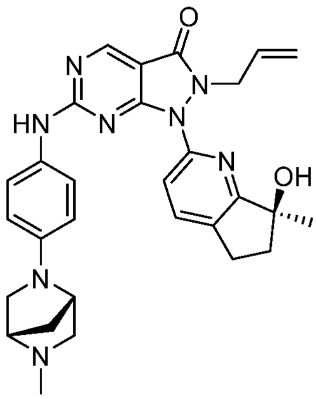 |
| WEE1-083 | 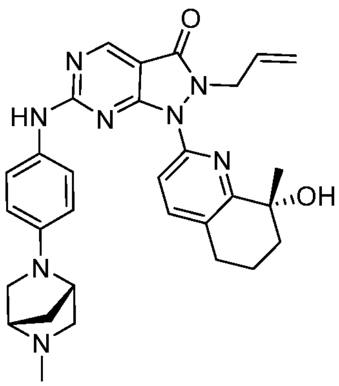 | WEE1-084 | 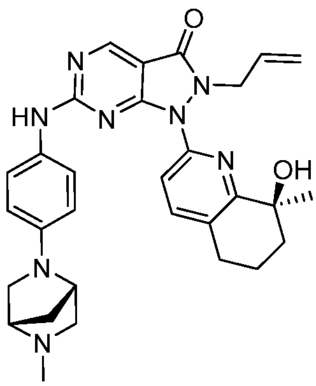 |
| WEE1-085 | 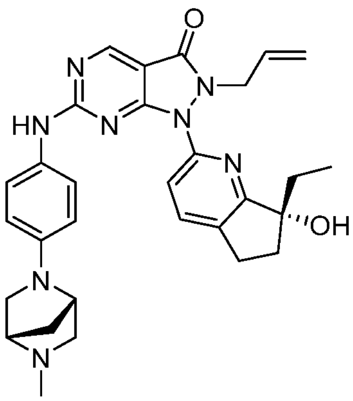 | WEE1-086 | 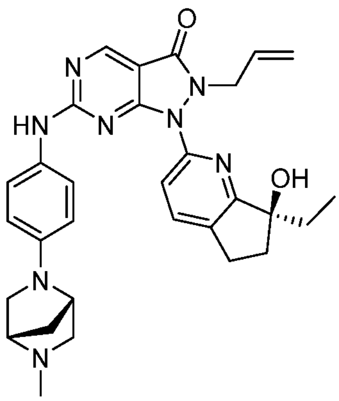 |

(continued)

| No. of the compound | Chemical structure | No. of the compound | Chemical structure |
|---|---|---|---|
| WEE1-087 | 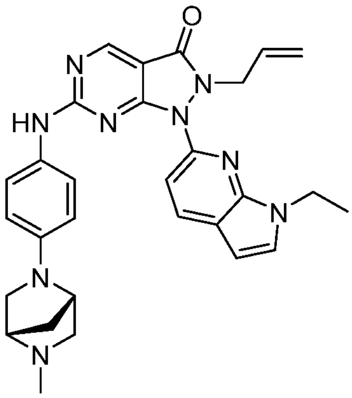 | WEE1-088 | 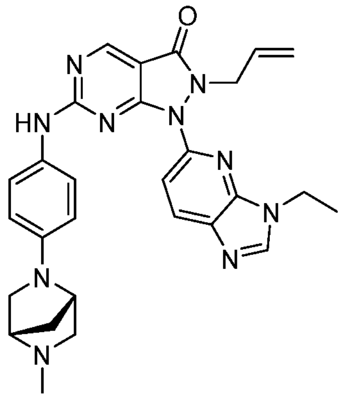 |
| WEE1-089 | 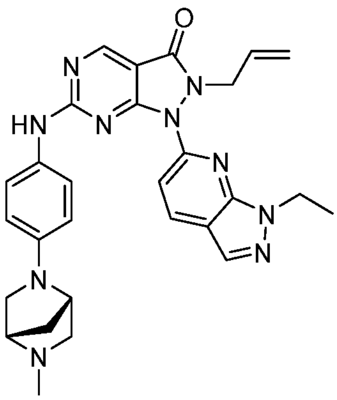 | WEE1-090 | 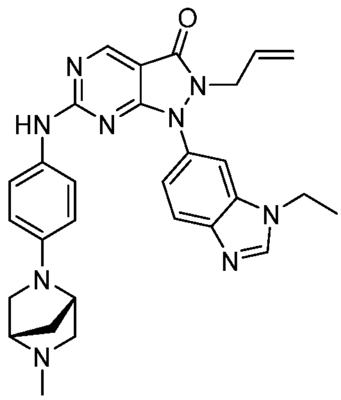 |
| WEE1-091 | 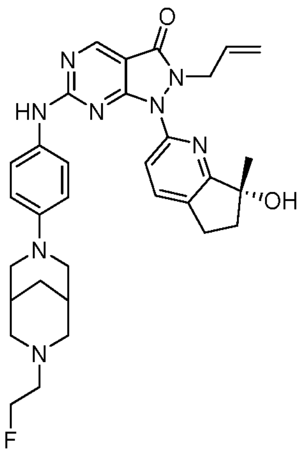 | WEE1-092 | 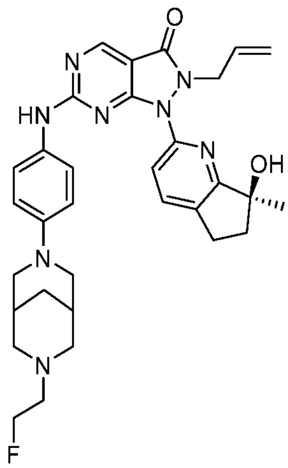 |

(continued)

| No. of the compound | Chemical structure | No. of the compound | Chemical structure |
|---|---|---|---|
| WEE1-093 | 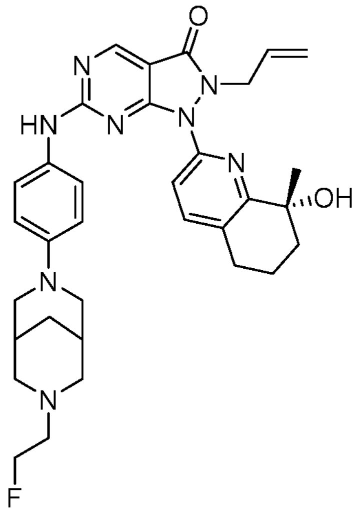 | WEE1-094 | 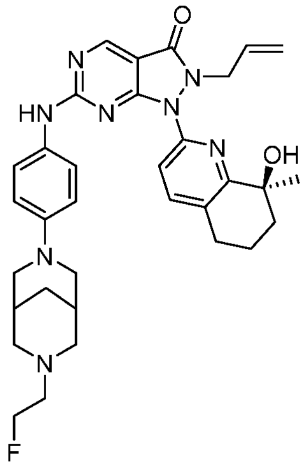 |
| WEE1-095 | 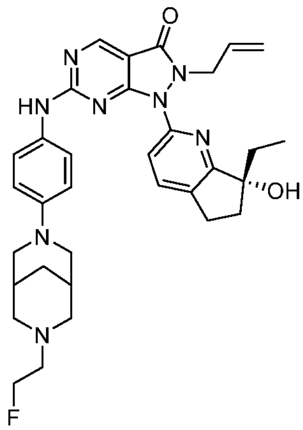 | WEE1-096 | 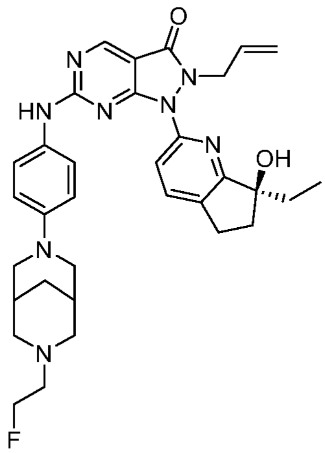 |
| WEE1-097 | 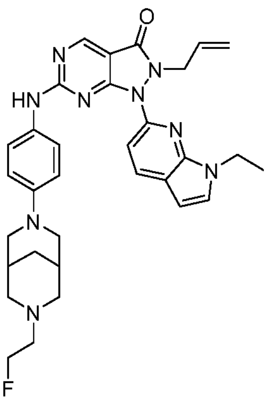 | WEE1-098 | 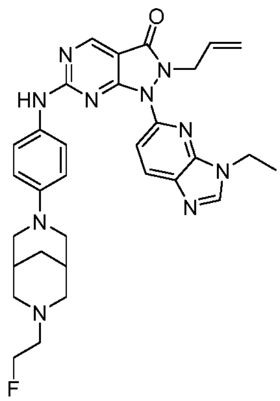 |

(continued)

| No. of the compound | Chemical structure | No. of the compound | Chemical structure |
|---|---|---|---|
| WEE1-099 | 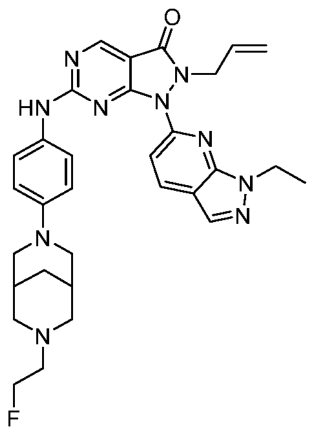 | WEE1-100 | 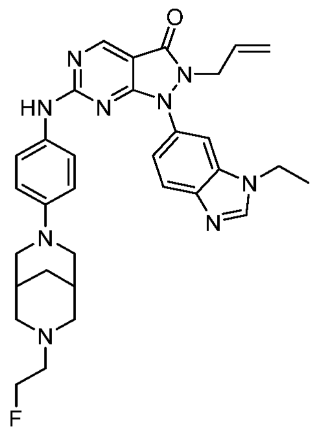 |
| WEE1-101 | 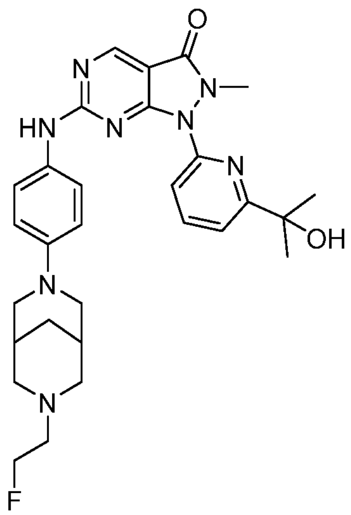 | WEE1-102 | 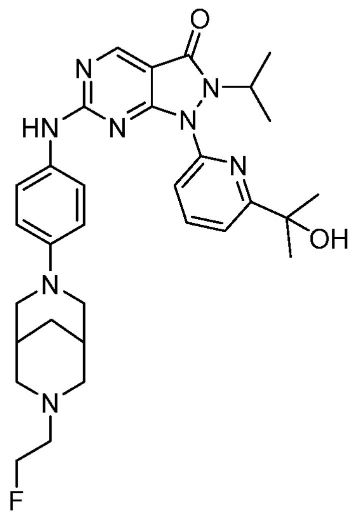 |
| WEE1-103 | 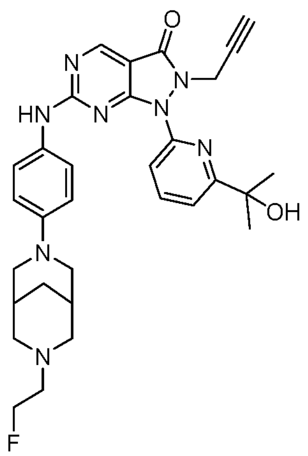 | WEE1-104 | 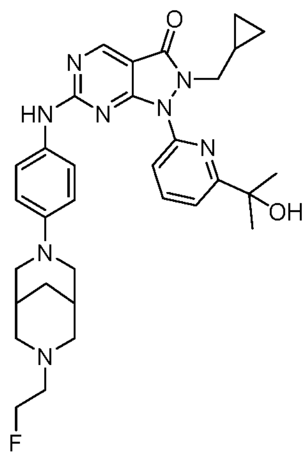 |

(continued)

| No. of the compound | Chemical structure | No. of the compound | Chemical structure |
|---|---|---|---|
| WEE1-105 | 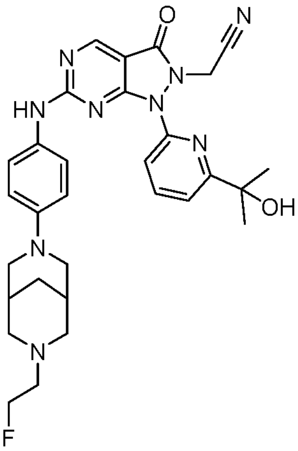 | WEE1-106 | 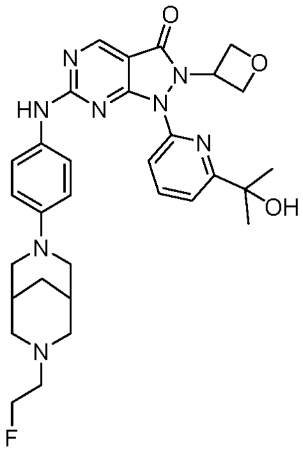 |
| WEE1-107 | 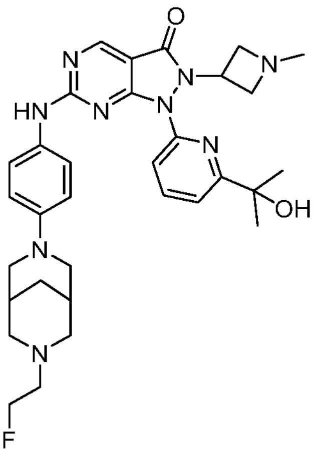 | WEE1-108 | 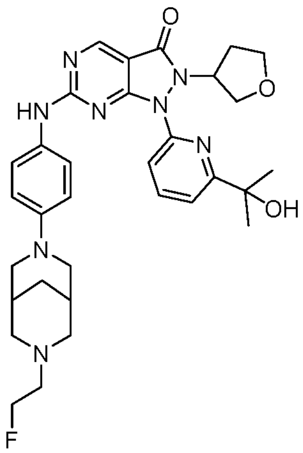 |
| WEE1-109 | 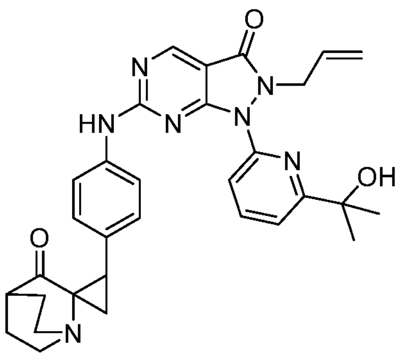 | WEE1-110 | 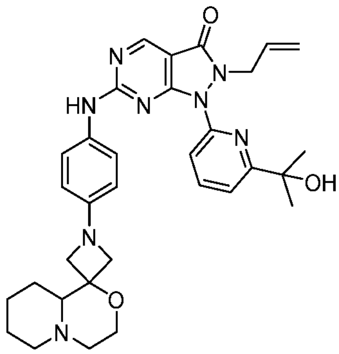 |

(continued)

| No. of the compound | Chemical structure | No. of the compound | Chemical structure |
|---|---|---|---|
| WEE1-111 | 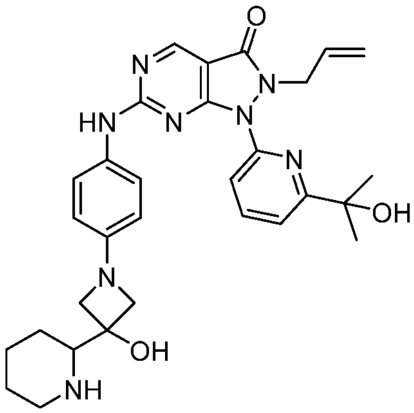 | WEE1-112 | 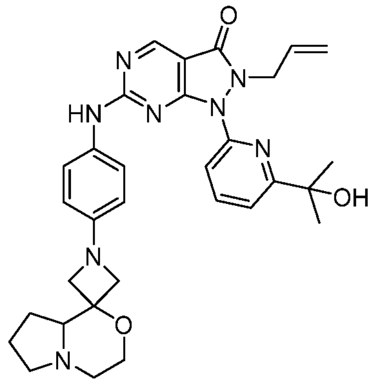 |
| WEE1-113 | 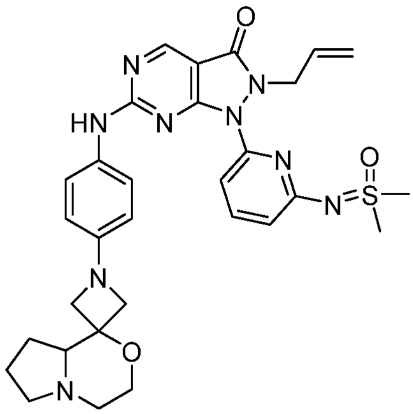 | WEE1-114 | 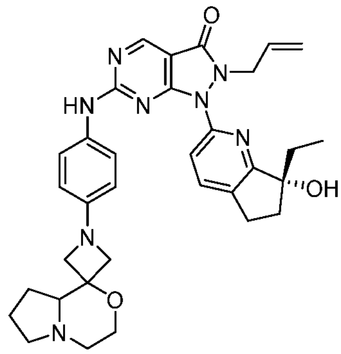 |
| WEE1-115 | 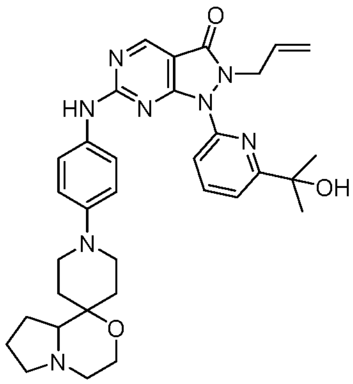 | WEE1-116 | 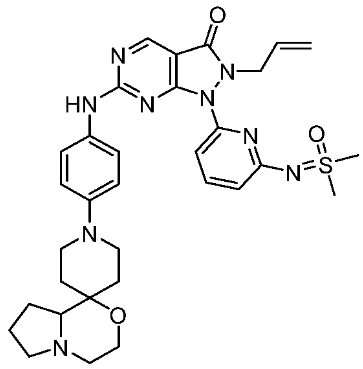 |
| WEE1-117 | 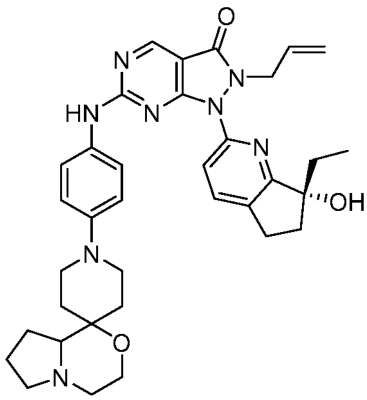 | WEE1-118 | 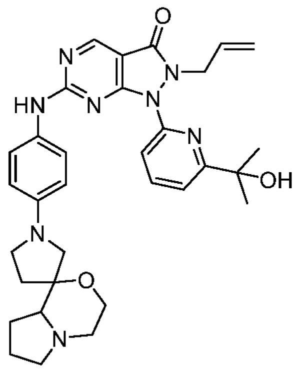 |

(continued)

| No. of the compound | Chemical structure | No. of the compound | Chemical structure |
|---|---|---|---|
| WEE1-119 | 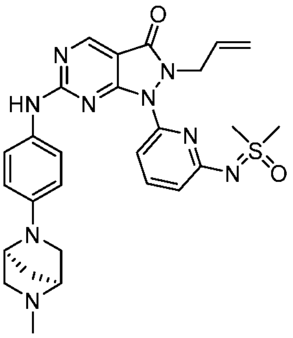 | WEE1-120 | 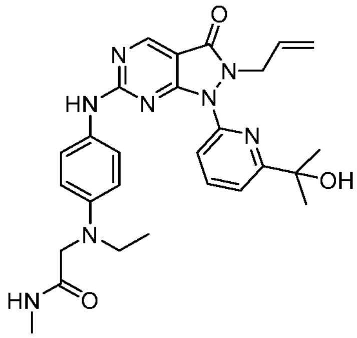 |
| WEE1-121 | 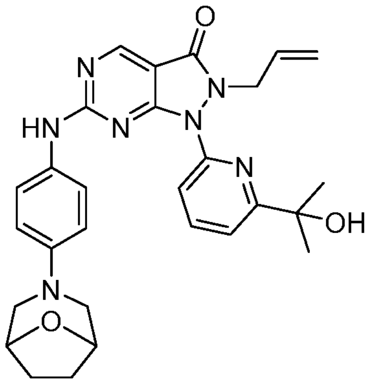 | WEE1-122 | 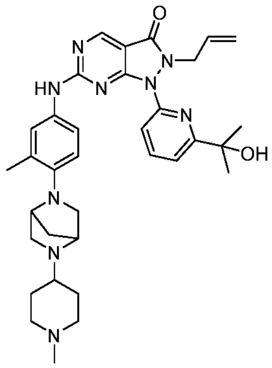 |
| WEE1-123 | 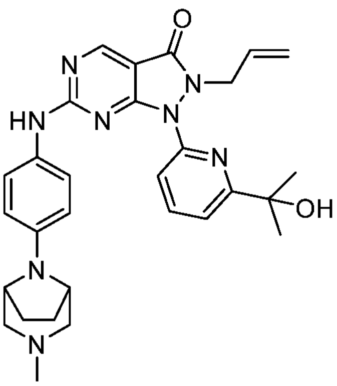 | WEE1-124 | 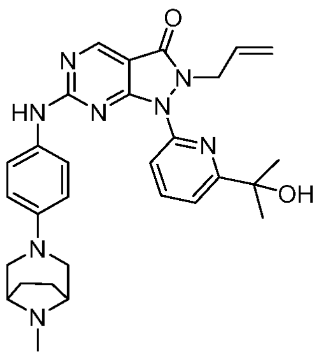 |
| WEE1-125 | 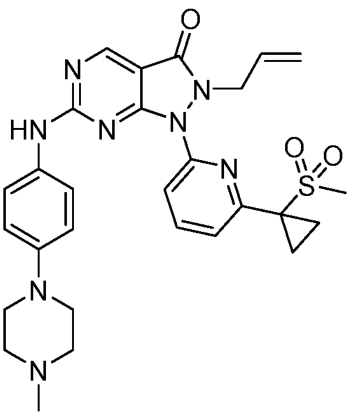 | WEE1-126 | 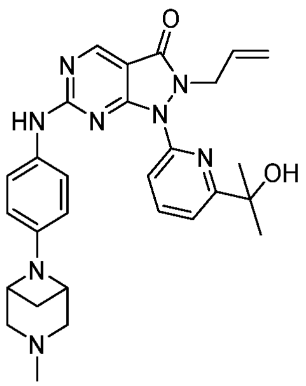 |

(continued)

| No. of the compound | Chemical structure | No. of the compound | Chemical structure |
|---|---|---|---|
| WEE1-127 | 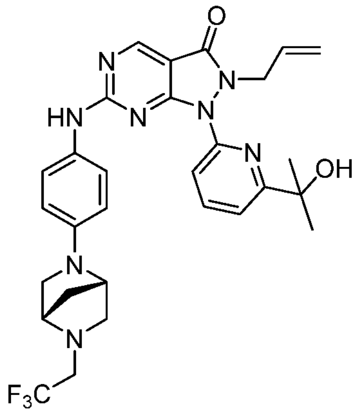 | WEE1-128 | 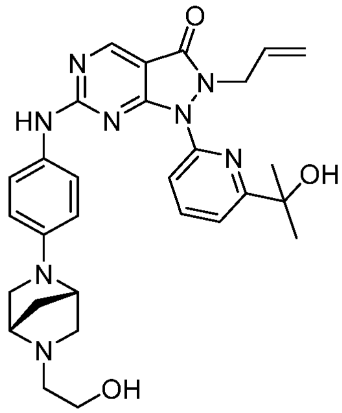 |
| WEE1-129 | 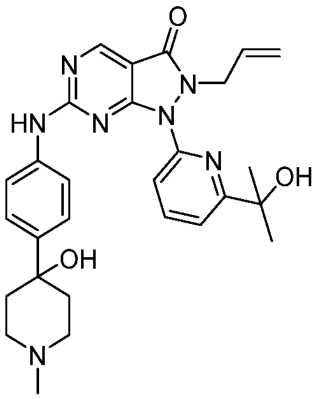 | WEE1-130 | 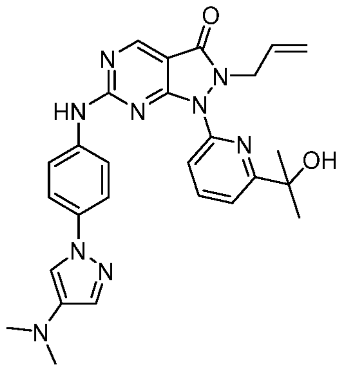 |
| WEE1-131 | 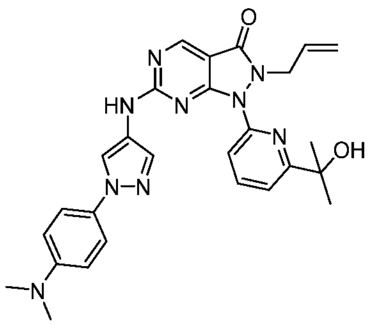 | WEE1-132 | 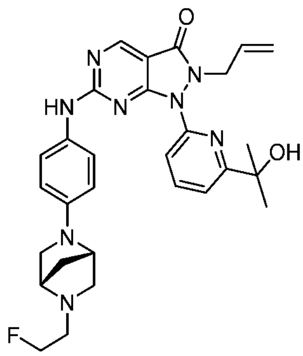 |
| WEE1-133 | 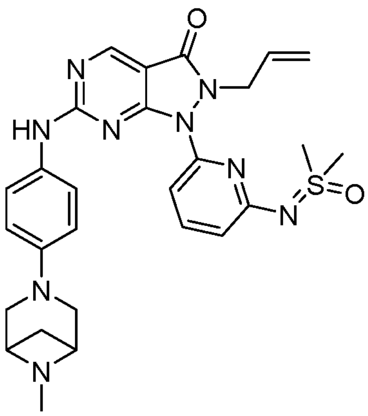 | WEE1-134 | 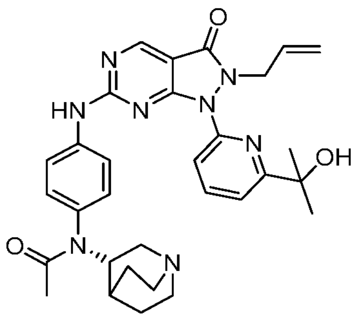 |

(continued)

| No. of the compound | Chemical structure | No. of the compound | Chemical structure |
|---|---|---|---|
| WEE1-135 | 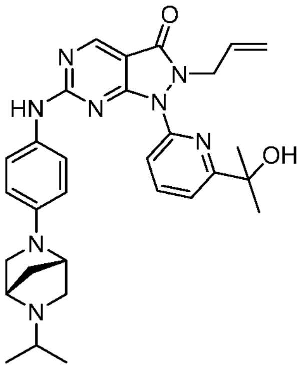 | WEE1-136 | 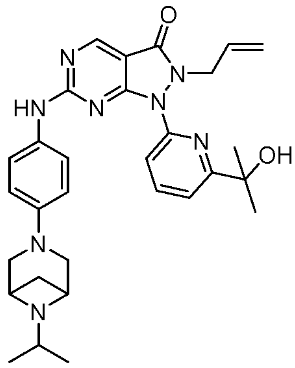 |
| WEE1-137 | 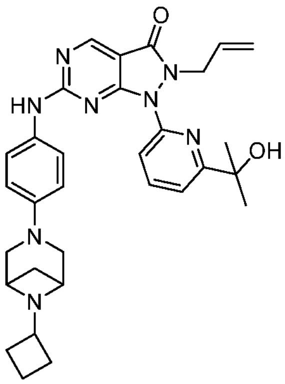 | WEE1-138 | 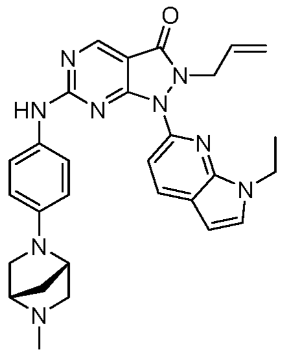 |
| WEE1-139 | 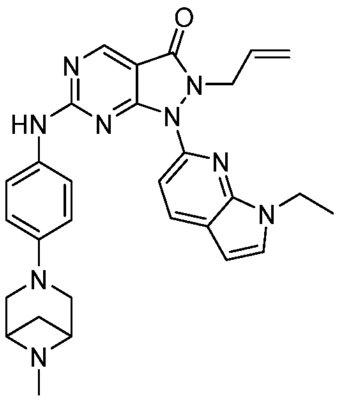 | WEE1-140 | 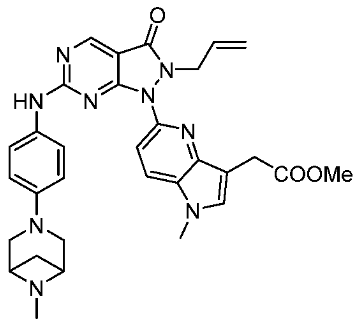 |
| WEE1-141 | 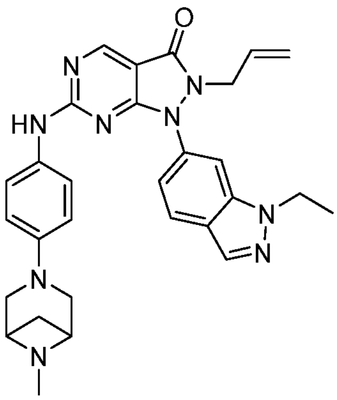 | WEE1-142 | 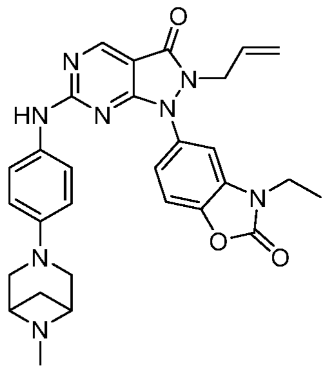 |

(continued)

| No. of the compound | Chemical structure | No. of the compound | Chemical structure |
|---|---|---|---|
| WEE1-143 | 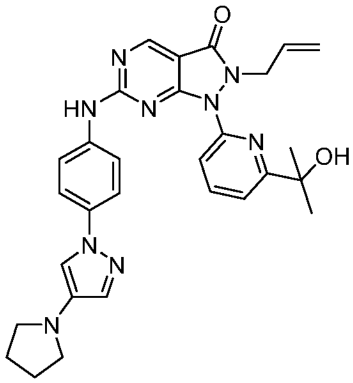 | WEE1-144 | 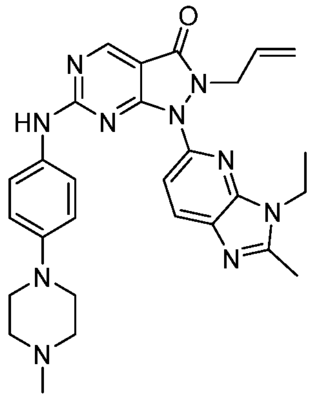 |
| WEE1-145 | 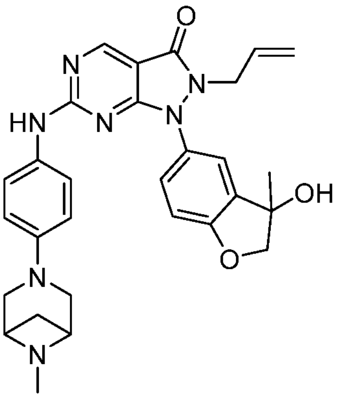 | WEE1-146 | 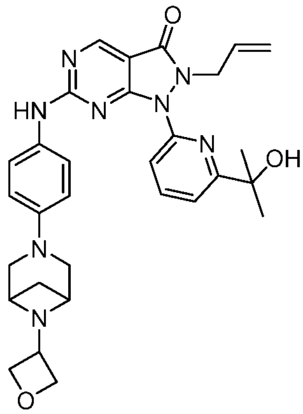 |
| WEE1-147 | 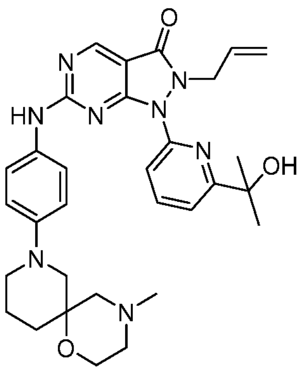 | WEE1-148 | 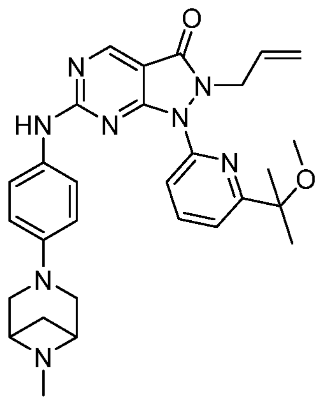 |
| WEE1-149 | 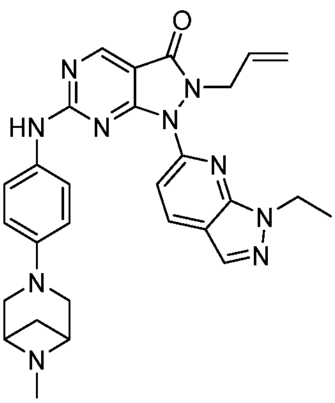 | WEE1-150 | 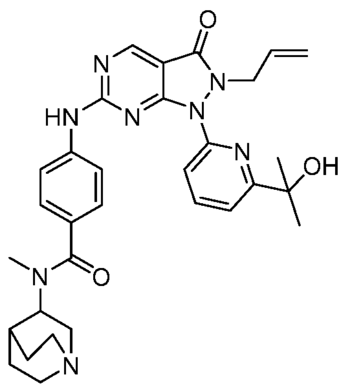 |

(continued)

| No. of the compound | Chemical structure | No. of the compound | Chemical structure |
|---|---|---|---|
| WEE1-151 | 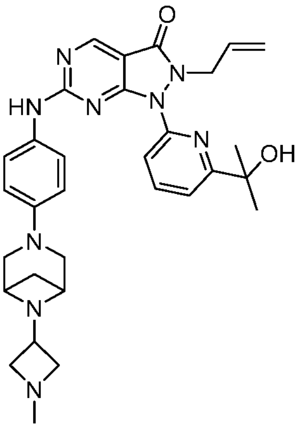 | WEE1-152 | 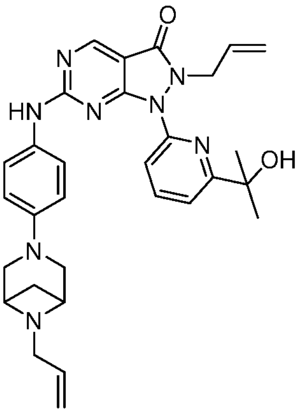 |
| WEE1-153 | 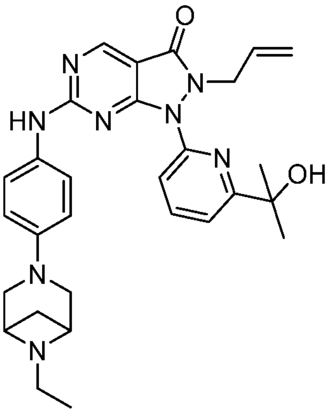 | WEE1-154 | 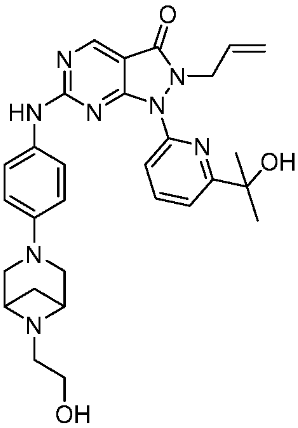 |
| WEE1-155 | 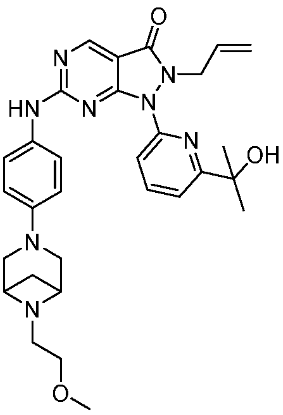 | WEE1-156 | 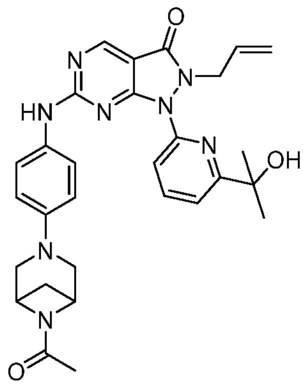 |

(continued)

| No. of the compound | Chemical structure | No. of the compound | Chemical structure |
|---|---|---|---|
| WEE1-157 | 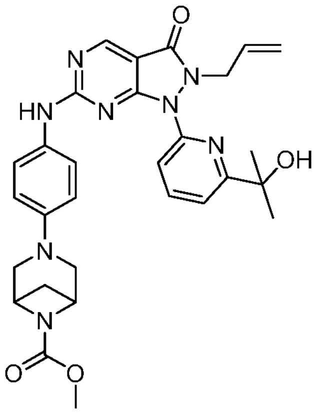 | WEE1-158 | 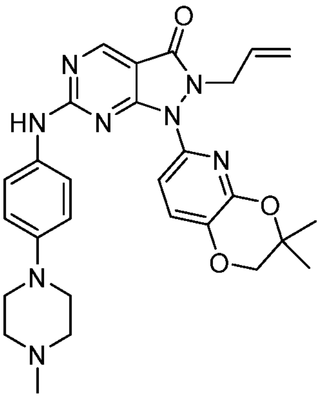 |
| WEE1-159 | 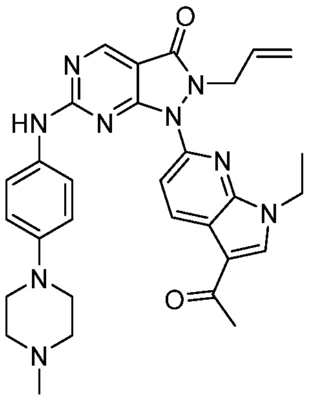 | WEE1-160 | 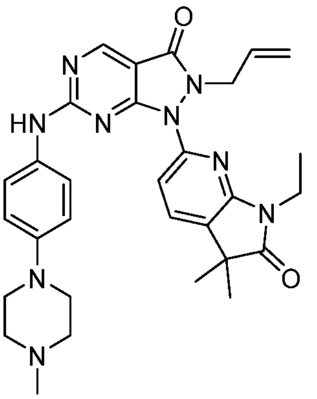 |
| WEE1-161 | 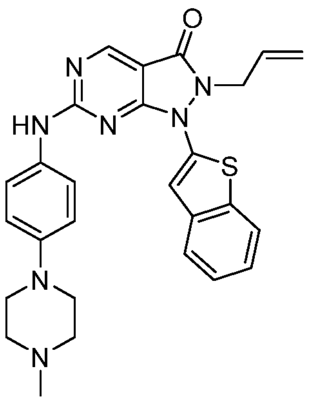 | WEE1-162 | 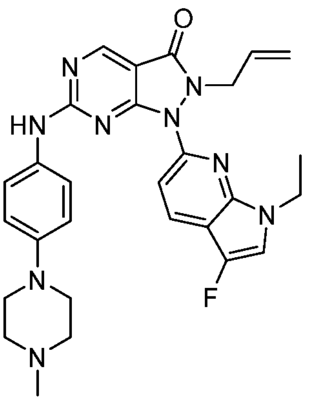 |
| WEE1-163 | 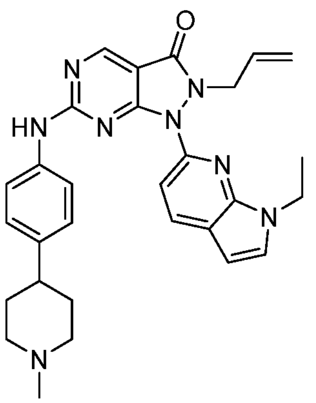 | WEE1-164 | 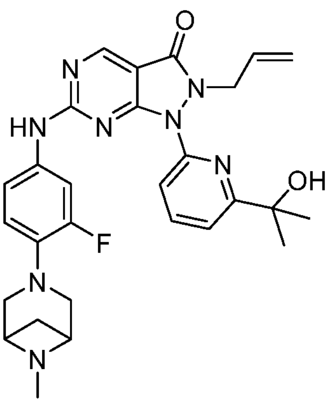 |

(continued)

| No. of the compound | Chemical structure | No. of the compound | Chemical structure |
|---|---|---|---|
| WEE1-165 | 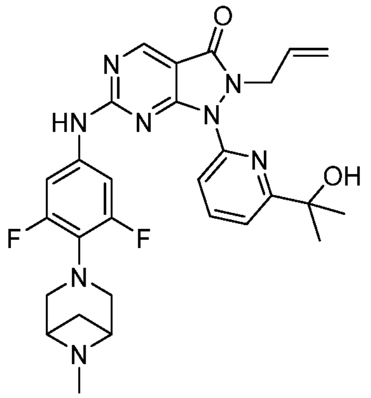 | WEE1-166 | 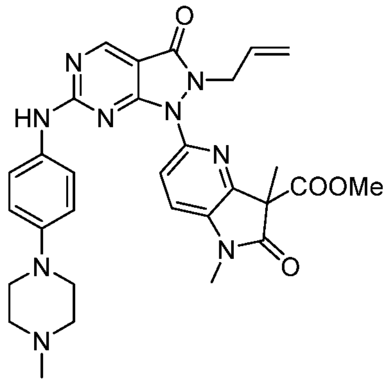 |
| WEE1-167 | 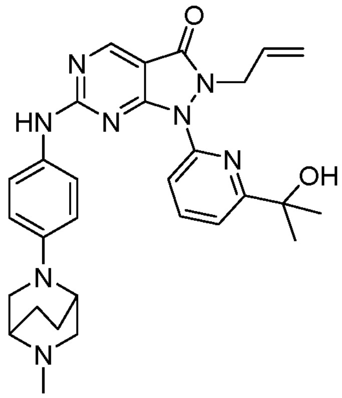 | WEE1-168 | 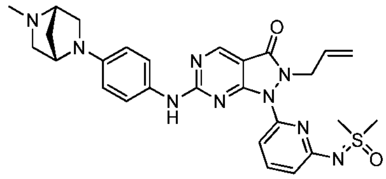 |

More preferably, in specific, the compound represented by Formula I of the present invention, or a stereoisomer thereof, or a pharmaceutically acceptable salt thereof, is

.

| | | |
|---|---|---|
| 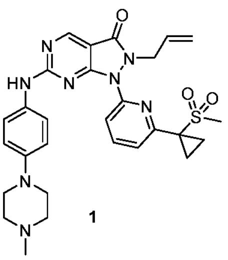 1 | 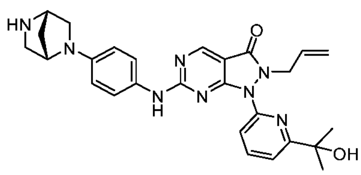 2 | 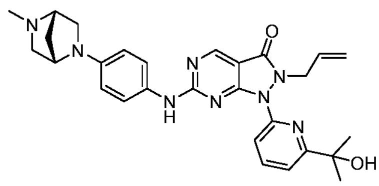 3 |

| | | |
|---|---|---|
| 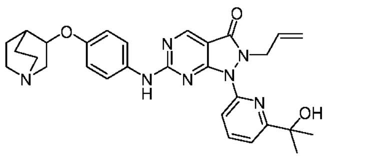 4 | 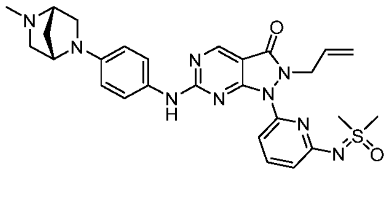 5 | 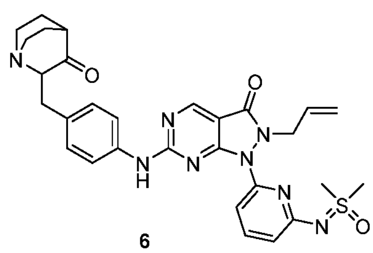 6 |
| 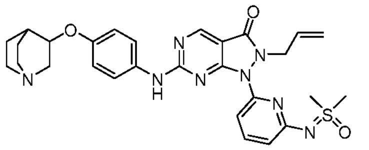 7 | 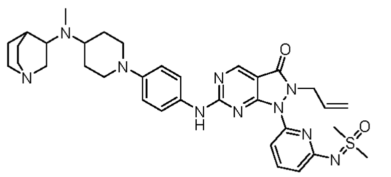 8 | 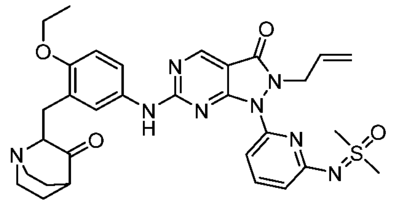 9 |
| 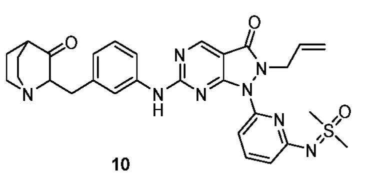 10 | 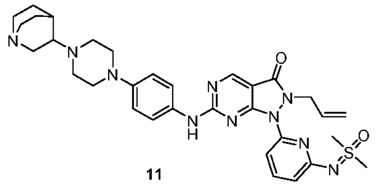 11 | 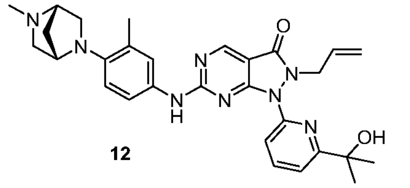 12 |
| 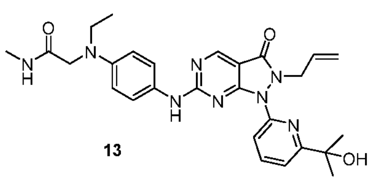 13 | 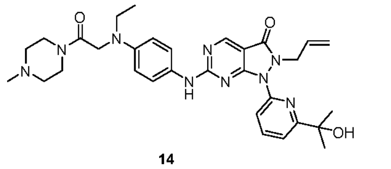 14 | 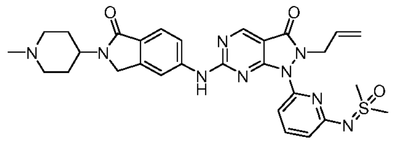 15 |
| 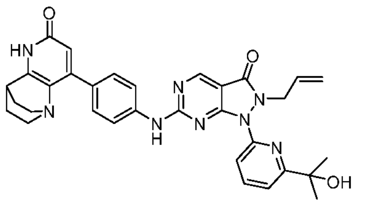 16 | 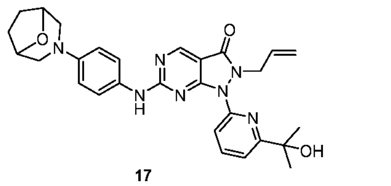 17 | 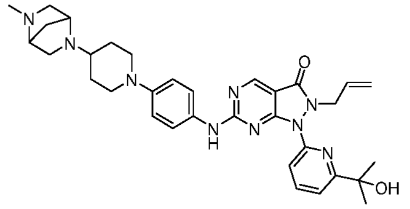 18 |
| 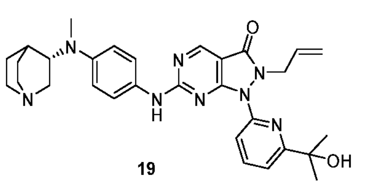 19 | 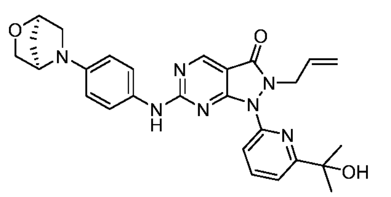 20 | 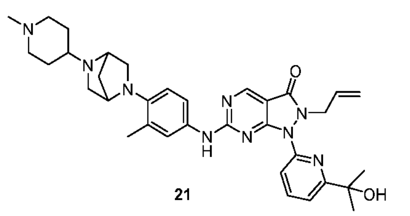 21 |
| 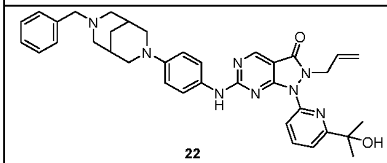 22 | 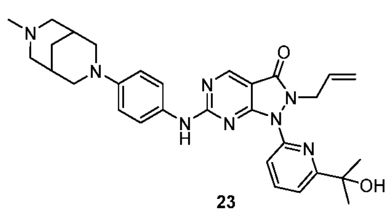 23 | 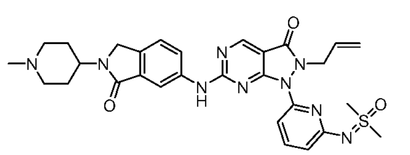 24 |

| | | |
|---|---|---|
| 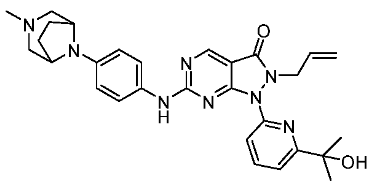 25 | 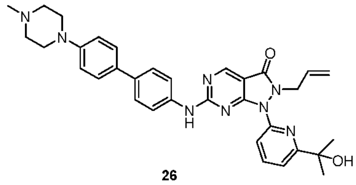 26 | 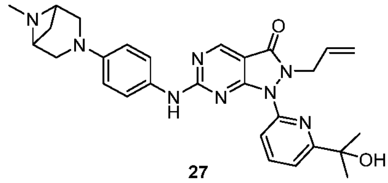 27 |
| 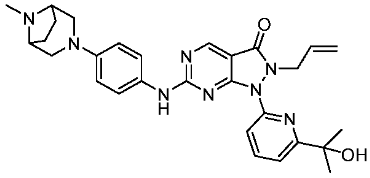 28 | 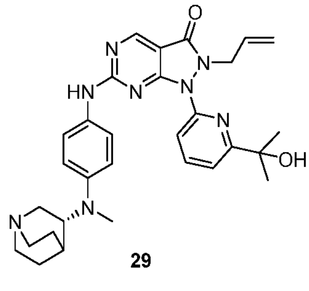 29 | 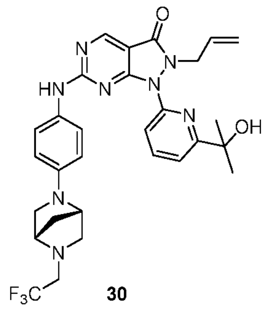 30 |
| 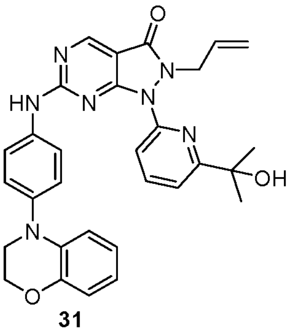 31 | 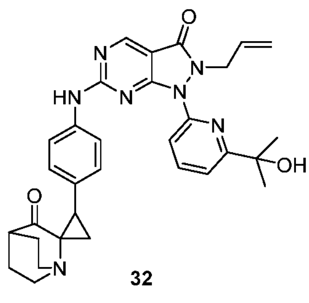 32 | 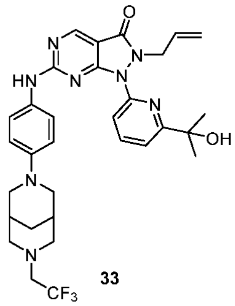 33 |
| 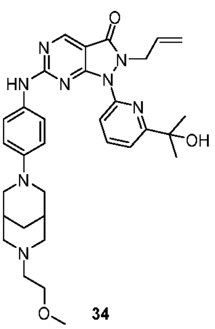 34 | 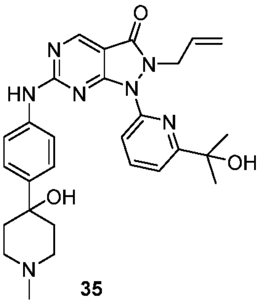 35 | 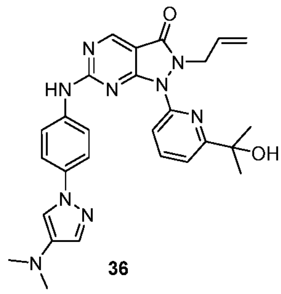 36 |
| 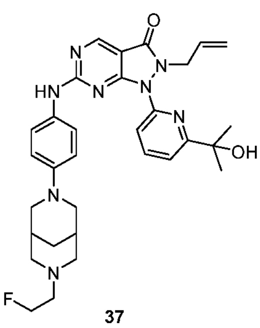 37 | 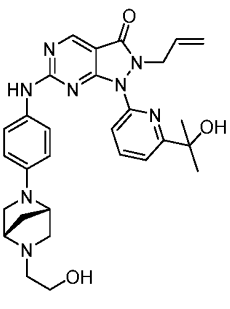 38 | 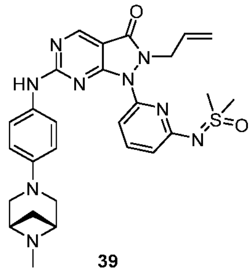 39 |

| | | |
|---|---|---|
| 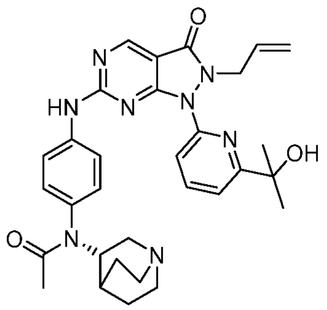 40 | 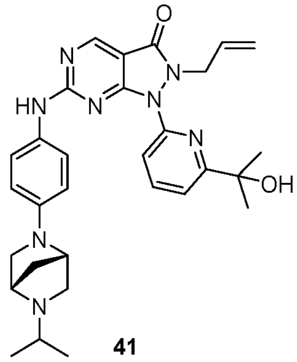 41 | 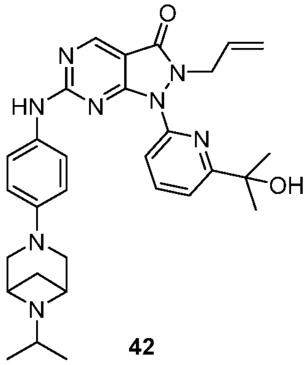 42 |
| 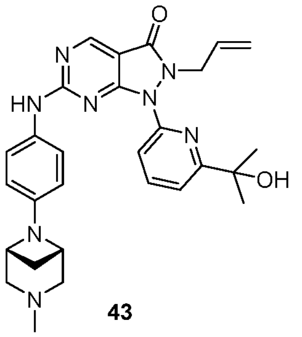 43 | 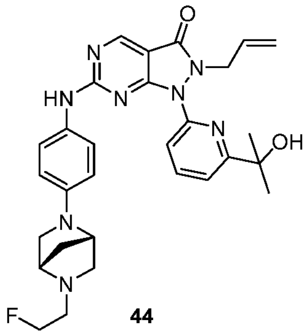 44 | 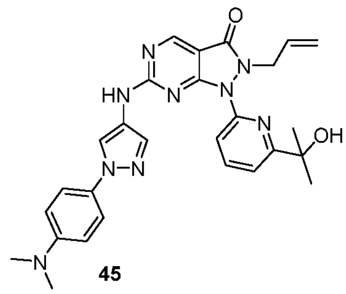 45 |
| 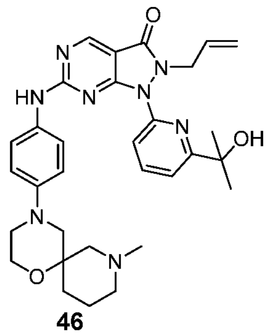 46 | 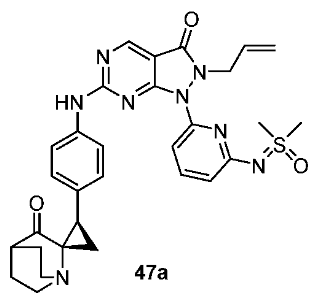 47a | 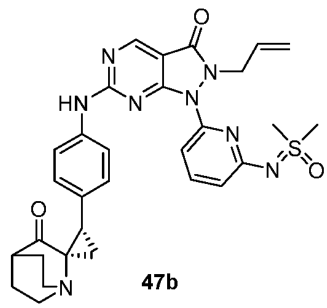 47b |
| 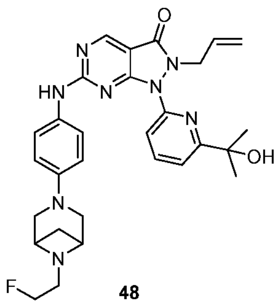 48 | 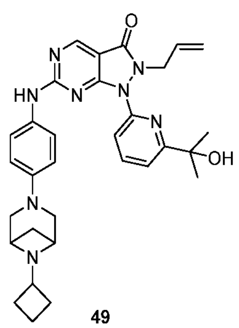 49 | 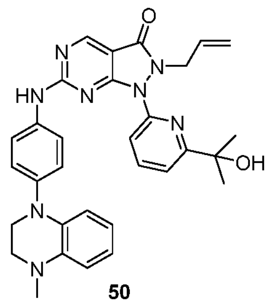 50 |

| | | |
|---|---|---|
| 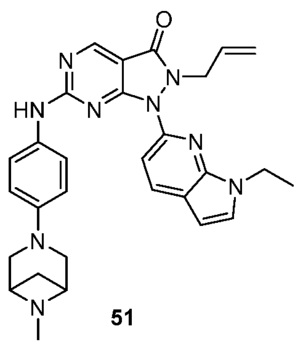 51 | 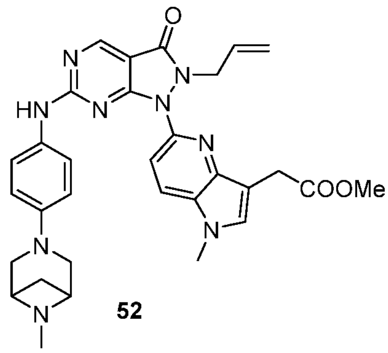 52 | 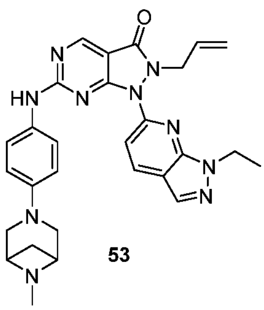 53 |
| 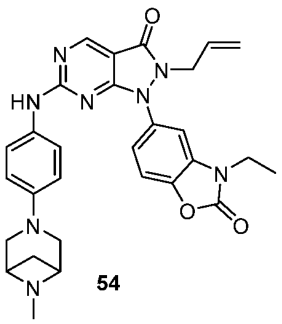 54 | 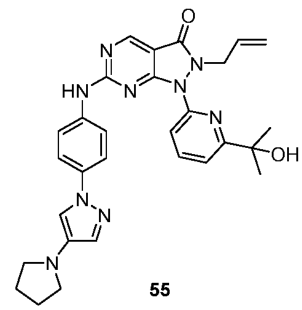 55 | 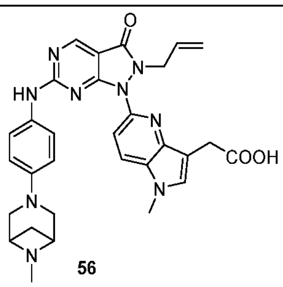 56 |
| 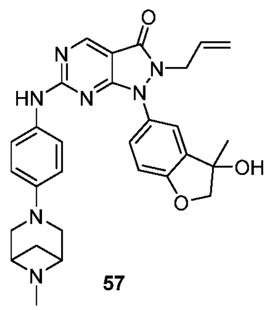 57 | 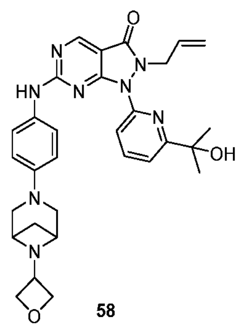 58 | 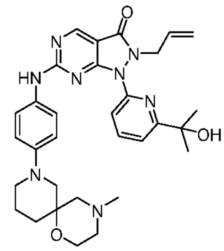 59 |
| 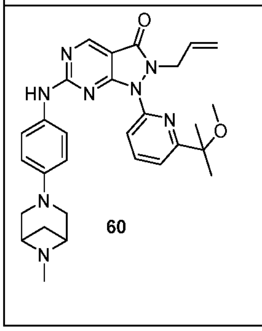 60 | 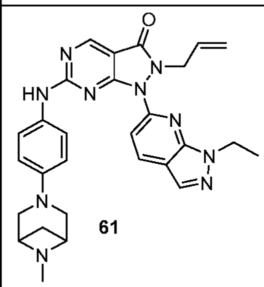 61 | 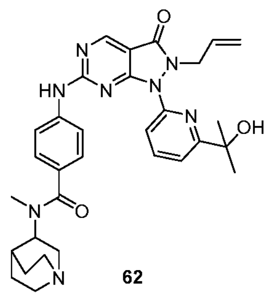 62 |

| | | |
|---|---|---|
| 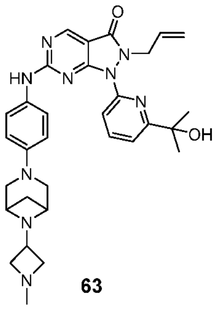 63 | 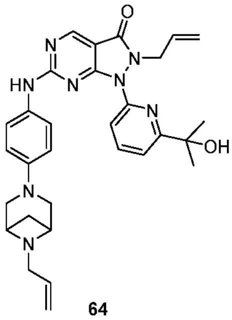 64 | 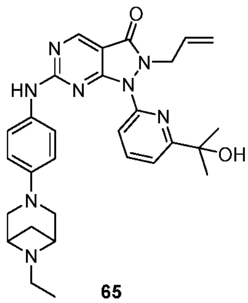 65 |
| 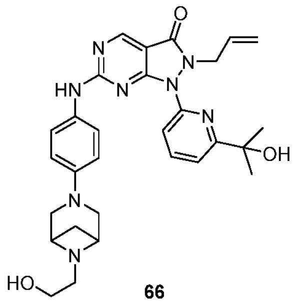 66 | 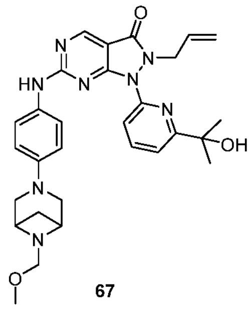 67 | 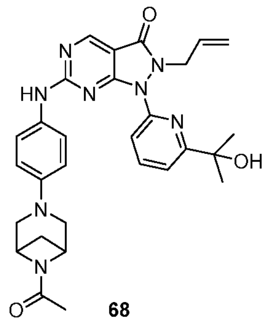 68 |
| 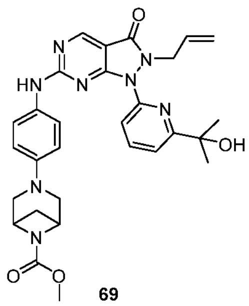 69 | 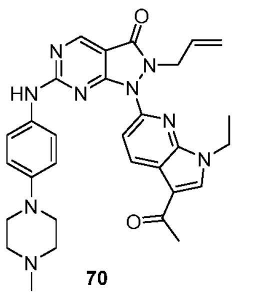 70 | 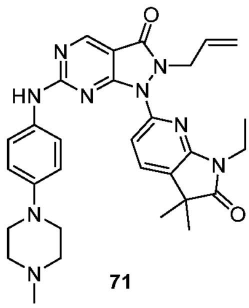 71 |
| 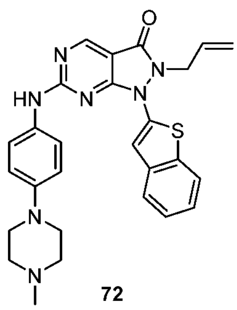 72 | 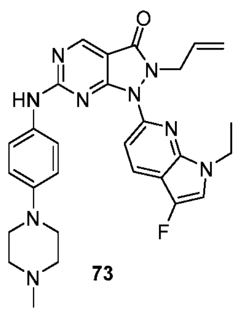 73 | 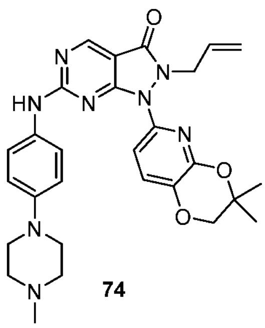 74 |
| 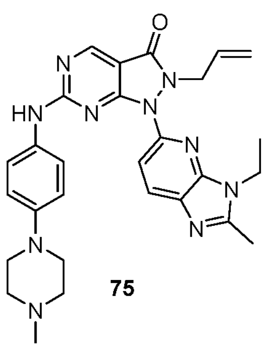 75 | 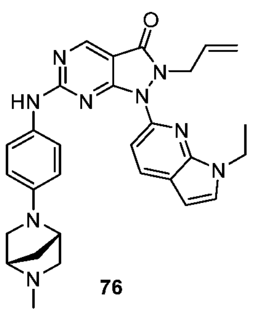 76 | 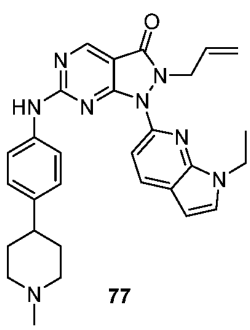 77 |

| | | |
|---|---|---|
| 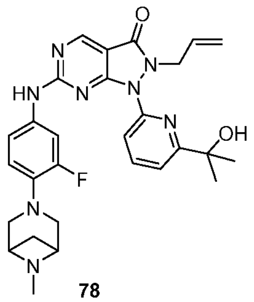 78 | 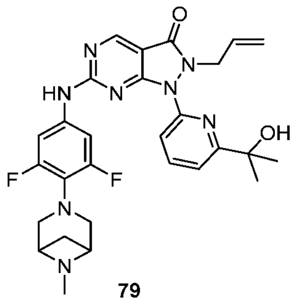 79 | 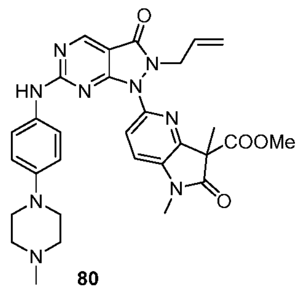 80 |

More preferably, in specific, the compound represented by Formula I of the present invention, or a stereoisomer thereof, or a pharmaceutically acceptable salt thereof, is

| | | |
|---|---|---|
| 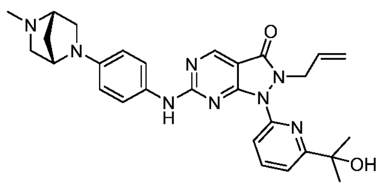 3 | 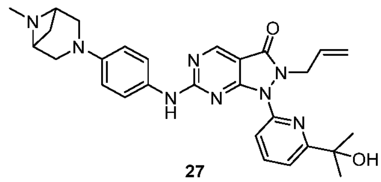 27 | 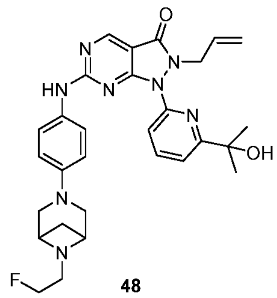 48 |
| 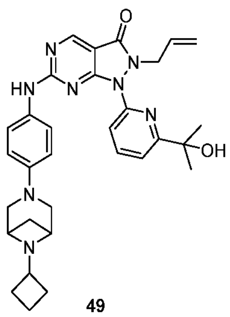 49 | 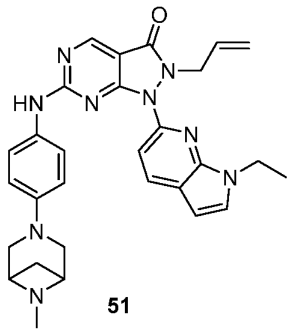 51 | 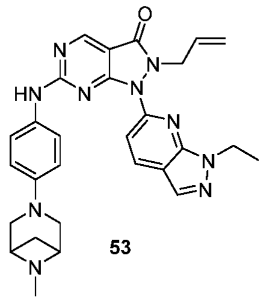 53 |

The present invention futher provides the use of any of the abovementioned compound, or a stereoisomer thereof, or a pharmaceutically acceptable salt thereof in the preparation of the drug for the treatment of WEE1-mediated disease(s).

The WEE1-mediated disease(s) is(are) one or more of the diseases associated with inflammation, autoimmune disease, infectious disease, cancer, precancer syndrome.

The present invention futher provides a pharmaceutical composition which is prepared with the compound of any of the abovementioned compound or a stereoisomer thereof, or a pharmaceutically acceptable salt thereof, as the pharmaceutically active ingredient, together with pharmaceutically acceptable excipients.

"Cancer" or "malignant neoplasm" means any of a number of diseases characterized by the uncontrolled abnormal cell proliferation which includes the spread of affected cells locally or through the bloodstream and lymphatic system to other parts of the body (i.e., metastasis) and any of many characteristic structural and/or molecular features. "Cancer cell" means a cell that undergoes multiple steps of tumor progression in the early, intermediate, or advanced phases. Cancers include sarcoma, breast cancer, lung cancer, brain cancer, cancer of bone, liver cancer, renal cancer, colon cancer and prostatic cancer. In some embodiments, compounds of Formula I are used to treat a cancer selected from colon cancer, brain cancer, breast cancer, fibrosarcoma, and squamous cell carcinoma. In some embodiments, the cancer is selected from melanoma, breast cancer, colon cancer, lung cancer, and ovarian cancer. In some embodiments, the cancer treated is a metastatic cancer.

Autoimmune diseases are caused by the body's immune response to substances and tissues normally present in the body. Examples of autoimmune diseases include myocarditis, lupus nephritis, primary biliary cirrhosis, psoriasis, type I diabetes mellitus, Grave's disease, celiac disease, Crohn's disease, autoimmune neutropenia, juvenile arthritis, rheumatoid arthritis, fibromyalgia, Guillain-Barre syndrome, multiple sclerosis and autoimmune retinopathy. Some embodiments of the present invention relate to the treatment of autoimmune diseases such as psoriasis or multiple sclerosis.

Inflammatory diseases include a wide range of conditions characterized by pathologic inflammation of tissues. Examples of inflammatory diseases include acne vulgaris, asthma, celiac disease, chronic prostatitis, glomerulonephritis, inflammatory bowel disease, pelvic inflammatory disease, reperfusion injury, rheumatoid arthritis, sarcoidosis, vasculitis, airway inflammation due to house dust mites, and interstitial cystitis. There is significant overlap between inflammatory and autoimmune diseases. Some embodiments of the present invention relate to the treatment of the inflammatory disease asthma. The immune system is usually involved in inflammatory disease and is manifested in allergic reactions and some myopathies. Many immune system diseases result in abnormal inflammation. IL-17A-mediated diseases also include autoimmune inflammatory diseases.

Compounds and derivatives provided in the present invention can be named according to IUPAC (International Union of Pure and Applied Chemistry) or CAS (Chemical Abstracts Service, Columbus, OH) nomenclature system.

Definition of terms used in the present invention: Unless otherwise specified, the initial definition provided by the group or term herein is applicable to the group or term in the whole specification. For terms that are not specifically defined herein, they should be given meanings that can be given by those skilled in the art according to the disclosure and context.

"Substitution" means that the hydrogen atom in the molecule is replaced by other different atoms or molecules.

The minimum and maximum values of the carbon atom content in the hydrocarbon group are indicated by prefixes. For example, the prefix $C_{a\sim b}$ alkyl indicates any alkyl group containing "a" to "b" carbon atoms. Therefore, for example, $C_{1\sim 4}$ alkyl refers to alkyl groups containing 1~4 carbon atoms.

"Alkyl" refers to a saturated hydrocarbon chain with a specified number of member atoms. For example, $C_{1\sim 6}$ alkyl refers to any alkyl group containing 1~6 member atoms, such as alkyl group containing 1~4 member atoms. Alkyl groups can be linear or branched. A representative branched alkyl group has one, two or three branches. Alkyl groups can be optionally substituted by one or more substituents as defined herein. Alkyl includes methyl, ethyl, propyl (n-propyl and isopropyl), butyl (n-butyl, isobutyl and tert-butyl), pentyl (n-pentyl, isopentyl and neopentyl) and hexyl. Alkyl group can also be a part of other groups, wherein said other group is for example -O($C_{1\sim 6}$ alkyl).

"Cycloalkyl", "cycloalkane" means a saturated or partially saturated cyclic group with carbon atoms and no cyclic heteroatoms, and with a single ring or multiple rings (including fused, combined). For polycyclic systems having aromatic and non-aromatic cyclyls without ring heteroatoms, the term "cycloalkyl" applies when the connection point is at a non-aromatic carbon atom(for example, 5,6,7,8,- tetrahydronaphthalen-5-yl). The term "cycloalkyl" includes cycloalkenyl groups such as cyclohexenyl. Examples of cycloalkyl groups include, for example, adamantyl, cyclopropyl, cyclobutyl, cyclohexyl, cyclopentyl, cyclooctyl, cyclopentenyl, and cyclohexenyl. Examples of cycloalkyl groups including polybicycloalkyl ring systems are bicyclohexyl, bicyclopentyl, bicyclooctyl, etc., such as

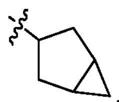, 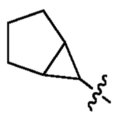, 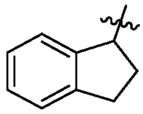

"Alkenyl" means a straight or branched chain hydrocarbon groups having from 2 to 10 carbon atoms and in some embodiments from 2 to 6 carbon atoms or from 2 to 4 carbon atoms and having at least 1 vinyl unsaturated site (>C=C<). For example, ($C_a$-$C_b$)alkenyl refers to an alkenyl group having a to b carbon atoms and is intended to include, for example, vinyl, propenyl, isopropenyl, 1 ,3-butadienyl, etc.

"Alkynyl" means a straight chain monovalent hydrocarbon group or a branched chain monovalent hydrocarbon group containing at least one triple bond. The term "alkynyl" is also intended to include those hydrocarbon groups having a triple bond and a double bond. For example, ($C_2$-$C_6$) alkynyl are intended to include ethynyl, propynyl, etc.

"Halogen" is fluorine, chlorine, bromine or iodine.

"Halogen substituted alkyl" refers to an alkyl wherein one or more hydrogen atoms are replaced by halogen; for example Halogen substituted $C_{1\sim 4}$ alkyl refers to an alkyl containing 1~4 carbon atoms wherein one or more hydrogen atoms are replaced by halogen.

"Heterocyclic group", "heterocycloalkyl", "heterocycloalkane" refers to a saturated or non-aromatic unsaturated ring containing at least one heteroatom; wherein a heteroatom refers to a nitrogen atom, an oxygen atom, or a sulfur atom.

"Aromatic heterocyclyl" means an aromatic unsaturated ring containing at least one heteroatom; wherein heteroatom means a nitrogen atom, an oxygen atom, a sulfur atom.

"Stereoisomer" includes both enantiomers and diastereomers. The compounds of the present invention may contain asymmetric or chiral centers, and thus different stereoisomers exist. All stereoisomeric forms of the compounds of the present invention include but not limited to diastereomers, enantiomers, atropisomerism, and mixtures thereof, such as racemic mixtures. They form part of the present invention. Many organic compounds exist in optically active forms, i.e., they have the ability to rotate planes of plane-polarized light. In describing optically active compounds, the prefixes D,L or R,S are used to indicate the absolute configuration of the chiral center of the molecule. These stereoisomers have the

same chemical structure, but their stereo structures are different. Specific stereoisomers may be enantiomers, and mixtures of isomers are often referred to as enantiomeric mixtures. A 50:50 mixture of enantiomers is referred to as a racemic mixture or racemate, which may result in a chemical reaction process that is not stereoselective or stereotactic. The terms "racemic mixture" and "racemate" refer to an equimolar mixture of two enantiomers lacking optical activity. For example, the C atom marked by the "*" on the ring in the compounds numbered Wee1-92 in the table of the present invention is the chiral center.

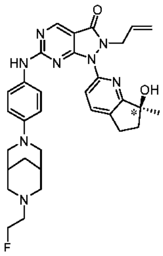

The term "pharmaceutically acceptable" refers to a medium, carrier, diluent, excipient, and/or a salt formed thereby chemically or physically compatible with other components constituting a pharmaceutical dosage form and physiologically compatible with the receptor.

The pharmaceutical compositions of the present invention may be in any one of a number of compoundable pharmaceutical dosage forms, e.g., oral, injectable, topical, etc.; the oral dosage forms include, but are not limited to: tablets, capsules, oral liquids, granules, pills, suspension; the injectable dosage forms are selected from point injection, powde injection; the topical dosage forms are selected from patches, creams. All dosage forms can be prepared according to common pharmaceutical techniques, such as using any of the compounds of the present invention, or stereoisomers thereof, or pharmaceutically acceptable salts thereof, as the active pharmaceutical ingredient, and, if necessary, incorporating pharmaceutically acceptable carriers, to form the above pharmaceutical dosage forms suitable for administration; wherein, the unit dose of the pharmaceutically active ingredient may be 0.1 mg-1000 mg, e.g. tablets containing 0.1 mg-1000 mg, preferably 5-500 mg of the pharmaceutically active ingredient per tablet.

The term "salt" and "pharmaceutically acceptable salt" refers to an acidic and/or basic salt formed by the abovementioned compound or a stereoisomer thereof, and inorganic and/or organic acid and base, also including zwitterionic salts (internal salts), also including quaternary ammonium salt, for example alkylammonium salt. These salts can be directly obtained in the final separation and purification of the compound. Alternatively, they can be obtained by mixing the abovementioned compound, or a stereoisomer thereof, and a certain amount of acid or base appropriately (for example in same equivalence). These salts may form precipitates in the solution and be collected by filtration, or be recovered after solvent evaporation, or be prepared by freeze-drying after the reaction in water medium. Said salt in the present invention can be compound hydrochloride salt, sulfate salt, citrate salt, benzene sulfonate salt, hydrobromide salt, hydrofluoride salt, phosphorate salt, acetate salt, propionate salt, succinate salt, oxalate salt, malate salt, succinate salt, fumarate salt, maleate salt, tartarate salt or trifluoroacetate salt.

In some embodiments, one or more compounds of the present invention can be used in combination with each other. Alternatively, the compound of the present invention can be used in combination with any other active agents. It is used to prepare drugs or pharmaceutical compositions for regulating cell functions or treating diseases. If a group of compounds are used, these compounds can be administered to the subjects simultaneously, separately and orderly.

Obviously, according to the above content of the present invention, according to the common technical knowledge and common means in this field, and without departing from the above basic technical idea of the present invention, other various forms of modification can be made to replace or change.

The advantagous effects of the present invention are:

Compared with the prior art, the compounds of the present invention have better WEE1 kinase inhibitory activity and tumor cell value-added inhibitory activity; the inhibition of cytochrome P450 enzymes and hERG potassium ion channels is reduced, and the safety is better.

In the following, the above content of the present invention will be further explained in detail through the concrete implementation in the form of examples. However, it should not be understood that the scope of the above theme of the present invention is limited to the following examples. All technologies realized based on the above content of the present invention belong to the scope of the present invention.

## DETAILED DESCRIPTION OF THE INVENTION

The structure of the compound was determined by nuclear magnetic resonance (NMR) and mass spectrometry (MS). The NMR shift (δ) was given in units of $10^{-6}$(ppm). The NMR was measured by (Bruker Avance III 400 and Bruker

Avance 300) nuclear magnetic apparatus. Deuterated methyl sulfoxide (DMSO-$d_6$), deuterated chloroform ($CDCl_3$) and deuterated methanol ($CD_3OD$) were used as the charaterization solvents, and tetramethylsilane (TMS) was used as the internal standard.

The LC-MS was determined by Shimadzu LC-MS 2020 (ESI). The HPLC was determined by Shimadzu LC-20A. MPLC (medium performance liquid chromatography) was conducted by Gilson GX-281 reverse phase preparative chromatography. Yantai Huanghai HSGF254 or Qingdao GF254 silica gel plate was used as the silica gel plate for thin-layer chromatography, and the specification of thin-layer chromatography separation and purification products was 0.4 mm~0.5 mm. Column chromatography generally used Yantai Huanghai silica gel 200~300 mesh silica gel as carrier.

The known starting materials of the present invention can be synthesized by or according to the methods known in the field, or can be purchased from Anneiji Chemical, Chengdu Kelon Chemical, Shaoyuan Chemical Technology, Bailingwei Technology etc.

Unless otherwise specified in Examples, the reaction was carried out under $N_2$ atmosphere. Unless otherwise specified in the examples, the solution refers to an aqueous solution. Unless otherwise specified in Examples, the reaction temperature was room temperature. Unless otherwise specified in Examples, M refers to mole per liter.

THF: Tetrahydrofuran; DIPEA: N,N-Diisopropylethylamine;
DCM: Dichloromethane; TFA: Trifluoroacetic acid; m-CPBA: m-Chloroperoxybenzoic acid;
DMF: Dimethylformamide; PTSA: p-Toluenesulfonamide;
DMSO: Dimethyl sulfoxide; NBS:N-Bromosuccinimide; AIBN: Azobisisobutyronitrile;
Unless otherwise specified in Examples, the HPLC test conditions were as follows:

Method A:

Column: Boston Green C18 150 mm*4.6 mm 5 um; Mobile Phase A: 0.05% TFA Water B:0.05% TFA Acétonitrile; Gradient: B from 5% to 95% in 10.0 min and hold 95% for 5.0 min; Flow rate: 1.5 mL/min; Column temperature: 40°C.

Method B:

Column: Boston Green ODS 150 mm*4.6 mm 5 μm; Mobile Phase A: 0.01M $NH_4HCO_3$ Water B: Acétonitrile; Gradient: B from 5% to 95% in 10.0 min and hold 95% for 5.0 min; Flow rate: 1.5 mL/min; Column temperature: 40°C.

Unless otherwise specified, the SFC splitting condition is: Column: 3 μm, 150 mm * 3 mm; Mobile Phase A: $CO_2$, B: ethanol; Flow rate: 1 mL/min; Column temperature: 40°C.

**Intermediate Example 1: Synthesis of Intermediate IM-1**

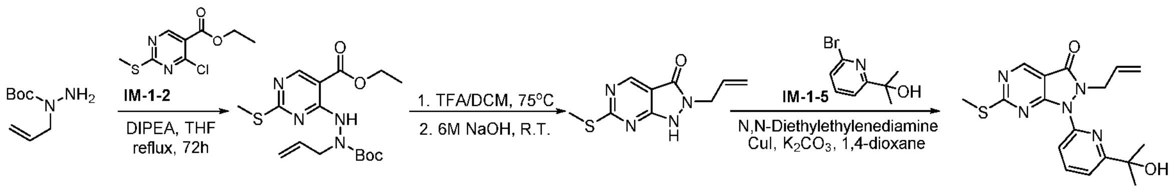


IM-1-1 IM-1-3 IM-1-4 IM-1

**Step 1: Synthesis of Compound IM-1-3:**

The substrate IM-**1-2** (8.15 g, 35 mmol) and THF (50mL) were added to a dry single-necked flask, stirred to dissolve, then added with IM-1-1 (6.3 g, 37 mol) and DIPEA (15 mL, 75 mol), heated to 110°C to react and monitored by LC-MS. After the reaction was completed, the solid was precipitated and filtered out after the system returned to room temperature, and oven-dried to give the crude product **IM-1-3** (9.87 g, 76.6% yield),LCMS ($ESI^+$) m/z: 369.2 $[M+H]^+$.

**Step 2: Synthesis of Compound IM-1-4:**

The substrate IM-**1-3** (9.16 g, 27.5 mmol) and DCM (18mL) were added to a dry single-necked flask, stirred to dissolve, then added slowly with TFA (18 mol), heated to 75°C to react and monitored by LC-MS. The organic solvents were concentrated under reduced pressure after the reaction was completed, dissolved with ethanol, then added with 6M NaOH solution, stirred at room temperature and monitored by LC-MS, concentrated under reduced pressure after the

reaction to precipitate the solid. The solid was filtered out, washed three times with water and cold ethanol respectively, dried at room temperature to give crude product **IM-1-4** (5 g, 81.7% yield), LCMS (ESI+) m/z: 223.1 [M+H]+.

**Step 3: Synthesis of Compound IM-1:**

The substrate IM-**1-4** (5.20 g, 23.4 mmol), $K_2CO_3$ (4.53 g, 32.8 mmol) and **IM-1-5** (5.55 g, 25.7 mmol) were added to a dry double-necked flask, and then added with CuI(4.46 g, 23.4 mmol), N,N'-Dimethylethylenediamine (4.13 g, 46.8 mmol) and 1,4-Dioxane (100 mL) after three $N_2$ replacements, stirred evenly and then heated to 120°C to react and monitored by LC-MS and TLC, . added with ammonia after the reaction was completed, stirred, then extracted three times with ethyl acetate. The organic phase was combined and dewatered by anhydrous $Na_2SO_4$ and then concentrated under reduced pressure. The residue was purified by medium pressure liquid chromatography (Alkali process) to give the compound **IM-1** (5.1 g, 61% yield), LCMS (ESI+) m/z: 358.1 [M+H]+.

**Intermediate Example 2: Synthesis of Intermediate IM-2**

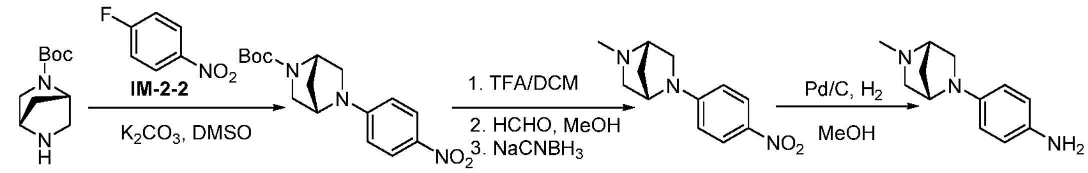


IM-2-1 IM-2-3 IM-2-4 IM-2

**Step 1: Synthesis of Compound IM-2-3:**

The substrate IM-2-**1** (7.12 g, 50 mmol) and DMSO (30 mL) were added to a dry single-necked flask, stiired to dissolve then added with **IM-2-2** (9.9 g, 50 mmol), heated to 90°C to react and monitored by LC-MS, cooled to room temperature after the reaction was completed, the precipitated solid was filtered out and washed three times with water and a small amount of methanol respectively, dried to give product **IM-2-3** (12.12 g, 76% yield), LCMS (ESI+) m/z: 320.3 [M+H]+.

**Step 2: Synthesis of Compound IM-2-4:**

The substrate IM-**2-3** (12.12 g, 38 mmol) and **DCM** (18mL) were added to a dry single-necked flask, stirred to dissolve then added slowly with TFA (18 mL) and monitored by LC-MS, concentrated under reduced pressure after the reaction to remove solvents to give thr residue. The residue was added with methanol (70 mL), stirred to dissolve then added with polyformaldehyde (7g) and acetic acid (1 mL), stirred for 30 min at room temperature, added with sodium cyanoborohydride (3.8g, 60mmol), and heated to 50°C to react, cooled to room temperature after the reaction to filter out the solids. The organic phase was concentrated under reduced pressure to remove solvents. The residue was purified by medium pressure liquid chromatography (Alkali process) to give the compound **IM-2-4** (7.6 g, 85.8% yield), LCMS (ESI+) m/z: 234.2[M+H]+.

**Step 3: Synthesis of Compound IM-2:**

The substrate **IM-2-4** (5 g, 21.5 mmol) and methanol (40mL) were added to a dry single-necked flask, stirred to dissolve then added with **Pd/C** (500 mg), stirred at room temperature after five $H_2$ displacements to react and monitored by LC-MS. After the reaction was completed, Pd/C was removed by diatomaceous earth and the organic phase was concentrated under reduced pressure to give the compound **IM-2** (4.0g, crude), LCMS (ESI+) m/z: 204.2[M+H]+.

**Intermediate Example 3: Synthesis of Intermediate IM-3**

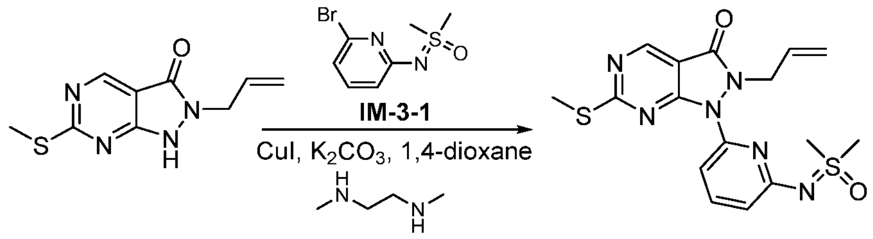


IM-1-4 IM-3

**Step 1: Synthesis of Compound IM-3:**

The substrate IM-**1-4** (138 mg, 620.88 μmol), $K_2CO_3$ (101 mg, 620.88 μmol) and **IM-3-1** (130 mg, 521.82 μmol) were added to a dry double-necked flask, $N_2$ displaced three times, then added with CuI (99 mg, 519.82 μmol), N,N'-Dimethylethylenediamine (106 mg, 1.20 mmol) and 1,4-Dioxane (10 mL), stirred evenly then heated to 120°C to react and monitored by LC-MS, returned to room temperature after the reaction and added with ammonia, extracted three times with ethyl acetate. After the organic phase was concentrated under reduced pressure, and the residue was purified by medium pressure liquid chromatography (Alkali process)to give the ompound **IM-3** (130 mg, 53.62% yield), LCMS ($ESI^+$) m/z: 391.2 $[M+H]^+$.

**Intermediate Example 4: Synthesis of Intermediate IM-4**

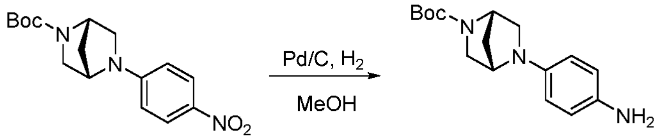


IM-2-3 IM-4

**Step 1: Synthesis of Compound IM-4:**

The substrate IM-**2-3** (1 g, 3.13 mmol) and methanol (10mL) were added to a dry single-necked flask, stirred to dissolve then added with **Pd/C**(200 mg), $H_2$ displaced five times, stirred at room temperature to react and monitored by LC-MS. After the reaction was completed, Pd/C was removed by diatomaceous earth and the organic phase was concentrated under reduced pressure to give the compound **IM-4** (700 mg, crude),LCMS ($ESI^+$) m/z: 290.2$[M+H]^+$.

**Intermediate Example 5: Synthesis of Intermediate IM-5**

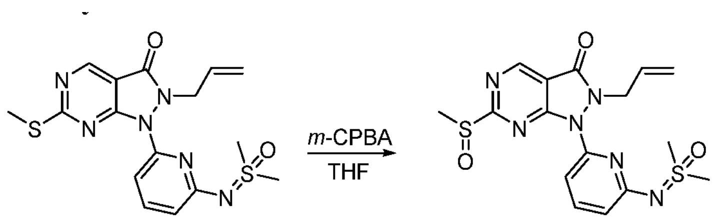


IM-3 IM-5

**Step 1: Synthesis of Compound IM-5:**

The substrate IM-3 (0.2 g, 0.51 mmol) and anhydrous THF (5ml) were added to a single-necked flask, stirred to dissolve then added with m-CPBA (208.2g, 1.02mmol, purity 85%), stirred for 40 min at room temperature and monitored by LC-MS. The reactants were concentrated under reduced pressure, then separated by medium pressure liquid

chromatographyto give to the compound **IM-5** (160 mg), LCMS (ESI$^+$) m/z: 407.1 $[M+H]^+$.

**Intermediate Example 6: Synthesis of Intermediate IM-6**

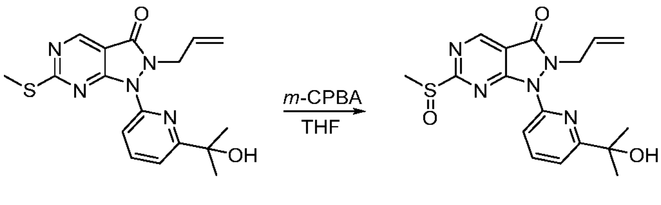


IM-1 IM-6

**Step 1: Synthesis of Compound IM-6:**

The substrate IM-1 (0.2g, 0.56mmol) and anhydrous THF (5ml) were added to a single-necked flask, stirred to dissolve then added with *m*-CPBA (144.9mg, 0.84mmol, purity 85%), stirred for 40 min at room temperature and monitored by LC-MS. The reactants were concentrated under reduced pressure, then separated by medium pressure liquid chromatography to give to the compound **IM-6** (150 mg, 0.40 mmol, 71.8% yield), LCMS (ESI$^+$) m/z: 374.1 $[M+H]^+$.

**Example 1: Synthesis of Compound 1**

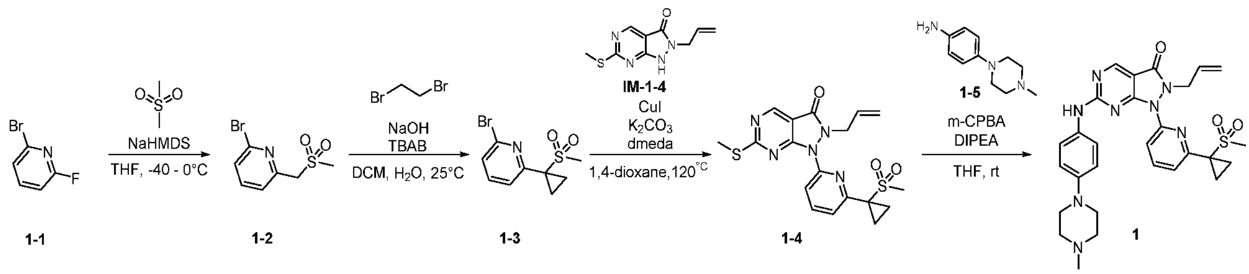


1-1 1-2 1-3 1-4 1

**Step 1: Synthesis of Compound 1-2:**

The substrate 2-bromo-6-fluoropyridine (3g, 15mmol) and tetrahydrofuran (25 mL) were added to dry round-bottomed flask, lowered to -40 °C and added slowly and dropwise with NaHMDS (12.79 g, 85.23 mmol) the system, stirred for 30 min at -40 °C thend added with dimethyl sulfoxide (3.21 g, 34.09 mmol), stirred continously for 3 hours at this temperature. The reaction was quenched with water, extracted three times with ethyl acetate, the organic phase was dried with anhydrous $Na_2SO_4$, filtered, distilled under reduced pressure and purified by MPLC to give the compound **1-2** (1.5 g, 6.00 mmol), LCMS (ESI) m/z: 252.0 $[M+H]^+$.

**Step 2: Synthesis of Compound 1-3:**

The Compounds **1-2** (1.5 g, 6.00 mmol) and Tetrabutylammonium bromide (193.1 mg, 0.6 mmol) and dichloromethane (10 mL) were added to a dry flask at room temperature, stirred for 10 min at room temperature then added with NaOH (9.60 g, 239.9 mmol) and water (10mL), stirred for 12 h at room temperature, the reaction was quenched with water, extracted three times with ethyl acetate, the combined organic phase was washed with saline and dried with anhydrous $Na_2SO_4$, filtered, distilled under reduced pressure. The residue was purified by MPLC to give the compound **1-3** (450mg, 1.63mmol), LCMS (ESI) m/z: 278.0 $[M+H]^+$.

**Step 3: Synthesis of Compound 1-4:**

The substrate **1-3** (93mg, 336.77μmol), **IM-1-4** (89.82mg, 404.13μmol), CuI (64.14mg, 336.77μmol), $K_2CO_3$ (65.17mg, 505.16μmol) and N,N'-Dimethylethylenediamine (29.64mg, 336.77μmol) were added to a dry microwave tube

under $N_2$ protection, then added with 1,4-Dioxane (5mL), heated to react at 120°C for 10 hours under $N_2$ atmosphere and monitored by LC-MS. After the reaction was completed, the reaction system was cooled to room temperature, filtered, extracted three times with ethyl acetate, the organic phase was dried with anhydrous $Na_2SO_4$, filtered, distilled under reduced pressure. The residue was purified by MPLC to give the compound **1-4** (50 mg, 119.76 μmol), LCMS (ESI) m/z: 418.1 $[M+H]^+$.

**Step 4: Synthesis of Compound 1:**

The m-chloroperoxybenzoic acid (18.74 mg, 107.8 μmol) was added to the tetrahydrofuran (1mL) containing dissolved **1-4** (30mg, 71.9μmol), stirred for 1 hour at room temperature and monitored by LC-MS, then added with N, N-diisopropylethylamine (43 mg, 0.33 mmol) and 1-5 (15.3 mg, 80.1 μmol) after the disappearance of the raw materials, stirred for 12 hours at room temperature and monitored by LC-MS, concentrated under reduced pressure after the reaction. The residue was purified by High Performance Liquid Chromatography (Acid) to give the compound 1 (30 mg, 53.51 μmol). LCMS (ESI) m/z: 561.3 $[M+H]^+$, HPLC method A: $R_T$= 5.05 min, purity: 98.2%. $^1H$ NMR (400 MHz, Methanol-$d_6$) δ 8.79 (s, 1H), 8.05 - 8.01 (m, 1H), 7.93 (d, *J* = 8.4 Hz, 1H), 7.61 (d, *J* = 7.6 Hz, 1H), 7.57 - 7.42 (m, 2H), 7.03 - 6.88 (m, 2H), 5.71 - 5.67(m, 1H), 5.06 - 5.05 (m, 1H), 4.98 - 4.95 (m, 1H), 4.80 - 4.78 (m, 2H), 3.20 - 3.18 (m, 5H), 3.04 (s, 4H), 2.65 - 2.63 (m, 5H), 2.36 (s, 4H), 1.92 - 1.75 (m, 2H), 1.53 (d, *J* = 2.4 Hz, 2H).

**Example 2: Synthesis of Compound 2**

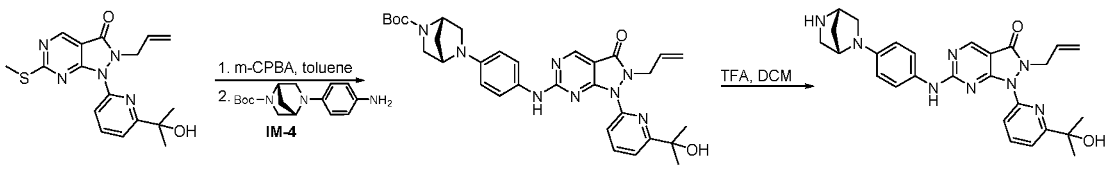


IM-1 2-1 2

**Step 1: Synthesis of Compound 2-1:**

The substrate **IM-1** (43 mg, 120.30 μmol) and methylbenzene (1mL) were added to a dry single-necked flask, stirred to dissolve then added with m-CPBA (43.60 mg, 252.64 μmol) to react for 30 min at room temperature and monitored by LC-MS, added with DIPEA (80.6 mg, 623.65 μmol, 108.63 μL) after the reaction and stirred for 10 min, then added with **IM-4** (45.14 mg, 0.156 mmol) and monitored by LC-MS. The reactants were concentrated under reduced pressure after the reaction. The residue was purified by medium pressure liquid chromatography (Alkali process) to give the compound **2-1** (45mg, 62.64% yield). **$^1$H NMR** (400 MHz, DMSO-$d_6$) δ 10.08 (s, 1H), 8.80 (s, 1H), 8.00 (t, *J* = 8.0 Hz, 1H), 7.74 (d, *J* = 8.2 Hz, 1H), 7.59 (d, *J* = 7.7 Hz, 1H), 7.51 (s, 2H), 6.61 (d, *J* = 8.5 Hz, 2H), 5.71-5.61 (m, 1H), 5.31 (s, 1H), 4.99 (dd, *J* = 10.2, 1.5 Hz, 1H), 4.83 (dd, *J* = 17.1, 1.5 Hz, 1H), 4.68 (d, *J* = 5.9 Hz, 2H), 4.49 (s, 1H), 4.42 (d, *J* = 23.5 Hz, 1H), 3.55 (t, *J* = 10.6 Hz, 1H), 3.27 - 3.20 (m, 2H), 2.96 (t, *J* = 9.0 Hz, 1H), 1.93 (s, 2H), 1.46 (s, 6H), 1.39 (s, 5H), 1.32 (s, 4H).**$^1$H NMR** (400 MHz, DMSO-$d_6$, $D_2O$) δ 8.81 (s, 1H), 8.02 (t, *J* = 7.9 Hz, 1H), 7.73 (d, *J* = 7.8 Hz, 1H), 7.60 (d, *J* = 7.7 Hz, 1H), 7.51 (s, 2H), 6.62 (d, *J* = 8.5 Hz, 2H), 5.71 - 5.61 (m, 1H), 5.04 - 5.01 (m, 1H), 4.85 - 4.81 (m, 1H), 4.69 (d, *J* = 5.9 Hz, 2H), 4.49 - 4.41 (m, 2H), 3.57 (t, *J* = 9.0 Hz, 1H), 3.36 - 3.25 (m, 2H), 2.96 (t, *J* = 8.4 Hz, 1H), 1.94 (s, 2H), 1.47 (s, 6H), 1.39 (s, 5H), 1.32 (s, 4H). LCMS (E+) m/z: 599.4 $[M+H]^+$, HPLC method B: $R_T$= 8.23 min, purity: 91.5%. **Step 2: Synthesis of Compound 2:**

The substrate **2-1** (40.00mg, 66.81μmol) and DCM (3mL) were added to a dry single-necked flask, stirred to dissolve then added slowly with TFA (3 mL) and monitored by LC-MS, concentrated under reduced pressure to remove the solvents after the reaction and give the residue. The residue was purified by medium pressure liquid chromatography (Acid process) to give the compound 2 (25 mg, 61.08% yield). **$^1$H NMR** (400 MHz, Methanol-$d_4$) δ 8.77 (s, 1H), 7.97 (t, *J* = 7.9 Hz, 1H), 7.76 (d, *J* = 8.1 Hz, 1H), 7.63 (dd, *J* = 7.8, 0.8 Hz, 1H), 7.54 (d, *J* = 8.4 Hz, 2H), 6.67 (d, *J* = 8.6 Hz, 2H), 5.70 (m, 1H), 5.03 (dq, *J* = 10.2, 1.2 Hz, 1H), 4.92 (d, *J* = 1.2 Hz, 1H), 4.80 (d, *J* = 6.1 Hz, 2H), 4.66 (s, 1H), 4.48 (s, 1H), 3.76 (dd, *J* = 10.6, 2.5 Hz, 1H), 3.42 - 3.34 (m, 3H), 2.32 (d, *J* = 11.1 Hz, 1H), 2.05 (d, *J* = 11.3 Hz, 1H), 1.57 (s, 6H). LCMS (E+) m/z: 499.2 $[M+H]^+$, HPLC method A: $R_T$= 5.03 min, purity: 99.2%.

**Example 3: Synthesis of Compound 3:**

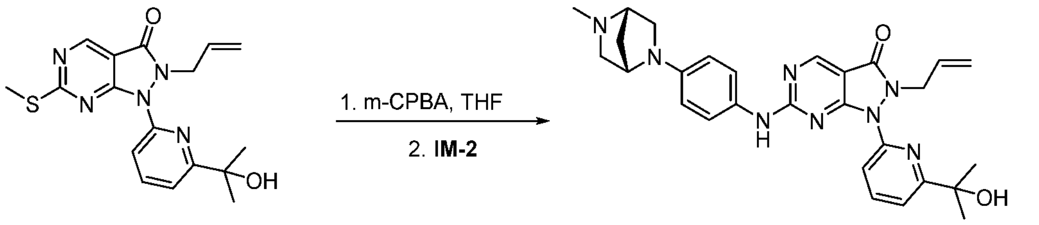


**IM-1** **3**

**Step 1: Synthesis of Compound 3:**

The substrate **IM-1** (3.57g, 10 mmol) and tetrahydrofuran (30mL) were added in a dry single-necked flask, stirred to dissolve then added with m-CPBA (2.5 g, 15 mmol, purity 85%) to react for 1 hour at room temperature and monitored by LC-MS, added with DIPEA (6.45 g, 50 mmol) after the reaction and stirred for 10 min, then added with **IM-2** (2.1 g, 10 mmol) and monitored by LC-MS, concentrated under reduced pressure after the reaction to remove the solvents. The residue was purified by medium pressure liquid chromatography (Alkali process) to give the compound 3 (2.1g, 41% yield). **$^1$H NMR** (400 MHz, DMSO-$d_6$) δ 10.04 (s, 1H), 8.79 (s, 1H), 8.00 (t, *J* = 7.9 Hz, 1H), 7.75 (s, 1H), 7.59 (d, *J* = 7.7 Hz, 1H), 7.48(s, 2H), 6.56 (d, *J* = 8.5 Hz, 2H), 5.71-5.61 (m, 1H), 5.32 (s, 1H), 4.99 (dd, *J* = 10.3, 1.5 Hz, 1H), 4.82 (d, *J* = 17.1, 1H), 4.67 (d, *J* = 6.0 Hz, 2H), 4.27 (s, 1H), 3.42 (s, 1H), 3.13 (d, *J* = 9.0 Hz, 1H), 2.78 (d, *J* = 9.5, 1H), 2.52 (d, *J* = 9.7 Hz, 2H), 2.25 (s, 3H), 1.87 (d, *J* = 9.3 Hz, 1H), 1.76 (d, *J* = 9.2 Hz, 1H), 1.46 (s, 6H). **$^1$H NMR** (400 MHz, DMSO-$d_6$, $D_2O$) δ 8.80 (s, 1H), 8.02 (t, *J* = 7.9 Hz, 1H), 7.73 (s, 1H), 7.61 (d, *J* = 7.8 Hz, 1H), 7.48 (s, 2H), 6.58 (d, *J* = 8.6 Hz, 2H), 5.71-5.60 (d, *J* = 10.3 Hz, 1H), 5.03 (m, 1H), 4.83 (d, *J* = 17.1 Hz, 1H), 4.68 (d, *J* = 5.9 Hz, 2H), 4.29 (s, 1H), 3.49 (s, 1H), 3.35 (d, *J* = 9.5 Hz, 1H), 3.17 (d, *J* = 9.4 Hz, 1H), 2.82 (d, *J* = 9.7 Hz, 1H), 2.56 (d, *J* = 9.7 Hz, 1H), 2.27 (s, 3H), 1.91 (d, *J* = 9.6 Hz, 1H), 1.81 (d, *J* = 9.6 Hz, 1H), 1.47 (s, 6H). LCMS (E+) m/z: 513.2 $[M+H]^+$, HPLC method B: $R_T$= 6.02 min, purity: 98.4%.

**Example 4: Synthesis of Compound 4:**

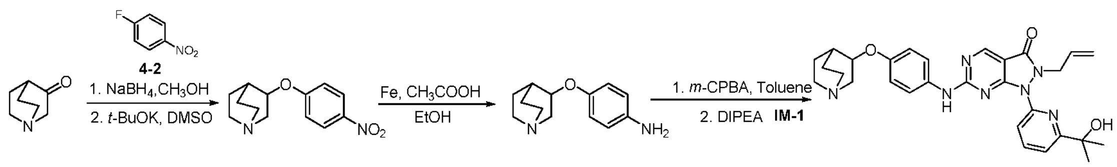


**4-1** **4-3** **4-4** **4**

**Step 1: Synthesis of Compound 4-3:**

The substrate **4-1** (114mg, 910.78μmol) and methanol (2mL) were added in a dry single-necked flask, stirred to dissolve with stirring and added with NaBH4 (38 mg, 1.00 mmol) to react for 1 hour at room temperature and monitored by LC-MS, quenched by slow addition of dilute hydrochloric acid in an ice bath after the reaction and concentrated under reduced pressure to remove the solvents. The residue was added with DMSO (4mL), stirred to dissolve with stirring, added with potassium tert-butoxide (337mg, 2.36 mmol) and then added with the compound **4-2** (141mg, 999.29μmol), heated up to 80°C to react and monitored by LC-MS, extracted three times with ethyl acetate after the reaction and concentrated under reduced pressure, then purified by medium pressure liquid chromatography (Alkali process) to give the compound **4-3** (99 mg, 50.71% yield), LCMS ($ESI^+$) m/z: 249.2$[M+H]^+$.

**Step 2: Synthesis of Compound 4-4:**

The substrate **4-3** (99mg, 398.75μmol) and ethanol (4mL) were added to a dry single-necked flask, stirred to dissolve then added with Fe (67mg, 1.20mmol) and $CH_3COOH$ (47.89mg, 797.50 μmol, 45.65μL), heated up to 80°C to react and monitored by LC-MS, filtered by diatomaceous earth after the reaction and washed with ethyl acetate, concentrated under reduced pressure to give the crude product **4-4** (80mg, crude),LCMS ($ESI^+$) m/z: 219.2$[M+H]^+$.

**Step 3: Synthesis of Compound 4:**

The substrate **IM-1** (71 mg, 0.2 mmol) and methylbenzene (2mL) were added in a dry single-necked flask, stirred

to dissolve then added with m-CPBA (52 mg, 0.3 mmol, purity 85%) to react for 1 hour at room temperature and monitored by LC-MS, added with DIPEA (83.58 mg, 646.69 μmol, 112.64 μL) after the reaction and stirred for 10 min, then added with the compound **4-4** (52.39 mg, 0.24 mmol) and monitored by LC-MS, concentrated under reduced pressure to remove the solvents after the reaction to give the residue. The residue was purified by medium pressure liquid chromatography (Alkali process) to give the compound **4** (8 mg, 6.32% yield). **$^1$H NMR** (400 MHz, DMSO-$d_6$) δ 10.18 (s, 1H), 8.84 (s, 1H), 8.04 (t, *J* = 7.9 Hz, 1H), 7.73 (d, *J* = 7.2, 1H), 7.62-7.60 (m, 3H), 6.90 (d, *J* = 7.7 Hz, 2H), 5.71-5.62 (m, 1H), 5.33 (s, 1H), 4.99 (dd, *J* = 10.3, 1.4 Hz, 1H), 4.82 (dd, *J* = 10.3, 1.4 Hz, 1H), 4.68 (d, *J* = 5.9 Hz, 2H), 4.44-4.42 (m, 1H), 2.77-2.60 (m, 6H), 2.04-2.03 (m, 1H), 1.84-1.81(m, 1H), 1.66 - 1.63(m, 1H), 1.57-1.54 (m, 1H), 1.46 (s, 6H), 1.36 - 1.33 (m, 1H). **$^1$H NMR** (400 MHz, DMSO-$d_6$, $D_2O$) δ 8.85 (s, 1H), 8.04 (t, *J* = 7.9 Hz, 1H), 7.72 (d, *J* = 7.6 Hz, 1H), 7.64-7.59 (m, 3H), 6.92 (d, *J* = 8.5 Hz, 2H), 5.72-5.62 (m, 1H), 5.03 (d, *J* = 10.3 Hz, 1H), 4.83 (d, *J* = 17.1 Hz, 1H), 4.69 (d, *J* = 5.9 Hz, 2H), 4.49 (s, 1H), 2.81- 2.63 (m, 6H), 2.06 (s, 1H), 1.86 (s, 1H), 1.70-1.68 (m, 1H), 1.62 (d, *J* = 7.4 Hz, 1H), 1.47 (s, 6H), 1.42-1.37 (m, **1**H). LCMS (E+) m/z: 528.2 [M+H]$^+$, HPLC method B: $R_T$= 6.34 min, purity: 82.9%.

**Example 5: Synthesis of Compound 5:**

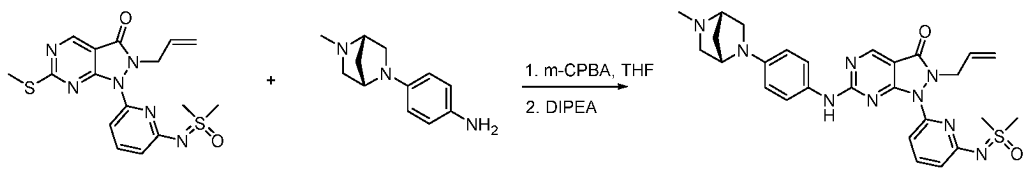


IM-3 IM-2 5

**Step 1: Synthesis of Compound 5:**

The substrate **IM-3** (30 mg, 77 μmol) and tetrahydrofuran (2mL) were added to a dry single-necked flask, stirred to dissolve then added with m-CPBA (20 mg, 116 μmol) to react for 1 hour at room temperature and monitored by LC-MS, added with DIPEA (50 mg, 385 μmol) after the reaction and stirred for 10 min, then added with the compound **IM-2** (20 mg, 98.39 μmol) and monitored by LC-MS, concentrated under reduced pressure after the reaction to give the residue. The residue was purified by medium pressure liquid chromatography (Alkali process) to give the compound 5 (15 mg, 55.87% yield). **$^1$H NMR** (400 MHz, DMSO-$d_6$) δ 10.01 (s, 1H), 8.77 (s, 1H), 7.76 (t, *J* = 8.0 Hz, 1H), 7.50 (s, 2H), 7.30 (d, *J* = 7.9 Hz, 1H), 6.59-6.55 (m, 3H), 5.68-5.58 (m, 1H), 5.00 (d, *J* = 10.1 Hz, 1H), 4.89 (d, *J* = 17.0 Hz, 1H), 4.74 (s, 2H), 4.26 (s, 1H), 3.40(s, 1H), 3.37 (s, 6H), 3.30 (s, 1H), 3.13 (d, *J* = 9.0 Hz, 1H), 2.78-2.76 (m, 1H), 2.47(s, 1H), 2.24 (s, 3H), 1.85 (d, *J* = 9.3 Hz, 1H), 1.75 (d, *J* = 9.1 Hz, 1H). **$^1$H NMR** (400 MHz, DMSO-$d_6$, $D_2O$) δ 8.79 (s, 1H), 7.80 (t, *J* = 7.9 Hz, 1H), 7.50-7.48 (m, 2H), 7.30 (d, *J* = 7.8 Hz, 1H), 6.64 (d, *J* = 8.0 Hz, 1H), 6.58 (d, *J* = 8.7 Hz, 2H), 5.69 - 5.59 (m, 1H), 5.05 - 5.03 (m, 1H), 4.92 - 4.87 (m, 1H), 4.74 (s, 2H), 4.27 (s, 1H), 3.44 (s, 1H), 3.36 (s, 6H), 3.35 - 3.32 (m, 1H), 3.16 (d, *J* = 9.4 Hz, 1H), 2.79 (d, *J* = 9.7 Hz, 1H), 2.48 (d, *J* = 9.9 Hz, 1H), 2.24 (s, 3H), 1.89 (d, *J* = 9.5 Hz, 1H), 1.78 (d, *J* = 9.4 Hz, 1H). LCMS (E+) m/z: 546.3 [M+H]$^+$, HPLC method B: $R_T$= 5.56 min, purity: 92%.

**Example 6: Synthesis of Compound 6:**

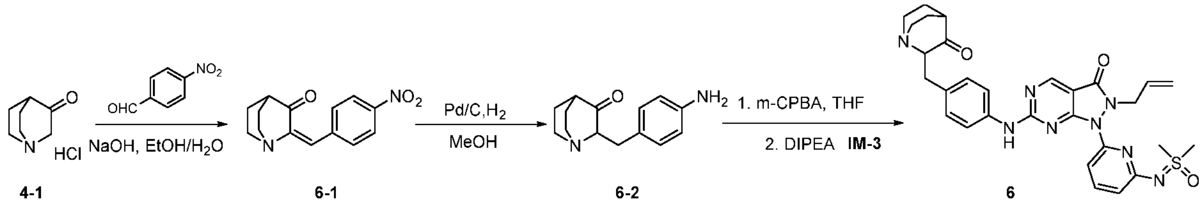


4-1 6-1 6-2 6

**Step 1: Synthesis of Compound 6-1:**

The substrate 4-**1** (1g, 6.2 mmol) and ethanol/water (10:1, 20mL) were added to a dry single-necked flask, stirred to dissolve then added with p-Nitrobenzaldehyde (1 g, 6.6 mmol) and NaOH (620 mg, 15.5 mmol) to react at room temperature and monitored by LC-MS. After the reaction was completed, the solid was precipitated and filtered out,

washed three times with a small amount of ethanol, and dried naturally to give the compound **6-1** (1g, 63% yield),LCMS (E+) m/z: 259.2 [M+H]+.

**Step 2: Synthesis of Compound 6-2:**

The substrate **6-1** (103mg, 0.4mmol) and methanol (5mL) were added to a dry single-necked flask, stirred to dissolve then added with **Pd/C**(15mg), $H_2$ displaced five times then stirred at room temperature to react and monitored by LC-MS. After the reaction was completed, Pd/C was removed by diatomaceous earth and the organic phase was concentrated under reduced pressure to give the compound **6-2** (80mg, crude), LCMS (ESI+) m/z: 231.2[M+H]+.

**Step 3: Synthesis of Compound 6:**

The substrate **IM-3** (30mg, 77μmol) and tetrahydrofuran (2mL) were added to a dry single-necked flask, stirred to dissolve then added with m-CPBA (20mg, 116μmol) to react for 1 hour at room temperature and monitored by LC-MS, added with DIPEA (50 mg, 385 μmol) after the reaction and stirred for 10 min, then added with **6-2** (20 mg, 86.84 μmol) and monitored by LC-MS, concentrated under reduced pressure after the reaction to give the residue. The residue was purified by medium pressure liquid chromatography (Alkali process) to give the compound 6 (5 mg, 17.74% yield).**$^1$H NMR** (400 MHz, DMSO-$d_6$) δ 10.18 (s, 1H), 8.85 (s, 1H), 7.78 (t, *J* = 7.9 Hz, 1H), 7.61 (d, *J* = 8.1 Hz, 2H), 7.30 (d, *J* = 7.9 Hz, 1H), 7.20 (d, *J* = 8.4 Hz, 2H), 6.61 (d, *J* = 7.9 Hz, 1H), 5.69 - 5.60 (m, 1H), 5.02-4.99 (m, 1H), 4.90 (dd, *J* = 17.1, 1.7 Hz, 1H), 4.74 (d, *J* = 6.0 Hz, 2H), 3.37 (s, 6H), 2.99 - 2.86 (m, 4H), 2.76 - 2.69 (m, 3H), 2.33 - 2.29 (m, 2H), 1.92 - 1.88 (m, 3H). **$^1$H NMR** (400 MHz, DMSO-$d_6$, $D_2O$) δ 8.86 (s, 1H), 7.83 (t, *J* = 7.9 Hz, 1H), 7.60 (d, *J* = 8.0 Hz, 2H), 7.31 (d, *J* = 7.8 Hz, 1H), 7.22 (d, *J* = 8.2 Hz, 2H), 6.68 (d, *J* = 8.0 Hz, 1H), 5.70 - 5.61(m, 1H), 5.07 - 5.04 (m, 1H), 4.90 (d, *J* = 17.2 Hz, 1H), 4.75 (d, *J* = 5.9 Hz, 2H), 3.36 (s, 6H), 3.09 - 2.85 (m, 4H), 2.79 - 2.72 (m, 3H), 2.38 - 2.32 (m, 2H), 1.91-1.89 (m, 3H). LCMS (E+) m/z: 573.6 [M+H]+, HPLC method B: $R_T$= 5.05 min, purity: 87.7%.

**Example 7: Synthesis of Compound** 7:

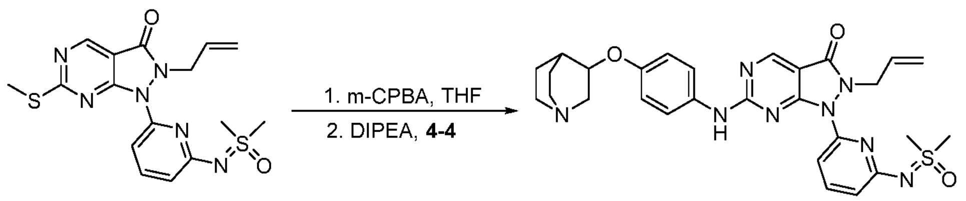


IM-3 7

**Step 1: Synthesis of Compound 7:**

The substrate **IM-3** (30mg, 77μmol) and tetrahydrofuran (2mL) were added to a dry single-necked flask, stirred to dissolve then added with m-CPBA (20mg, 116μmol) to react for 1 hour at room temperature and monitored by LC-MS, added with DIPEA (50 mg, 385 μmol) after the reaction and stirred for 10 min, then added with **4-4** (20 mg, 91.62 μmol) and monitored by LC-MS, concentrated under reduced pressure after the reaction to remove the solvents. The residue was purified by medium pressure liquid chromatography (Alkali process) to give the compound 7 (5 mg, 19.08% yield).**$^1$H** NMR (400 MHz, DMSO-$d_6$) δ 10.16 (s, 1H), δ 8.82 (s, 1H), 7.79 (t, *J* = 8.0 Hz, 1H), 7.61 (s, 2H), 7.28 (d, *J* = 7.8 Hz, 1H), 6.89 (d, *J* = 9.0 Hz, 2H), 6.60 (d, *J* = 8.1 Hz, 1H), 5.69 - 5.59 (m, 1H), 5.02- 4.99 (m, 1H), 4.89 (d, *J* = 17.2 Hz, 1H), 4.74 (s, 2H), 4.41 (d, *J* = 7.8 Hz, 1H), 3.18 (s, 3H), 3.16 (s, 3H), 2.79-2.70(m, 6H), 2.03-2.02 (m, 1H), 1.91-1.80 (m, 1H), 1.67-1.63 (m, 1H), 1.57-1.54(m, 1H), 1.38-1.33 (m, 1H). **$^1$H NMR** (400 MHz, DMSO-$d_6$, $D_2O$) δ 8.83 (s, 1H), 7.82 (t, *J* = 8.0 Hz, 1H), 7.60 (s, 2H), 7.29 (d, *J* = 7.8 Hz, 1H), 6.92 (d, *J* = 8.8 Hz, 2H), 6.66 (d, *J* = 8.0 Hz, 1H), 5.68-5.62 (m, 1H), 5.05 (d, *J* = 10.4 Hz, 1H), 4.89 (d, *J* = 16.9 Hz, 1H), 4.75 (s, 2H), 4.48 (s, 1H), 3.36 (s, 6H), 2.78-2.72 (m, 6H), 2.05 (s, 1H), 1.84 (s, 1H), 1.70-1.59 (m, 2H), 1.40-1.33 (m, 1H). LCMS (E+) m/z: 561.5 [M+H]+, HPLC method B: $R_T$= 5.10 min, purity: 90.2%.

**Example 8: Synthesis of Compound 8:**

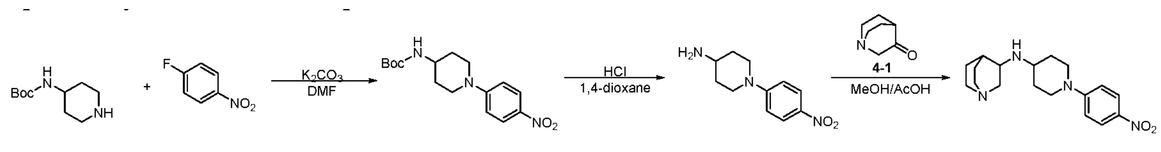


8-1 4-2 8-2 8-3 8-4

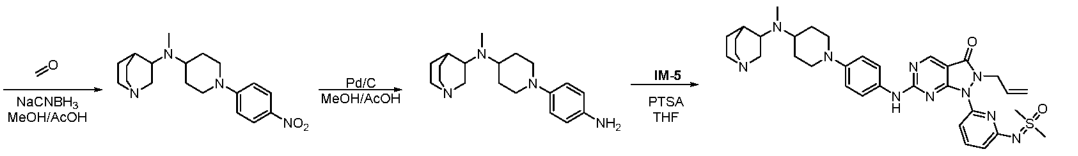


8-5 8-6 8

**Step 1: Synthesis of Compound 8-2:**

The substrate **4-2** (1g, 7.09 mmol), compound **8-1** (1.70g, 8.50mmol) and $K_2CO_3$ (2.45 g, 17.72 mmol) were added to a dry single-necked flask, dissolved with DMF (10 mL) and heated up to 90 °C to react for 1 hour and monitored by LC-MS, extracted three times with water and ethyl acetate after the reaction. The organic phase was combined, washed with saturated saline and dried with anhydrous $Na_2SO_4$, then concentrated under reduced pressure with organic solvents to give the crude product which can be used without purification for the following reaction directly to give the compound **8-2** (2.1g, crude), a kind of yellow oil. LCMS (E+) m/z: 322.4 $[M+H]^+$.

**Step 2: Synthesis of Compound 8-3:**

The substrate **8-2** (2.1g, 6.53 mmol) was added to a dry single-necked flask, dissolved with HCl/1,4-Dioxane (10 mL) to react at room temperature for 1 hour and monitored by LC-MS, concentrated under reduced pressure after the reaction, extracted three times with saturated sodium carbonate solution and ethyl acetate. The organic phase was combined, washed with saturated saline and dried with anhydrous $Na_2SO_4$, then concentrated under reduced pressure with organic solvents to give crude product which can be used without purification for the following reaction directly to give the compound **8-3** (1.3 g, crude), a kind of yellow oil. LCMS (E+) m/z: 222.4 $[M+H]^+$.

**Step 3: Synthesis of Compound 8-4:**

The substrate **8-3** (700 mg, 3.16 mmol) and compound **4-1** (396.00 mg, 3.16 mmol) were added to a dry single-necked flask, dissolved with MeOH/AcOH (10 mL) to react at room temperature for 1 hour and monitored by LC-MS, concentrated under reduced pressure after the reaction and extracted three times with water and ethyl acetate. The organic phase was combined, washed with saturated saline and dried with anhydrous $Na_2SO_4$. The organic solvents were concentrated under reduced pressure. The residual impurities were removed with MPLC to give the compound **8-4** (300 mg, 907.93μmol, 28.70% yield), a kind of yellow oil. LCMS (E+) m/z: 331.4 $[M+H]^+$.

**Step 4: Synthesis of Compound 8-5:**

The substrate **8-4** (300 mg, 907.93 μmol) and formaldehyde (136.31 mg, 4.54 mmol) were added to a dry single-necked flask, dissolved with MeOH/AcOH (8mL), heated up to 50 °C to react for 3 hours, then added with NaCNBH3 (142.64 mg, 2.27 mmol) in the system and heated up to 70°C to react overnight and monitored by LC-MS, concentrated under reduced pressure after the reaction and extracted three times with water and ethyl acetate. The organic phase was combined, washed with saturated saline and dried with anhydrous $Na_2SO_4$, then concentrated under reduced pressure with organic solvents. The residual impurities were removed with MPLC to give the compound **8-5** (283 mg, 739.44 μmol, 81.44% yield), a kind of yellow solid. LCMS (E+) m/z: 345.5 $[M+H]^+$.

**Step 5: Synthesis of Compound 8-6:**

The substrate **8-5** (1.5 g, 4.74 mmol) was added to a dry single-necked flask, dissolved with MeOH/AcOH (10mL), then added with Pd/C (172.73 mg, 1.42 mmol), $H_2$ displaced three times then reacted for 3 hours at room temperature and monitored by LC-MS, filtered with diatomaceous earth after the reaction. The filterate was concentrated under reduced pressure. The crude product can be used without purification for the following reaction directly to give the compound **8-6** (1.5 g, crude), a kind of black oil. LCMS (E+) m/z: 315.5 $[M+H]^+$.

**Step 6: Synthesis of Compound 8:**

The substrate **8-6** (50 mg, 159.00 μmol) and compound **IM-5** (64.63 mg, 159.00 μmol) were added to a dry single-necked flask, dissolved with THF (3mL) then added with PTSA (54.76 mg, 318.00 μmol), heated up to 110°C to react with stirring for 5 hours and monitored by LC-MS. After the reaction was completed, the organic solvents were concentrated under reduced pressure and extracted three times with water and ethyl acetate. The organic phase was combined, washed with saturated saline and dried with anhydrous $Na_2SO_4$, then concentrated under reduced pressure. The residual impurities were removed with MPLC to give the compound **8** (23 mg, 35.02 μmol, 22.02% yield), a kind of yellow solid. **$^1$H NMR** (600 MHz, DMSO-*d*$_6$) δ 10.11 (s, 1H), 8.80 (s, 1H), 7.79 (s, 1H), 7.56 (s, 2H), 7.30 (d, *J* = 7.9 Hz, 1H), 6.91 (d, *J* = 8.6 Hz, 2H), 6.59 (d, *J* = 8.2 Hz, 1H), 5.68 - 5.59 (m, 1H), 5.00 (d, *J* = 10.1 Hz, 1H), 4.89 (d, *J* = 17.0 Hz, 1H), 4.74 (s, 2H), 3.74 - 3.66 (m, 2H), 3.37 (s, 6H), 2.94 - 2.91 (m, 1H), 2.79 - 2.57 (m, 9H), 2.06 (s, 3H), 1.97 - 1.93 (m, 1H), 1.75 - 1.53 (m, 6H), 1.42 (d, *J* = 11.1 Hz, 1H), 1.24 (d, *J* = 11.7 Hz, 1H). LCMS(ESI+)m/z: 657.4 $[M+H]^+$, HPLC method B:$R_T$= 5.54min,purity: 98.9%.

**Example 9: Synthesis of Compound 9:**

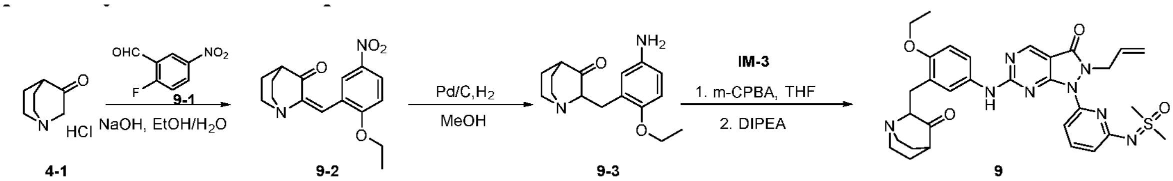


4-1 9-2 9-3 9

**Step 1: Synthesis of Compound 9-2:**

The substrate **4-1** (161 mg, 1 mmol) was added to a dry single-necked flask, dissolved with ethanol/water (10:1, 10 mL) then added with **9-1** (186 mg, 1.1 mmol) and NaOH (100 mg, 2.5mmol) to react at room temperature and monitored by LC-MS. After the reaction was completed, the solid was precipitated and filtered out, washed three times with a small amount of ethanol, and dried to give the compound **9-2** (150 mg, 50% yield), LCMS (E+) m/z: 303.1 $[M+H]^+$.

**Step 2: Synthesis of Compound 9-3:**

The substrate **9-2** (81 mg, 0.27 mmol) and methanol (5mL) were added to a dry single-necked flask, stirred to dissolve then added with Pd/C (15mg), $H_2$ displaced five times then stirred to react at room temperature and monitored by LC-MS. After the reaction was completed, Pd/C was removed by diatomaceous earth, the organic solvents were concentrated under reduced pressure to give the compound **9-3** (42 mg, crude), LCMS ($ESI^+$) m/z: 275.2 $[M+H]^+$.

**Step 3: Synthesis of Compound 9:**

The substrate **IM-3** (40mg, 110.71μmol) and THF (2mL) were added to a dry single-necked flask, stirred to dissolve then added with m-CPBA (20mg, 116μmol) to react for 1 hour at room temperature and monitored by LC-MS, added with DIPEA (50 mg, 385 μmol) after the reaction and stirred for 10 min, then added with the compound **9-3** (36.45mg, 132.85μmol) and monitored by LC-MS, concentrated under reduced pressure after the reaction to give the residue which was purified by medium pressure liquid chromatography (Alkali process) to give the compound 9 (35 mg, 51.26% yield).**$^1$H NMR** (400 MHz, DMSO-*d*$_6$) δ 10.12 (s, 1H), 8.82 (s, 1H), 7.77-7.75 (m, 1H), 7.59 (s, 1H), 7.45 (d, *J* = 8.9 Hz, 1H), 7.29 (d, *J* = 7.4 Hz, 1H), 6.89 (d, *J* = 8.8 Hz, 1H), 6.58 (d, *J* = 8.0 Hz, 1H), 5.67-5.60 (m, 1H), 5.00 (d, *J* = 10.2 Hz, 1H), 4.88 (d, *J* = 17.1 Hz, 1H), 4.74 (s, 2H), 4.01 (q, *J* = 7.0 Hz, 2H), 3.37 (s, 6H), 3.06-3.03 (m, 2H), 2.85-2.84 (m, 1H), 2.71-2.64 (m, 4H), 2.28 (s, 1H), 1.95 - 1.87 (m, 4H), 1.32 (t, *J* = 6.9 Hz, 3H). **$^1$H NMR** (400 MHz, DMSO-*d*$_6$, $D_2O$) δ 8.82 (s, 1H), 7.81-7.77 (m, 1H), 7.58 (s, 1H), 7.45 (d, *J* = 8.7 Hz, 1H), 7.29 (d, *J* = 7.9 Hz, 1H), 6.90 (d, *J* = 8.9 Hz, 1H), 6.61 (d, *J* = 8.1 Hz, 1H), 5.67-5.60 (m, 1H), 5.02(d, *J* = 10.2 Hz, 1H ), 4.88(d, *J* =17.1 Hz, 1H ) , 4.73(s, 1H ), 4.01 (q, *J* = 7.0 Hz, 2H), 3.36 (s, 6H), 3.03 (d, *J* = 14.9 Hz, 2H), 2.86 (s, 1H), 2.71-2.65 (m, 4H), 2.29 (s, 1H), 1.96-1.87 (m, 4H), 1.32 (t, *J* = 6.9 Hz, 3H). LCMS (E+) m/z: 617.2 $[M+H]^+$, HPLC method B: $R_T$= 6.66 min, purity: 95.4%.

**Example 10: Synthesis of Compound 10:**

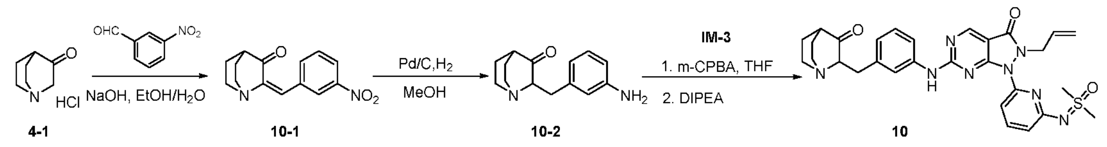

4-1 10-1 10-2 10

**Step 1: Synthesis of Compound 10-1:**

The substrate **4-1** (1g, 6.2 mmol) and ethanol/water (10:1, 20mL) were added to a dry single-necked flask, stirred to dissolve then added with p-Nitrobenzaldehyde (1g, 6.6 mmol) and NaOH (620 mg, 15.5 mmol) to react at room temperature and monitored by LC-MS. After the reaction was completed, the solid was precipitated and filtered out, washed three times with a small amount of ethanol, and dried naturally to give the compound **10-1** (1 g, 63% yield), LCMS (E+) m/z: 259.2 $[M+H]^+$.

**Step 2: Synthesis of Compound 10-2:**

The substrate **10-1** (103mg, 0.4mmol) and methanol (5mL) were added to a dry single-necked flask, stirred to dissolve then added with **Pd/C**(15mg), $H_2$ displaced five times then stirred at room temperature to react and monitored by LC-MS. After the reaction was completed, Pd/C was removed by diatomaceous earth and the organic phase was concentrated under reduced pressure to give the compound **10-2** (65 mg, crude), LCMS ($ESI^+$) m/z: 231.2$[M+H]^+$.

**Step 3: Synthesis of Compound 10:**

The substrate **IM-3** (40mg, 110.71μmol) and THF (2mL) were added to a dry single-necked flask, stirred to dissolve then added with m-CPBA (29mg, 116μmol) to react for 1 hour at room temperature and monitored by LC-MS, added with DIPEA (85.85mg, 664.24μmol) after the reaction and stirred for 10 min, then added with **10-2** (30.6mg, 132.85 μmol) and monitored by LC-MS, concentrated under reduced pressure after the reaction. The residue was purified by medium pressure liquid chromatography (Alkali process) to give the compound 10 (15 mg, 22.22% yield).**$^1$H NMR** (400 MHz, DMSO-$d_6$) δ 10.22 (s, 1H), 8.87 (s, 1H), 7.83 (t, *J* = 7.9 Hz, 1H), 7.76 (s, 1H), 7.47 (d, *J* = 8.2 Hz, 1H), 7.31 (d, *J* = 7.8 Hz, 1H), 7.21 (t, *J* = 7.8 Hz, 1H), 6.95 (d, *J* = 7.6 Hz, 1H), 6.61 (d, *J* = 8.1 Hz, 1H), 5.69-5.60 (m, 1H), 5.01 (d, *J* = 10.1 Hz, 1H), 4.89 (d, *J* = 17.0 Hz, 1H), 4.74 (d, *J* = 6.0 Hz, 2H), 3.41 (s, 6H) 3.03 - 2.98 (m, 2H), 2.89-2.86 (m, 1H), 2.78 - 2.71 (m, 4H), 2.32-2.31 (m, 1H), 1.97-1.89 (m, 4H). **$^1$H NMR** (400 MHz, DMSO-$d_6$, $D_2O$) δ 8.87 (s, 1H), 7.84 (t, *J* = 7.9 Hz, 1H), 7.74 (s, 1H), 7.47 (d, *J* = 8.2 Hz, 1H), 7.31 (d, *J* = 7.7 Hz, 1H), 7.23 (t, *J* = 7.8 Hz, 1H), 6.96 (d, *J* = 7.7 Hz, 1H), 6.64 (d, *J* = 8.0 Hz, 1H), 5.70-5.60 (m, 1H), 5.03 (d, *J* = 10.2 Hz, 1H), 4.89 (d, *J* = 17.0 Hz, 1H), 4.74 (d, *J* = 6.0 Hz, 2H), 3.36 (d, *J* = 2.1 Hz, 6H), 3.03 - 2.98 (m, 2H), 2.93-2.86 (m, 1H), 2.78 - 2.69 (m, 4H), 2.33-2.32 (m, 1H), 1.99-1.84 (m, 4H). LCMS (E+) m/z: 573.1 $[M+H]^+$, HPLC method B: $R_T$= 6.24 min, purity: 93.9%.

**Example 11: Synthesis of Compound 11:**

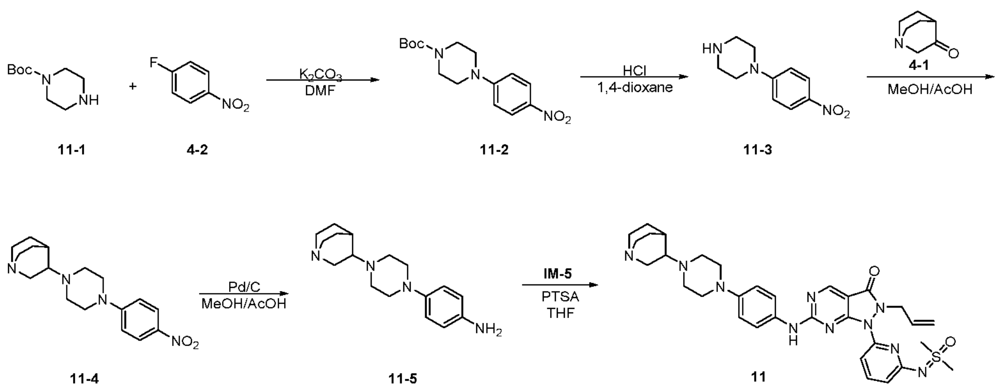

11-1 4-2 11-2 11-3 11-4 11-5 11

**Step 1: Synthesis of Compound 11-2:**

The substrate **4-2** (2g, 14.17mmol), compound **11-1** (3.17g, 17.01mmol) and $K_2CO_3$ (4.89g, 35.44 mmol) were

added to a dry single-necked flask, dissolved with DMF (10 mL) and heated up to 90°C to react for 1 hour and monitored by LC-MS, extracted three times with water and ethyl acetate after the reaction, and the organic phase was combined, washed with saturated saline and dried with anhydrous $Na_2SO_4$, then the organic solvents were concentrated under reduced pressure to give crude product which can be used without purification for the following reaction directly to give the compound **11-2** (4g, crude), a kind of yellow solid. LCMS (E+) m/z: 308.3 $[M+H]^+$.

**Step 2: Synthesis of Compound 11-3:**

The substrate **11-2** (4g, 13.01mmol) was added to a dry single-necked flask, dissolved with HCl/1,4-Dioxane (10mL) to react at room temperature for 1 hour and monitored by LC-MS. The organic phase was concentrated under reduced pressure after the reaction, extracted three times with saturated sodium carbonate solution and ethyl acetate, then combined, washed with saturated saline and dried with anhydrous $Na_2SO_4$. The organic solvents were concentrated under reduced pressure to give crude product which can be used without purification for the following reaction directly to give the compound **11-3** (2.5g, crude), a kind of yellow solid, LCMS (E+) m/z: 208.2 $[M+H]^+$.

**Step 3: Synthesis of Compound 11-4:**

The substrate **11-3** (1g, 4.83mmol) and free base of compound **4-1** (724.81 mg, 5.79mmol) were added to a dry single-necked flask, dissolved with MeOH/AcOH (10mL) to react at room temperature for 1 hour and monitored by LC-MS. The organic phase was concentrated under reduced pressure after the reaction, extracted three times with saturated sodium carbonate solution and ethyl acetate, then combined, washed with saturated saline and dried with anhydrous $Na_2SO_4$. The organic solvents were concentrated under reduced pressure to remove the residual impurities by MPLC to give the compound **11-4** (1.5 g, 4.27 mmol, 88.42% yield), a kind of yellow solid. LCMS (E+) m/z: 317.4 $[M+H]^+$.

**Step 4: Synthesis of Compound 11-5:**

The substrate **11-4** (1.5g, 4.74mmol) and Pd/C (172.73mg, 1.42mmol) were added to a dry single-necked flask, dissolved with MeOH/AcOH (10mL), $H_2$ displaced five times to react at room temperature for 3 hours and monitored by LC-MS, filtered with diatomaceous earth after the reaction. The filterate was concentrated under reduced pressure. The crude product can be used without purification for the following reaction directly to give the compound **11-5** (1.5 g, crude), a kind of black oil. LCMS (E+) m/z: 287.4 $[M+H]^+$.

**Step 5: Synthesis of Compound 11:**

The substrate **11-5** (35mg, 122.20μmol) and compound **IM-5** (49.67mg, 122.20μmol) were added to a dry single-necked flask and dissolved with THF (3mL), added with PTSA (52.61 mg, 305.50 μmol) and heated up to 110°C to react with stirring for 5 hours and monitored by LC-MS. The organic phase was concentrated under reduced pressure after the reaction, extracted three times with water and ethyl acetate, then combined, washed with saturated saline and dried with anhydrous $Na_2SO_4$, then concentrated under reduced pressure to remove the residual impurities by MPLC to give the compound **11** (21 mg, 32.56 μmol, 26.65% yield), a kind of green solid. **$^1$H NMR** (600 MHz, DMSO-$d_6$) δ 10.13 (s, 1H), 8.80 (s, 1H), 7.80 (s, 1H), 7.59 (s, 2H), 7.30 (d, *J* = 7.7 Hz, 1H), 6.91 (d, *J* = 8.5 Hz, 2H), 6.59 (d, *J* = 7.9 Hz, 1H), 5.64 (ddt, *J* = 18.3, 13.6, 6.4 Hz, 1H), 5.00 (d, *J* = 10.1 Hz, 1H), 4.90 (d, *J* = 17.0 Hz, 1H), 4.75 (s, 2H), 3.37 (s, 6H), 3.13 - 3.09 (m, 6H), 2.98 - 2.91 (m, 2H), 2.80 - 2.63 (m, 4H), 2.63 - 2.55 (m, 2H), 2.03 - 2.00 (m, 2H), 1.77 - 1.60 (m, 2H), 1.43 - 1.39 (m, 1H), 1.29 - 1.25 (m, 1H). LCMS (ESI$^+$) m/z: 629.5 $[M+H]^+$, HPLC method B: $R_T$= 5.94 min, purity: 97.5%.

**Example 12: Synthesis of Compound 12:**

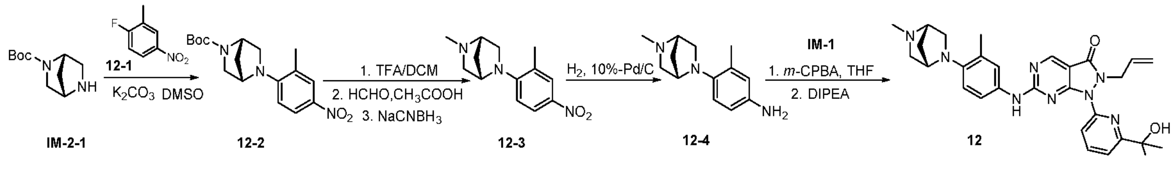


**Step 1: Synthesis of Compound 12-2:**

The substrate IM-2-1 (416 mg, 2.1 mmol) and DMSO (8mL) were added to a dry single-necked flask, stirred to

dissolve then added with $K_2CO_3$ (387 mg, 2 mmol) and compound **12-1** (310 mg, 2 mmol), heated up to 90°C to react and monitored by LC-MS, cooled to room temperature after the reaction, added with water and stirred until solid precipitated. The solids were filtered out and washed three times with water and a small amout of methanol respectively, then dried to give the product **12-2** (200 mg, 30% yield), LCMS (ESI+) m/z: 334.2 [M+H]+.

**Step 2: Synthesis of Compound 12-3:**

The substrate **12-2** (200 mg, 0.6 mmol) and DCM (5mL) were added to a dry single-necked flask, stirred to dissolve, added slowly with TFA (5mL) and monitered by LC-MS, concentrated under reduced pressure after the reaction. The residue was added with methanol (5mL), stirred to dissolve, added with polyformaldehyde (180mg) and acetic acid (500 μL) , stirred for 30 min at room temperature, then added with sodium cyanoborohydride (75mg, 1.2 mmol), heated up 50°C to react and monitered by LC-MS, cooled to room temperature after the reaction and filtered to remove the solids. The organic phase was concentrated under reduced pressure and purified by medium pressure liquid chromatography (Alkali process) to give the compound **12-3** (120 mg, 81% yield), LCMS (ESI+) m/z: 248.2[M+H]+.

**Step 3: Synthesis of Compound 12-4:**

The substrate **12-3** (120mg, 0.48mmol) and methanol (5mL) were added to a dry single-necked flask, stirred to dissolve then added with **Pd/C**(12mg), $H_2$ displaced five times, stirred at room temperature to react and monitored by LC-MS. After the reaction, Pd/C was removed by diatomaceous earth and the organic phase was concentrated under reduced pressure to give the compound **12-4** (90 mg, 86.4% yield). LCMS (ESI+) m/z: 218.2 [M+H]+.

**Step 4: Synthesis of Compound 12:**

The substrate **IM-1** (52 mg, 0.15 mmol) and THF (3mL) were added to a dry single-necked flask, stirred to dissolve then added with m-CPBA (121mg, 0.6mmol, purity 85%) to react at room temperature for 1 hour and monitered by LC-MS, added with DIPEA (98 mg, 758.28 μmol) after the reaction and stirred for 10 min, added with 12-4 (33 mg, 151.86 μmol) and monitered by LC-MS, then concentrated under reduced pressure after the reaction. The residue was purified by medium pressure liquid chromatography (Alkali process) to give the compound **12** (13 mg, 15.38% yield). **$^1$H NMR** (600 MHz, DMSO-$d_6$) δ 10.09 (s, 1H), 8.82 (s, 1H), 8.00-7.98 (m, 1H), 7.78 (d, *J* = 8.1 Hz, 1H), 7.61 (d, *J* = 7.7 Hz, 2H), 7.28 (dd, *J* = 8.8, 2.6 Hz, 1H), 6.74 (d, *J* = 8.7 Hz, 1H), 5.69-5.63 (m, 1H), 5.32 (s, 1H), 4.99 (dd, *J* = 10.3, 1.5 Hz, 1H), 4.82 (d, *J* = 17.1 Hz,1H), 4.68 (d, *J* = 6.0 Hz, 2H), 3.97 (s, 1H), 3.39 (s, 1H), 3.25 - 3.21 (m, 2H), 2.80-2.74 (m, 2H), 2.32 (s, 3H), 2.22 (s, 3H), 1.83 (d, *J* = 9.3 Hz, 1H), 1.72 (d, *J* = 9.2 Hz, 1H), 1.46 (s, 6H). **$^1$H NMR** (600 MHz, DMSO-$d_6$, $D_2O$) δ 8.82 (s, 1H), 8.03-8.01 (m, 1H), 7.76 (d, *J* = 8.1 Hz, 1H), 7.65 - 7.57 (m, 2H), 7.29 (dd, *J* = 8.7, 2.7 Hz, 1H), 6.76 (d, *J* = 8.7 Hz, 1H), 5.70-5.63 (m, 1H), 5.03-5.01 (m, 1H), 4.82 (d, *J* = 17.1 Hz, 1H), 4.69 (d, *J* = 5.8 Hz, 2H), 3.98 (s, 1H), 3.42 (s, 1H), 3.27 - 3.23 (m, 2H), 2.81 - 2.74 (m, 2H), 2.31 (s, 3H), 2.23 (s, 3H), 1.86 (d, *J* = 9.5 Hz, 1H), 1.75 (d, *J* = 9.4 Hz, 1H) , 1.47 (s, 6H). LCMS (E+) m/z: 527.3 [M+H]+, HPLC method B: $R_T$= 6.53 min, purity: 94.6%.

**Example 13: Synthesis of Compound 13:**

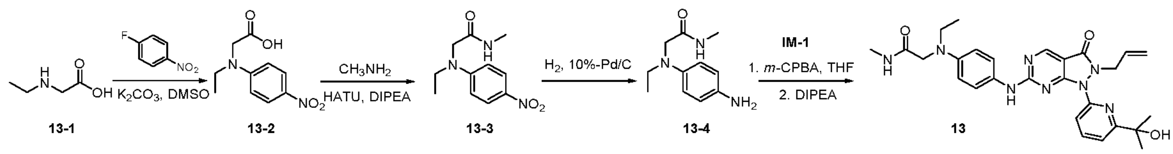


13-1 13-2 13-3 13-4 13

**Step 1: Synthesis of Compound 13-2:**

The substrate 13-1 (1.03g, 10mmol) and DMSO (15mL) were added to a dry single-necked flask, stirred to dissolve then added with $K_2CO_3$ (2g, 14mmol) and p-fluoronitrobenzene (1.43 g, 10 mmol), heated up to 100°C to react and monitored by LC-MS, cooled to room temperature after the reaction to filter out the solids. The filterate was concentrated under reduced pressure and purified by medium pressure liquid chromatography (Acid process) to give the compound **13-2** (2 g, 89% yield), LCMS (ESI+) m/z: 225.1 [M+H]+.

**Step 2: Synthesis of Compound 13-3:**

The substrate **13-2** (44 mg, 0.2 mmol) and THF (2mL) were added to a dry single-necked flask, stirred to dissolve then added with HATU(84 mg, 0.22 mmol) and DIPEA (28 mg, 0.22 mmol), . stirred for 30 min at room temperature and added with methylamine hydrochloride (67mg, 1mmol), heated up to 65°C to react and monitored by LC-MS, cooled to room temperature after the reaction and washed three times with ethyl acetate. The organic phase was concentrated under reduced pressure to give the crude product 13-3 (24 mg, crude), LCMS ($ESI^+$) m/z: 238.3 $[M+H]^+$.

**Step 3: Synthesis of Compound 13-4:**

The substrate **13-3** (24 mg, 0.1 mmol) and methanol (4mL) were added to a dry single-necked flask, stirred to dissolve then added with Pd/C(5mg), stirred at room temperature to react after five $H_2$ displacements and monitored by LC-MS. After the reaction, Pd/C was removed by diatomaceous earth and the organic phase was concentrated under reduced pressure to give the crude product **13-4** (18 mg, crude), LCMS ($ESI^+$) m/z: 208.2 $[M+H]^+$.

**Step 4: Synthesis of Compound 13:**

The substrate **IM-1** (31mg, 86μmol) and THF (2mL) were added to a dry single-necked flask, stirred to dissolve then added with m-CPBA (26 mg, 129 μmol) to react for 1 hour at room temperature and monitered by LC-MS, added with DIPEA (55 mg, 425 μmol) after the reaction and stirred for 10 min, added with **13-4** (18 mg, 85 μmol), heated up to 80°C to react and monitered by LC-MS, then concentrated under reduced pressure after the reaction. The residue was purified by medium pressure liquid chromatography (Alkali process) to give the compound **13** (7 mg, 16% yield).**$^1$H NMR** (600 MHz, DMSO-$d_6$) δ 10.05 (s, 1H), 8.79 (s, 1H), 7.97-4.95 (m, 1H), 7.75 (d, *J* = 4.4 Hz, 2H), 7.60 (d, *J* = 7.6 Hz, 1H), 7.49 (s, 2H), 6.59 (d, *J* = 8.0 Hz, 2H), 5.69-5.62 (m, 1H), 5.33 (s, 1H), 5.04 - 4.95 (m, 1H), 4.98 (d, *J* = 10.0 Hz, 1H), 4.82 (d, *J* = 17.0 Hz, 1H), 4.67 (d, *J* = 5.9 Hz, 2H), 3.78 (s, 2H), 3.41-3.40 (m, 2H), 2.60 (d, *J*= 4.7 Hz, 3H), 1.46 (s, 6H), 1.08 (t, *J* = 7.0 Hz, 3H). **$^1$H NMR** (600 MHz, DMSO-$d_6$, $D_2O$) δ 8.80 (s, 1H), 8.00-7.97 (m, 1H), 7.82 (d, *J* = 4.4 Hz, 1H), 7.72 (s, 1H), 7.61 (d, *J* = 7.6 Hz, 1H), 7.49 (s, 1H), 6.59 (d, *J* = 8.2 Hz, 2H), 5.69-5.63 (m, 1H), 5.02 (d, *J* = 10.2 Hz, 1H), 4.83 (d, *J* = 17.0 Hz, 1H), 4.68 (s, 2H), 3.81 (s, 2H), 3.42 (q, *J* = 7.0 Hz, 2H), 2.62 (d, *J* = 4.6 Hz, 3H), 1.47 (s, 6H), 1.08 (t, *J* = 7.0 Hz, 3H). LCMS (E+) m/z: 517.3 $[M+H]^+$, HPLC method B: $R_T$ = 6.25 min, purity: 90.5%.

**Example 14: Synthesis of Compound 14:**

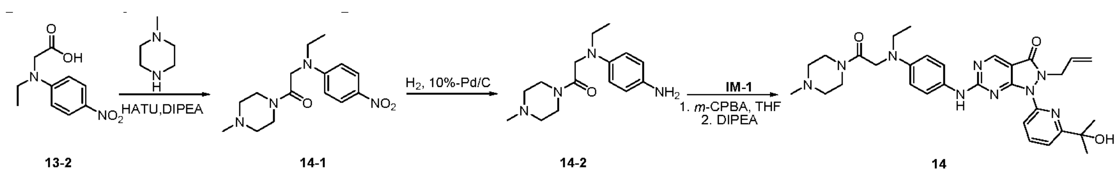


13-2 14-1 14-2 14

**Step 1: Synthesis of Compound 14-1:**

The substrate **13-2** (44 mg, 0.2 mmol) and THF (2mL) were added to a dry single-necked flask, stirred to dissolve then added with HATU(84 mg, 0.22 mmol) and DIPEA (28 mg, 0.22 mmol), . stirred for 30 min at room temperature and added with N-methylpiperazine (40mg, 0.4mmol), heated up to 65°C to react and monitored by LC-MS, cooled to room temperature after the reaction and washed three times with ethyl acetate. The organic phase was concentrated under reduced pressure to give the crude product **14-1** (50 mg, crude), LCMS ($ESI^+$) m/z: 307.2 $[M+H]^+$.

**Step 2: Synthesis of Compound 14-2:**

The substrate **14-1** (50mg, 0.16mmol) and methanol (4mL) were added to a dry single-necked flask, stirred to dissolve then added with Pd/C(5mg), stirred at room temperature to react after five $H_2$ displacements and monitored by LC-MS. After the reaction, Pd/C was removed by diatomaceous earth and the organic phase was concentrated under reduced pressure to give the product **14-2** (40 mg, crude), LCMS ($ESI^+$) m/z: 277.2 $[M+H]^+$.

**Step 3: Synthesis of Compound 14:**

The substrate **IM-1** (38 mg, 106 μmol) and THF (2mL) were added to a dry single-necked flask, stirred to dissolve then added with m-CPBA (40mg, 200μmol) to react for 1 hour at room temperature and monitered by LC-MS, added with DIPEA (65 mg, 500 μmol) after the reaction and stirred for 10 min, added with 14-2 (40 mg, crude), heated up to 80°C to react and monitered by LC-MS, then concentrated under reduced pressure after the reaction. The residue was purified by medium pressure liquid chromatography (Alkali process) to give the compound **14** (12.2 mg, 17.58% yield). **$^1$H NMR** (600 MHz, Methanol-*$d_4$*) δ 8.76 (s, 1H), 7.96-7.93 (m, 1H), 7.76 (s, 1H), 7.62 (d, *J* = 7.7 Hz, 1H), 7.43 (d, *J* = 8.4 Hz, 2H), 6.68 (d, *J* = 8.6 Hz, 2H), 5.73-5.67 (m, 1H), 5.03 (d, *J* = 10.2 Hz, 1H), 4.91 (d, *J* = 10.2 Hz, 1H), 4.80 (d, *J* = 5.8 Hz, 2H), 4.59 (s, 2H), 4.23 (s, 2H), 3.76 (s, 2H), 3.44 (q, *J* = 7.0 Hz, 2H), 2.90-2.85 (m, 4H), 2.62 (s, 3H), 1.57 (s, 6H), 1.18 (t, *J* = 7.0 Hz, 3H). LCMS (E+) m/z: 586.4 $[M+H]^+$, HPLC method B: $R_T$ = 6.23 min, purity: 91.9%.

**Example 15: Synthesis of Compound 15:**

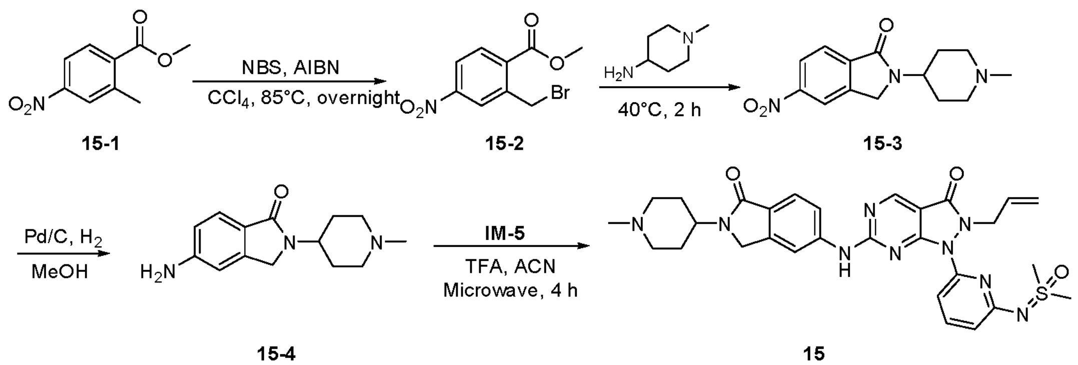


15-1 15-2 15-3 15-4 15

**Step 1: Synthesis of Compound 15-2:**

The substrate **15-1** (2.44g, 12.50mmol), NBS (3.33g, 18.70mmol) and AIBN (0.2g, 1.21mmol) were added to a single-necked flask, dissolved with $CCl_4$ (20 ml) and $N_2$ disreplaced three times, stirred overnight under $N_2$ protection and monitored by TLC as the flask was placed in an oil bath at 85°C, concentrated under reduced pressure to remove the solvents. The crude product can be used without purification for the following reaction directly to give the crude product **15-2** (3 g, 11.03 mmol, 88.1% yield).

**Step 2: Synthesis of Compound 15-3:**

The crude product **15-2** (3 g, 11.03 mmol) was added with 4-amino-1-methylpiperidine (1.8 g, 15.77 mmol) to dissolve, then heated up to 40°C to react with stirring for 2 h. The reaction was monitored by LC-MS until completion. The reaction was separated by column chromatography to give the compound **15-3** (1.3 g, 4.72 mmol, 42.86% yield), LCMS ($ESI^+$) m/z: 276.1 $[M+H]^+$.

**Step 3: Synthesis of Compound 15-4:**

The substrate **15-3** (0.6 g, 1.70 mmol) was added to a flask and dissolved with anhydrous methanol (10ml), then added with Pd/C (10% wt.) (60 mg), stirred overnight at room temperature after 3 times of $H_2$ displacements and monitored by LC-MS, then filtered with diatomaceous earth after the reaction and the filtrate was concentrated under reduced pressure and purified by column chromatography to give the compound **15-4** (474 mg, 1.92 mmol, 88.7% yield), LCMS ($ESI^+$) m/z: 246.2 $[M+H]^+$.

**Step 4: Synthesis of Compound 15:**

The substrate **IM-5** (39.3 mg, 0.097 mmol), The substrate **15-4** (47.43 mg, 0.19 mmol), anhydrous acetonitrile (4 ml) and TFA (55.18 mg, 0.48 mmol) were added to a microwave reaction tube for microwave reaction for 4 h at 90°C and monitored by LC-MS, quenched with saturated NaHCOs solution after the reaction, extracted three times with ethyl acetate, the organic phase was combined, washed with saturated saline and dried with anhydrous $Na_2SO_4$, then

concentrated under reduced pressure and purified by medium pressure liquid chromatography to give the compound **15** (10 mg, 0.017 mmol, 17.6% yield).**$^1$H NMR** (600 MHz, Chloroform-*$d_6$*) δ 8.89 (s, 1H), 8.03 (s, 1H), 7.79 (d, *J* = 8.3 Hz, 1H), 7.75 (s, 1H), 7.71 - 7.68 (m, 1H), 7.56 (d, *J* = 8.2 Hz, 1H), 7.31 (d, *J* = 7.7 Hz, 1H), 6.74 (d, *J* = 8.0 Hz, 1H), 5.69 - 5.63 (m, 1H), 5.05 - 4.96 (m, 2H), 4.92 (d, *J* = 6.3 Hz, 2H), 4.37 (s, 3H), 3.34 (s, 6H), 3.11 (s, 2H), 2.44 (s, 3H), 2.33 (s, 2H), 2.05 (s, 2H), 1.89 (d, *J* = 12.5 Hz, 2H). LCMS (ESI$^+$) m/z: 588.1 $[M+H]^+$, HPLC method B: $R_T$ = 5.34 min, purity: 96.5%.

**Example 16: Synthesis of Compound 16:**

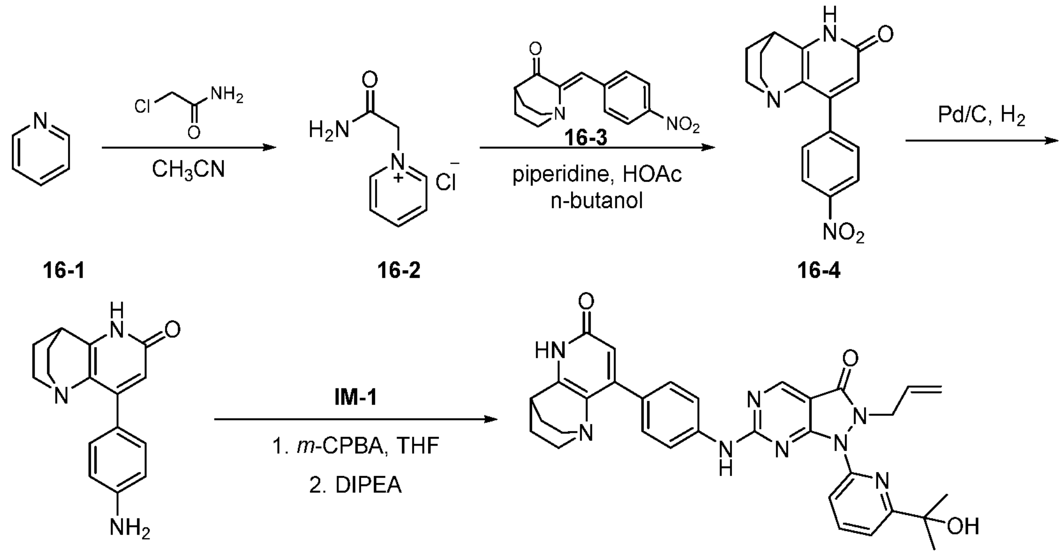


16-1 16-2 16-4

16-5 16

**Step 1: Synthesis of Compound 16-2:**

Pyridine (7.91g, 100 mmol) and acetonitrile (40 mL) were added to a dry single-necked flask, stirred well, added with 2-chloroacetamide (9.35 g, 100 mmol), and then heated to 90 °C to react overnight, cooled to room temperature after the reaction, filtered out the solid, washed three times with petroleum ether, and recrystallized with ethanol to give the product **16-2** (10.8 g, 62% yield)

**Step 2: Synthesis of Compound 16-4:**

The substrate **16-2** (3.6g, 21.15mmol) and n-butanol (50mL) were added to a dry single-necked flask, stirred well, added with **16-3** (1.82 g, 7.05 mmo), acetic acid (1.5 mL) and piperidine (2.5 mL), and heated up to 120 °C to react and monitored by LC-MS. After the reaction, the solid was filtered out, the filtrate was extracted with $CHCl_3$ (containing 5% MeOH) three times, the organic phase was concentrated under reduced pressure, and the resulting solid was recrystallized with methanol to give the product **16-4**(**1.92** g, 91% yield), LCMS (ESI$^+$) m/z: 298.2 $[M+H]^+$.

**Step 3: Synthesis of Compound 16-5:**

The substrate **16-4** (500mg, 1.68mmol) and methanol (15mL) were added to a dry single-necked flask, stirred well, added with Pd/C (50 mg), $H_2$ displaced five times, stirred to react at room temperature and monitored by LC-MS. After the reaction, Pd/C was removed with diatomaceous earth, washed with methanol, and the organic phase was concentrated under reduced pressure to give the product **16-5** (310 mg, 69% yield), LCMS (ESI$^+$) m/z: 268.1 $[M+H]^+$.

**Step 4: Synthesis of Compound 16:**

The substrate **IM-1** (70 mg, 200 μmol) and tetrahydrofuran (4 mL) were added into a dry single-necked flask, stirred to dissolve, then added with m-CPBA (60 mg, 300 μmol, 85% purity) to react for 1 hour at room temperature and monitored by LC-MS, added with DIPEA (129.24 mg, 1.00 mmol) after the reaction, stirred for 10 min, then added with **16-5** (53.47 mg, 200.00 μmol) to react at 80°C in microwave and monitored by LC-MS, concentrated under reduced pressure

after the reaction, and the residue was purified by medium pressure liquid chromatography (Alkali process) to give the compound **16** (6.3mg, 5.22% yield).**$^1$H NMR** (600 MHz, DMSO-$d_6$) δ 11.81 (s, 1H), 10.43 (s, 1H), 8.93 (s, 1H), 8.08-8.05 (m, 1H), 7.81-7.78 (m, 3H), 7.64 (d, *J* = 7.7 Hz, 1H), 7.58 (d, *J* = 8.2 Hz, 2H), 6.22 (s, 1H), 5.71-5.65 (m, 1H), 5.35 (s, 1H), 5.01 (d, *J* = 10.2 Hz, 1H), 4.84 (d, *J* = 17.2 Hz, 1H), 4.69 (d, *J* = 5.9 Hz, 2H), 3.47 (s, 2H), 3.09 (s, 1H), 3.00 (s, 2H), 1.84 (s, 2H), 1.56 (s, 2H), 1.47 (s, 6H). **$^1$H NMR** (600 MHz, DMSO-$d_6$, $D_2O$) δ 8.93 (s, 1H), 8.09-8.06 (m, 1H), 7.81-7.76 (m, 3H),7.65 (d, *J* = 7.6 Hz, 1H) 7.59 (d, *J* = 8.4 Hz, 2H), 6.27 (s, 1H), 5.71-5.64 (m, 1H), 5.04 (d, *J* = 10.2 Hz, 1H), 4.84 (d, *J* = 17.1 Hz, 1H), 4.70 (d, *J* = 5.8 Hz, 2H), 3.93 (s, 2H), 3.16 (s, 1H), 3.02 (s, 2H), 1.88 (s, 2H), 1.57 (s, 2H), 1.48 (s, 6H). LCMS (E+) m/z: 577.3 $[M+H]^+$, HPLC method B: $R_T$ = 5.96 min, purity: 95.5%.

**Example 17: Synthesis of Compound 17:**

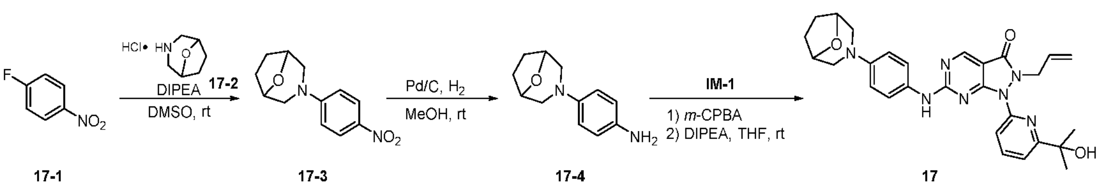


17-1 17-3 17-4 17

**Step 1: Synthesis of Compound 17-3:**

The compound **17-2** (130 mg, 0.88 mmol), DIPEA (0.58 g, 4.4 mmol) were sequentially dissolved in DMSO (1 ml) in a dry round-bottomed flask, then added with **17-1** (1.1 mmol, 148 mmol) and stirred for 2 hours at room temperatue and monitored by LC-MS, diluted with water after the reaction, extracted three times with ethyl acetate, washed with saturated brine, and the organic phase was dried with anhydrous $Na_2SO_4$ and concentrated under reduced pressure for the following reaction directly without purification to give the crude product **17-3** (150 mg, 0.64 mmol, 65.6% yield), LCMS (ESI+) m/z: 235.1 [M+H]+ .

**Step 2: Synthesis of Compound 17-4:**

The compound **17-3** (150 mg, 0.64 mmol), a catalytic amount of Pd/C (45 mg), and MeOH (3 mL) were added to a 50 mL round-bottomed flask, $H_2$ displaced 3 times, and stirred vigorously at room temperature for 12 hours under $H_2$ atmosphere, monitored by LC-MS, filtered with diatomaceous earth, and the filtrate was concentrated under reduced pressure and purified by medium pressure liquid chromatography to give the compound **17-4** (100 mg, 0.49 mmol, 76.1% yield), LCMS (ESI+) m/z: 205.1 [M+H]+.

**Step 3: Synthesis of Compound 17:**

A solution of THF (1 mL) containing dissolved **IM-1** (51 mg, 0.18 mmol) was added with m-CPBA (39 mg, 0.18 mmol) and stirred for 10 min at room temperature, monitered by LC-MS then by TLC, added with DIPEA (122 mg, 0.75 mmol) after the disappearance of the raw materials, and stirred for 20 min at room temperature. Subsequently, **17-4** (51 mg, 0.15 mmol) was added and stirred for 1 hour at room temperature, monitored by LC-MS, added with water after the reaction, extracted with ethyl acetate for three times, and the organic phase was dried with anhydrous $Na_2SO_4$ and then concentrated under reduced pressure, and the crude product was purified by acidic preparative high-performance liquid chromatography (AP-HPLC) to give the compound **17** (26 mg, 0.05 mmol, 35.5% yield).**$^1$H NMR** (600 MHz, DMSO-$d_6$) δ 10.13 (s, 1H), 8.81 (s, 1H), 8.04 (s, 1H), 7.74 (d, *J* = 7.6 Hz, 1H), 7.60 (d, *J* = 7.7 Hz, 1H), 7.57 (s, 1H), 6.82 (d, *J* = 8.5 Hz, 2H), 5.69 - 5.64 (m, 1H), 5.32 (s, 1H), 4.99 (d, *J*= 10.2 Hz, 1H), 4.82 (d, *J*= 17.1 Hz, 1H), 4.68 (s, 2H), 4.42 (s, 2H), 2.82 - 2.72 (m, 2H), 1.84 (s, 4H), 1.49 (s, 1H), 1.46 (s, 6H), 1.38 (s, 2H). LCMS (E+) m/z: 514.5$[M+H]^+$, HPLC method A: $R_T$ = 7.13 min, purity: >99.9%.

**Example 18: Synthesis of Compound 18:**

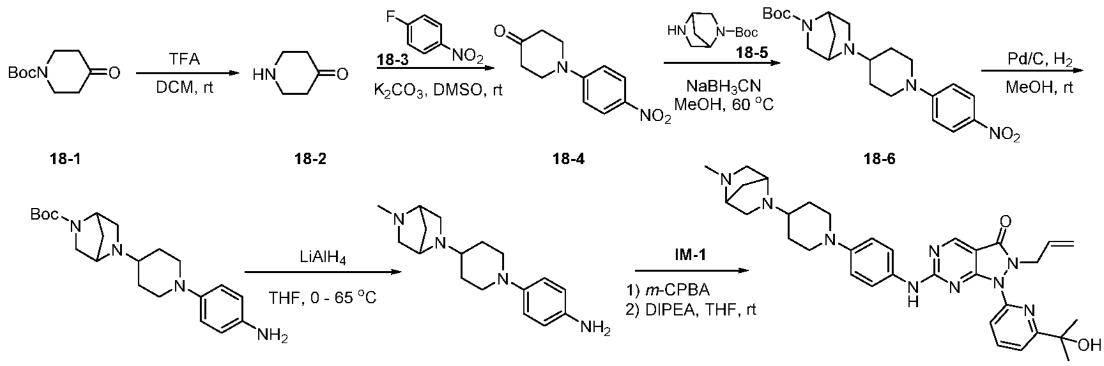


18-1 18-2 18-4 18-6

18-7 18-8 18

**Step 1: Synthesis of Compound 18-2:**

The compound 18-1 (4 g, 20 mmol) was added to a dry single-necked flask, dissolved in DCM (50 ml) , added with TFA (25mL) at room temperatue and stirred for 30 min, and monitored by LC-MS, the solvents were removed by vacuum after the reaction, then diluted with water, extracted three times with ethyl acetate, and the organic phase was dried with anhydrous $Na_2SO_4$ and concentrated under reduced pressure to give the crude product **18-2** (1.8 g, crude)for the following reaction directly without purification. LCMS (ESI+) m/z: 100.1 $[M+H]^+$.

**Step 2: Synthesis of Compound 18-4:**

The compound **18-2** (1g, 7mmol) was added to a dry single-necked flask, dissolved in DMSO (10 mL), added subsequently with **18-3** (1g, 10.5 mmol) and $K_2CO_3$(4.8 g, 35 mmol). The reaction mixture was stirred overnight at room temperature and monitored by LC-MS, diluted with 100 mL water after the reaction, extracted three times with ethyl acetate, and the organic phase was dried with anhydrous $Na_2SO_4$ and concentrated under reduced pressure to give the crude product **18-4** (1.35 g, crude) for the following reaction directly without purification. LCMS (ESI+) m/z: 221.1 $[M+H]^+$.

**Step 3: Synthesis of Compound 18-6:**

The compound **18-4** (1.35 g, 6.2 mmol), **18-5** (1.47 g, 7.4 mmol) were dissolved in methanol (15ml) in a dry round-bottomed flask, added with acetic acid (1ml) to react for 30 min, then added with Sodium cyanoborohydride (584 mg, 9.3 mmol) and heated up to 60°C, stirred for 4 h, monitored by LC-MS. The solvents were removed under reduced pressure after the reaction, then added with water, extracted three times with ethyl acetate, and the organic phase was dried with anhydrous $Na_2SO_4$ and concentrated under reduced pressure to give the crude product **18-6**(1.75g, crude), LCMS (ESI+) m/z: 403.2 $[M+H]^+$ .

**Step 4: Synthesis of Compound 18-7:**

The compound **18-6** (1.75 g, 4.4 mmol), a catalytic amount of Pd/C (200 mg), and MeOH (20mL) were added to a 50 mL round-bottomed flask, and stirred for 12 hours at room temperature under $H_2$ atmosphere, monitored by LC-MS. The reaction solution was concentrated under reduced pressure after the reaction. The residue was purified by column chromatography to give the compound **18-7** (1.46 g, 3.9 mmol, 90.2% yield). LCMS (ESI+) m/z: 373.3 $[M+H]^+$ .

**Step 5: Synthesis of Compound 18-8:**

The compound **18-7** (1.75 g, 4.4 mmol) was dissolved in THF in a round-bottomed flask, added slowly with a tetrahydrofuran solution (10 ml, 20 mmol) of 2 M $LiAlH_4$ under $N_2$ protection at 0 °C, heated up to 65 °C and stirred for 6 h at this temperature, monitored by LC-MS, cooled to 0 °C After the reaction, subsequently added with water (0.76ml) and 10% aqueous solution of NaOH, quenched with water (2.3ml), stirred for 30 min at room temperature, dried with anhydrous $Na_2SO_4$, and filtered with diatomaceous earth. The filtrate was concentrated under vacuum then purified by column chromatography to give the compound **18-8** (736 mg, 2.5 mmol, 65.6% yield). LCMS (ESI+) m/z: 287.2 $[M+H]^+$.

**Step 6: Synthesis of Compound 18:**

Following the synthesis method of step 3 in **Example 17, 17-4** (51 mg, 0.15 mmol) in step 3 was replaced with

**18-8** (42.1 mg, 0.15 mmol) in the same synthesis method to give the compound **18** (20 mg, 33.6 μmol, 22.9% yield).**$^1$H NMR** (600 MHz, DMSO-$d_6$) δ 10.19 (s, 1H), 8.84 (s, 1H), 8.04 (s, 1H), 7.76 (d, *J* = 8.1 Hz, 1H), 7.62 (d, *J* = 7.7 Hz, 3H), 7.00 (d, *J* = 8.5 Hz, 3H), 5.70 - 5.63 (m, 1H), 5.00 (d, *J* = 10.2 Hz, 1H), 4.83 (d, *J* = 17.2 Hz, 1H), 4.68 (s, 3H), 4.45 (s, 1H), 3.82 (s, 6H), 3.37 (s, 2H), 2.92 (s, 3H), 2.69 (t, *J* = 12.4 Hz, 2H), 2.23 (d, *J* = 64.4 Hz, 2H), 2.07 (d, *J* = 11.8 Hz, 1H), 1.78 - 1.63 (m, 2H), 1.47 (s, 6H). LCMS (ESI+) m/z: 596.4 $[M+H]^+$.HPLC method A: $R_T$ = 6.35 min, purity: 96.9 %.

**Example 19: Synthesis of Compound 19:**

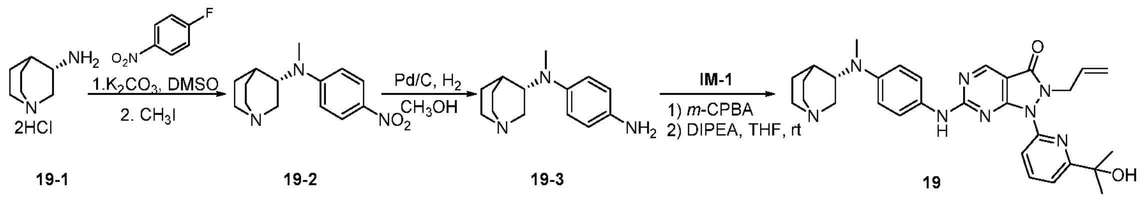


19-1 19-2 19-3 19

**Step 1: Synthesis of Compound 19-2:**

The compound 19-1 (200 mg, 0.1 mmol) and DMSO (5 mL) were added to a dry single-necked flask, then added with $K_2CO_3$ (470 mg, 3.4 mmol) and p-fluoronitrobenzene (141 mg, 1 mmol) under stirring, heated up to 110°C to react and monitored by LC-MS, cooled to room temperature after the reaction, added with iodomethane($CH_3I$) (141 mg, 1 mmol), then heated up to 90°C to react and monitored by LC-MS, cooled to room temperature after the reaction to filter out the solid. The filterate was concentrated under reduced pressure and purified by medium pressure liquid chromatography (Alkali process) to give the product **19-2** (50 mg, 19% yield), LCMS ($ESI^+$) m/z: 262.3 $[M+H]^+$.

**Step 2: Synthesis of Compound 19-3:**

The substrate **19-2** (50 mg, 0.19 mmol) and methanol (2mL) were added to a dry single-necked flask, stirred to dissolve, added with Pd/C (10 mg), $H_2$ displaced five times, stirred at room temperature to react and monitored by LC-MS. After the reaction, Pd/C was removed with diatomaceous earth, the organic phase was concentrated under reduced pressure to give the product **19-3** (40 mg, crude), LCMS ($ESI^+$) m/z: 232.2 $[M+H]^+$.

**Step 3: Synthesis of Compound 19:**

The substrate **IM-1** (52 mg, 0.15 mmol) and tetrahydrofuran (3 mL) were added into a dry single-necked flask, stirred to dissolve, then added with m-CPBA (121 mg, 0.6 mmol, 85% purity) to react for 1 hour at room temperature and monitored by LC-MS, added with DIPEA (95.17 mg, 736.42 μmol) after the reaction, stirred for 10 min, then added with **19-3** (40 mg, crude) and monitored by LC-MS, concentrated under reduced pressure after the reaction, and the residue was purified by medium pressure liquid chromatography (Alkali process) to give the compound **19** (35 mg, 40.96% yield). **$^1$H NMR** (600 MHz, DMSO-$d_6$) δ 10.11 (s, 1H), 8.80 (s, 1H), 7.99-7.96 (m, 1H), 7.75 (s, 1H), 7.62 (d, *J* = 7.7 Hz, 1H), 7.47 (s, 2H), 6.62 (d, *J* = 8.3 Hz, 2H), 6.19 (d, *J* = 23.4 Hz, 1H), 5.69-5.63 (m, 1H), 4.99 (d, *J* = 10.2 Hz, 1H), 4.83 (d, *J* = 17.1 Hz, 1H), 4.67 (s, 2H), 3.89 (s, 2H), 3.51 (s, 2H), 3.29 (s, 3H), 2.97 (s, 3H), 2.22 (s, 2H), 2.00 (d, *J* = 7.8 Hz, 2H), 1.76 (d, *J* = 14.1 Hz, 1H), 1.46 (s, 6H). **$^1$H NMR** (600 MHz, DMSO-$d_6$, $D_2O$) δ 8.81 (s, 1H), 8.01-7.98 (m, 1H), 7.74 (s, 1H), 7.62 (d, *J* = 7.8 Hz, 1H), 7.49 (s, 2H), 6.64 (d, *J* = 8.4 Hz, 2H), 5.69-5.63 (m, 1H), 5.02 (d, *J* = 10.2 Hz, 1H), 4.83 (d, *J* = 17.0 Hz, 1H), 4.68 (s, 2H), 3.90-3.88 (m, 2H), 3.43-3.42 (m, 4H), 3.14 - 3.12 (m, 1H), 2.97 (s, 3H), 2.23 - 2.20 (m, 2H), 2.03 (s, 2H), 1.82(s, 1H), 1.47 (s, 6H). LCMS (E+) m/z: 541.3 $[M+H]^+$, HPLC method B: $R_T$ = 5.65 min, purity: 93.2%.

**Example 20: Synthesis of Compound 20:**

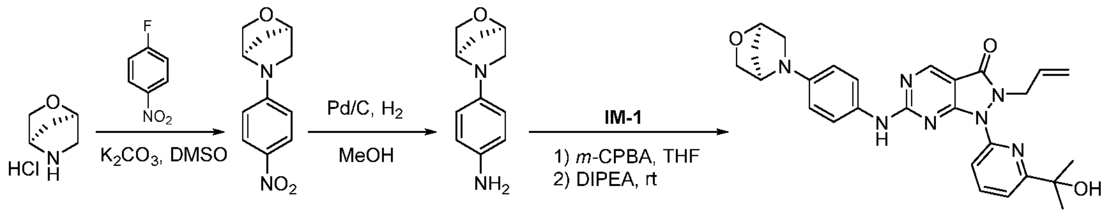


20-1 20-2 20-3 20

**Step 1: Synthesis of Compound 20-2:**

The compound **20-1** (136 mg, 1 mmol) and DMSO (8 mL) were added to a dry single-necked flask, stirred to dissolve, then added with $K_2CO_3$ (332 mg, 2.4 mmol) and p-fluoronitrobenzene (141 mg, 1 mmol), heated up to 90°C to react and monitored by LC-MS, cooled to room temperature after the reaction, added with water and stirred till the solid precipitated, washed three times with water, then washed with a small amount of methanol, and dried to give the product **20-2** (180 mg, 81.8% yield), LCMS ($ESI^+$) m/z: 221.2 $[M+H]^+$.

**Step 2: Synthesis of Compound 20-3:**

The substrate **20-2** (180 mg, 0.82 mmol) and methanol (7mL) were added to a dry single-necked flask, stirred to dissolve, added with Pd/C (18mg), $H_2$ displaced five times, stirred at room temperature to react and monitored by LC-MS. After the reaction, Pd/C was removed with diatomaceous earth, the organic phase was concentrated under reduced pressure to give the crude product **20-3** (120 mg, crude), LCMS (ESI+) m/z:191.1 [M+H]+.

**Step 3: Synthesis of Compound 20:**

The substrate **IM-1** (52mg, 0.15mmol) and tetrahydrofuran (3mL) were added into a dry single-necked flask, stirred to dissolve, then added with m-CPBA (121 mg, 0.6 mmol, 85% purity) to react for 1 hour at room temperature and monitored by LC-MS, added with DIPEA (98 mg, 758.28 μmol) after the reaction and stirred for 10 min, then added with **20-3** (46 mg, 151.86 μmol) and monitored by LC-MS, concentrated under reduced pressure after the reaction, and the residue was purified by medium pressure liquid chromatography (Alkali process) to give the compound **20** (25 mg, 0.05 mmol, 33.84% yield).**$^1$H NMR** (600 MHz, DMSO-$d_6$) δ 10.07 (s, 1H), 8.80 (s, 1H), 8.02-8.01 (m, 1H), 7.75 (s, 1H), 7.60 (d, *J* = 7.6 Hz, 1H), 7.53 - 7.38 (m, 2H), 6.62 (d, *J* = 7.2 Hz, 2H), 5.70-5.63 (m, 1H), 5.33 (s, 1H), 5.00 (d, *J* = 10.2 Hz, 1H), 4.83 (d, *J* = 17.1 Hz, 1H), 4.69 (s, 2H), 4.60 (s, 1H), 4.53 (s, 1H), 3.75 (d, *J* =6.8 Hz, 1H), 3.67 (d, *J* = 7.4 Hz, 1H), 3.50 (d, *J* = 9.0Hz, 1H), 2.95 (d, *J* = 9.0 Hz, 1H), 1.93 (d, *J* = 9.2 Hz, 1H), 1.84 (d, *J* = 8.8 Hz, 1H), 1.47 (s, 6H). **$^1$H NMR** (600 MHz, DMSO-$d_6$, $D_2O$) δ 8.82 (s, 1H), 8.05-8.02 (m, 1H), 7.74 (s, 1H), 7.62 (d, *J* = 7.6 Hz, 1H), 7.52 (s, 2H), 6.63 (d, *J* = 7.6 Hz, 2H), 5.70-5.64 (m, 1H), 5.03 (d, *J* = 10.2 Hz, 1H), 4.84 (d, *J* = 17.0 Hz, 1H), 4.70 (s, 2H), 4.62 (s, 1H), 4.51 (s, 1H), 3.78 (d, *J* = 7.4 Hz, 1H), 3.68 (d, *J* = 7.6 Hz, 1H), 3.52 (d, *J* = 9.4 Hz, 1H), 2.96 (d, *J* = 9.2 Hz, 1H), 1.95 (d, *J* = 9.0 Hz, 1H), 1.86 (d, *J* = 9.0 Hz, 1H), 1.48 (s, 6H). LCMS (E+) m/z: 500.1 $[M+H]^+$, HPLC method B: $R_T$ = 6.69 min, purity: 99.6%.

**Example 21: Synthesis of Compound 21:**

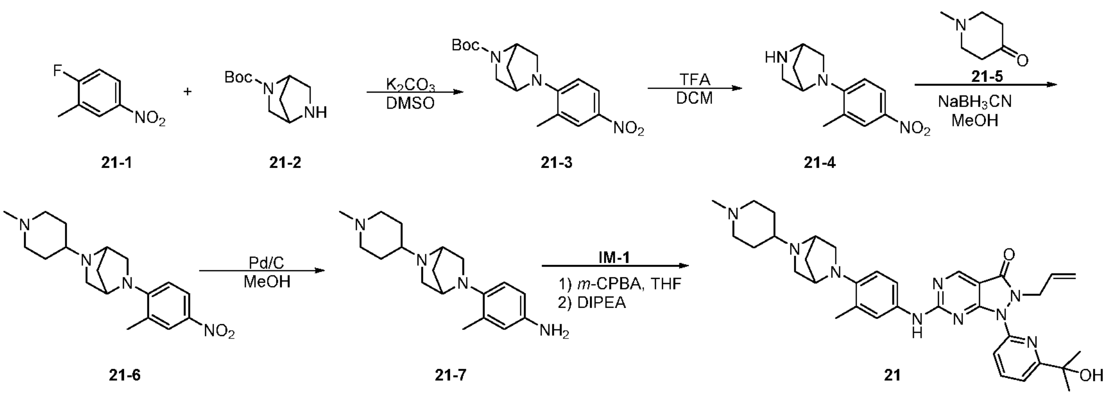


21-1 21-2 21-3 21-4

21-6 21-7 21

**Step 1: Synthesis of Compound 21-3:**

The compound **21-1** (3.0 g, 19.34 mmol) and DMSO (20mL) were added to a dry single-necked flask, stirred to dissolve, then added with $K_2CO_3$ (2.67 g, 19.34 mmol) and 21-2 (3.82 g, 19.34 mmol), heated up to 100°C to react for 3 h and monitored by LC-MS, cooled to room temperature after the reaction, added with water and stirred till the solid precipitated and filtered out, washed three times with water, then washed with a small amount of methanol, and dried to give the product **21-3** (6 g, 18.00 mmol, 93.06% yield), LCMS ($ESI^+$) m/z: 334.4 $[M+H]^+$.

**Step 2: Synthesis of Compound 21-4:**

The compound **21-3** (6.67 g, 20.01 mmol), TFA (2.28 g, 20.01 mmol) and DCM (50 mL) were added to a dry single-necked flask, stirred to dissolve to react for 18 hours at room temperature and monitored by LC-MS, concentrated under reduced pressure after the reaction, extracted three times with water and ethyl acetate. The orgnanic phase was combined, washed with saturated saline, dried with anhydrous $Na_2SO_4$. The filterate was concentrated under reduced pressure after filteration to give the crude product **21-4** (4 g, crude), LCMS ($ESI^+$) m/z: 234.3 $[M+H]^+$.

**Step 3: Synthesis of Compound 21-6:**

The compound **21-4** (3g, 12.86mmol), compound **21-5** (1.46 g, 12.86 mmol), $NaBH_3CN$ (1.2 g, 19.11 mmol) and methanol (30mL) were added to a dry single-necked flask, stirred to dissolve, heated up to 60°C to react for 18 hours and monitored by LC-MS, concentrated under reduced pressure after the reaction, extracted three times with water and ethyl acetate. The orgnanic phase was combined, washed with saturated saline, dried with anhydrous $Na_2SO_4$. The filterate was concentrated under reduced pressure after filteration and purified by medium pressure liquid chromatography to give the crude product **21-6** (3 g, 9.08 mmol, 70.60% yield), LCMS ($ESI^+$) m/z: 331.4 $[M+H]^+$.

**Step 4: Synthesis of Compound 21-7:**

The substrate **21-6** (200 mg, 0.61 mmol) and methanol (10mL) were added to a dry single-necked flask, stirred well, added with Pd/C (40mg), $H_2$ displaced five times, stirred at room temperature to react and monitored by LC-MS. After the reaction, Pd/C was removed with diatomaceous earth, washed with methanol, the organic phase was concentrated under reduced pressure to give the crude product **21-7** (50 mg, 27.5% yield), LCMS ($ESI^+$) m/z: 301 $[M+H]^+$.

**Step 5: Synthesis of Compound 21:**

The substrate **IM-1** (17.8 mg, 50.08 μmol) and tetrahydrofuran (2mL) were added into a dry single-necked flask, stirred to dissolve, then added with m-CPBA (12.9 mg, 74.74 μmol) to react for 1 hour at room temperature and monitored by LC-MS, added with DIPEA (98 mg, 758.28 μmol) after the reaction and stirred for 10 min, then added with **21-7** (15.05 mg, 50.08 μmol) and monitored by LC-MS, concentrated under reduced pressure after the reaction, and the residue was purified by medium pressure liquid chromatography (Alkali process) to give **21** (5 mg, 8.16 μmol, 16.29% yield). **$^1$H NMR** (600 MHz, DMSO-*d*$_6$) δ 10.09 (s, 1H), 8.82 (s, 1H), 7.99 - 7.97 (m, 1H), 7.78 (d, *J* = 7.8 Hz, 1H), 7.60 (d, *J* = 7.8 Hz, 1H), 7.57 (s, 1H), 7.29 - 7.27 (m, 1H), 6.72 (d, *J* = 9.0 Hz, 1H), 5.69 - 5.63 (m, 1H), 5.33 (s, 1H), 4.99 (d, *J* = 10.2 Hz, 1H), 4.82 (d, *J* = 17.4 Hz, 1H), 4.68 (d, *J* = 5.9 Hz, 2H), 3.95 (s, 1H), 3.66 (s, 1H), 3.20 (q, *J* = 8.4 Hz, 2H), 3.02 (d, *J* = 8.4 Hz, 1H), 2.68 (d, *J* = 7.8 Hz, 2H), 2.56 (d, *J* = 9.0 Hz, 1H), 2.22 (s, 3H), 2.16 (s, 1H), 2.12 (s, 3H), 1.91 (s, 1H), 1.88 (s, 1H), 1.74 -1.69 (m, 4H), 1.46 (s, 6H), 1.38 - 1.25 (m, 2H). LCMS ($ESI^+$) m/z: 610.6 $[M+H]^+$. HPLC method A: $R_T$ = 4.45 min, purity: 99.5%.

**Example 22: Synthesis of Compound 22:**

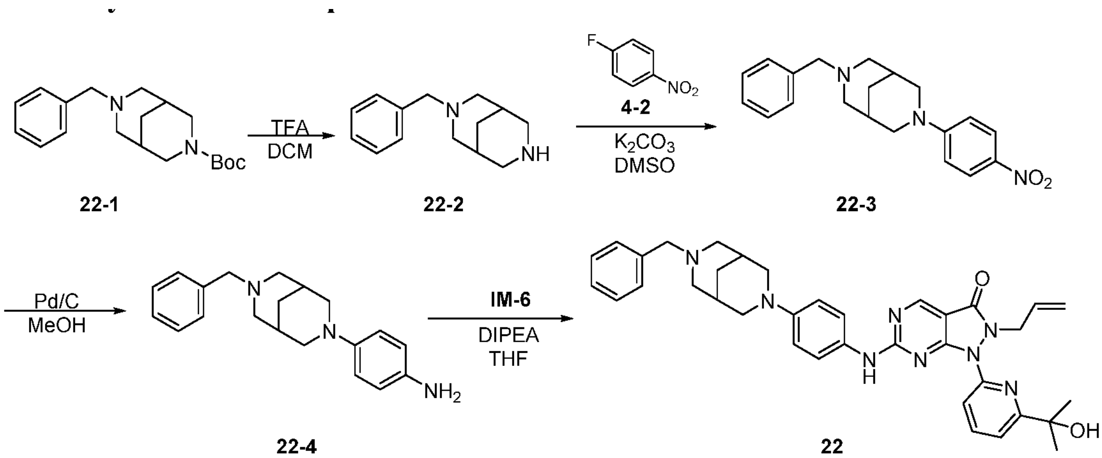


22-1 22-2 22-3 22-4 22

**Step 1: Synthesis of Compound 22-2:**

The compound **22-1** (158 mg, 499.31 μmol), TFA (3 mL) and DCM (3 mL) were added to a dry single-necked flask, stirred to dissolve to react for 18 hours at room temperature and monitored by LC-MS, concentrated under reduced pressure after the reaction, extracted three times with water and ethyl acetate. The orgnanic phase was combined, washed with saturated saline, dried with anhydrous $Na_2SO_4$. The filterate was concentrated under reduced pressure after filteration to give the crude product **22-2** (100 mg, crude), LCMS ($ESI^+$) m/z: 217.3 $[M+H]^+$.

**Step 2: Synthesis of Compound 22-3:**

The compound **22-2** (100 mg, 462.28 μmol) and DMSO (4mL) were added to a dry single-necked flask, stirred to dissolve, then added with $K_2CO_3$ (97 mg, 701.88 μmol) and compound **4-2** (72 mg, 510.28 μmol), heated up to 100°C to react for 3 hours and monitored by LC-MS, cooled to room temperature after the reaction, added with water and stirred till the solid precipitated then filtered out, washed three times with water, then washed with a small amount of methanol, and dried to give the product **22-3** (150 mg, 444.56 μmol, 96.17% yield), LCMS ($ESI^+$) m/z: 338.4 $[M+H]^+$.

**Step 3: Synthesis of Compound 22-4:**

The substrate **22-3** (50 mg, 148.19 μmol) and methanol (4mL) were added to a dry single-necked flask, stirred well, added with Pd/C (8mg), $H_2$ displaced five times, stirred at room temperature to react and monitored by LC-MS. After the reaction, Pd/C was removed with diatomaceous earth, washed with methanol, the organic phase was concentrated under reduced pressure to give the crude product **22-4** (20 mg, 65.06 μmol, 43.90% yield), LCMS ($ESI^+$) m/z: 308.4 $[M+H]^+$.

**Step 4: Synthesis of Compound 22:**

The substrate **22-4** (24.70 mg, 80.34 μmol) and tetrahydrofuran (2mL) were added into a dry single-necked flask, stirred to dissolve, then added with DIPEA (51.91 mg, 401.68 μmol, 69.96 μL) and compound IM-6 (30 mg, 80.34 μmol) to react for 1 hour at room temperature and monitored by LC-MS, concentrated under reduced pressure after the reaction, and the residue was purified by medium pressure liquid chromatography to give the compound **22** (5 mg, 7.30 μmol, 9.09% yield). **$^1$H NMR** (600 MHz, DMSO-$d_6$) δ 10.10 (s, 1H), 8.80 (s, 1H), 8.02 (s, 1H), 7.79-7.78 (m, 1H), 7.61-7.55 (m, 3H), 7.18-7.17 (m, 5H), 6.77 (d, *J* = 8.4 Hz, 2H), 5.70 - 5.63 (m, 1H), 5.33 (s, 1H), 5.00 (d, *J* = 10.3 Hz, 1H), 4.83 (d, *J* = 17.2 Hz, 1H), 4.68 (s, 2H), 3.59 (d, *J* = 11.3 Hz, 2H), 3.38 (s, 2H) 3.08 (d, *J* = 11.3 Hz, 2H), 2.87 (d, *J*= 10.6 Hz, 2H), 2.26 (d, *J* = 10.7 Hz, 2H), 2.09 (s, 2H), 1.70 (d, *J* = 12.2 Hz, 1H), 1.60 (d, *J* = 12.1 Hz, 1H), 1.47 (s, 6H). **$^1$H NMR** (600 MHz, DMSO-$d_6$, $D_2O$) δ 8.81 (s, 1H), 8.03 (s, 1H), 7.78 (s, 1H), 7.61-7.58 (m, 3H), 7.18 (s, 5H), 6.78 (d, *J* = 8.6 Hz, 2H), 5.69-5.63 (m, 1H), 5.02 (d, *J* = 10.3 Hz, 1H), 4.83 (d, *J* = 17.1 Hz, 1H), 4.68 (s, 2H), 3.64 (s, 2H), 3.39 (s, 2H), 3.08 (d, *J* = 10.9 Hz, 2H), 2.87 (d, *J* = 10.8 Hz, 2H), 2.28 (s, 2H), 2.10 (s, 2H), 1.70 (d, *J* = 11.9 Hz, 1H), 1.60 (d, *J* = 12.2 Hz, 1H), 1.47 (s, 6H). LCMS (E+) m/z: 617.4 $[M+H]^+$, HPLC method B: $R_T$ = 6.01 min, purity: 90.1%.

**Example 23: Synthesis of Compound 23:**

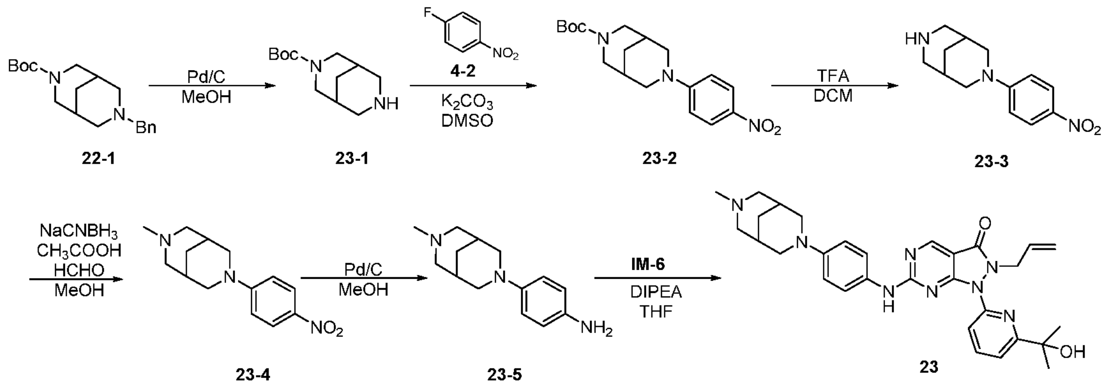


22-1 23-1 23-2 23-3

23-4 23-5 23

**Step 1: Synthesis of Compound 23-1:**

The substrate **22-1** (200 mg, 632.04 μmol) and methanol (5mL) were added to a dry single-necked flask, stirred well, added with Pd/C (20mg), $H_2$ displaced five times, stirred at room temperature to react and monitored by LC-MS. After the reaction, Pd/C was removed with diatomaceous earth, washed with methanol, the organic phase was concentrated under reduced pressure to give the crude product **23-1** (140 mg, crude), LCMS ($ESI^+$) m/z: 227.3 $[M+H]^+$.

**Step 2: Synthesis of Compound 23-2:**

The compound **23-1** (128.31 mg, 566.97 μmol) and DMSO (3mL) were added to a dry single-necked flask, stirred to dissolve, then added with $K_2CO_3$ (96.74 mg, 0.7 mmol) and p-fluoronitrobenzene (4-2, 80 mg, 566.97 μmol), heated up to 90°C to react for 6 hours and monitored by LC-MS, cooled to room temperature after the reaction, added with water and stirred till the solid precipitated then filtered out, washed three times with water, then washed with a small amount of methanol, and dried to give the product **23-2** (120 mg, crude), LCMS ($ESI^+$) m/z: 348.4 $[M+H]^+$.

**Step 3: Synthesis of Compound 23-3:**

The compound **23**-**2** (69 mg, 198.61 μmol), TFA (3 mL) and DCM (3 mL) were added to a dry single-necked flask, stirred to dissolve to react for 18 hours at room temperature and monitored by LC-MS, concentrated under reduced pressure after the reaction, extracted three times with water and ethyl acetate. The orgnanic phase was combined, washed with saturated saline, dried with anhydrous $Na_2SO_4$. After filteration, the filterate was concentrated under reduced pressure to give the crude product **23-3**(49 mg, crude), LCMS ($ESI^+$) m/z: 248.3 $[M+H]^+$.

**Step 4: Synthesis of Compound 23-4:**

The substrate **23-3** (49.46 mg, 0.2 mmol) was added into a dry single-necked flask, stirred to dissolve with methanol (4 mL), then added with formaldehyde (34.06 mg, 1.00 mmol) and acetic acid (11mL), stirred for 10 min then added with $NaCNBH_3$ (25.14 mg, 400.00 μmol), heated up to 50°C to react for 2 hours and monitored by LC-MS, concentrated under reduced pressure after the reaction, extracted three times with water and ethyl acetate. The orgnanic phase was combined, washed with saturated saline, dried with anhydrous $Na_2SO_4$. After filteration, the filterate was concentrated under reduced pressure and purified by medium pressure liquid chromatography to give the compound **23-4** (30 mg, 114.80 μmol, 57.40% yield), LCMS ($ESI^+$) m/z: 262.3 $[M+H]^+$.

**Step 5: Synthesis of Compound 23-5:**

The substrate **23-4** (30 mg, 114.80 μmol) and methanol (3mL) were added to a dry single-necked flask, stirred well, added with Pd/C (8mg), $H_2$ displaced five times, stirred at room temperature to react and monitored by LC-MS. After the reaction, Pd/C was removed with diatomaceous earth, washed with methanol, the organic phase was concentrated under reduced pressure to give the product **23-5** (20 mg, crude), LCMS ($ESI^+$) m/z: 232.3 $[M+H]^+$.

**Step 6: Synthesis of Compound 23:**

The substrate **23-5** (18.58 mg, 80.34 μmol) and tetrahydrofuran (2mL) were added into a dry single-necked flask, stirred to dissolve, then added with DIPEA (51.91 mg, 401.68 μmol, 69.96 μL) and compound **IM-6** (30 mg, 80.34 μmol) to

react for 1 h at room temperature and monitored by LC-MS, concentrated under reduced pressure after the reaction, and the residue was purified by medium pressure liquid chromatography to give the compound **23** (8 mg, 13.58 μmol, 16.91% yield).**$^1$H NMR** (600 MHz, DMSO-$d_6$) δ 10.09 (s, 1H), 8.80 (s, 1H), 8.04 (s, 1H), 7.76 (d, *J* = 8.0 Hz, 1H), 7.61-7.56 (m, 3H), 6.73 (d, *J* = 8.6 Hz, 2H), 5.65-5.63 (m, 1H), 5.33 (s, 1H), 4.99 (dd, *J* = 10.3, 1.6 Hz, 1H), 4.83 (dd, *J* = 17.1, 1.7 Hz, 1H), 4.68 (d, *J* = 6.1 Hz, 2H), 3.57 (d, *J* = 11.2 Hz, 2H), 3.46 (s, 3H), 3.00 (dd, *J* = 11.3, 4.8 Hz, 2H), 2.88 (s, 2H), 2.17-2.09 (m, 4H), 1.66-1.54 (m, 2H), 1.46 (s, 6H). **$^1$H NMR** (600 MHz, DMSO-$d_6$, $D_2O$) δ 8.80 (s, 1H), 8.05 (s, 1H), 7.76 (s, 1H), 7.62 - 7.55 (m, 3H), 6.75 (s, 2H), 5.67-5.63 (m, 1H), 5.02-5.01 (m, 1H), 4.84 - 4.82 (m, 1H), 4.68 (d, *J* = 6.1 Hz, 2H), 3.71 (s, 3H), 3.58 (d, *J* = 11.4 Hz, 2H), 2.99-2.89 (m, 4H), 2.12-2.04 (m, 4H), 1.66 - 1.57 (m, 2H), 1.47 (s, 6H). LCMS (E+) m/z: 541.3 $[M+H]^+$, HPLC method B: $R_T$ = 5.29 min, purity: 94.1%.

**Example 24: Synthesis of Compound 24:**

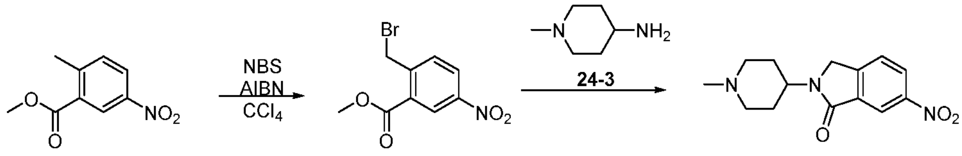


**24-1** **24-2** **24-4**

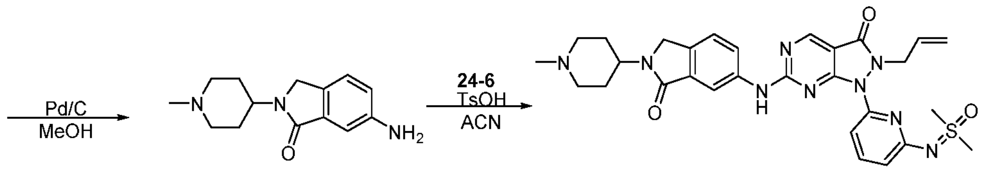


**24-5** **24**

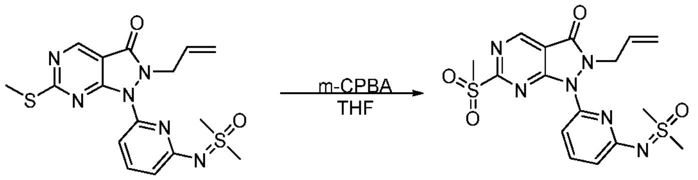


**IM-3** **24-6**

**Step 1: Synthesis of Compound 24-2:**

The substrate **24-1** (2g, 10.25mmol) and carbon tetrachloride (40mL) were added to a dry single-necked flask, stirred well, added with NBS (2.74g, 15.37mmol) and AIBN (269.24mg, 1.64mmol), and $N_2$ displaced five times, heated up to 70 °C with stirring and refluxing for 12 hours and monitored by LC-MS, concentrated under reduced pressure after the reaction, and the residue was purified by medium pressure liquid chromatography to give thr compound **24-2** (1.3 g, 4.74 mmol, 46.3% yield), LCMS ($ESI^+$) m/z: 274.1 $[M+H]^+$.

**Step 2: Synthesis of Compound 24-4:**

The substrate **24-2** (200 mg, 0.73 mmol) and **24-3** (166.42 mg, 1.46 mmol) were added to a dry single-necked flask, heated up to 50 °C with stirring and refluxing for 1 hour and monitored by LC-MS, concentrated under reduced pressure after the reaction, and added with a small amount of ethyl acetate. The solid was filtered out, the residue was purified by medium pressure liquid chromatography to give the compound **24-4** (180 mg, 0.65 mmol, 89.67% yield), LCMS ($ESI^+$) m/z: 276.1 $[M+H]^+$.

**Step 3: Synthesis of Compound 24-5:**

The substrate **24-4** (180 mg, 0.65 mmol) and methanol (3mL) were added to a dry single-necked flask, stirred well, added with Pd/C (8mg), $H_2$ displaced five times, stirred at room temperature to react and monitored by LC-MS. After the reaction, Pd/C was removed with diatomaceous earth, washed with methanol, the organic phase was concentrated under reduced pressure to give the product **24-5** (100 mg, crude), LCMS ($ESI^+$) m/z: 246.3 $[M+H]^+$.

**Step 4: Synthesis of Compound 24-6:**

The substrate **IM-3** (0.2 g, 0.51 mmol) and anhydrous THF (5mL) were added into a single-necked flask, stirred to dissolve, then added with m-CPBA (208.2 g, 1.02 mmol, 85% purity), stirred for 40 min at room temperature and monitored by LC-MS. After the reaction, the reactant was concentrated under reduced pressure and separated by medium pressure liquid chromatography (Alkali process) to give the compound **24-6** (130 mg, 0.31 mmol, 60.0% yield), LCMS (ESI$^+$) m/z: 423.5 [M+H]$^+$.

**Step 5: Synthesis of Compound 24:**

The substrate **24-6** (31 mg, 73.38 μmol), **24-5** (36.00 mg, 146.75 μmol), TsOH (50.48 mg, 293.50 μmol) and anhydrous ACN (0.3 mL) were added to a dry microwaveable tube, stirred to dissolve , heated up to 110 °C after the tube closed to react for 4 hours by microwave and monitored by LC-MS, cooled to room temperature after the reeaction, quenched by adding saturated sodium bicarbonate solution, extracted 3 times with ethyl acetate, the organic phase was combined, washed with saturated brine, dried with anhydrous $Na_2SO_4$, concentrated under reduced pressure, and then separated by medium pressure liquid chromatography to give the product 24 (1.5 mg, 2.55 μmol, 3.48% yield). **$^1$H NMR** (600 MHz, DMSO-$d_6$) δ 10.52 (s, 1H), 8.91 (s, 1H), 8.46 (s, 1H), 7.95 (s, 1H), 7.77 (dd, *J* = 8.2, 2.2 Hz, 1H), 7.55 (d, *J* = 8.3 Hz, 1H), 7.40 (d, *J* = 7.9 Hz, 1H), 6.64 (d, *J* = 8.0 Hz, 1H), 5.66 (ddt, *J* = 16.4, 10.2, 6.0 Hz, 1H), 5.02 (dd, *J* = 10.3, 1.6 Hz, 1H), 4.90 (dd, *J* = 17.1, 1.6 Hz, 1H), 4.80 (s, 2H), 4.42 (s, 2H), 4.08 - 3.97 (m, 1H), 3.38 (s, 6H), 2.89 (d, *J* = 11.1 Hz, 2H), 2.22 (s, 3H), 2.07 (t, *J* = 11.8 Hz, 2H), 1.82 (dd, *J* = 12.1, 3.8 Hz, 2H), 1.71 (d, *J* = 13.3 Hz, 2H). LCMS (ESI$^+$) m/z: 588.1 [M+H]$^+$, HPLC method B: $R_T$ = 5.22 min, purity: 92.3%.

**Example 25: Synthesis of Compound 25:**

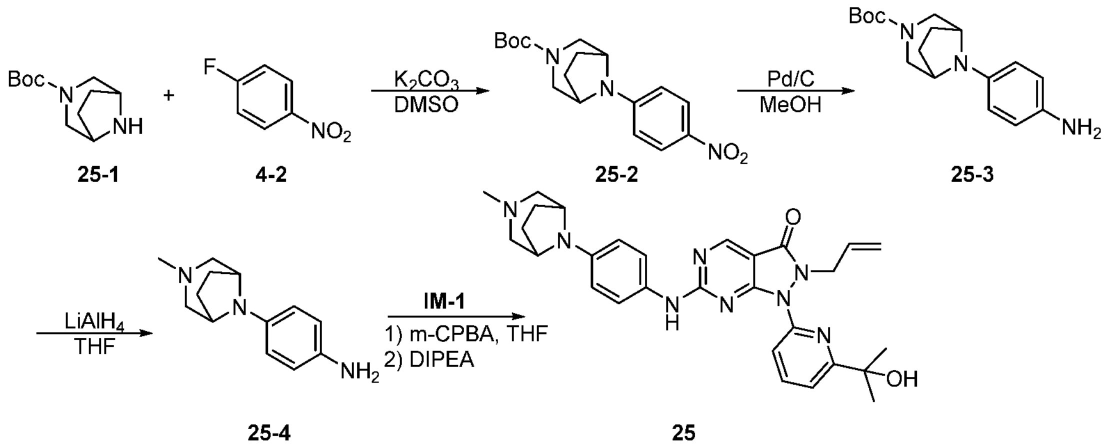


25-1 4-2 25-2 25-3 25-4 25

**Step 1: Synthesis of Compound 25-2:**

The compound **25-1** (0.5 g, 2.36 mmol) and DMSO (10mL) were added to a dry single-necked flask, stirred to dissolve, then added with $K_2CO_3$ (651.05 mg, 4.71 mmol) and p-fluoronitrobenzene (4-2, 332.33 mg, 2.36 mmol), heated up to 100°C to react for 6 hours and monitored by LC-MS, cooled to room temperature after the reaction, added with water and stirred till the solid precipitated then filtered out, washed three times with water, then washed with a small amount of methanol, and dried to give the product **25-2** (0.7 g, 2.10 mmol, 89.15% yield), LCMS (ESI$^+$) m/z: 333.4 [M+H]$^+$.

**Step 2: Synthesis of Compound 25-3:**

The substrate **25-2** (0.5 g, 1.50 mmol) and methanol (10mL) were added to a dry single-necked flask, stirred well, added with Pd/C (910mg), $H_2$ displaced five times, stirred at room temperature to react and monitored by LC-MS. After the reaction, Pd/C was removed with diatomaceous earth, washed with methanol, the organic phase was concentrated under reduced pressure to give the product **25-3** (0.4 g, 1.32 mmol, 87.91% yield), LCMS (ESI$^+$) m/z: 304.2 [M+H]$^+$.

**Step 3: Synthesis of Compound 25-4:**

The substrate **25-3** (150 mg, 0.5 mmol) and THF (4mL) were added into a single-necked flask, stirred to dissolve, then added with $LiAlH_4$(112.57 mg, 2.97 mmol), heated up to 65°C to react for 2 hours and monitored by LC-MS, cooled to room temperature after the reaction, added with water and stirred till the solid precipitated then filtered out, washed three times with water, then washed with a small amount of methanol, and dried to give the product **25-4** (80 mg, 0.36 mmol, 74.46% yield), LCMS ($ESI^+$) m/z: 218.3 $[M+H]^+$.

**Step 4: Synthesis of Compound 25:**

The substrate **IM-1** (21.70 mg, 99.88 μmol) and tetrahydrofuran (2mL) were added into a dry single-necked flask, stirred to dissolve, then added with m-CPBA (25.86 mg, 149.82 μmol) to react for 1 hour at room temperature and monitored by LC-MS, added with DIPEA (64.54 mg, 499.40 μmol, 86.98 μL) after the reaction and stirred for 10 min, then added with **25-4** (21.70 mg, 99.88 μmol) and monitored by LC-MS, concentrated under reduced pressure after the reaction, and the residue was purified by medium pressure liquid chromatography (Alkali process) to give the compound **25** (9 mg, 16.06 μmol, 16.08% yield). **$^1$H NMR** (600 MHz, DMSO-$d_6$) δ 10.07 (s, 1H), 8.80 (s, 1H), 8.03 - 8.01 (m, 1H), 7.75 (s, 1H), 7.60 (d, *J* = 7.8 Hz, 1H), 7.51 (s, 2H), 6.80 (d,*J* = 8.4 Hz, 2H), 5.70 - 5.99 (m, 1H), 5.31 (s, 1H), 5.00 - 4.98 (m, 1H), 4.84 - 4.81 (m, 1H), 4.68 (d, *J* = 6.0 Hz, 2H), 4.20 (s, 2H), 2.47 - 2.44 (m, 2H), 2.30 - 2.28 (m, 2H), 2.06 (s, 3H), 1.88 (d, *J* = 6.6 Hz, 2H), 1.83 (d, *J* = 6.0 Hz, 2H), 1.46 (s, 6H). LCMS ($ESI^+$) m/z : 527.3 $[M+H]^+$. HPLC method B: $R_T$: 7.83 min, purity: 94.0%.

**Example 26: Synthesis of Comnound 26:**

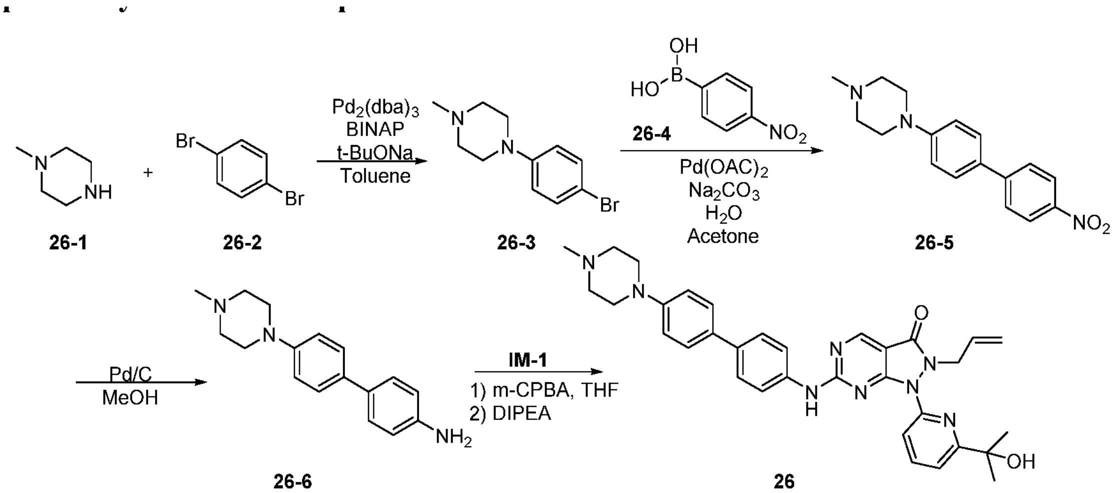


26-1 26-2 26-3 26-5

26-6 26

**Step 1: Synthesis of Compound 26-3:**

The substrate **26-1** (1.27 g, 12.72 mmol), **26-2** (2 g, 8.48 mmol), t-BuONa (1 g, 10.17 mmol) and toluene (15 mL) were added to a dry microwaveable tube, stirred to dissolve, deoxidized with $N_2$ for 10 min, added with $Pd_2(dba)_3$ (778 mg, 0.85 mmol), BINAP (264.6 mg, 0.43 mmol), and then deoxidized with $N_2$ for 10 min, heated to 110 °Cin an oil bath after the tube closed to react for 12 hours and monitored by LC-MS, cooled to room temperature after the reaction, filtered with diatomaceous earth, the filterate was concentrated under reduced pressure, and then separated by medium pressure liquid chromatography to give the product **26-3** (1.57 g, 6.15 mmol, 72.58% yield), LCMS ($ESI^+$) m/z: 255.2 $[M+H]^+$.

**Step 2: Synthesis of Compound 26-5:**

The substrate **26-3** (650 mg, 2.55 mmol) was added to a dry single-necked flask, dissolved with acetone (6 mL) and water (7 mL), then added with **26-4** (637.87 mg, 3.82 mmol), Pd(OAc) 2 (57.25 mg, 0.25 mmol), and sodium carbonate (542 mg, 2.55 mmol), $N_2$ displaced three times, then heated to 50 °C for 12 hours under $N_2$ protection and monitored by LC-MS, cooled to room temperature after the reaction, extracted three times with water and ethyl acetate, the organic phase was combined, washed with saturated brine, dried with anhydrous $Na_2SO_4$. The residue was purified by medium pressure

liquid chromatography to give the product **26-5** (240 mg, 807.13 μmol, 31.68% yield), LCMS (ESI+) m/z: 298.4 [M+H]+.

**Step 3: Synthesis of Compound 26-6:**

The substrate **26-5** (240 mg, 807.13 μmol) and methanol (10mL) were added to a dry single-necked flask, stirred well, added with Pd/C (30mg), $H_2$ displaced five times, stirred at room temperature to react and monitored by LC-MS. After the reaction, Pd/C was removed with diatomaceous earth, washed with methanol, the organic phase was concentrated under reduced pressure to give the product **26-6** (160 mg, 598.43 μmol, 74.14% yield), LCMS (ESI+) m/z: 268.4 [M+H]+.

**Step 4: Synthesis of Compound 26:**

The substrate **IM-1** (47.5 mg, 132.89 μmol) and tetrahydrofuran (0.5mL) were added into a dry single-necked flask, stirred to dissolve, then added with m-CPBA (54 mg, 265.79 μmol) to react for 1 h at room temperature and monitored by LC-MS, added with DIPEA (85.88 mg, 664.47 μmol, 115.74 μL) after the reaction and stirred for 10 min, then added with **26-6** (21.70 mg, 99.88 μmol) and monitored by LC-MS, concentrated under reduced pressure after the reaction, and the residue was purified by medium pressure liquid chromatography (Alkali process) to give the compound 26 (1.5 mg, 2.35 μmol, 1.77% yield). **$^1$H NMR** (600 MHz, DMSO-$d_6$) δ 10.37 (s, 1H), 8.90 (s, 1H), 8.11 (t, *J* = 8.2 Hz, 1H), 7.80 (dd, *J* = 8.3, 6.2 Hz, 2H), 7.63 (d, *J* = 7.7 Hz, 1H), 7.58 (d, *J* = 8.4 Hz, 2H), 7.54 (d, *J* = 8.5 Hz, 2H), 7.01 (d, *J* = 8.8 Hz, 2H), 5.68 (ddt, *J* = 16.5, 10.3, 5.9 Hz, 1H), 5.35 (s, 1H), 5.01 (d, *J* = 10.2 Hz, 1H), 4.88 - 4.79 (m, 1H), 4.70 (d, *J* = 6.0 Hz, 2H), 3.18 (t, *J* = 5.0 Hz, 4H), 2.47 (t, *J* = 5.0 Hz, 4H), 2.23 (s, 3H), 1.47 (s, 6H). LCMS (ESI) m/z: 577.3 [M+H]+, HPLC method B: $R_T$ = 9.20 min, purity: 97.8%.

**Example 27: Synthesis of Compound 27:**

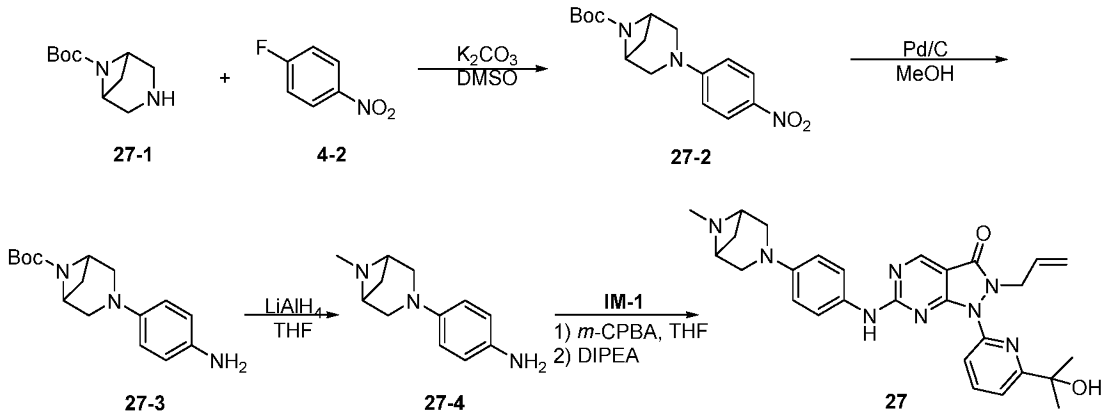


27-1 4-2 27-2

27-3 27-4 27

**Step 1: Synthesis of Compound 27-2:**

The compound **27-1** (400mg, 2.02mmol) and DMSO (5mL) were added to a dry single-necked flask, stirred to dissolve, then added with $K_2CO_3$ (836.50 mg, 6.05 mmol) and p-fluoronitrobenzene (313.14mg, 2.22mmol), heated up to 65°C to react for 10 hours and monitored by LC-MS, extracted three times with water and ethyl acetate after the reaction, the organic phase was combined, washed with saturated brine, dried with anhydrous $Na_2SO_4$, concentrated under reduced pressure and purified by medium pressure liquid chromatography to give the product **27-2** (633 mg, 1.98 mmol, 98.24% yield), LCMS (ESI+) m/z: 320.4 [M+H]+.

**Step 2: Synthesis of Compound 27-3:**

The substrate **27-2** (633mg, 1.98mmol) and methanol (5mL) were added to a dry single-necked flask, stirred well, added with Pd/C (60mg), $H_2$ displaced five times, stirred at room temperature to react and monitored by LC-MS. After the reaction, Pd/C was removed with diatomaceous earth, washed with methanol, the organic phase was concentrated under reduced pressure to give the product **27-3** (459 mg, crude), LCMS (ESI+) m/z: 290.4 [M+H]+.

**Step 3: ynthesis of Compound 27-4:**

The substrate **27-3** (150 mg, 518.36 μmol) and THF (5mL) were added into a single-necked flask, stirred to dissolve, then added with $LiAlH_4$(19.67mg, 518.36μmol), heated up to 65°C to react for 4 hours and monitored by LC-MS, quenched by adding 10% aqueous sodium hydroxide solution after the reaction under an ice bath, filtered, and the filtrate was concentrated under reduced pressure and purified by medium pressure liquid chromatography to give the product **27-4** (36.59 mg, 180.00 μmol, 34.73% yield), LCMS ($ESI^+$) m/z: 204.3 $[M+H]^+$.

**Step 4: Synthesis of Compound 27:**

The substrate **IM-1** (53.61 mg, 0.15 mmol) and tetrahydrofuran (0.5mL) were added into a dry single-necked flask, stirred to dissolve, then added with m-CPBA (38.83 mg, 225.00 μmol) to react for 1 hour at room temperature and monitored by LC-MS, added with DIPEA (96.93 mg, 750.00 μmol, 130.63 μL) after the reaction and stirred for 10 min, then added with **27-4** (36.59 mg, 180.00 μmol) and monitored by LC-MS, concentrated under reduced pressure after the reaction, and the residue was purified by medium pressure liquid chromatography (Alkali process) to give the compound **27** (18.9 mg, 33.81 μmol, 22.54% yield). **$^1$H NMR** (600 MHz, DMSO-$d_6$) δ 10.10 (s, 1H), 8.80 (s, 1H), 8.03 (s, 1H), 7.77 (s, 1H), 7.60 (d, *J* = 7.7 Hz, 3H), 6.72 (d, *J* = 8.5 Hz, 2H), 5.70 - 5.61 (m, 1H), 5.32 (s, 1H), 4.99 (dd, *J* = 10.3, 1.6 Hz, 1H), 4.83 (dd, *J* = 17.1, 1.7 Hz, 1H), 4.68 (d, *J* = 6.1 Hz, 2H), 3.64 (s, 2H), 3.46 (d, *J* = 10.9 Hz, 2H), 2.51 (s, 3H), 2.05 - 1.98 (m, 2H), 1.57 (s, 1H), 1.46 (s, 6H). LCMS(E+) m/z: 513.2 $[M+H]^+$, HPLC Method B $R_T$ = 11.32 min, purity: 92.6%.

**Example 28: Synthesis of Compound 28:**

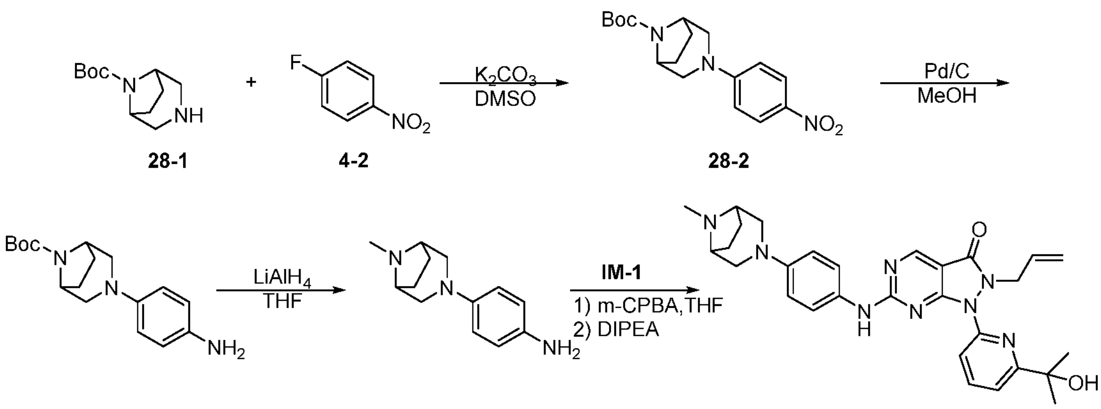


28-1 4-2 28-2

28-3 28-4 28

**Step 1: Synthesis of Compound 28-2:**

The susstrate **28-1** (332 mg, 1.56 mmol) and DMSO (5mL) were added to a dry single-necked flask, stirred to dissolve, then added with $K_2CO_3$ (648.42 mg, 4.69 mmol) and p-fluoronitrobenzene (242.73 mg, 1.72 mmol), heated up to 80°C to react for 12 hours and monitored by LC-MS, extracted three times with water and ethyl acetate after the reaction, the organic phase was combined, washed with saturated brine, dried with anhydrous $Na_2SO_4$, concentrated under reduced pressure and purified by medium pressure liquid chromatography to give the product **28-2** (512 mg, 1.54 mmol, 98.20% yield), LCMS ($ESI^+$) m/z: 334.4 $[M+H]^+$.

**Step 2: Synthesis of Compound 28-3:**

The substrate **28-2** (640 mg, 1.92 mmol) and methanol (5mL) were added to a dry single-necked flask, stirred well, added with Pd/C (60mg), $H_2$ displaced five times, stirred at room temperature to react and monitored by LC-MS. After the reaction, Pd/C was removed with diatomaceous earth, washed with methanol, the organic phase was concentrated under reduced pressure to give the product **28-3** (570 mg, crude), LCMS ($ESI^+$) m/z: 304.2 $[M+H]^+$.

**Step** 3: **Synthesis of Compound 28-4:**

The substrate **28-3** (200 mg, 659.20 μmol) and THF (5mL) were added into a single-necked flask, stirred to

dissolve, then added with $LiAlH_4$(125.08 mg, 3.30 mmol), heated up to 65°C to react for 4 hours and monitored by LC-MS, quenched by adding 10% aqueous sodium hydroxide solution after the reaction under an ice bath, filtered, and the filtrate was concentrated under reduced pressure and purified by medium pressure liquid chromatography to give the product **28-4** (89 mg, 409.55 μmol, 62.13% yield), LCMS ($ESI^+$) m/z: 218.2 $[M+H]^+$.

**Step 4: Synthesis of Compound 28:**

The substrate **IM-1** (53.61 mg, 0.15 mmol) and tetrahydrofuran (1mL) were added into a dry single-necked flask, stirred to dissolve, then added with m-CPBA (38.83 mg, 225.00 μmol) to react at room temperature for 1 hour and monitored by LC-MS, added with DIPEA (96.93 mg, 750.00 μmol, 130.63 μL) after the reaction and stirred for 10 min, then added with **28-4** (32.60 mg, 150.00 μmol) and monitored by LC-MS, concentrated under reduced pressure after the reaction, and the residue was purified by medium pressure liquid chromatography (Alkali process) to give the compound **28** (26.8 mg, 48.90 μmol, 32.60% yield). **$^1$H NMR** (600 MHz, DMSO-*d*$_6$) δ 10.11 (s, 1H), 8.81 (s, 1H), 8.04 (s, 1H), 7.75 (s, 1H), 7.65 - 7.43 (m, 3H), 6.79 (d, *J* = 8.6 Hz, 2H), 5.66 (ddt, *J* = 16.4, 10.2, 6.0 Hz, 1H), 5.32 (s, 1H), 4.99 (dd, *J* = 10.3, 1.4 Hz, 1H), 4.82 (dd, *J* = 17.1, 1.5 Hz, 1H), 4.68 (d, *J* = 5.9 Hz, 2H), 3.35 (d, *J* = 10.5 Hz, 2H), 3.24 (s, 2H), 2.81 (d, *J* = 10.3 Hz, 2H), 2.25 (s, 3H), 2.00 - 1.94 (m, 2H), 1.66 (d, *J* = 7.6 Hz, 2H), 1.46 (s, 6H). LCMS (E+) m/z: 527.4 $[M+H]^+$, HPLC Method B $R_T$ = 10.46 min, purity: 96.1%.

**Example 29: Synthesis of Compound 29:**

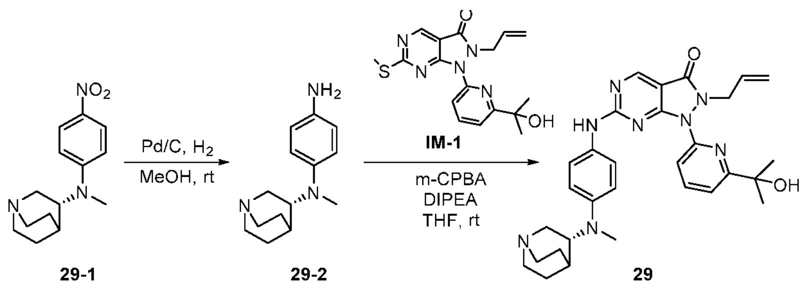


29-1 29-2 29

**Step 1: Synthesis of Compound 29-2:**

The substrate **29-1** (233 mg, 891.63 μmol) and methanol (10mL) were added to a dry single-necked flask, stirred to dissolve, added with Pd/C (10mg), $H_2$ displaced five times, stirred at room temperature to react for 2 hours and monitored by LC-MS. After the reaction, Pd/C was removed with diatomaceous earth, washed with methanol, the organic phase was concentrated under reduced pressure to give the compound **29-2** (200 mg, 866 μmol), LCMS (ESI) m/z: 232.2 $[M+H]^+$.

**Step 2: Synthesis of Compound 29:**

The substrate **IM-1** (38.63 mg, 108.07 μmol) was added to a dry single-necked vial, and added with tetrahydrofuran (1mL) to dissolve, then added with M-chloroperoxybenzoic acid (30 mg, 162.10 μmol) to react for 1 hour at room temperature, then N,N-diisopropylethylamine (20.95 mg, 162.10 μmol) and 29-2 (25 mg, 0.18 mmol) were added to the reaction solution to react overnight at room temperature and monitored by LC-MS, then the reaction solution was cooled to room temperature after the reaction, concentrated under reduced pressure. The residue was purified by preparative high-performance liquid chromatography (alkaline) to give the compound **29** (18 mg, 33.29 μmol). LCMS(ESI) m/z: 541.4 $[M+H]^+$,HPLC Method B $R_T$ = 5.14 min, purity >90.0%.**$^1$H NMR** (600 MHz, Methanol-*d*$_4$) δ 8.76 (s, 1H), 7.95 - 7.93 (m, 1H), 7.76 - 7.74 (m, 1H), 7.62 (d, *J* = 7.8 Hz, 1H), 7.44 (s, 2H), 6.70 - 6.62 (m, 2H), 5.72 - 5.68 (m, 1H), 5.03 (d, *J* = 10.2 Hz, 1H), 4.95 - 4.88 (m, 2H), 4.80 (d, *J* = 6.0 Hz, 2H), 4.00 (d, *J* = 8.6 Hz, 1H), 3.94 - 3.89 (m, 1H), 3.53 - 3.45 (m, 4H), 3.35 (s, 3H), 3.22 - 3.18 (m, 1H), 2.41 - 2.33 (m, 2H), 2.16 - 2.12 (m, 2H), 1.98 - 1.95 (m, 1H), 1.57 (s, 6H).

**Example 30: Synthesis of Compound 30:**

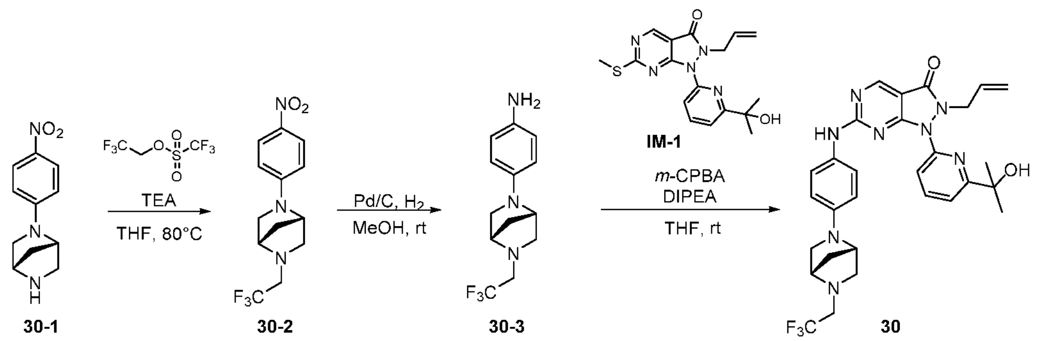


30-1 30-2 30-3 30

**Step 1: Synthesis of Compound 30-2:**

The substrate **30-1** (200 mg, 912.24 μmol) was added to a dry single-necked flask and dissolved in tetrahydrofuran (3mL), then added with triethylamine (461 mg, 4.56 mmol) and trifluoroethyl trifluoromethanesulfonateto (317 mg, 1.37 mmol), heated from room temperature to 80°C, stirred overnight and monitored by LC-MS, concentrated by distillation under reduced pressure after the reaction to give the compound 30-2(235mg,780 μmol), LCMS (ESI) m/z: 302.1[M+H]$^+$.

**Step 2: Synthesis of Compound 30-3:**

The substrate **30-2** (235 mg,780 μmol) and methanol (3mL) were added to a dry single-necked flask, stirred to dissolve, added with Pd/C (25mg), $H_2$ displaced five times to react for 2 hours at room temperature and monitored by LC-MS. After the reaction, Pd/C was removed with diatomaceous earth, washed with methanol, the organic phase was concentrated under reduced pressure to give the compound **30-3** (183 mg, 675 μmol), LCMS (ESI) m/z: 272.2[M+H]$^+$.

**Step 3: Synthesis of Compound 30:**

The substrate **IM-1** (27 mg, 75.54 μmol) was added to a dry single-necked vial, and added with tetrahydrofuran (1mL) to dissolve, then added with M-chloroperoxybenzoic acid (23 mg, 113.31 μmol) to react for 1 hour at room temperature, then N,N-diisopropylethylamine (48.81 mg, 377.1 μmol) and 30-3 (30.7mg, 113.31 μmol) were added to the reaction solution to react overnight at room temperature and monitored by LC-MS, then the reaction solution was cooled to room temperature after reeaction, concentrated under reduced pressure. The residue was purified by preparative high-performance liquid chromatography (alkaline) to give the compound **30** (10.7 mg, 18.43 μmol). LCMS(ESI) m/z: 581.3 [M+H]$^+$,HPLC Method B Rt = 8.07 min, purity >89.7%. $^1$H NMR (600 MHz, DMSO-$d_6$) δ 10.11 (s, 1H), 8.80 (s, 1H), 7.99 - 7.96 (m, 1H), 7.75 (s, 1H), 7.62 (d, *J* = 7.8 Hz, 1H), 7.47 (s, 2H), 6.62 (d, *J* = 8.4 Hz, 2H), 6.19 (d, *J* = 23.4 Hz, 1H), 5.69 - 5.63 (m, 1H), 4.99 (d, *J* = 10.2 Hz, 1H), 4.83 (d, *J* = 17.4 Hz, 1H), 4.67 (s, 2H), 3.89 (s, 2H), 3.51 (s, 2H), 3.29 (s, 3H), 2.97 (s, 3H), 2.22 (s, 2H), 2.00 (d, *J* = 7.8 Hz, 2H), 1.76 (d, *J* = 14.4 Hz, 1H), 1.46 (s, 6H). $D_2O$: $^1$H NMR (600 MHz, DMSO-$d_6$) δ 8.81 (s, 1H), 8.01 - 7.98 (m, 1H), 7.74 (s, 1H), 7.62 (d, *J* = 7.8 Hz, 1H), 7.49 (s, 2H), 6.64 (d, *J* = 8.4 Hz, 2H), 5.69 - 5.63 (m, 1H), 5.02 (d, *J* = 10.2 Hz, 1H), 4.83 (d, *J* = 17.4 Hz, 1H), 4.68 (s, 2H), 3.90-3.88 (m, 2H), 3.43 - 3.42 (m, 4H), 3.14 - 3.12 (m, 1H), 2.97 (s, 3H), 2.23 - 2.20 (m, 2H), 2.03 (s, 2H), 1.82(s, 1H), 1.47 (s, 6H).

**Example 31: Synthesis of Compound 31:**

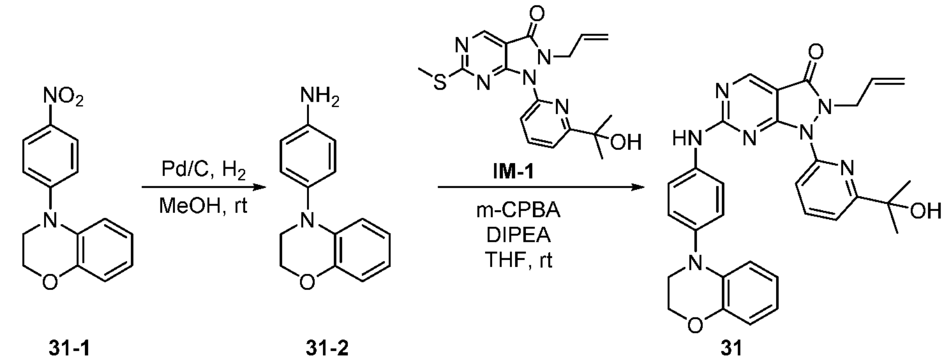


31-1 31-2 31

**Step 1: Synthesis of Compound 31-2:**

The substrate **31-1** (2.0 g, 7.80 mmol) and methanol (10mL) were added to a dry single-necked flask, stirred to dissolve, added with Pd/C (200mg), $H_2$ displaced five times to react for 2 hours at room temperature and monitored by LC-MS. After the reaction, Pd/C was removed with diatomaceous earth, washed with methanol, the organic phase was concentrated under reduced pressure to give the compound **31-2** (1.6 g, 7.07 mmol), LCMS (ESI) m/z: 232.2[M+H]$^+$.

**Step 2: Synthesis of Compound 31:**

The substrate **IM-1** (100 mg, 279.78 μmol) was added to a dry single-necked flask, and added with tetrahydrofuran (1.5mL) to dissolve, then added with M-chloroperoxybenzoic acid (120 mg, 559.55 μmol) to react for 1 hour at room temperature, then N,N-diisopropylethylamine (180.79 mg, 1.40 mmol) and 31-2 (126.61 mg, 559.55 μmol) were added to the reaction solution to react overnight at room temperature and monitored by LC-MS, then the reaction solution was cooled to room temperature after the reaction, concentrated under reduced pressure. The residue was purified by preparative high-performance liquid chromatography (alkaline) to give the compound **31** (40 mg, 74.68 μmol). LCMS (ESI) m/z: 536.2 [M+H]$^+$, HPLC method B: $R_T$ = 8.58 min, purity: >96.1%. $^1$H NMR (600 MHz, DMSO-$d_6$) δ 10.33 (s, 1H), 8.89 (s, 1H), 8.04 - 8.02 (m , 1H), 7.80 - 7.71 (m, 3H), 7.61 (d, *J* = 7.8 Hz, 1H), 7.23 (d, *J* = 8.4 Hz, 2H), 6.81 (d, *J* = 7.4 Hz, 1H), 6.73 - 6.71(m, 1H), 6.70 - 6.67 (m, 2H), 5.68 - 5.64 (m, 1H), 5.32 (s, 1H), 5.00 (d, *J* = 10.2 Hz, 1H), 4.83 (d, *J* = 17.4 Hz, 1H), 4.69 (d, *J* = 6.0 Hz, 2H), 4.27 - 4.26 (m, 2H), 3.68 - 3.66 (m, 2H), 1.46 (s, 7H).

**Example 32: Synthesis of Compound 32:**

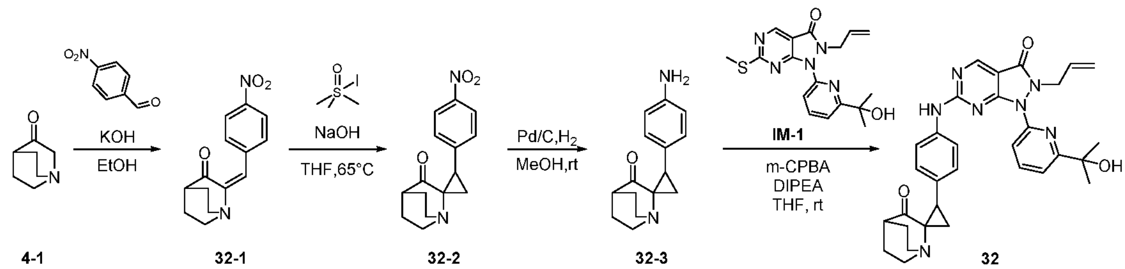


4-1 32-1 32-2 32-3 32

**Step 1: Synthesis of Compound 32-1:**

The compound **4-1** (800 mg, 6.39 mmol) was dissolved in ethanol (10 mL) in a dry single-necked flask, then added sequentially with potassium hydroxide (717 mg, 12.78 mmol), and p-nitrobenzaldehyde (1.16 g, 7.67 mmol). The reaction mixture was stirred overnight at room temperature and monitored by LC-MS, then concentrated under reduced pressure. The residue was purified by MPLC to give the compound **32-1** (863 mg, 3.34 mmol). LCMS (ESI) m/z: 259.1 [M+H]$^+$.

**Step 2: Synthesis of Compound 32-2:**

Trimethylsulfoxonium iodide (2.94g, 13.37mmol), sodium hydroxide (1.07g, 26.73mmol) was dissolved in

tetrahydrofuran (20 mL) in a single-necked flask, $N_2$ displaced to react for 1 hour at room temperature, then added with **32-1** (863mg, 3.34mmol), $N_2$ displaced again, stirred at 65°C for 1 hour, the reaction was monitored by LC-MS, concentrated under reduced pressure after the reaction to remove solvents, then extracted three times with ethyl acetate and water, the organic phase was dried with anhydrous $Na_2SO_4$, filtered, the residue was purified by column chromatography to give the compound **32-2** (493 mg,1.81 mmo). LCMS (ESI) m/z:273.1[M+H]$^+$.

**Step 3: Synthesis of Compound 32-3:**

The substrate **32-2** (493 mg,1.81 mmol) and methanol (5mL) were added to a dry single-necked flask, stirred to dissolve, added with Pd/C (50mg), $H_2$ displaced five times, stirred at room temperature to react for 2 hours and monitored by LC-MS. After the reaction, Pd/C was removed with diatomaceous earth, washed with methanol, the organic phase was concentrated under reduced pressure to give the compound **32-3** (395 mg, 1.63 mmol), LCMS (ESI) m/z: 243.2[M+H]$^+$.

**Step 4: Synthesis of Compound 32:**

The substrate **IM-1** (36 mg, 100.72 μmol) was added to a dry single-necked flask, and added with tetrahydrofuran (1.5mL) to dissolve, then added with M-chloroperoxybenzoic acid (26 mg, 151 μmol) to react for 1 hour at room temperature, then N,N-diisopropylethylamine (65 mg, 504 μmol) and 32-3 (48.8 mg, 201μmol) were added to the reaction solution to react overnight at room temperature and monitored by LC-MS, then the reaction solution was cooled to room temperature after the reaction, concentrated under reduced pressure. The residue was purified by preparative high-performance liquid chromatography (alkaline) to give the compound 32 (7.2 mg, 13.05 μmol). LCMS (ESI) m/z: 552.3 [M+H]$^+$, HPLC method A: $R_T$ = 9.90 min, purity: >99.1%. $^1$H NMR (600 MHz, DMSO-$d_6$) δ 10.26 (s, 1H), 8.88 (s, 1H), 8.04 - 8.02 (m 1H), 7.75 (d, *J* = 8.4 Hz, 1H), 7.62 (d, *J* = 8.4 Hz, 3H), 7.26 (d, *J* = 8.4 Hz, 2H), 5.67 - 5.65 (m, 1H), 5.34 (s, 1H), 5.00 (d, *J* = 10.2 Hz, 1H), 4.83 (d, *J* = 17.4 Hz, 1H), 4.69 (d, *J* = 6.0 Hz, 2H), 2.96 - 2.93 (m, 2H), 2.77 - 2.70 (m, 1H), 2.49 (d, *J* = 12.6 Hz, 1H), 2.45 - 2.43 (m, 1H), 2.21 - 2.20 (m, 1H), 2.01-1.97 (m, 1H), 1.92 - 1.89 (m, 2H), 1.87 - 1.79 (m, *J* = 2.6 Hz, 1H), 1.67 - 1.62 (m, 1H), 1.59 (d, *J* = 9.6 Hz, 1H), 1.46 (s, 6H).

**Example 33: Synthesis of Compound 33:**

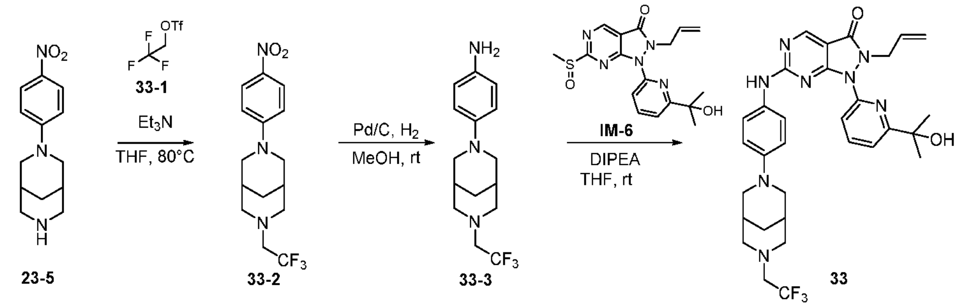


**Step 1: Synthesis of Compound 33-2:**

The compound **23-5** (100 mg, 404.38 μmol) was dissolved in tetrahydrofuran (5 ml) in a dry single-necked flask, then added sequentially with triethylamine (409.19 mg, 4.04 mmol) and **33-1** (140.79 mg, 606.57 μmol). The reaction mixture was stirred for 3 hours at 80°C, then concentrated under reduced pressure after reaction. The residue was purified by MPLC to give the compound **33-2** (70 mg, 212.56 μmol). LCMS (ESI) m/z: 330.1 [M+H]$^+$.

**Step 2: Synthesis of Compound 33-3:**

The compound **33-2** (80 mg, 0.24 mmol) was dissolved in methanol (3mL) in a round-bottomed flask, added with Pd/C (10mg), stirred for 2 hours at room temperature under $H_2$ atmosphere and monitored by LC-MS. The reaction solution was filtered with diatomaceous earth and washed with methanol for 2 times. The filterate was concentrated under reduced pressure to give the compound **33-3** (20 mg, 0.17 mmol). LCMS (ESI) m/z: 300.2 [M+H]$^+$.

**Step 3: Synthesis of Compound 33:**

The substrate **IM-6** (48.55 mg, 130.00 μmol) and tetrahydrofuran (3mL) were added to a dry single-necked flask, stirred to dissolve, then N,N-diisopropylethylamine (84.01 mg, 650.00 μmol) and 33-3 (44 mg, 146.99 μmol) were added to the reaction solution to react for 2 hours at room temperature and monitored by LC-MS, then concentrated under reduced pressure to remove the solvents after the reeaction. The residue was purified by preparative high-performance liquid chromatography (alkaline) to give the compound 33 (20 mg, 31.77 μmol). LCMS (ESI) m/z: 609.2 $[M+H]^+$, HPLC method A: $R_T$ = 5.8 min, purity: 96.7%. $^1H$ NMR (600 MHz, DMSO-$d_6$) δ 10.08 (s, 1H), 8.80 (s, 1H), 8.01 - 8.00 (m, 1H), 7.77 (s, 1H), 7.60 (d, *J* = 7.8 Hz, 1H), 7.54 - 7.40 (m, 2H), 6.75 (d, *J* = 8.4 Hz, 2H), 5.67-5.63 (m, 1H), 5.37 (s, 1H), 5.00 (d, *J* = 10.2 Hz, 1H), 4.83 (d, *J* = 16.8 Hz, 1H), 4.69 (s, 2H), 3.47 (s, 2H), 3.08 (s, 4H), 2.95 (s, 2H), 2.70 (s, 2H), 2.07 (s, 2H), 1.66-1.59 (m, 2H), 1.47 (s, 6H). $D_2O$: $^1H$ NMR (600 MHz, DMSO-$d_6$) δ 8.81 (s, 1H), 8.02 - 8.01 (m, 1H), 7.75 (s, 1H), 7.61 (d, *J* = 7.2 Hz, 1H), 7.54 (s, 2H), 6.75 (d, *J* = 7.8 Hz, 2H), 5.68 - 5.64 (m, 1H), 5.02 (d, *J* = 10.2 Hz, 1H), 4.84 (d, *J* = 16.8 Hz, 1H), 4.70 (s, 2H), 3.52 (d, *J* = 10.8 Hz, 2H), 3.09 - 3.04 (m, 4H), 2.94 (d, *J* = 10.2 Hz, 2H), 2.69 (d, *J* = 10.2 Hz, 2H), 2.08 (s, 2H), 1.67 - 1.58 (m, 2H), 1.48 (s, 6H).

**Example 34: Synthesis of Compound 34:**

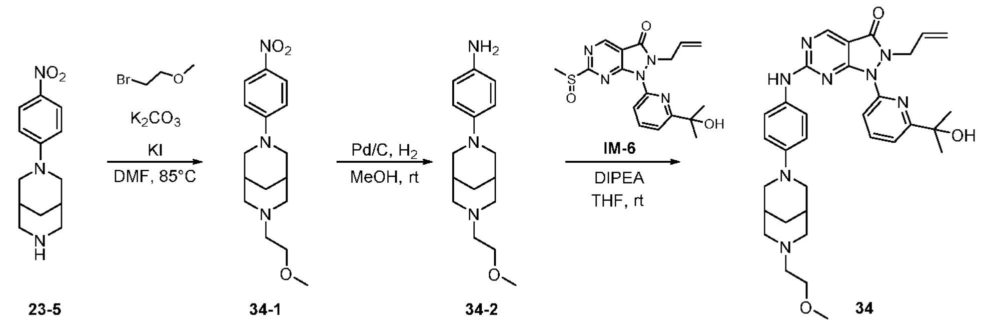


23-5 34-1 34-2 34

**Step 1: Synthesis of Compound 34-1:**

The substrate **23-5** (100 mg, 404.38 μmol) was dissolved in N,N-dimethylformamide (3 mL) in a dry single-necked flask, then added with 2-bromoethyl methyl ether (84 mg, 606 μmol), $K_2CO_3$ (111.77 mg, 808.76 μmol) and KI (13.43 mg, 80.88 μmol). The reaction was carried out at 85°C for 3 hours and monitored by LC-MS, then the reaction solution was cooled to room temperature after reeaction, extracted three times with water and ethyl acetate, the organic phase was dried with anhydrous $Na_2SO_4$, filtered, the residue was purified by column chromatography to give the compound **34-1** (65 mg, 212.86 μmol). LCMS (ESI) m/z: 306.2 $[M+H]^+$.

**Step 2: Synthesis of Compound 34-2:**

The substrate **34-1** (80 mg, 261.98 μmol) was added to a 50mL round-bottomed flask, dissolved in methanol (5mL), added with a catalytic amount of Pd/C (10mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **34-2** (29 mg, 0.10 mmol). LCMS (ESI) m/z: 276.2 $[M+H]^+$.

**Step 3: Synthesis of Compound 34:**

The substrate **IM-6** (37 mg, 99.08 μmol) and tetrahydrofuran (3mL) were added to a dry single-necked flask, stirred to dissolve, then added with N,N-diisopropylethylamine (64.03 mg, 495.41 μmol) and 34-2 (28.65 mg, 104.04 μmol) to react for 2 hours at room temperature and monitored by LC-MS, then concentrated under reduced pressure to remove solvents after the reeaction. The residue was purified by preparative high-performance liquid chromatography (alkaline) to give the compound **34** (10 mg, 17.10 μmol). LCMS (ESI) m/z: 585.3 $[M+H]^+$, HPLC method A: $R_T$ = 5.4 min, purity: 96.4%. $^1H$ NMR (600 MHz, DMSO-$d_6$) δ 10.09 (s, 1H), 8.80 (s, 1H), 8.03 (s, 1H), 7.76 (d, *J* = 7.2 Hz, 1H), 7.61 - 7.56 (m, 3H), 6.75 (s, 2H), 5.69-5.63 (m, 1H), 5.32 (s, 1H), 4.99 (d, *J* = 10.2 Hz, 1H), 4.83 (d, *J* = 17.4, 1H), 4.68 (d, *J* = 4.2 Hz, 2H), 3.54 (d, *J* = 10.2 Hz, 2H), 3.40 (s, 4H), 3.18 (s, 3H), 3.06 - 2.98 (m, 4H), 2.44 - 2.29 (m, 2H), 2.13 (s, 2H), 1.68 - 1.57 (m, 2H), 1.46 (s, 6H).

$D_2O$: $^1H$ NMR (600 MHz, DMSO-$d_6$) δ 8.81 (s, 1H), 8.03 (s, 1H), 7.76-7.75 (m, 1H), 7.61 (d, *J* = 7.8 Hz, 1H) 7.56 - 7.50 (m, 2H), 6.77 (s, 2H), 5.69 - 5.63 (m, 1H), 5.01 (d, *J* = 10.2 Hz, 1H), 4.83 (d, *J* = 16.8 Hz,, 1H), 4.68 (d, *J* = 4.8 Hz, 2H), 3.63 (s, 2H), 3.55 (d, *J* = 10.8 Hz, 2H), 3.41 (s, 2H), 3.18 (s, 3H), 3.05 - 2.96 (m, 4H), 2.45 - 2.36(m, 2H), 2.14 (s, 2H), 1.66 - 1.63 (m, 2H), 1.47 (s, 6H).

**Example 35: Synthesis of Compound 35:**

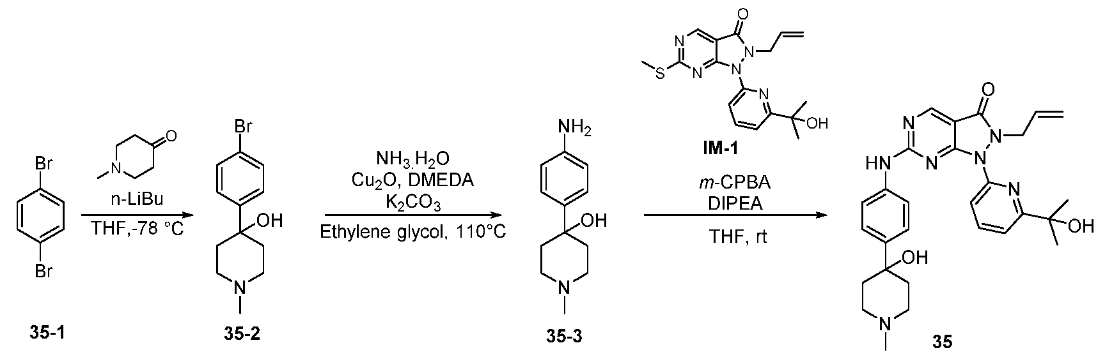


35-1 35-2 35-3 35

**Step 1: Synthesis of Compound 35-2:**

The compound **35-1** (2.59 g, 11 mmol) was added under $N_2$ protection to a 100 mL dry round-bottomed flask, then added with tetrahydrofuran (25 mL) to dissolve, lowered to -78 °C and slowly added dropwise with n-LiBu (5.5 mL, 2 M in THF, 11 mmol) into the reaction system, stirred for 30 min at -78 °C then added with N-methyl-4-piperidone (1.24 g, 11.00 mmol) , slowly heated to room temperature and stirred for 1 hour, quenched with saturated $NH_4Cl$ solution and extracted three times with ethyl acetate. The organic phase was dried with anhydrous $Na_2SO_4$ and concentrated under reduced pressure, and the residue was purified by column chromatography to give the compound **35-2** (2.63 g, 9.73 mmol). LCMS (ESI) m/z: 270.1[M+H]$^+$.

**Step 2: Synthesis of Compound 35-3:**

The substrate **35-2** (675.4 mg, 2.5 mmol), ammonia (1.75 g, 50.00 mmol), $Cu_2O$ (17.9 mg, 0.125 mmol), NaI (242.66 mg, 1.62 mmol), $K_2CO_3$ (69.10 mg, 0.5 mmol) and N,N ' - dimethylethylenediamine (22 mg, 0.25 mmol) were added to a dry microwave tube under $N_2$ protection, then added with ethylene glycol (5 mL) , heated up 110°C to react for 12 hours under $N_2$ atmosphere and monitored by LC-MS. The reaction system was cooled to room temperature after the reaction, filtered, extracted three times with ethyl acetate. The organic phase was dried with anhydrous $Na_2SO_4$, filtered, then concentrated under reduced pressure. The residue was purified by column chromatography to give the compound **35-3** (346 mg, 1.68 mmol). LCMS (ESI) m/z: 207.2 [M+H]$^+$.

**Step 3: Synthesis of Compound** 35:

The substrate **IM-1** (71.5 mg, 0.2 mmol) was added to a dry single-necked flask, and added with tetrahydrofuran (1mL) to dissolve, then added with M-chloroperoxybenzoic acid (51.8 mg, 0.3 mmol) to react for 30 min at room temperature, then N,N-diisopropylethylamine (129.2 mg, 1.00 mmol) and 35-3 (61.9 mg, 0.3 mmol) were added to the reaction solution to react for 4 hours at room temperature and monitored by LC-MS, then concentrated under reduced pressure after the reaction. The residue was purified by preparative high-performance liquid chromatography (alkaline) to give the compound **35** (16 mg, 31.03 μmol). $^1H$ NMR (600 MHz, DMSO-$d_6$) δ 10.27 (s, 1H), 8.88 (s, 1H), 8.06 - 8.04 (m, 1H), 7.76 (d, *J* = 8.4 Hz, 1H), 7.65 (d, *J* = 26.4 Hz, 3H), 7.43 (d, *J* = 8.4 Hz, 2H), 5.67 - 5.64 (m, 1H), 5.34 (s, 1H), 5.00 (d, *J* = 10.2 Hz, 1H), 4.83 (d, *J* = 17.4 Hz, 1H), 4.73 (s, 1H), 4.68 (d, *J* = 6.0 Hz, 2H), 2.58 (d, *J* = 10.8 Hz, 2H), 2.40 - 2.38 (m, 2H), 2.23 (s, 3H), 2.01 - 1.91 (m, 2H), 1.61 - 1.55 (m, 2H), 1.46 (s, 6H). LCMS(ESI) m/z: 516.3 [M+H]$^+$,HPLC $R_T$ = 4.68 min, purity > 92.7%.

**Example 36: Synthesis of Compound 36:**

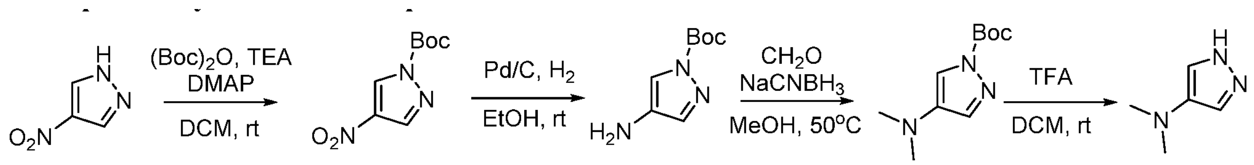


36-1 36-2 36-3 36-4 36-5

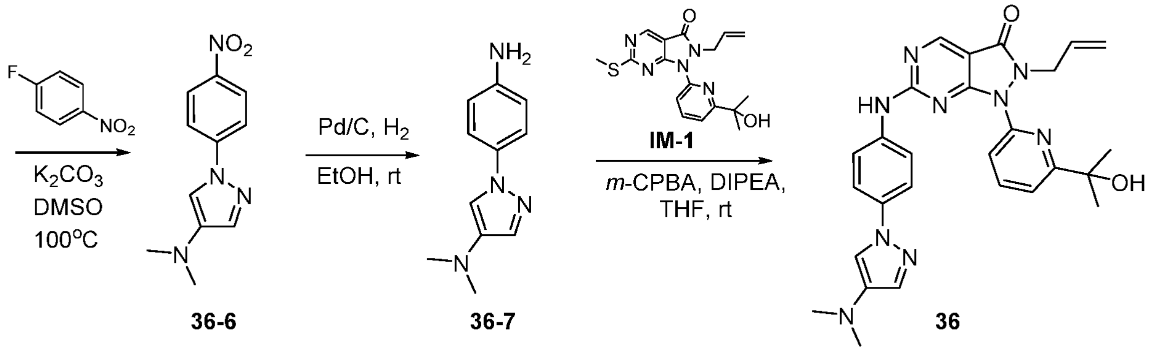


36-6 36-7 36

**Step 1: Synthesis of Compound 36-2:**

The compound 36-1 (5 g, 44.22 mmol) was added under $N_2$ protection to a dry single-necked flask, dissolved in dichloromethane (50mL), then added sequentially with triethylamine (8.95 g, 88.44 mmol, 12.33 mL), 4-dimethylaminopyridine (540.22 mg, 4.42 mmol) and di-tert-butyl dicarbonate (10.62 g, 48.64 mmol). The reaction mixture was stirred for 2 hours at room temperature and monitored by LC-MS, then diluted with water after the reaction and extracted three times with dichloromethane. The organic phase was dried with anhydrous $Na_2SO_4$, filtered and concentrated under reduced pressure, and the residue was purified by column chromatography to give the compound **36-2** (4.62 g, 21.67 mmol). LCMS (ESI) m/z: 214.1[M+H]$^+$.

**Step 2: Synthesis of Compound 36-3:**

The substrate **36-2** (4.62 g, 21.67 mmol) was added to a 50mL round-bottomed flask, dissolved in ethanol (3mL), added with a catalytic amount of Pd/C (400mg), stirred for 6 hours at room temperature under $H_2$ atmosphere and monitored by LC-MS. After the reaction, the reacton solution was filtered with diatomaceous earth, washed two times with ethanol, the filterate was concentrated under reduced pressure to give the compound **36-3** (3.85 g, 21.01 mmol). LCMS (ESI) m/z: 184.1 [M+H]$^+$ .

**Step 3: Synthesis of Compound 36-4:**

The substrate **36-3** (493 mg,1.81 mmol) was added to a dry single-necked flask and dissolved in methanol (40mL), added with formaldehyde (1.26 g, 42.03 mmol) and acetic acid (2 mL), stirred for 30 min at room temperature, added with sodium cyanoborohydride (3.30 g, 52.54 mmol), heated up to 50°C to react for 12 hours, then diluted with water after the reaction, added with saturated NaHCOs to make it alkaline, extracted three times with dichloromethane. The organic phase was dried with anhydrous $Na_2SO_4$, filtered and concentrated under reduced pressure, and the residue was purified by column chromatography to give the compound **36-4** (4.01 g, 18.98 mmol). LCMS (ESI) m/z: 212.1[M+H]$^+$.

**Step 4: Synthesis of Compound 36-5:**

The substrate **36-4** (4.01 g, 18.98 mmol) was added to a dry single-necked flask and dissolved in dichloromethane (40ml), added with trifluoroacetic acid (21.64 g, 189.81 mmol, 14.53 mL) to react for 30 min at room temperature and monitored by LC-MS, the solvent was removed by distillation under reduced pressure to give the compound **36-5** (1.86 g, 16.73 mmol). LCMS (ESI) m/z: 112.1 [M+H]$^+$.

**Step 5: Synthesis of Compound 36-6:**

The substrate **36-5** (1.86 g, 16.73 mmol) was added in a dry single-necked flask and dissolved in dimethyl sulfoxide (15 ml), added sequentially with p-fluoronitrobenzene (2.36 g, 16.73 mmol) and $K_2CO_3$ (5.78 g, 41.84 mmol). The reaction mixture was stirred overnight at 100°Cand monitored by LC-MS, then diluted with water after the reaction and

extracted three times with ethyl acetate. The organic phase was dried with anhydrous $Na_2SO_4$, then concentrated under reduced pressure. The residue was purified by column chromatography to give the compound **36-6** (3.56 g, 15.33 mmol). LCMS (ESI) m/z: 233.1[M+H]$^+$.

**Step 6: Synthesis of Compound 36-7:**

The substrate **36-6** (3.56 g, 15.33 mmol) was added to a dry single-necked flask, dissolved in ethanol (35mL), added with a catalytic amount of Pd/C (350mg), stirred for 3 hours at room temperature under $H_2$ atmosphere and monitored by LC-MS. After the reaction, the reacton solution was filtered with diatomaceous earth, washed two times with ethanol, the filterate was concentrated under reduced pressure to give the compound **36-7** (2.95 g, 14.59 mmol). LCMS (ESI) m/z: 203.1 [M+H]$^+$.

**Step 7: Synthesis of Compound 36:**

The substrate **IM-1** (45.51 mg, 225.00 μmol) was added to a dry single-necked flask, and added with tetrahydrofuran (1mL) to dissolve, then added with M-chloroperoxybenzoic acid (38.83 mg, 225.00 μmol) to react for 30 min at room temperature, then N,N-diisopropylethylamine (96.93 mg, 750.00 μmol) and 36-7 (53.61 mg, 0.15 mmol) were added to the reaction solution to react for 4 hours at room temperature and monitored by LC-MS, then concentrated under reduced pressure after the reaction. The residue was purified by preparative high-performance liquid chromatography (alkaline) to give the compound **36** (9 mg, 17.59 μmol). LCMS(ESI) m/z: 512.2 [M+H]$^+$, HPLC Method B $R_T$ = 7.26 min, purity > 98.0 %. $^1$H NMR (600 MHz, DMSO-*d*$_6$) δ 10.39 (s, 1H), 8.91 (s, 1H), 8.09 (s, 1H), 7.87 (s, 1H), 7.82 (d, *J* = 8.4 Hz, 2H), 7.78 (d, *J* = 8.4 Hz, 1H), 7.72 (d, *J* = 9.0 Hz, 2H), 7.64 (d, *J* = 7.8 Hz, 1H), 7.45 (s, 1H), 5.71 - 5.66 (m, 1H), 5.34 (s, 1H), 5.00 (d, *J* = 10.2 Hz, 1H), 4.84 (d, *J* = 17.4 Hz, 1H), 4.70 (d, *J* = 6.0 Hz, 2H), 2.70 (s, 6H), 1.47 (s, 6H).

**Example 37: Synthesis of Compound 37:**

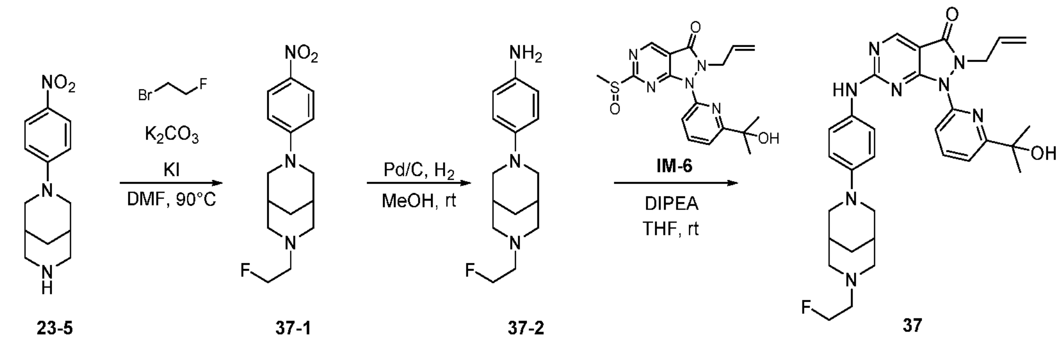


23-5 37-1 37-2 37

**Step 1: Synthesis of Compound 37-1:**

The substrate **23-5** (150 mg, 606.57 μmol) was added to a dry single-necked flask, dissolved in N,N-Dimethylformamide (3mL), then added with 1,2-Fluoroethane bromide (84 mg, 661.65 μmol), $K_2CO_3$ (165 mg, 1.19 mmol) and KI (20 mg, 120.5 μmol) to react for 3 hours at 90°C and monitored by LC-MS. The reaction solution was cooled to room temperature after the reaction, added with water, filtered and washed with methanol. The organic phase was concentrated under reduced pressure to give the compound **37-1** (80 mg, 272.7 μmol). LCMS (ESI) m/z: 394.2 [M+H]$^+$.

**Step 2: Synthesis of Compound 37-2:**

The substrate **37-1** (80 mg, 272.7 μmol) was added to a 50mL round-bottomed flask, dissolved in methanol (3mL), added with a catalytic amount of Pd/C (10mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **37-2** (50 mg, 0.19 mmol). LCMS (ESI) m/z: 264.2 [M+H]$^+$.

**Step 3: Synthesis of Compound 37:**

The substrate **IM-6** (42 mg, 112.47 μmol) and tetrahydrofuran (3mL) were added to a dry single-necked flask, stirred to dissolve, then added with N,N-diisopropylethylamine (72.68 mg, 562.36 μmol) and 37-2 (30 mg, 113.92 μmol) to react for 2 hours at room temperature and monitored by LC-MS, then concentrated under reduced pressure to remove solvents after the reaction. The residue was purified by preparative high-performance liquid chromatography (alkaline) to give the compound 37 (25 mg, 42.65 μmol). LCMS (ESI) m/z: 585.3 $[M+H]^+$, HPLC method A: $R_T$ = 5.4 min, purity: 96.4%. $^1$H NMR (600 MHz, Chloroform-*d*) δ 8.74 (s, 1H), 7.82 (s, 1H), 7.71 (d, *J* = 7.8 Hz, 1H), 7.36 (s, 2H), 7.26 (d, *J* = 7.2 Hz, 1H), 6.76 (s, 2H), 5.67 - 5.60 (m, 1H), 4.97 (d, *J* = 10.2 Hz, 1H), 4.87 (d, *J* = 17.4 Hz, 1H), 4.67 (d, *J* = 6.0 Hz, 2H), 4.55 - 4.74 (m, 2H), 3.94 (brs, 1H), 3.57 - 3.55 (m, 2H), 3.10 - 2.93 (m, 4H), 2.70 - 2.51 (m, 4H), 2.12 (s, 2H), 1.67 (s, 2H), 1.51 (s, 6H), 1.25 (brs, 1H).

**Example 38: Synthesis of Compound 38:**

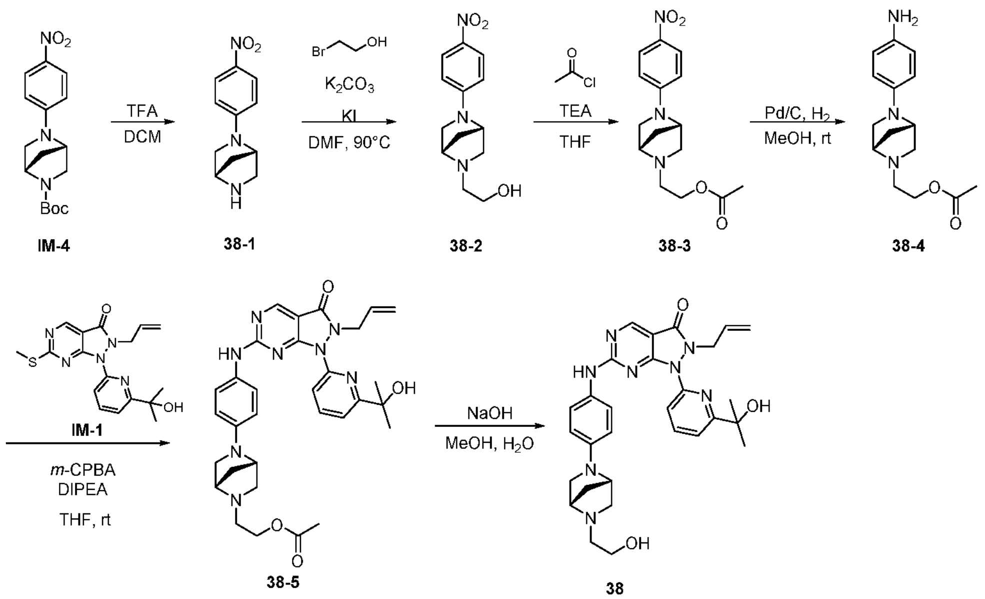


IM-4 38-1 38-2 38-3 38-4

38-5 38

**Step 1: Synthesis of Compound 38-1:**

The substrate **IM-4** (500 mg, 1.57 mmol) was added to a dry single-necked flask, dissolved in dichloromethane (3ml), then added with trifluoroacetic acid (1 ml) to react for 1 hour at room temperature and monitored by LC-MS, the solvent was removed after the reaction by distillation under reduced pressure to give the compound **38-1** (330 mg, 1.51 mmol). LCMS (ESI) m/z: 220.1 $[M+H]^+$.

**Step 2: Synthesis of Compound 38-2:**

The substrate **38-1** (100 mg, 456.12 μmol) was added to a dry single-necked flask, dissolved in N,N-dimethylformamide (2mL) , then added with 2-Bromoethanol (57.00 mg, 456.12 μmol), $K_2CO_3$ (94.55 mg, 684.18 μmol) and KI (15.14 mg, 91.22 μmol) to react for 2 hours at 90°C and monitored by LC-MS, then the reaction solution was cooled to room temperature after the reaction, added with water, filtered and washed with methanol. The organic phase was concentrated under reduced pressure to give the compound **38-2** (93 mg, 353 μmol). LCMS (ESI) m/z: 264.1 $[M+H]^+$.

**Step 3: Synthesis of Compound 38-3:**

The substrate **38-2** (93 mg, 353.22 μmol) was added under $N_2$ protection to a dry single-necked flask, dissolved in tetrahydrofuran (2.5 mL), then added with triethylamine (142.97 mg, 1.41 mmol), lowered to 0 °C and added with acetyl chloride (41.59 mg, 529.83 μmol) , then slowly heated to room temperature and stirred for 1 hour, monitored by LC-MS, then concentrated under reduced pressure after the reaction to give the compound **38-3** (96 mg, 314.8 μmol). LCMS (ESI) m/z: 292.1 $[M+H]^+$.

**Step 4: Synthesis of Compound 38-4:**

The substrate **38-3** (277 mg, 907.22 μmol) was added to a 50mL round-bottomed flask, dissolved in methanol (3mL), added with a catalytic amount of Pd/C (30mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **38-4** (177 mg, 642 μmol). LCMS (ESI) m/z: 262.2 $[M+H]^+$.

**Step 5: Synthesis of Compound 38-5:**

The substrate **IM-1** (40 mg, 111.91 μmol) was added to a dry single-necked flask, and added with tetrahydrofuran (1mL) to dissolve, then added with M-chloroperoxybenzoic acid (38.62 mg, 223.82 μmol) to react for 30 min at room temperature, then N,N-diisopropylethylamine (72.32 mg, 559.55 μmol) and 38-4 (30.81 mg, 111.91 μmol) were added to the reaction solution to react for 4 h at room temperature and monitored by LC-MS, then concentrated under reduced pressure to give the crude product **38-5** (300 mg, crude). LCMS (ESI) m/z: 571.3 $[M+H]^+$.

**Step 6: Synthesis of Compound 38:**

The substrate **38-5** (300 mg, crude) was added to a dry single-necked flask, and added with methanol (3mL) to dissolve, then added with NaOH (41.05 mg, 1.03 mmol) and water (0.2 mL) to react for 30 min at room temperature and monitored by LC-MS, then concentrated under reduced pressure after the reaction, diluted with water and extracted three times with ethyl acetate. The crude product was purified by preparative high-performance liquid chromatography (alkaline) to give the compound **38** (10.36 mg, 19.09 μmol). LCMS (ESI) m/z: 543.2 $[M+H]^+$. HPLC method A: $R_T$=4.82 min, purity: 100.0%. $^1$H NMR (600 MHz, DMSO-$d_6$) δ 10.04 (s, 1H), 8.79 (s, 1H), 8.02 - 7.99 (m, 1H), 7.74 (s, 1H), 7.59 (d, *J* =7.2 Hz, 1H), 7.49 (s, 2H), 6.55 (d, *J* =7.8 Hz, 2H), 5.67 - 5.63 (m, 1H), 5.32 (s, 1H), 4.99 (d, *J* =10.2 Hz, 1H), 4.82 (d, *J* =17.4 Hz, 1H), 4.69 - 4.67 (m, 2H), 4.37-4.35 (m, 1H), 4.26 (s, 1H), 3.54 (s, 1H), 3.38 - 3.36 (m, 3H), 3.14 (d, *J* =9.0 Hz, 1H), 2.87 (d, *J* =9.0 Hz, 1H), 2.48 - 2.46 (m, 2H), 1.83 - 1.77 (m, 2H), 1.46 (s, 6H).

**Example 39: Synthesis of Compound 39:**

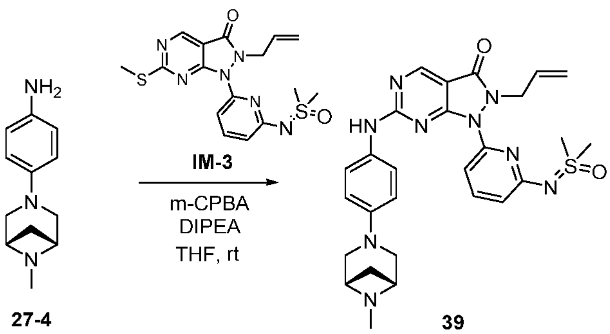


27-4 39

**Step 1: Synthesis of Compound 39:**

The substrate IM-3 (30.49 mg, 150.00 μmol) was added to a dry single-necked flask, added with tetrahydrofuran (1mL) to dissolve, added with m-Chloroperoxybenzoic acid (25.88 mg, 150.00 μmol) to react for 30 min at room temperature, then added with N,N-diisopropylethylamine (19.39 mg, 150.00 μmol) and 27-4 (30.49 mg, 150.00 μmol) to react for 4 hours at room temperature and monitored by LC-MS, then concentrated under reduced pressure after the reaction. The residue was purified by preparative high-performance liquid chromatography (alkaline) to give the compound **39** (26 mg, 38.83 μmol). LCMS(ESI) m/z: 546.2$[M+H]^+$,HPLC Method B $R_T$= 5.93 min, purity > 81.5 %. $^1$H NMR (600 MHz, DMSO-$d_6$) δ 10.07 (s, 1H), 8.78 (s, 1H), 7.78 (s, 1H), 7.61 (s, 2H), 7.32 (d, *J* = 7.8 Hz, 1H), 6.71 (d, *J* = 7.8 Hz, 2H), 6.59 (d, *J* = 8.4 Hz, 1H), 5.68 - 5.60 (m, 1H), 5.00 (d, *J* = 10.2 Hz, 1H), 4.90 (d, *J* = 17.4 Hz, 1H), 4.75 (s, 2H), 3.60 (s, 2H), 3.44 (*d, J* = 11.4 Hz, 2H), 3.38 (s, 6H), 3.35 (d, *J* = 8.4 Hz, 2H), 2.44 (s, 1H), 2.00 (s, 3H), 1.55 - 1.53 (m, 1H).

**Example 40: Synthesis of Compound 40:**

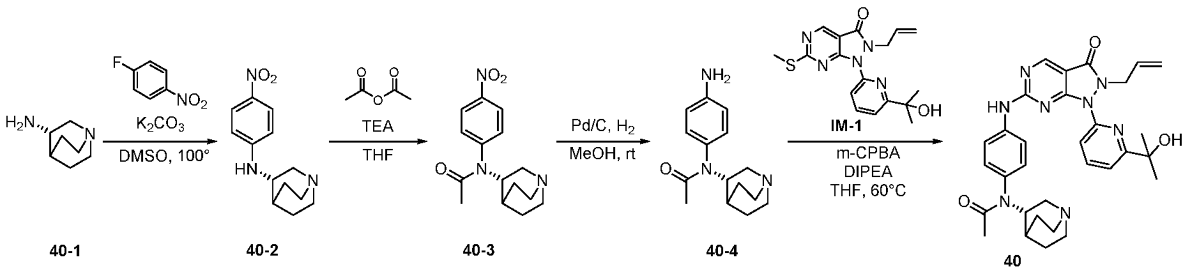


40-1 40-2 40-3 40-4 40

**Step 1: Synthesis of Compound 40-2:**

The substrate **40-1** (0.4 g, 2 mmol) was added to a dry single-necked flask, dissolved in dimethyl sulfoxide (15mL) , then added sequentially with p-Fluoronitrobenzene (0.28 g, 2 mmol) and $K_2CO_3$ (1.11g, 4 mmol). The reaction mixture was stirred overnight at 100°C and monitored by LC-MS, then diluted with water after the reaction, extracted three times with ethyl acetate. The organic phase was dried with anhydrous $Na_2SO_4$, then concentrated under reduced pressure. The residue was purified by column chromatography to give the compound **40-2** (0.63 g, 2.56 mmol). LCMS (ESI) m/z: 248.1$[M+H]^+$.

**Step 2: Synthesis of Compound 40-3:**

The substrate **40-2** (71.4 mg, 300 μmol) was added under $N_2$ protection to a dry single-necked flask, dissolved in tetrahydrofuran (2.5 mL), then added with triethylamine (121.4 mg, 1.2 mmol), lowered to 0 °C and added with acetic anhydride (61.2 mg, 600 μmol), then slowly heated to room temperature and stirred for 12 hours, monitored by LC-MS, then concentrated under reduced pressure after the reaction. The resude was purified by MPLC to give the compound **40-3** (25.3 mg, 87.4 μmol). LCMS (ESI) m/z: 290.2 $[M+H]^+$.

**Step 3: Synthesis of Compound 40-4:**

The substrate **40-3** (173 mg,598 μmol) was added to a 50mL round-bottomed flask, dissolved in methanol (5mL), added with a catalytic amount of Pd/C (18mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **40-4** (122 mg, 470 μmol). LCMS (ESI) m/z: 260.2 $[M+H]^+$.

**Step 4: Synthesis of Compound 40:**

The substrate **IM-1** (35.7 mg, 100 μmol) was added to a dry single-necked flask, added with tetrahydrofuran (1mL) to dissolve, added with m-Chloroperoxybenzoic acid (25.85 mg, 150 μmol) to react for 30 min at room temperature, then added with N,N-diisopropylethylamine (64.54 mg, 500 μmol) and 40-4 (51.81 mg, 0.2 mmol), heated up to 60°C to react for 4 hours and monitored by LC-MS, then concentrated under reduced pressure after the reaction. The residue was purified by preparative high-performance liquid chromatography (alkaline) to give the compound **40** (18 mg, 31.65 μmol). LCMS(ESI) m/z: 569.3$[M+H]^+$,HPLC Method B Rt = 6.28 min, purity > 63.4 %.$^1$H NMR (600 MHz, DMSO-$d_6$) δ 10.14 (s, 1H), 8.82 (s, 1H), 8.04 (s, 1H), 7.89 (s, 1H), 7.75 (s, 1H), 7.67 - 7.47 (m, 3H), 6.93 (d, *J* = 8.4 Hz, 2H), 5.68 - 5.64 (m, 1H), 5.32 (s, 1H), 4.99 (d, *J* = 10.2 Hz, 1H), 4.82 (d, *J* = 17.4 Hz, 1H), 4.68 (s, 2H), 4.37 - 4.35 (m, 1H), 4.13 - 4.06 (m, 1H), 3.69 (s, 2H), 3.62 (d, *J* = 7.8 Hz, 1H), 2.66 - 2.55 (m, 3H), 2.39 (s, 1H), 1.79 (d, J= 13.2 Hz, 1H), 1.59 (d, *J* = 13.2 Hz, 1H), 1.46 (s, 6H), 1.45 - 1.42 (m, 1H), 1.36 - 1.24 (m, 2H).

**Example 41: Synthesis of Compound 41:**

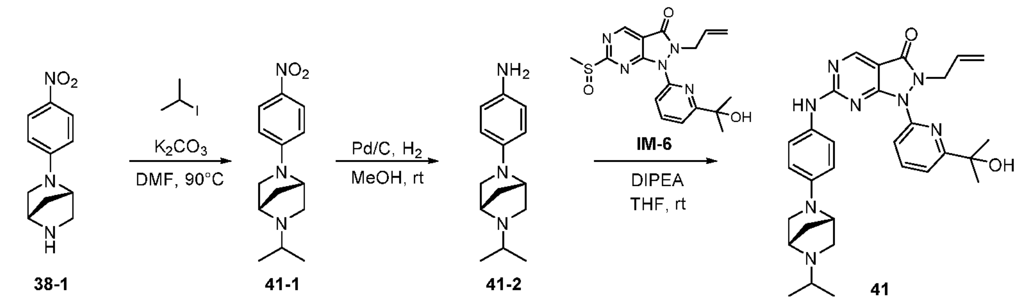


38-1 41-1 41-2 41

**Step 1: Synthesis of Compound 41-1:**

The substrate **38-1** (140 mg, 638.57 μmol) was added to a dry single-necked flask, dissolved in N,N-Dimethylformamide (3mL), then added with 2-Iodopropane (131 mg, 770.63 μmol) and $K_2CO_3$ (124 mg, 897.25 μmol) to react for 3 hours at 90°C and monitored by LC-MS. The reaction solution was cooled to room temperature after the reaction, added with water and extracted three times with ethyl acetate. The organic phase was dried with anhydrous $Na_2SO_4$, filtered, and concentrated under reduced pressure. The residue was purified by column chromatography to give the compound **41-1** (80 mg, 306.14 μmol). LCMS (ESI) m/z: 262.2 $[M+H]^+$.

**Step 2: Synthesis of Compound 41-2:**

The substrate **41-1** (80 mg, 306.14 μmol) was added to a 50mL round-bottomed flask, dissolved in methanol (3mL), added with a catalytic amount of Pd/C (20mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **41-2** (60 mg, crude). LCMS (ESI) m/z: 232.2 $[M+H]^+$ .

**Step 3: Synthesis of Compound 41:**

The substrate **IM-6** (45 mg, 120.50 μmol) and tetrahydrofuran (3mL) were added to a dry single-necked flask, stirred to dissolve, then added with N,N-diisopropylethylamine (77.87 mg, 602.52 μmol) and 41-2 (30 mg, 129.68 μmol) to react for 2 hours at room temperature and monitored by LC-MS, then concentrated under reduced pressure to remove solvents after the reaction. The residue was purified by preparative high-performance liquid chromatography (alkaline) to give the compound **41** (20 mg, 36.99 μmol). LCMS (ESI) m/z: 541.3 $[M+H]^+$, HPLC method A: $R_T$ = 5.1 min, purity: 95.3%. $^1$H NMR (600 MHz, DMSO-$d_6$) δ 10.05 (s, 1H), 8.79 (s, 1H), 8.02 - 7.99 (m, 1H), 7.78 - 7.75 (m, 1H), 7.59 (d, *J* = 7.8 Hz, 1H), 7.55 - 7.41 (m, 2H), 6.57 (d, *J* = 7.8 Hz, 2H), 5.69 - 5.63 (m, 1H), 5.33 (s, 1H), 4.99 (d, *J* = 10.2 Hz, 1H), 4.84 (d, *J* = 16.8 Hz, 1H), 4.68 (s, 2H), 4.26 (s, 1H), 3.71 (s, 1H), 3.32 - 3.30 (m, 1H), 3.16 (d, *J* = 9.0 Hz, 1H), 3.01 (d, *J* = 7.8 Hz, 1H), 2.41 - 2.36 (m, 2H), 1.81 (s, 2H), 1.46 (s, 6H), 0.98 - 0.93 (m, 3H). $D_2O$: $^1$H NMR (600 MHz, DMSO-$d_6$) δ 8.80 (s, 1H), 8.03 - 7.99 (m, 1H), 7.73 (s, 1H), 7.60 (d, *J* = 7.2 Hz, 1H), 7.49 - 7.37 (m, 2H), 6.59 (d, *J* = 7.2 Hz, 2H), 5.69 - 5.63 (m, 1H), 5.02 (d, *J* = 10.2 Hz, 1H), 4.83 (d, *J* = 16.8 Hz, 1H), 4.68 (s, 2H), 4.26 (s, 1H), 3.78 (s, 1H), 3.32 (d, *J* = 7.8 Hz, 1H), 3.19 (d, *J* = 9.0 Hz, 1H), 3.03 (d, *J* = 9.0 Hz, 1H), 2.42 - 2.35 (m, 2H), 1.84 (d, *J* = 6.6 Hz, 2H), 1.47 (s, 6H), 1.0 - 0.94 (m, 6H).

**Example 42: Synthesis of Compound 42:**

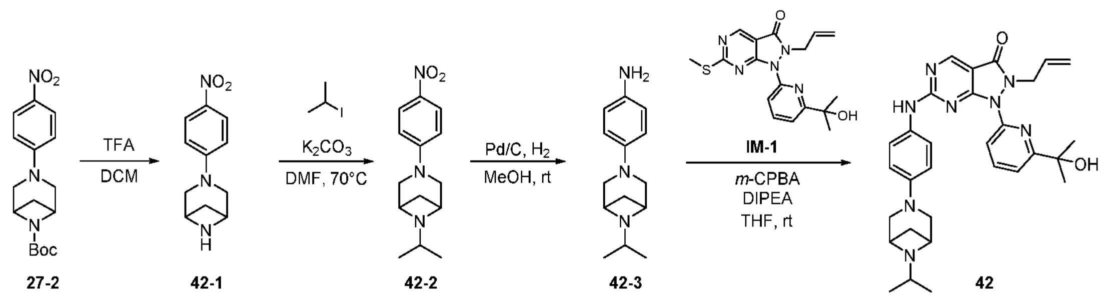


27-2 42-1 42-2 42-3 42

**Step** 1: **Synthesis of Compound 42-1:**

The compound **27-2** (330 mg, 1.03 mmol) was added to a dry single-necked flask, dissolved in dichloromethane (5ml), then added with trifluoroacetic acid (2ml) to react for 30 min at room temperature and monitored by LC-MS. After the reaction, the solvents were removed by distillation under reduced pressure to give the compound **42-1** (280 mg, crude).LCMS (ESI) m/z: 220.1 $[M+H]^+$.

**Step 2**: **Synthesis of Compound 42-2:**

The substrate **42-1** (300 mg, 1.37 mmol) was added to a dry single-necked flask, dissolved in N,N-Dimethylformamide (3mL), then added with 2-Iodopropane (348.92 mg, 2.05 mmol) and $K_2CO_3$ (567.35 mg, 4.11 mmol) to react for 12 hours at 70°C and monitored by LC-MS. The reaction solution was cooled to room temperature after the reaction, added with water and extracted three times with ethyl acetate. The organic phase was dried with anhydrous $Na_2SO_4$, filtered, and concentrated under reduced pressure. The residue was purified by column chromatography to give the compound **42-2** (280 mg, 1.07 mmol). LCMS (ESI) m/z: 262.2 $[M+H]^+$.

**Step 3: Synthesis of Compound 42-3:**

The substrate **42-2** (0.8 g, 3.06 mmol) was added to a 50mL round-bottomed flask, dissolved in methanol (10mL), added with a catalytic amount of Pd/C (20mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **42-3** (500 mg, 2.16 mmol). LCMS (ESI) m/z: 232.2 $[M+H]^+$.

**Step 4: Synthesis of Compound 42:**

The substrate **IM-1** (42.89 mg, 120.00 μmol) was added to a dry single-necked flask, and added with tetrahydrofuran (1mL) to dissolve, then added with M-chloroperoxybenzoic acid (31.07 mg, 180.00 μmol) to react for 30 min at room temperature, then N,N-diisopropylethylamine (77.54 mg, 600.00 μmol) and 42-3 (33.31 mg, 144.00 μmol) were added to the reaction solution to react for 4 hours at room temperature and monitored by LC-MS, then concentrated under reduced pressure after the reaction. The residue was purified by preparative high-performance liquid chromatography (alkaline) to give the compound **42** (30 mg, 51.77 μmol). LCMS(ESI) m/z: 541.4$[M+H]^+$,HPLC Method B $R_T$ = 7.02 min, purity >93.3 %. $^1H$ NMR (600 MHz, DMSO-$d_6$) δ 10.09 (s, 1H), 8.80 (s, 1H), 8.03 (s, 1H), 7.76 (s, 1H), 7.60 - 7.47 (m, 3H), 6.69 (d, *J* = 8.4 Hz, 2H), 5.68 - 5.64 (m, 1H), 5.31 (s, 1H), 5.00 - 4.98 (m, 1H), 4.86 - 4.79 (m, 1H), 4.68 (d, *J* = 5.4 Hz, 2H), 3.71 (d, *J* = 6.0 Hz, 2H), 3.42 (d, *J* = 11.2 Hz, 2H), 3.24 (d, *J* = 10.8 Hz, 2H), 2.50 - 2.43 (m, 1H), 2.37 - 2.35 (m, 1H), 1.50 (d, *J* = 8.4 Hz, 1H), 1.46 (s, 6H), 0.88 (d, *J* = 6.0 Hz, 6H).

**Example 43: Synthesis of Compound 43:**

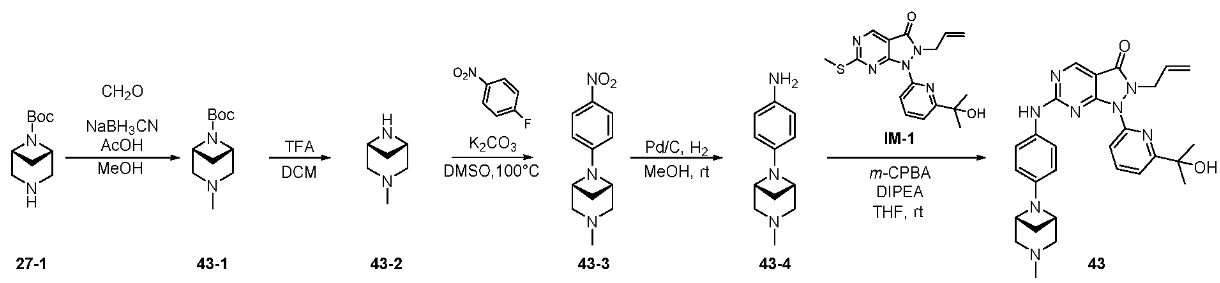


**27-1** **43-1** **43-2** **43-3** **43-4** **43**

**Step 1: Synthesis of Compound 43-1:**

The substrate **27-1** (0.33 g, 1.66 mmol) was added to a dry single-necked flask and dissolved in methanol (4mL), added with formaldehyde (226.77 mg, 6.66 mmol) and acetic acid (0.5 mL), stirred for 10 min at room temperature, then added with Sodium cyanoborohydride (1.41 g, 6.66 mmol) to react for 12 hours at room temperature, diluted with water after the reaction, extracted three times with dichloromethane, and the organic phase was dried with anhydrous $Na_2SO_4$, filtered and concentrated under reduced pressure. The residue was purified by column chromatography to give the

compound **43-1** (300 mg, 1.41 mmol). LCMS (ESI) m/z: 213.2[M+H]+.

**Step 2: Synthesis of Compound 43-2:**

The compound **43-1** (300 mg, 1.41 mmol) was added to a dry single-necked flask and dissolved in dichloromethane (3 ml), added with trifluoroacetic acid (1ml) to react for 30 min at room temperature and monitered by LC-MS. The solvents were removed by distillation under reduced pressure after the disappearance of the raw materials to give the compound **43-2** (0.12 g, 1.07 mmol).

**Step 3: Synthesis of Compound 43-3:**

The compound **43-2** (0.22 g, 1.96 mmol) was added in a dry single-necked flask and dissolved in dimethyl sulfoxide (5 ml), added sequentially with p-fluoronitrobenzene (304.41 mg, 2.16 mmol) and $K_2CO_3$ (1.36 g, 9.81 mmol). The reaction mixture was stirred for 3 hours at 100°Cand monitored by LC-MS, then diluted with water after the reaction and extracted three times with ethyl acetate. The organic phase was dried with anhydrous $Na_2SO_4$, filtered, concentrated under reduced pressure. The residue was purified by column chromatography to give the compound **43-3** (260 mg, 1.11 mmol). LCMS (ESI) m/z: 234.1[M+H]+.

**Step 4: Synthesis of Compound 43-4:**

The compound **43-3** (0.3 g, 1.29 mmol) was added to a 50mL round-bottomed flask, dissolved in methanol (5mL), added with a catalytic amount of Pd/C (15mg), stirred for 3 hours at room temperature under $H_2$ atmosphere and monitored by LC-MS. After the reaction, the reacton solution was filtered with diatomaceous earth, washed two times with methanol, the filterate was concentrated under reduced pressure to give the compound **43-4** (200 mg, 983.85 μmol). LCMS (ESI) m/z: 204.2 [M+H]+ .

**Step 5: Synthesis of Compound 43:**

The substrate **IM-1** (42.89 mg, 120.00 μmol) was added to a dry single-necked flask, and added with tetrahydrofuran (1mL) to dissolve, then added with M-chloroperoxybenzoic acid (31.07 mg, 180.00 μmol) to react for 30 min at room temperature, then N,N-diisopropylethylamine (77.54 mg, 600.00 μmol) and 43-4 (29.27 mg, 144.00 μmol) were added to the reaction solution to react for 4 hours at room temperature and monitored by LC-MS, then concentrated under reduced pressure after the reaction. The residue was purified by preparative high-performance liquid chromatography (alkaline) to give the compound **43** (32 mg, 55.12 μmol). LCMS(ESI) m/z: 513.2[M+H]+, HPLC Method B $R_T$ = 6.47 min, purity > 88.3%. $^1$H NMR (600 MHz, DMSO-$d_6$) δ 10.05 (s, 1H), 8.80 (s, 1H), 7.99 - 7.97 (m, 1H), 7.74 (s, 1H), 7.60 - 7.58 (m, 1H), 7.48 (s, 2H), 6.45 (d, *J*= 8.4 Hz, 2H), 5.64 - 5.63 (m, 1H), 5.31 (s, 1H), 4.99 (d, *J* = 10.2 Hz, 1H), 4.83 (d, *J* = 17.4 Hz, 1H), 4.68 (d,*J* = 5.6 Hz, 2H), 4.23 (d, *J* = 5.6 Hz, 2H), 2.97 (d, *J* = 10.8 Hz, 2H), 2.71 (d, *J* = 11.4 Hz, 2H), 2.42 - 2.39(m, 1H), 2.08 (s, 3H), 1.96 (d, *J* = 7.2 Hz, 1H), 1.46 (s, 6H).

**Example 44: Synthesis of Compound 44:**

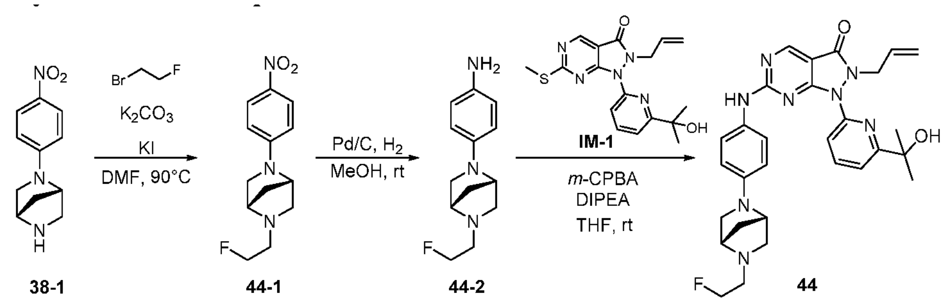


38-1 44-1 44-2 44

**Step 1: Synthesis of Compound 44-1:**

The substrate **38-1** (300 mg, 1.37 mmol) was added to a dry single-necked flask and dissolved in N,N-dimethylformamide (2 mL), then added with 1,2-Fluoroethane bromide (173.72 mg, 1.37 mmol), $K_2CO_3$ (378 mg,

2.74 mmol) and KI (45.43 mg, 273.67 μmol) to react overnight at 90°C and monitored by LC-MS, then the reaction solution was cooled to room temperature after the reaction, added with water, filtered, washed by methanol. The organic phase was concentrated under reduced pressure to give the compound **44-1** (320 mg, 1.21 mmol). LCMS (ESI) m/z: 266.1 $[M+H]^+$.

**Step 2: Synthesis of Compound 44-2:**

The compound **44-1** (320 mg, 1.21 mmol) was added to a round-bottomed flask, dissolved in methanol (5mL), added with a catalytic amount of Pd/C (30mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **44-2** (200 mg, 849.98 μmol). LCMS (ESI) m/z: 236.2 $[M+H]^+$.

**Step 3: Synthesis of Compound 44:**

The substrate **IM-1** (74 mg, 207.03 μmol) was added to a dry single-necked flask, and added with tetrahydrofuran (1mL) to dissolve, then added with M-chloroperoxybenzoic acid (90 mg, 414.07 μmol) to react for 30 min at room temperature, then N,N-diisopropylethylamine (133.79 mg, 1.04 mmol, 180.30 μL) and 44-2 (97.43 mg, 414.07 μmol) were added to the reaction solution to react for 4 h at room temperature and monitored by LC-MS, then concentrated under reduced pressure after the reaction. The residue was purified by preparative high-performance liquid chromatography (alkaline) to give the compound **44** (11.5 mg, 21.12 μmol). LCMS (ESI) m/z: 545.3 $[M+H]^+$, HPLC method A: $R_T$ = 4.95 min, purity: >84.1%. $^1H$ NMR (600 MHz, DMSO-$d_6$) δ 10.05 (s, 1H), 8.79 (s, 1H), 8.02 - 8.00 (m, 1H), 7.74 (s, 1H), 7.59 (d, *J* = 7.8 Hz, 1H), 7.50 (s, 2H), 6.57 (d, *J* = 8.4 Hz, 2H), 5.68 - 5.65 (m, 1H), 5.31 (s, 1H), 4.99 (d, *J* = 10.2, 1H), 4.82 (d, *J* = 17.4 Hz, 1H), 4.67 (s, 2H), 4.49 - 4.42 (m, 1H), 4.38 - 4.36 (m, 1H), 4.29 (s, 1H), 3.57 (s, 1H), 3.35 (d, *J* = 9.0 Hz, 1H), 3.12 (d, *J* = 9.0Hz, 1H), 2.90 (d, *J* = 9.6 Hz, 1H), 2.83 - 2.64 (m, 2H), 2.53 (d, *J* = 9.6 Hz, 1H), 1.84 (d, *J* = 9.6 Hz, 1H), 1.79 (d, *J* = 9.0 Hz, 1H).

**Example 45: Synthesis of Compound 45:**

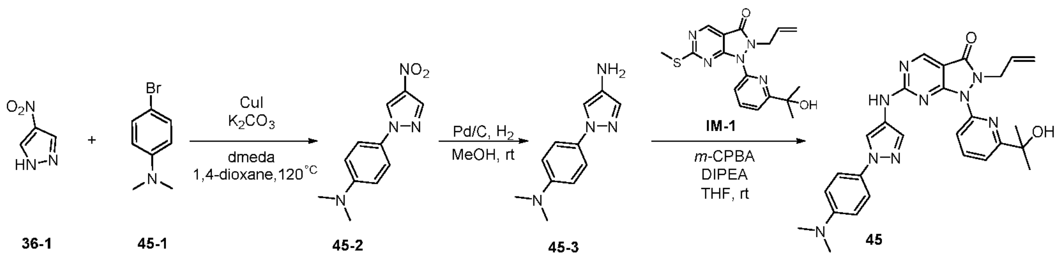


36-1 45-1 45-2 45-3 45

**Step 1: Synthesis of Compound 45-2:**

The substrate **36-1** (1.13 g, 9.99 mmol), 45-1 (2.20 g, 10.99 mmol), CuI(1.90 g, 9.99 mmol), $K_2CO_3$ (1.38 g, 9.99 mmol) and N,N'-Dimethylethylenediamine (880.93 mg, 9.99 mmol) were added to a dry microwave tube under $N_2$ protection, then added with 1,4-Dioxane (15mL), heated up to 120°C to react for 3 hours under $N_2$ atmosphere and monitored by LC-MS. After the reaction was completed, the reaction system was cooled to room temperature, filtered, extracted three times with ethyl acetate, the organic phase was dried with anhydrous $Na_2SO_4$, filtered, concentrated under reduced pressure. The residue was purified by MPLC to give the compound **45-2** (0.8 g, 3.44 mmol). LCMS (ESI) m/z:233.1 $[M+H]^+$.

**Step 2: Synthesis of Compound 45-3:**

The compound **45-2** (800 mg, 3.44 mmol) was added to a 50 mL round-bottomed flask, dissolved in methanol (10mL), added with a catalytic amount of Pd/C (40mg), stirred for 3 hours at room temperature under $H_2$ atmosphere and monitored by LC-MS. After the reaction, the reacton solution was filtered with diatomaceous earth, washed two times with methanol, the filterate was concentrated under reduced pressure to give the compound **45-3** (500 mg, 2.47 mmol). LCMS (ESI) m/z: 203.1 $[M+H]^+$.

**Step 3: Synthesis of Compound 45:**

The substrate **IM-1** (39 mg, 109.11 μmol) was added to a dry single-necked flask, and added with tetrahydrofuran (1mL) to dissolve, then added with M-chloroperoxybenzoic acid (28.25 mg, 163.67 μmol) to react for 30 min at room temperature, then N,N-diisopropylethylamine (70.51 mg, 545.56 μmol, 95.02 μL) and **45-3** (33.10 mg, 163.67 μmol) were added to the reaction solution to react for 4 hours at room temperature and monitored by LC-MS, then concentrated under reduced pressure after the reaction. The residue was purified by preparative high-performance liquid chromatography (alkaline) to give the compound **45** (32 mg, 62.55 μmol). LCMS(ESI) m/z: 512.2[M+H]$^+$, HPLC Method B $R_T$ = 7.60 min, purity > 100.0%. $^1$H NMR (600 MHz, DMSO-$d_6$) δ 10.47 (s, 1H), 8.85 (s, 1H), 8.41 (s, 1H), 8.04 - 8.01 (m, 1H), 7.88 (d, *J* = 7.8 Hz, 1H), 7.79 - 7.68 (m, 2H), 7.52 (d, *J* = 9.0 Hz, 2H), 6.88 (d, *J* = 8.4 Hz, 2H), 5.71 - 5.64 (m, 1H), 5.34 (s, 1H), 5.00 (d, *J* = 10.2 Hz, 1H), 4.85 - 4.77 (m, 1H), 4.65 (d, *J* = 6.0 Hz, 2H), 2.97 (s, 6H), 1.46 (s, 6H).

**Example 46: Synthesis of Compound 46:**

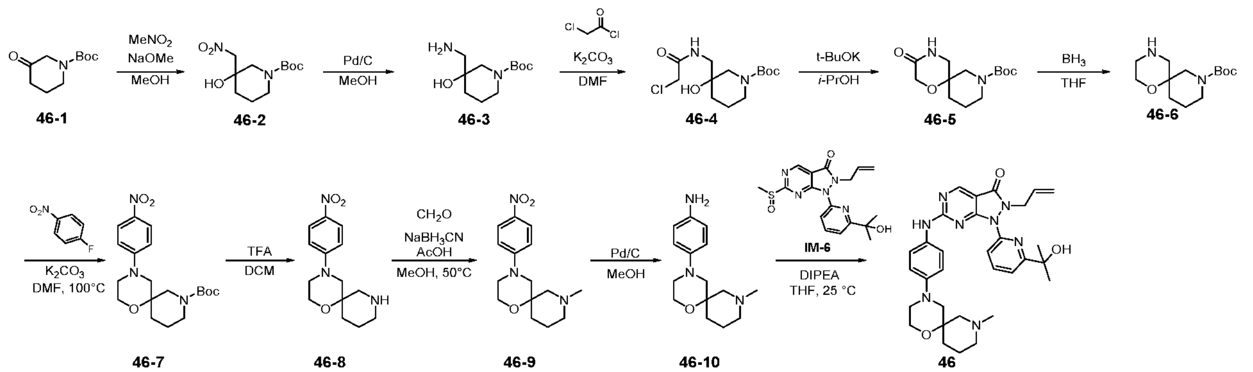


**Step 1: Synthesis of Compound 46-2:**

CHsONa (297 mg, 5.52 mmol), $CH_3NO_2$ (306.35 mg, 5.02 mmol) and methanol (8 mL) were added to a dry single-necked flask. The reaction mixture was stirred for 30 min at room temperature, then added with **46-1** (1 g, 5.02 mmol), stirred overnight at room temperature, concentrated under reduced pressure after the reaction and the pH was adjusted dropwise with acetic acid to 7.0, then extracted three times with dichloromethane. The organic phase was dried with anhydrous $Na_2SO_4$, filtered, concentrated under reduced pressure. The residue was purified by column chromatography to give the compounds **46-2** (640 mg, 2.46 mmol).

**Step 2: Synthesis of Compound 46-3:**

The compound **46-2** (100 mg, 384.19 μmol) was added to a 50 mL round-bottomed flask, dissolved in methanol (10mL), added with a catalytic amount of Pd/C (40mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **46-3** (80 mg, 347.4 μmol). LCMS (ESI) m/z: 236.2 [M+H]$^+$ .

**Step 3: Synthesis of Compound 46-4:**

The substrate **46-3** (1 g, 4.34 mmol) was added to a dry single-necked flask and dissolved in N,N-dimethylformamide (10mL) , then added with $K_2CO_3$ (1.2 g, 8.68 mmol) and Chloroacetyl chloride (540.40 mg, 4.78 mmol) to react for 5 hours at room temperature, diluted with water after the reaction, extracted three times with ethyl acetate. The organic phase was dried with anhydrous $Na_2SO_4$, filtered, concentrated under reduced pressure. The residue was purified by column chromatography to give the compound **46-4** (480 mg, 1.56 mmol). LCMS (ESI) m/z: 266.1 [M+H]$^+$.

**Step 4: Synthesis of Compound 46-5:**

The substrate **46-4** (60 mg, 195.58 μmol) was added to a dry single-necked flask and dissolved in isopropanol (6mL) , then added with t-BuOK (112 mg, 998.13 μmol), stirred for 3 hours at room temperature, concentrated under

reduced pressure after the reaction and extracted three times with ethyl acetate. The organic phase was dried with anhydrous $Na_2SO_4$, filtered, concentrated under reduced pressure to give the crude product **46-5** (50 mg, crude). LCMS (ESI) m/z: 215.1 $[M+H]^+$.

**Step 5: Synthesis of Compound 46-6:**

The substrate **46-5** (26 mg, 96.18 μmol) was added to a dry single-necked flask, and dissolved in tetrahydrofuran (4.4mL), then added with 2M Borane tetrahydrofuran solution (0.19 mL, 384.72 μmol), stirred for 2 hours at 70°C, concentrated under reduced pressure after the reaction, added with methanol and N,N,N',N'-Tetramethylethylenediamine, stirred overnight at 78 °C , then concentrated under reduced pressure after the reaction and extracted three times with ethyl acetate. The organic phase was dried with anhydrous $Na_2SO_4$, filtered, concentrated under reduced pressure to give the crude product **46-6** (25 mg, crude). LCMS (ESI) m/z: 257.2 $[M+H]^+$.

**Step 6: Synthesis of Compound 46-7:**

The compound **46-6** (25 mg, 97.53 μmol) was added in a dry single-necked flask and dissolved in dimethyl sulfoxide (2 ml), added sequentially with p-fluoronitrobenzene (13.76 mg, 97.53 μmol) and $K_2CO_3$ (19 mg, 137.48 μmol). The reaction mixture was stirred for 5 hours at 100°C and monitored by LC-MS, then diluted with water after the reaction and extracted three times with ethyl acetate. The organic phase was dried with anhydrous $Na_2SO_4$, filtered, concentrated under reduced pressure. The residue was purified by column chromatography to give the compound **46-7** (30 mg, 79.48 μmol). LCMS (ESI) m/z:234.1$[M+H]^+$.

**Step 7: Synthesis of Compound 46-8:**

The compound **46-7** (18 mg, 47.69 μmol) was added to a dry single-necked flask and dissolved in dichloromethane (1 ml) , added with trifluoroacetic acid (1ml) to react for 30 min at room temperature and monitered by LC-MS. The solvents were removed by distillation under reduced pressure after the disappearance of the raw materials to give the compound **46-8** (13 mg, crude).

**Step 8: Synthesis of Compound 46-9:**

The substrate **46-8** (13 mg, 46.88 μmol) was added to a dry single-necked flask and dissolved in methanol (2mL), added with formaldehyde (16 mg, 469.76 μmol) and acetic acid (50 μL), stirred for 10 min at room temperature, then added with Sodium cyanoborohydride (10 mg, 159.13 μmol) to react for 2 hours at 50°C, concentrated under reduced pressure after the reaction. The residue was purified by MPLC to give the compound **46-9** (10 mg, 34.32 μmol). LCMS (ESI) m/z: 262.2$[M+H]^+$.

**Step 9: Synthesis of Compound 46-10:**

The compound **46-9** (50 mg, 171.62 μmol) was added to a 50 mL round-bottomed flask, dissolved in methanol (3mL), added with a catalytic amount of Pd/C (15mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **46-10** (30 mg, 114.7 μmol). LCMS (ESI) m/z: 236.2 $[M+H]^+$ .

**Step 10: Synthesis of Compound 46:**

The substrate **IM-6** (42 mg, 112.47 μmol) and tetrahydrofuran (4mL) were added to a dry single-necked flask, stirred to dissolve, then N,N-diisopropylethylamine (72.68 mg, 562.36 μmol) and 46-10 (30 mg, 114.78 μmol) were added to react for 2 hours at room temperature and monitored by LC-MS, then the solvents were removed by distillation under reduced pressure after the reaction. The residue was purified by preparative high-performance liquid chromatography (alkaline) to give the compound **46** (32 mg, 56.07 μmol). LCMS (ESI) m/z: 571.2 $[M+H]^+$, HPLC method B: $R_T$ = 6.53 min, purity: 99.5%. $^1$H NMR (600 MHz, DMSO-$d_6$) δ 10.15 (s, 1H), 8.83 (s, 1H), 8.04 (s, 1H), 7.75 (d, *J* = 6.6 Hz, 1H), 7.62 - 7.59 (m, 3H), 6.91 (d, *J* = 8.4 Hz, 2H), 5.70-5.63 (m, 1H), 5.34 (s, 1H), 5.02 - 4.99 (m, 1H), 4.83 (d, *J* = 16.8 Hz, 1H), 4.68 (d, *J* = 5.4 Hz, 2H), 3.81 - 3.74 (m, 2H), 3.20 (d, *J* = 12.0 Hz, 1H), 3.16 - 3.14 (m, 1H), 2.99 - 2.96 (m, 1H), 2.89 (d, *J* = 12.0 Hz, 1H), 2.66 - 2.64 (m, 1H), 2.32 (s, 1H), 2.32 - 2.21 (m, 5H), 1.74 - 1.66 (m, 2H), 1.57 - 1.52 (m, 1H), 1.46 (s, 6H), 1.44 - 1.38 (m, 1H). $D_2O$:$^1$H NMR (600 MHz, DMSO-$d_6$) δ 8.83 (s, 1H), 8.05 (s, 1H), 7.74 (d, *J* = 7.2 Hz, 1H), 7.63 - 7.59 (m, 3H), 6.92 (d, *J* = 9.0 Hz, 2H), 5.70 - 5.63 (m, 1H), 5.02 (d, *J* = 10.2 Hz, 1H), 4.83 (d, *J* = 17.4 Hz, 1H), 4.69 (d, *J* = 5.4 Hz, 2H), 3.82 - 3.77 (m, 2H),

3.18 - 3.13 (m, 2H), 3.00 - 2.96 (m, 1H), 2.91 (d, *J* = 12.0 Hz, 1H), 2.67 - 2.64 (m, 1H), 2.51 - 2.44 (m, 2H), 2.35 - 2.26 (m, 4H), 1.71 - 1.67 (m, 2H), 1.57 - 1.54 (m, 1H), 1.50 - 1.47 (m, 7H).

**Example 47: Synthesis of Compound 47:**

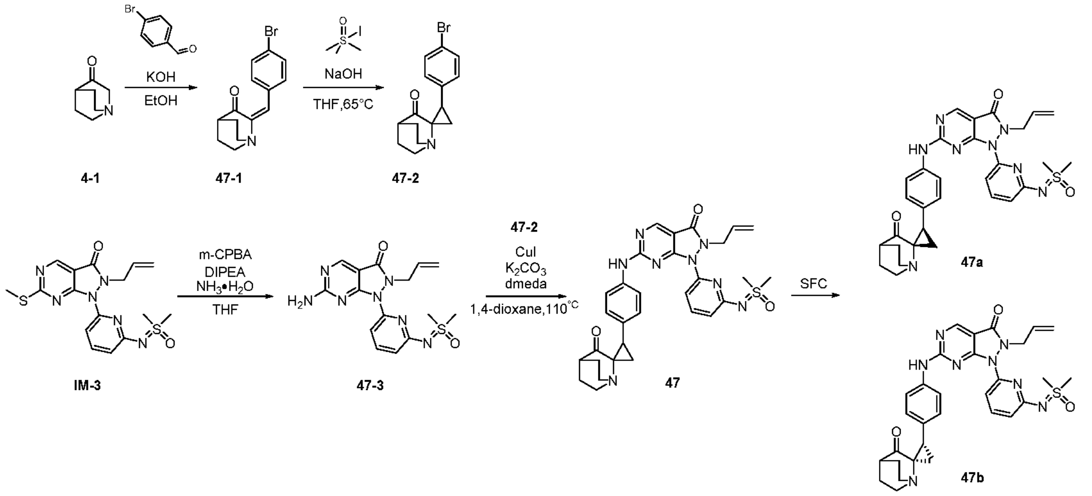

4-1 47-1 47-2
IM-3 47-3 47 47a 47b

**Step 1: Synthesis of Compound 47-1:**

The compound **4-1** (323 mg,2 mmol) was dissolved in ethanol (10 ml) in a dry single-necked flask, added sequentially with KOH (337 mg,6 mmol) and p-Bromo benzaldehyde (370 mg,2 mmol). The reaction mixture was stirred overnight at room temperature and monitored by LC-MS, then concentrated under reduced pressure after the reaction. The residue was purified by MPLC to give the compound **47-1** (456 mg, 1.6 mmol). LCMS (ESI) m/z: 292.1[M+H]$^+$.

**Step 2: Synthesis of Compound 47-2:**

Trimethylsulfoxonium iodide (1.6 g,8 mmol), NaOH (0.64 g,16 mmol) were dissolved in tetrahydrofuran (20 mL) in a single-necked flask, $N_2$ displaced to react for 1 hour at room temperature, then added with **47-1** (582 mg, 2 mmol), $N_2$ displaced again, stirred for 1 hour at 65°C and monitored by LC-MS, concentrated under reduced pressure after the reaction to remove solvents, then extracted three times with ethyl acetate and water respectively, the organic phase was dried with anhydrous $Na_2SO_4$, filtered, the residue was purified by column chromatography to give the compound **47-2** (284 mg,0.93 mmol). LCMS (ESI) m/z: 306.1[M+H]$^+$.

**Step 3: Synthesis of Compound 47-3:**

The substrate **IM-3** (78 mg, 0.2 mol) was added to a dry single-necked flask, then added with tetrahydrofuran (1mL) to dissolve, added with m-Chloroperoxybenzoic acid (52 mg, 0.3 mmol) to react for 30 min at room temperature, then N,N-diisopropylethylamine (129 mg, 1 mmol) and ammonia (0.1mL, 0.4 mmol) were added to the reaction solution to react for 2 hours at room temperature and monitored by LC-MS, then concentrated under reduced pressure after the reaction. The residue was purified by preparative high-performance liquid chromatography (alkaline) to give the compound **47-3** (58 mg, 0.16 mmol). LCMS (ESI) m/z: 585.2[M+H]$^+$.

**Step 4: Synthesis of Compound 47:**

The substrate **47-3** (58 mg, 0.16 mmol), **47-2** (70 mg, 0.23 mmol), CuI(61 mg, 320.29 μmol), $K_2CO_3$ (44.2 mg, 319.83 μmol) and N,N'-Dimethylethylenediamine (56 mg, 635.28 μmol) were added to a dry microwave tube under $N_2$ protection, then added with 1,4-Dioxane (4mL), heated up to 110°C to react for 6 hours under $N_2$ atmosphere and monitored by LC-MS. After the reaction was completed, the reaction system was cooled to room temperature, the solvents were removed under reduced pressure. The residue was purified by MPLC to give the compound 47 (61 mg, 104.33 μmol). LCMS (ESI) m/z: 585.2 [M+H]$^+$, HPLC method B: $R_T$ = 6.54 min, purity: 100%. $^1$H NMR (600 MHz, DMSO-$d_6$) δ 10.23 (s, 1H), 8.86 (s, 1H), 7.80 - 7.77 (m, 1H), 7.63 (d, *J* = 8.4 Hz, 2H), 7.30 - 7.25 (m, 3H), 6.61 (d, *J* = 8.0 Hz, 1H), 5.65 - 5.62 (m, 1H),

5.00 (d, $J$ = 10.2 Hz, 1H), 4.90 (d, $J$ = 17.4 Hz, 1H), 4.75 (d, $J$ = 6.0 Hz, 2H), 3.37 (s, 6H), 2.96 - 2.92 (m, 2H), 2.74 - 2.72 (m, 1H), 2.50 - 2.47 (m, 1H), 2.44 - 2.43 (m, 1H), 2.22 - 2.21 (m, 1H), 1.99 - 1.98 (m, 1H), 1.93 - 1.86 (m, 2H), 1.83 - 1.82 (m, 1H), 1.65 - 1.63 (m, 1H), 1.60 - 1.57 (m, 1H).

**Step 5: Synthesis of Compounds 47a and 47b:**

The compound **47** (20 mg) was split by SFC to give **47a** (6 mg), LCMS (ESI) m/z: 585.2 [M+H ]+, HPLC method B: RT=6.72 min, purity: 98.7%, and **47b** (8.6 mg), LCMS (ESI) m/z: 585.2 [M+H ]+, HPLC method B: RT=6.75 min, purity: 99.6%.

**Example 48: Synthesis of Compound 48:**

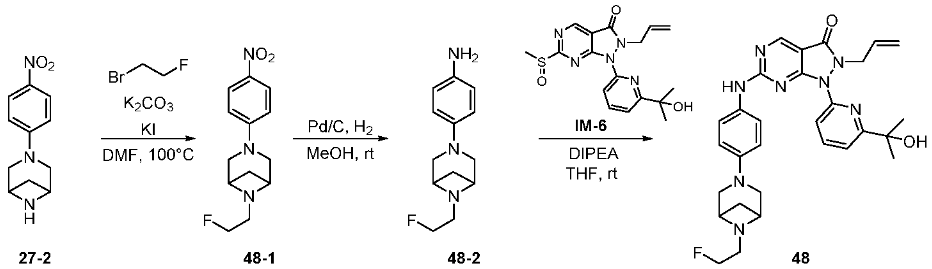


27-2 48-1 48-2 48

**Step 1: Synthesis of Compound 48-1:**

The substrate **27-2** (100 mg, 456.12 μmol) was added to a dry single-necked flask and dissolved in N,N-dimethylformamide (2 mL) , then added with 1-Bromo-2-fluoroethane (86.86 mg, 684.18 μmol), $K_2CO_3$ (88.25 mg, 638.57 μmol) and KI (8 mg, 48.19 μmol) to react for 7.5 hours at 100°C and monitored by LC-MS, then the reaction solution was cooled to room temperature after the reaction, extracted three times with ethyl acetate, the organic phase was dried with anhydrous $Na_2SO_4$, filtered, concentrated under reduced pressure to give the compound **48-1** (60 mg, 226.17 μmol). LCMS (ESI) m/z: 266.2 $[M+H]^+$.

**Step 2: Synthesis of Compound 48-2:**

The compound **48-1** (60 mg, 226.17 μmol) was added to a 50 mL round-bottomed flask and dissolved in methanol (5mL), added with a catalytic amount of Pd/C (15mg), stirred for 1 hours at room temperature under $H_2$ atmosphere and monitored by LC-MS. After the reaction, the reacton solution was filtered with diatomaceous earth, washed two times with methanol, the filterate was concentrated under reduced pressure to give the compound **48-2** (45 mg, crude). LCMS (ESI) m/z: 236.2 $[M+H]^+$.

**Step 3: Synthesis of Compound 48:**

The substrate **IM-6** (42 mg, 112.47 μmol) and tetrahydrofuran (4mL) were added to a dry single-necked flask, stirred to dissolve, then N,N-diisopropylethylamine (72.68 mg, 562.36 μmol) and 48-2 (42 mg, 112.47 μmol) were added to react for 4 hours at room temperature and monitored by LC-MS, then the solvents were removed by distillation under reduced pressure after the reaction. The residue was purified by preparative high-performance liquid chromatography (alkaline) to give the compound **48** (45 mg, 74.45 μmol). LCMS (ESI) m/z: 545.2 $[M+H]^+$, HPLC method B: $R_T$ = 6.81 min, purity: 90.1%. $^1$H NMR (600 MHz, Chloroform-*d*) δ 8.81 (s, 1H), 7.86 - 7.84 (m, 1H), 7.78 - 7.77 (m, 1H), 7.48 (s, 2H), 7.33 (d, $J$ = 7.8 Hz, 1H), 6.73 (d, $J$ = 9.0 Hz, 2H), 5.73 - 5.67 (m, 1H), 5.04 (d, $J$ = 10.2 Hz, 1H), 4.94 (d, $J$ = 16.8 Hz, 1H), 4.74 (d, $J$ = 6.0 Hz, 2H), 4.62 - 4.60 (m, 1H), 4.54 - 4.52 (m, 1H), 3.99 (s, 1H), 3.92 (d, $J$ = 4.8 Hz, 2H), 3.64 (d, J= 10.8 Hz, 2H), 3.39 (d, $J$ = 10.8 Hz, 2H), 2.78 - 2.70 (m, 3H), 1.68 (d, $J$ = 8.4 Hz, 1H), 1.58 (s, 6H).

**Example 49: Synthesis of Compound 49:**

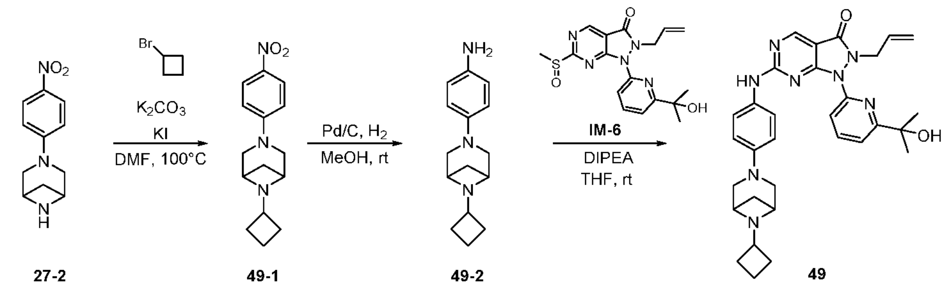


27-2 49-1 49-2 49

**Step 1: Synthesis of Compound 49-1:**

The substrate **27-2** (100 mg, 456.12 μmol) was added to a dry single-necked flask and dissolved in N,N-dimethylformamide (2 mL) , then added with Cyclobutyl bromide (92.37 mg, 684.18 μmol), $K_2CO_3$ (158 mg, 1.14 mmol) and KI (8 mg, 48.19 μmol) to react for 32 hours at 100°C and monitored by LC-MS, then the reaction solution was cooled to room temperature after the reaction, extracted three times with ethyl acetate, the organic phase was dried with anhydrous $Na_2SO_4$, filtered, concentrated under reduced pressure to give the compound **49-1** (32 mg, 117.07 μmol). LCMS (ESI) m/z: 274.2 $[M+H]^+$.

**Step 2: Synthesis of Compound 49-2:**

The compound **49-1** (32 mg, 117.07 μmol) was added to a 50 mL round-bottomed flask and dissolved in methanol (5mL), added with a catalytic amount of Pd/C (10mg), stirred for 1 hour at room temperature under $H_2$ atmosphere and monitored by LC-MS. After the reaction, the reacton solution was filtered with diatomaceous earth, washed two times with methanol, the filterate was concentrated under reduced pressure to give the compound **49-2** (28 mg, crude). LCMS (ESI) m/z: 244.1 $[M+H]^+$.

**Step 3: Synthesis of Compound 49:**

The substrate **IM-6** (35.7 mg, 95.60 μmol) and tetrahydrofuran (3mL) were added to a dry single-necked flask, stirred to dissolve, then N,N-diisopropylethylamine (61.78 mg, 478.00 μmol) and 49-2 (25.59 mg, 105.16 μmol) were added to react for 4 hours at room temperature and monitored by LC-MS, then the solvents were removed by distillation under reduced pressure after the reaction. The residue was purified by preparative high-performance liquid chromatography (alkaline) to give the compound **49** (20.8 mg, 33.31 μmol). LCMS (ESI) m/z: 553.2 $[M+H]^+$, HPLC method B: $R_T$ = 7.50 min, purity: 88.5 %. $^1H$ NMR (600 MHz, Chloroform-*d*) δ 8.82 (s, 1H), 7.86 - 7.84 (m, 1H), 7.78 - 7.77 (m, 1H), 7.48 (s, 2H), 7.33 (d, *J* = 7.8 Hz, 1H), 6.71 (d, *J* = 8.4 Hz, 2H), 5.74 - 5.67 (m, 1H), 5.04 (d, *J* = 10.2 Hz, 1H), 4.94 (d, *J* = 19.4 Hz, 1H), 4.67 (d, *J* = 5.4 Hz, 2H), 3.94 - 3.88 (m, 3H), 3.47 (d, *J* = 10.2 Hz, 2H), 3.35 (d, *J* = 10.2 Hz, 2H), 3.21 (s, 1H), 2.81 - 2.72 (m, 1H), 2.00 - 1.90 (m, 5H),1.78 - 1.74 (m, 1H), 1.69 - 1.64 (m, 1H), 1.58 (s, 6H).

**Example 50: Synthesis of Compound 50:**

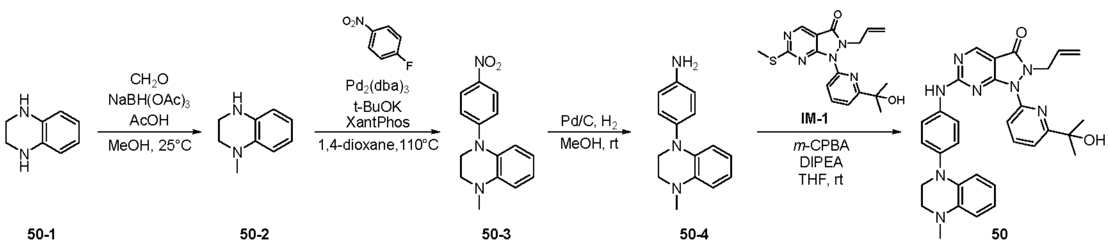


50-1 50-2 50-3 50-4 50

**Step 1: Synthesis of Compound 50-2:**

The substrate **50-1** (2 g, 14.91 mmol) was added to a dry single-necked flask and dissolved in methanol (40mL), added with formaldehyde (358.09 mg, 11.92 mmol) and acetic acid (89.51 mg, 1.49 mmol), stirred for 15 min at room temperature, then added with Sodium triacetoxyborohydride (15.80 g, 74.53 mmol) to react for 3 hours at room temperature, concentrated under reduced pressure after the reaction and extracted three times with dichloromethane.

The organic phase was dried with anhydrous $Na_2SO_4$, filtered, concentrated under reduced pressure. The residue was purified by MPLC to give the compound **50-2** (200 mg, 1.35 mmol). LCMS (ESI) m/z: 149.1[M+H]$^+$.

**Step 2: Synthesis of Compound 50-3:**

The substrate **50-2** (138 mg, 0.93 mmol), p-fluoronitrobenzene (282 mg, 1.4 mmol), $Pd_2(dba)_3$ (85 mg, 0.093 mmol), XantPhos (108 mg, 0.18 mmol), t-BuOK (358 mg, 3.72 mmol) and 1,4-Dioxane (3 mL) were added to a dry single-necked flask under $N_2$ protection. The reaction mixture was stiired for 3 hours at 110°C under $N_2$ atmosphere and monitored by LC-MS. The reaction system was cooled to room temperature after the reaction, extracted three times with ethyl acetate. The organic phase was dried with anhydrous $Na_2SO_4$, filtered, concentrated under reduced pressure. The residue was purified by column chromatography to give the compound **50-3** (20 mg, 0.074 mmol). LCMS (ESI) m/z: 270.1 [M+H]$^+$.

**Step 3: Synthesis of Compound 50-4:**

The compound **50-3** (20 mg, 74 μmol) was added to a 50 mL round-bottomed flask and dissolved in methanol (5mL), added with a catalytic amount of Pd/C (10mg), stirred for 1 hour at room temperature under $H_2$ atmosphere and monitored by LC-MS. After the reaction, the reacton solution was filtered with diatomaceous earth, washed two times with methanol, the filterate was concentrated under reduced pressure to give the compound **50-4** (7.80 mg, 32.59 μmol). LCMS (ESI) m/z: 240.2 [M+H]$^+$.

**Step 4: Synthesis of Compound 50:**

The substrate **IM-1** (13.98 mg, 39.11 μmol) was added to a dry single-necked flask, and added with tetrahydrofuran (1mL) to dissolve, then added with M-chloroperoxybenzoic acid (24.75 mg, 143.41 μmol) to react for 30 min at room temperature, then N,N-diisopropylethylamine (42.12 mg, 325.93 μmol) and **50-4** (7.80 mg, 32.59 μmol) were added to the reaction solution to react for 2 hours at room temperature and monitored by LC-MS, then concentrated under reduced pressure after the reaction. The residue was purified by preparative high-performance liquid chromatography (alkaline) to give the compound **50** (2 mg, 3.65 μmol). LCMS (ESI) m/z: 549.2 [M+H]$^+$, HPLC method B: $R_T$ = 9.17 min, purity: 95.7 %. $^1$H NMR (600 MHz, DMSO-$d_6$) δ 10.28 (s, 1H), 8.87 (s, 1H), 8.03 - 7.99 (m, 1H), 7.75 (d, $J$ = 8.4 Hz, 1H), 7.70 (d, $J$ = 8.4 Hz, 2H), 7.61 (d, $J$ = 7.8 Hz, 1H), 7.15 (d, $J$ = 8.4 Hz, 2H), 6.70 - 6.60 (m, 2H), 6.53 (d, $J$ = 8.4 Hz, 1H), 6.49 - 6.42 (m, 1H), 5.71 - 5.62 (m, 1H), 5.32 (s, 1H), 5.02 - 4.96 (m, 1H), 4.85 - 4.79 (m, 1H), 4.68 (d, $J$ = 6.0 Hz, 2H), 3.68 - 3.65 (m, 2H), 3.30 (d, $J$ = 1.8 Hz, 2H), 2.87 (s, 3H), 1.46 (s, 6H), 1.24 (s, 1H).

**Example 51: Synthesis of Compound 51:**

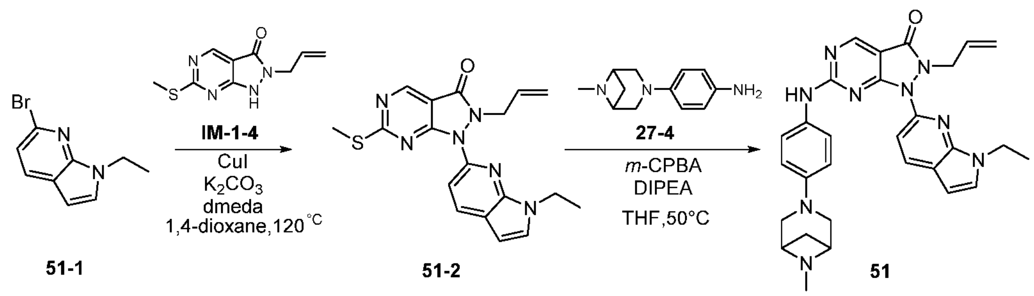


51-1 51-2 51

**Step 1: Synthesis of Compound 51-2:**

The substrate **51-1** (44 mg, 195.48 μmol), **IM-1-4** (44 mg, 197.96 μmol), CuI(37.70 mg, 197.96 μmol), $K_2CO_3$ (38.3 mg, 227.14 μmol) and N,N'-Dimethylethylenediamine (34.90 mg, 395.92 μmol) were added to a dry microwave tube under $N_2$ protection, then added with 1,4-Dioxane (2mL), heated up to 120°C to react for 4 hours under $N_2$ atmosphere and monitored by LC-MS. After the reaction was completed, the reaction system was cooled to room temperature, concentrated under reduced pressure to remove the solvents. The residue was purified by MPLC to give the compound **51-2** (26 mg, 70.95 μmol). LCMS (ESI) m/z:367.1[M+H]$^+$.

**Step 2: Synthesis of Compound 51:**

The substrate **51-2** (56 mg, 152.82 μmol) was added to a dry single-necked flask, and added with tetrahydrofuran (4mL) to dissolve, then added with M-chloroperoxybenzoic acid (42.06 mg, 244.52 μmol) to react for 2 hours at 50°C, then N,N-diisopropylethylamine (98.75 mg, 764.11 μmol) and 27-4 (37.28 mg, 183.39 μmol) were added to the reaction solution to react for 16 hours at 50°C and monitored by LC-MS, then concentrated under reduced pressure after the reaction. The residue was purified by preparative high-performance liquid chromatography (alkaline) to give the compound 51(11 mg, 21.09 μmol). LCMS (ESI) m/z: 522.2 $[M+H]^+$, HPLC method B: $R_T$ = 8.05 min, purity: 96.8%. $^1H$ NMR (600 MHz, DMSO-$d_6$) δ 10.05 (s, 1H), 8.80 (s, 1H), 8.22 (d, *J* = 8.4 Hz, 1H), 7.64 - 7.59 (m, 4H), 6.67 (d, *J* = 8.4 Hz, 2H), 6.56 (d, *J* = 3.6 Hz, 1H), 5.75 - 5.65 (m, 1H), 5.00 (d, *J* = 10.2 Hz, 1H), 4.86 (d, *J* = 17.4 Hz, 1H), 4.64 (s, 2H), 4.30 - 4.26 (m, 2H), 3.57 (d, *J* = 6.0 Hz, 2H), 3.41 (d, *J* = 10.8 Hz, 2H), 3.25 (d, *J* = 11.4 Hz, 2H), 2.42 - 2.41 (m, 1H), 1.98 (s, 3H), 1.52 (d, *J* = 8.4 Hz, 1H), 1.40 - 1.38 (m, 3H).

**Example 52: Synthesis of Compound 52:**

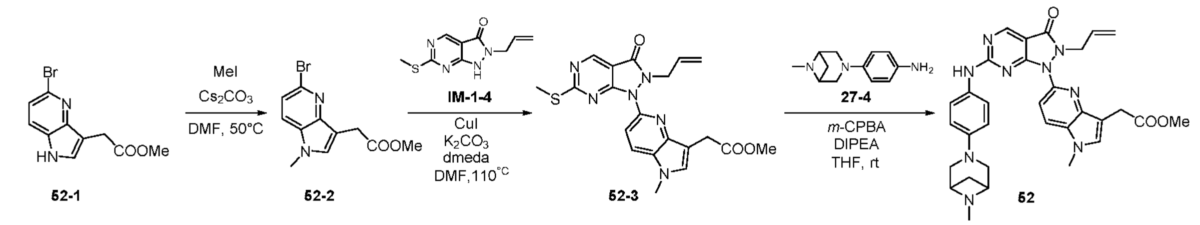


**Step 1: Synthesis of Compound 52-2:**

The substrate **52-1** (1 g, 3.94 mmol) was added in a dry single-necked flask and dissolved in N,N-diisopropylethylamine (10 mL), added with $CH_3I$ (1.40 g, 9.84 mmol) and $Cs_2CO_3$ (5.13 g, 15.74 mmol) to react for 2 hours at 50°C and monitored by LC-MS. The reaction solution was cooled to room temperature after the reaction, then diluted with water and extracted three times with ethyl acetate. The organic phase was dried with anhydrous $Na_2SO_4$, filtered, concentrated under reduced pressure. The residue was purified by column chromatography to give the compound **52-2** (1 g, 3.54 mmol). LCMS (ESI) m/z: 283.1 $[M+H]^+$.

**Step 2: Synthesis of Compound 52-3:**

The substrate **52-2** (167.55 mg, 593.88 μmol), **IM-1-4** (110 mg, 494.90 μmol), CuI(9.43 mg, 49.49 μmol), $K_2CO_3$ (95.76 mg, 692.86 μmol) and N,N'-Dimethylethylenediamine (8.73 mg, 98.98 μmol) were added to a dry microwave tube under $N_2$ protection, then added with N,N-diisopropylethylamine (2mL), heated up to 110°C to react for 12 hours under $N_2$ atmosphere and monitored by LC-MS. After the reaction was completed, the reaction system was cooled to room temperature, concentrated under reduced pressure to remove the solvents. The residue was purified by MPLC to give the compound **52-3** (16 mg, 37.78 μmol). LCMS (ESI) m/z: 425.1 $[M+H]^+$.

**Step 3: Synthesis of Compound 52:**

The substrate **52-3** (18.00 mg, 42.50 μmol) was added to a dry single-necked flask, and added with tetrahydrofuran (0.5mL) to dissolve, then added with M-chloroperoxybenzoic acid (11.00 mg, 63.76 μmol) to react for 10 min at room temperature, then N,N-diisopropylethylamine (27.47 mg, 212.52 μmol) and **27-4** (8.64 mg, 42.50 μmol) were added to the reaction solution to react for 4 hours at room temperature and monitored by LC-MS, then concentrated under reduced pressure after the reaction. The residue was purified by preparative high-performance liquid chromatography (alkaline) to give the compound **52** (7.2 mg, 12.44 μmol). LCMS(ESI) m/z: 579.1$[M+H]^+$,HPLC Method B $R_T$ = 7.30 min, purity > 98.0%. $^1H$ NMR (600 MHz, DMSO-$d_6$) δ 9.92 (s, 1H), 8.77 (s, 1H), 7.61 (d, *J* = 1.8 Hz, 1H), 7.56 (d, *J* = 8.4 Hz, 1H), 7.52 (s, 1H), 7.39 (s, 1H), 7.19 (s, 1H), 6.59 (s, 3H), 5.68 - 5.66 (m, 1H), 5.09 (s, 1H), 4.93 (s, 1H), 4.24 (s, 2H), 3.81 (s, 3H), 3.80 (s, 2H), 3.60 (s, 3H), 3.56 (d, *J* = 5.4 Hz, 2H), 3.37 (d, *J* = 10.8 Hz, 2H), 3.24 - 3.18 (m, 2H), 2.39 (d, *J* = 2.4 Hz, 1H), 1.96 (s, 3H), 1.50 (d, *J* = 8.4 Hz, 1H).

**Example 53: Synthesis of Compound 53:**

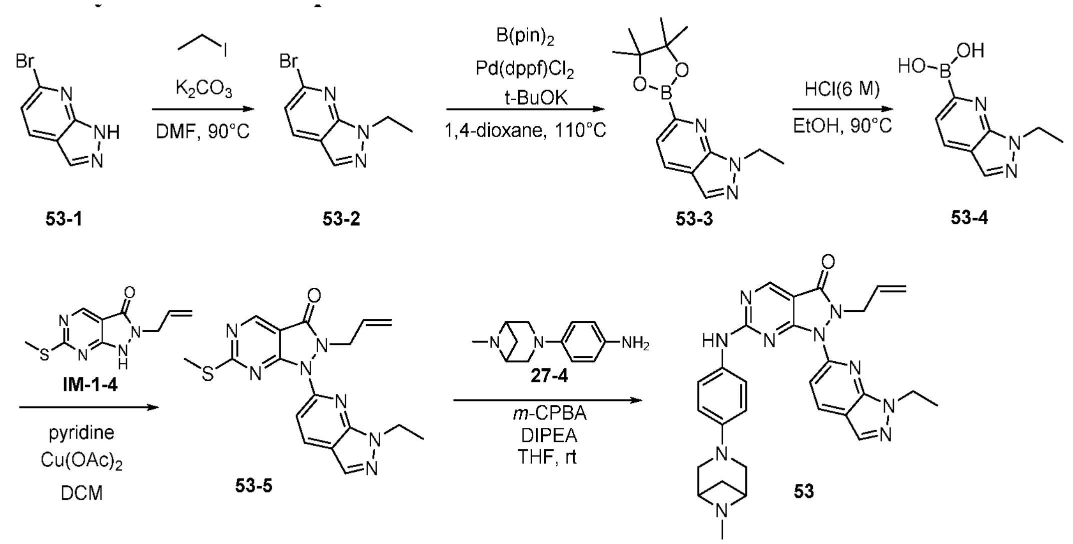


53-1 53-2 53-3 53-4 53-5 53

**Step 1: Synthesis of Compound 53-2:**

The substrate **53-1** (2.1 g, 10.66 mmol) was added in a dry single-necked flask and dissolved in N,N-diisopropylethylamine (25 mL), added with $C_2H_5I$ (4.99 g, 31.97 mmol) and $K_2CO_3$ (4.22 g, 31.97 mmol) to react for 12 hours at 90°C and monitored by LC-MS. The reaction solution was cooled to room temperature after the reaction, then diluted with water and extracted three times with ethyl acetate. The organic phase was dried with anhydrous $Na_2SO_4$, filtered, concentrated under reduced pressure. The residue was purified by column chromatography to give the compound 53-2(1.38 g, 6.13 mmol). LCMS (ESI) m/z: 226.0 $[M+H]^+$.

**Step 2: Synthesis of Compound 53-3:**

The substrate **53-2** (849 mg, 8.66 mmol), $B(pin)_2$ (1.61 g, 6.35 mmol), $Pd(dppf)Cl_2$ (236 mg, 0.29 mmol), t-BuOK (845 mg, 8.66 mmol) and 1,4-Dioxane (30 mL) were added to a dry single-necked flask under $N_2$ protection. The reaction mixture was stirred for 16 hours at 110°C under $N_2$ atmosphere and monitored by LC-MS, then cooled to room temperature after the reaction, extracted three times with ethyl acetate. The organic phase was dried with anhydrous $Na_2SO_4$, filtered, concentrated under reduced pressure. The residue was purified by column chromatography to give the compound **53-3** (680 mg, 2.5 mmol). LCMS (ESI) m/z: 274.2$[M+H]^+$.

**Step 3: Synthesis of Compound 53-4:**

The substrate **53-3** (0.47 g, 1.73 mmol) was added to a dry single-necked flask and dissolved in ethanol (12 mL) , added with 6M HCl (0.3 mL, 1.73 mmol) to react for 7 hours at 90°C and monitored by LC-MS, then concentrated under reduced pressure after the reaction. The residue was purified by MPLC to give the compound **53-4** (180 mg, 0.95 mmol). LCMS (ESI) m/z: 192.1$[M+H]^+$.

**Step 4: Synthesis of Compound 53-5:**

The substrate **53-4** (217.99 mg, 1.15 mmol) was added to a dry single-necked flask and dissolved in $CH_2Cl_2$ (20 mL), added with **IM-1-4** (170 mg, 764.85 μmol), pyridine (242.00 mg, 3.06 mmol) and $Cu(Ac)_2$ (277.84 mg, 1.53 mmol), stirred for 24 hours at room temperature and monitored by LC-MS, then concentrated under reduced pressure after the reaction. The residue was purified by MPLC to give the compound **53-5** (31 mg, 84.60 μmol). LCMS (ESI) m/z: 368.1 $[M+H]^+$.

**Step 5: Synthesis of Compound 53:**

The substrate **53-5** (39 mg, 106.43 μmol) was added to a dry single-necked flask, and added with tetrahydrofuran (6mL) to dissolve, then added with M-chloroperoxybenzoic acid (20.20 mg, 117.07 μmol) to react for 30 min at room temperature, then N,N-diisopropylethylamine (137.55 mg, 1.06 mmol) and 27-4 (23.80 mg, 117.07 μmol) were added to the reaction solution to react for 2 hours at room temperature and monitored by LC-MS, then concentrated under reduced pressure after the reaction. The residue was purified by preparative high-performance liquid chromatography (alkaline) to give the compound **53** (8.3 mg, 15.91 μmol). LCMS (ESI) m/z: 523.3[M+H]$^+$, HPLC method B: $R_T$=7.18 min, purity:98.3%. $^1$H NMR (600 MHz, DMSO-$d_6$) δ 10.14 (s, 1H), 8.80 (s, 1H), 8.14 (s, 1H), 7.92 (d, *J* = 8.4 Hz, 1H), 7.80 (s, 1H), 7.56 - 7.43 (m, 2H), 7.28 (s, 1H), 6.60 (s, 2H), 5.76 - 5.62 (m, 1H), 5.08 (d, *J* = 10.2 Hz, 1H), 4.95 (d, *J* = 17.4 Hz, 1H), 4.51 - 4.47 (m, 2H), 4.35 (s, 1H), 3.56 (d, *J* = 5.4 Hz, 2H), 3.38 (d, *J* = 10.8 Hz, 2H), 3.22 (d, *J* = 11.2 Hz, 2H), 2.45 - 2.38 (m, 1H), 1.96 (s, 3H), 1.50 (d, *J* = 8.4 Hz, 1H), 1.44 - 1.37 (m, 3H).

**Example 54: Synthesis of Compound 54:**

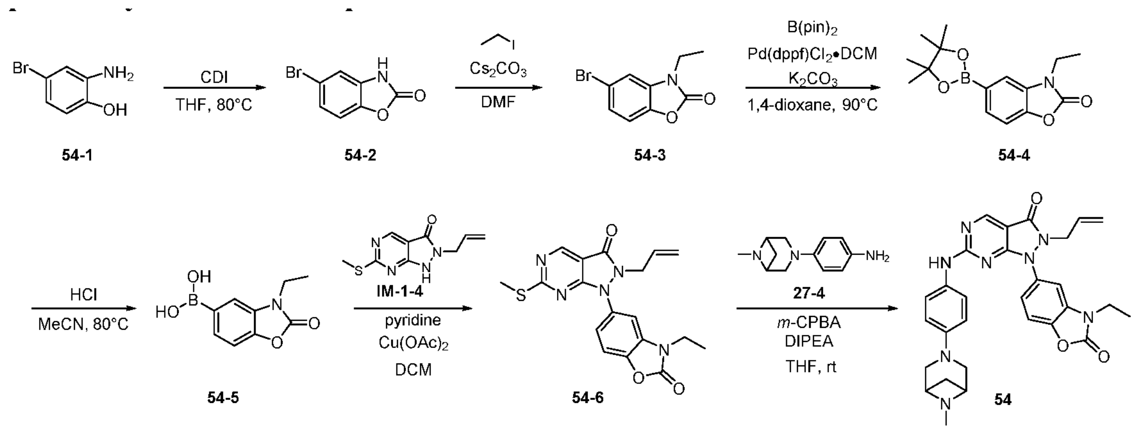


54-1 54-2 54-3 54-4 54-5 54-6 54

**Step** 1: **Synthesis of Compound 54-2:**

The substrate **54-1** (300 mg, 1.60 mmol) was added in a dry single-necked flask and dissolved in tetrahydrofuran (3 mL), added with N,N'-Carbonyldiimidazole (344.52 mg, 2.39 mmol) to react for 2 hours at 80 °C and monitored by LC-MS. The reaction solution was cooled to room temperature after the reaction, then diluted with water and extracted three times with ethyl acetate. The organic phase was dried with anhydrous $Na_2SO_4$, filtered, concentrated under reduced pressure. The residue was purified by column chromatography to give the compound **54-2** (260 mg, 1.22 mmol). LCMS (ESI) m/z: 213.9 [M+H]$^+$.

**Step 2: Synthesis of Compound 54-3:**

The substrate **54-2** (620 mg, 2.90 mmol) was added in a dry single-necked flask and dissolved in N,N-diisopropylethylamine (6 mL), added with $CH_3I$ (451.83 mg, 2.90 mmol) and $Cs_2CO_3$ (1.12 g, 3.19 mmol) to react for 12 hours at room temperature and monitored by LC-MS. The reaction solution was cooled to room temperature after the reaction, then diluted with water and extracted three times with ethyl acetate. The organic phase was dried with anhydrous $Na_2SO_4$, filtered, concentrated under reduced pressure. The residue was purified by MPLC to give the compound **54-3** (279 mg, 1.15 mmol). LCMS (ESI) m/z: 242.0 [M+H]$^+$.

**Step 3: Synthesis of Compound 54-4:**

The substrate **54-3** (220 mg, 908.83 μmol), $B(pin)_2$ (346.18 mg, 1.36 mmol), $Pd(dppf)Cl_2 \cdot CH_2Cl_2$ (74.22 mg, 90.88 μmol), $K_2CO_3$ (408.20 mg, 2.95 mmol) and 1,4-Dioxane (7 mL) were added to a dry single-necked flask under $N_2$ protection. The reaction mixture was stirred for 16 hours at 90°C under $N_2$ atmosphere and monitored by LC-MS, then cooled to room temperature after the reaction, added with water, extracted three times with ethyl acetate. The organic phase was dried with anhydrous $Na_2SO_4$, filtered, concentrated under reduced pressure. The residue was purified by column chromatography to give the compound **54-4** (140 mg, 484.20 μmol). LCMS (ESI) m/z: 290.2[M+H]$^+$.

**Step 4: Synthesis of Compound 54-5:**

The substrate **54-4** (140 mg, 484.20 μmol) was added to a dry single-necked flask and dissolved in acetonitrile (2 mL) , added with 6M HCl (80.70 μL) to react for 4 hours at 80°C and monitored by LC-MS, then concentrated under reduced pressure after the reaction. The residue was purified by MPLC to give the compound **54-5** (33 mg, 159.43 μmol). LCMS (ESI) m/z: 208.1[M+H]+.

**Step 5: Synthesis of Compound 54-6:**

The substrate **54-5** (30 mg, 144.93 μmol) was added to a dry single-necked flask and dissolved in $CH_2Cl_2$ (3 mL), added with **IM-1-4** (32.2 mg, 222.27 μmol), pyridine (11.46 mg, 144.93 μmol) and $Cu(Ac)_2$ (52.65 mg, 289.87 μmol), stirred for 24 hours at room temperature and monitored by LC-MS, then concentrated under reduced pressure after the reaction. The residue was purified by MPLC to give the compound **54-6** (30 mg, 78.24 μmol). LCMS (ESI) m/z: 384.1[M+H]+.

**Step 6: Synthesis of Compound 54:**

The substrate **54-6** (30 mg, 78.24 μmol) was added to a dry single-necked flask, and added with tetrahydrofuran (2mL) to dissolve, then added with M-chloroperoxybenzoic acid (20.25 mg, 117.36 μmol) to react for 30 min at room temperature, then N,N-diisopropylethylamine (50.56 mg, 391.21 μmol) and 27-4 (19.09 mg, 93.89 μmol) were added to the reaction solution to react for 1.5 hours at room temperature and monitored by LC-MS, then concentrated under reduced pressure after the reaction. The residue was purified by preparative high-performance liquid chromatography (alkaline) to give the compound **54** (4.3 mg, 6.99 μmol). LCMS (ESI) m/z: 539.1 [M+H]+, HPLC method A: $R_T$=7.01 min, purity:89.4%. $^1$H NMR (600 MHz, DMSO-$d_6$) δ 10.01 (s, 1H), 8.79 (s, 1H), 7.53 - 7.48 (m, 4H), 7.26 - 7.24 (m, 1H), 6.63 - 6.62 (m, 2H), 5.71 - 5.66 (m, 1H), 5.08 (d, *J* =10.2 Hz, 1H), 4.98 - 4.95 (m, 1H), 4.28 - 4.19 (m, 2H), 3.89 - 3.86 (m, 2H), 3.56 (d, *J* =4.8 Hz, 2H), 3.38 (d, *J* =10.8 Hz, 2H), 3.22 (d, *J* =10.8 Hz, 2H), 2.41 - 2.38 (m, 1H), 1.96 (s, 3H), 1.50 (d, *J* =8.4 Hz, 1H), 1.27 - 1.24 (m, 3H).

**Example 55: Synthesis of Compound 55:**

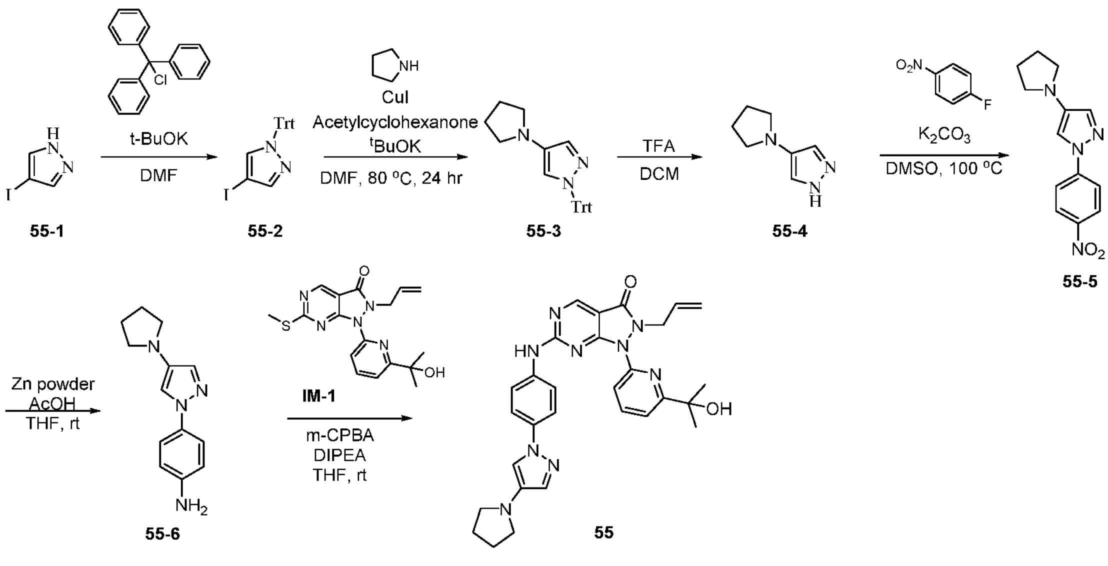


**55-1** **55-2** **55-3** **55-4** **55-5** **55-6** **55**

**Step 1: Synthesis of Compound 55-2:**

The substrate **55-1** (0.83 g, 4.28 mmol) was added to a dry single-necked flask, added with N,N-diisopropylethylamine (6 mL) to dissolve, added with t-BuOK (960.29 mg, 8.56 mmol) and Triphenylchloromethane (1.43 g, 5.13 mmol) to react overnight at room temperature and monitored by LC-MS, then cooled to room temperature after the reaction, added with water and extracted three times with $CH_2Cl_2$. The organic phase was dried with anhydrous $Na_2SO_4$, filtered, concentrated under reduced pressure. The residue was recrystallized to give the compound **55-2** (1.53 g, 3.51 mmol).LCMS (ESI) m/z: 437.1[M+H]+.

**Step 2: Synthesis of Compound 55-3:**

The substrate **55-2** (0.88 g, 2.02 mmol), Tetrahydropyrrole (573.81 mg, 8.07 mmol), CuI(38.41 mg, 201.70 μmol), t-BuOK (452.67 mg, 4.03 mmol) and 2-Acetylcyclohexanone (56.55 mg, 403.40 μmol) were added to a dry microwave tube under $N_2$ protection, then added with N,N-diisopropylethylamine (5mL), heated up to 80°C to react for 24 hours under $N_2$ atmosphere and monitored by LC-MS. After the reaction was completed, the reaction system was cooled to room temperature, concentrated under reduced pressure to remove the solvents. The residue was purified by MPLC to give the compound **55-3** (250 mg, 658.77 μmol). LCMS (ESI) m/z: 380.2[M+H]$^+$.

**Step 3: Synthesis of Compound 55-4:**

The compound **55-3** (140 mg, 368.91 μmol) was added to a dry single-necked flask and dissolved in $CH_2Cl_2$ (2 ml) , added with trifluoroacetic acid (1ml) to react for 30 min at room temperature and monitered by LC-MS. The solvents were removed by distillation under reduced pressure after the disappearance of the raw materials to give the compound **55-4** (22 mg, 160.37 μmol). LCMS (ESI) m/z: 138.1[M+H]$^+$.

**Step 4: Synthesis of Compound 55-5:**

The substrate **55-4** (250 mg, 1.82 mmol) was added in a dry single-necked flask and dissolved in dimethyl sulfoxide (5 ml), added sequentially with p-fluoronitrobenzene (282.85 mg, 2.00 mmol) and $K_2CO_3$ (2.52 g, 18.22 mmol). The reaction mixture was stirred for 12 hours at 100°C and monitored by LC-MS, then diluted with water after the reaction and extracted three times with ethyl acetate. The organic phase was dried with anhydrous $Na_2SO_4$, filtered, concentrated under reduced pressure. The residue was purified by column chromatography to give the compound **55-5** (200 mg, 774.37 μmol). LCMS (ESI) m/z: 259.1[M+H]$^+$.

**Step 5: Synthesis of Compound 55-6:**

The compound **55-5** (9 mg, 34.85 μmol) was dissolved tetrahydrofuran (0.5mL) and acetic acid (0.1 mL) in a dry single-necked flask, added with zinc powder (45.57 mg, 696.93 μmol), stirred for 30 min at room temperature and monitored by LC-MS, filtered with diatomaceous earth after the reaction, the filterate was concentrated under reduced pressure to give the compound **55-6** (6 mg, 26.28 μmol). LCMS (ESI) m/z: 229.2[M+H]$^+$.

**Step 6: Synthesis of Compound 55:**

The substrate **IM-1** (42.27 mg, 118.27 μmol) was added to a dry single-necked flask, and added with tetrahydrofuran (1mL) to dissolve, then added with M-chloroperoxybenzoic acid (30.61 mg, 177.40 μmol) to react for 30 min at room temperature, then N,N-diisopropylethylamine (76.43 mg, 591.35 μmol) and **55-6** (27 mg, 118.27 μmol) were added to the reaction solution to react for 4 hours at room temperature and monitored by LC-MS, then concentrated under reduced pressure after the reaction. The residue was purified by preparative high-performance liquid chromatography (alkaline) to give the compound **55** (2 mg, 3.72 μmol). LCMS(ESI) m/z: 538.2[M+H]$^+$,HPLC Method B $R_T$ = 8.23 min, purity > 97.9%. $^1$H NMR (600 MHz, DMSO-$d_6$) δ 10.39 (s, 1H), 8.90 (s, 1H), 8.10 (s, 1H), 7.82 (d, *J* = 8.4 Hz, 2H), 7.78 - 7.77 (m, 2H), 7.74 - 7.69 (m, 2H), 7.64 (d, *J* = 7.8Hz, 1H), 7.35 (s, 1H), 5.72 - 5.64 (m, 1H), 5.34 (s, 1H), 5.00 (d, *J*= 10.2 Hz, 1H), 4.83 (d, *J*= 17.4 Hz, 1H), 4.69 (d, *J*= 6.0 Hz, 2H), 3.08 - 3.06 (s, 4H), 1.92 - 1.89 (m, 4H), 1.47 (s, 6H).

**Example 56: Synthesis of Compound 56:**

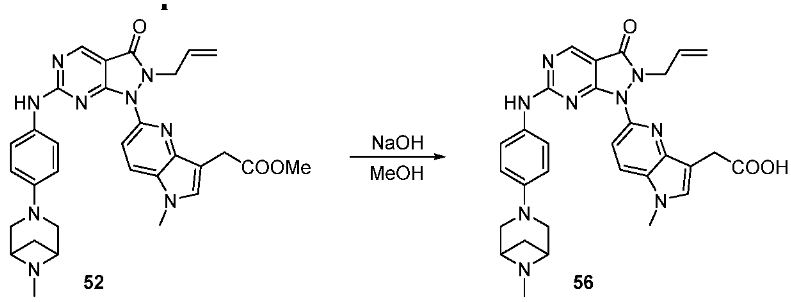


52

56

**Step 1: Synthesis of Compound 56:**

The substrate **52** (2 mg, 3.46 μmol) was added to a dry single-necked flask and dissolved in methanol (1 mL) , added with 1M NaOH (5 μl, 5.18 μmol), stirred for 30 min at room temperature and monitored by LC-MS, then concentrated under reduced pressure after the reaction. The residue was purified by preparative high-performance liquid chromatography (alkaline) to give the compound **56** (1.2 mg, 2.13 μmol). LCMS(ESI) m/z: 565.3[M+H]$^+$,HPLC Rt = 4.68 min, purity > 83.3%. $^1$H NMR (600 MHz, Methanol-*d*$_4$) δ 8.65 (s, 1H), 7.60 (d, *J* = 2.1 Hz, 1H), 7.37 (d, *J* = 8.6 Hz, 3H), 7.10 (s, 1H), 7.07 (d, *J* = 8.2 Hz, 1H), 6.52 (d, *J* = 14.6 Hz, 2H), 5.67 - 5.59 (m, 1H), 5.06 - 5.00 (m, 1H), 4.87 (d, *J*= 17.4 Hz, 1H), 4.26 (d, *J*= 5.5 Hz, 2H), 3.73 (s, 3H), 3.57 (s, 2H), 3.50 (d, *J* = 2.8 Hz, 2H), 3.39 (d, *J* = 11.0 Hz, 2H), 2.46 (s, 1H), 1.84 (s, 2H), 1.79 (s, 3H), 1.27 (d, *J* = 2.9 Hz, 1H).

**Example 57: Synthesis of Compound 57:**

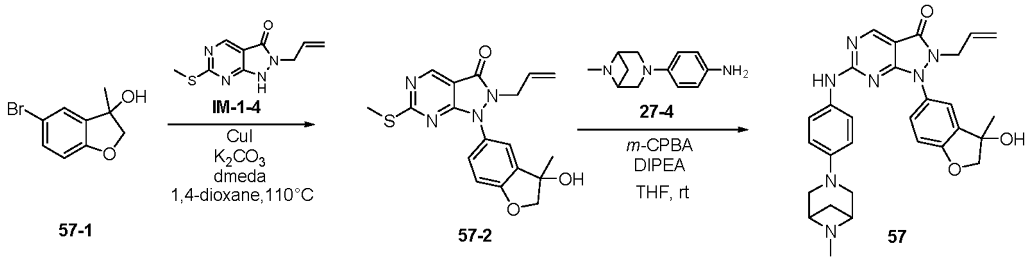


57-1 57-2 57

**Step 1: Synthesis of Compound 57-2:**

The substrate **57-1** (546.59 mg, 2.39 mmol), **IM-1-4** (444 mg, 2.00 mmol), CuI(762 mg, 4.00 mmol), $K_2CO_3$ (552 mg, 2.00 mmol) and N,N'-Dimethylethylenediamine (705 mg, 2.00 mmol) were added to a dry microwave tube under $N_2$ protection, then added with 1,4-Dioxane (10mL), heated up to 110°C to react for 16 hours under $N_2$ atmosphere and monitored by LC-MS. After the reaction was completed, the reaction system was cooled to room temperature, filtered, the filterate was concentrated under reduced pressure. The residue was purified by MPLC to give the compound **57-2** (140 mg, 377.94 μmol). LCMS (ESI) m/z: 371.1[M+H]$^+$.

**Step 2: Synthesis of Compound 57:**

The substrate **57-2** (7.4 mg, 19.98 μmol) was added to a dry single-necked flask, and added with tetrahydrofuran (0.5mL) to dissolve, then added with M-chloroperoxybenzoic acid (7.64 mg, 44.27 μmol) to react for 10 min at room temperature, then N,N-diisopropylethylamine (5.72 mg, 44.27 μmol) and **27-4** (6 mg, 29.52 μmol) were added to the reaction solution to react for 4 hours at room temperature and monitored by LC-MS, then concentrated under reduced pressure after the reaction. The residue was purified by preparative high-performance liquid chromatography (alkaline) to give the compound **57** (8 mg, 15.22 μmol). LCMS (ESI) m/z: 526.3[M+H]$^+$, HPLC $R_T$ = 6.38 min, purity > 94.2%.$^1$H NMR (600 MHz, DMSO-*d*$_6$) δ 9.95 (s, 1H), 8.76 (s, 1H), 7.54 (s, 2H), 7.38 (d, *J* = 9.0 Hz, 1H), 7.27 (d, *J* = 8.4 Hz, 1H), 6.99 (d, *J* = 8.5 Hz, 1H), 6.64 (d, *J* = 8.6 Hz, 2H), 5.69 - 5.67 (m, 1H), 5.62 (d, *J* = 18.0 Hz, 1H), 5.10 (d, *J* = 10.2 Hz, 1H), 4.94 (d, *J* = 17.4 Hz, 1H), 4.55 (s, 1H), 4.41 (d, *J* = 9.6 Hz, 1H), 4.36 (d, *J* = 9.6 Hz, 1H), 4.22 (s, 1H), 3.55 (d, *J* = 6.0 Hz, 2H), 3.39 (d, *J* = 11.2 Hz, 2H), 3.22 (d, *J* = 11.2 Hz, 2H), 2.43 - 2.38 (m, 1H), 1.96 (s, 3H), 1.57 (s, 3H), 1.50 (d, *J* = 8.4 Hz, 1H).

**Example 58: Synthesis of Compound 58:**

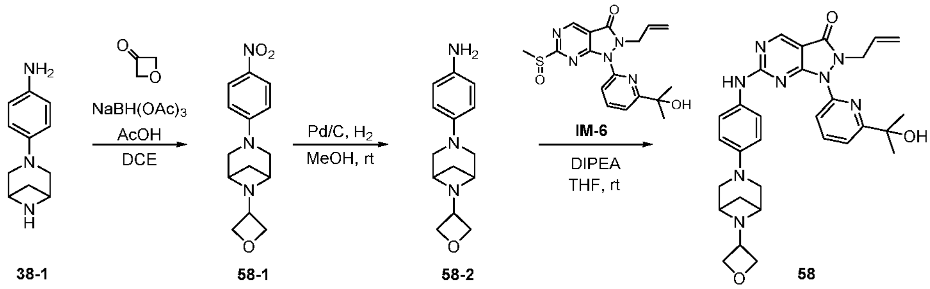


38-1 58-1 58-2 58

**Step 1: Synthesis of Compound 58-1:**

The substrate **38-1** (65.29 mg, 297.82 μmol) was added to a dry single-necked flask and dissolved in 1,2-Dichloroethane (5 mL), added with 3-Oxetanone (108 mg, 1.5 mmol) and acetic acid (0.5 mL), stirred for 15 min at room temperature, then added with Sodium triacetoxyborohydride (315.60 mg, 1.49 mmol) to react for 10 hours at room temperature, then concentrated under reduced pressure after the reaction. The residue was purified by MPLC to give the compound **58-1** (60 mg, 217.94 μmol). LCMS (ESI) m/z: 276.1[M+H]$^+$.

**Step 2: Synthesis of Compound 58-2:**

The compound **58-1** (60 mg, 217.94 μmol) was added to a 50 mL round-bottomed flask and dissolved in methanol (5mL), added with a catalytic amount of Pd/C (15mg), stirred for 1 hour at room temperature under $H_2$ atmosphere and monitored by LC-MS. After the reaction, the reacton solution was filtered with diatomaceous earth, washed two times with methanol, the filterate was concentrated under reduced pressure to give the compound **58-2**(45 mg, 183.43 μmol). LCMS (ESI) m/z: 246.2 [M+H]$^+$.

**Step 3: Synthesis of Compound 58:**

The substrate **IM-6** (41.84 mg, 112.04 μmol) and tetrahydrofuran (2mL) were added to a dry single-necked flask, stirred to dissolve, then N,N-diisopropylethylamine (72.40 mg, 560.20 μmol) and **58-2** (30.23 mg, 123.24 μmol) were added to react for 2 hour at room temperature and monitored by LC-MS, then the solvents were removed by distillation under reduced pressure after the reaction. The residue was purified by preparative high-performance liquid chromatography (alkaline) to give the compound **58** (35 mg, 62.16 μmol). LCMS (ESI) m/z:555.2 [M+H]$^+$, HPLC method B: $R_T$ = 6.27 min, purity: 98.5 %. $^1$H NMR (600 MHz, DMSO-$d_6$) δ 10.10 (s, 1H), 8.80 (s, 1H), 8.03 (s, 1H), 7.77 (s, 1H), 7.61-7.47 (m, 3H), 6.67 (d, *J* = 7.8 Hz, 2H), 5.70 - 5.63 (m, 1H), 5.32 (s, 1H), 4.99 (d, *J*= 10.2 Hz, 1H), 4.83 (d, *J*= 16.8 Hz, 1H), 4.68 (d, *J*= 2.4 Hz, 2H), 4.58 - 4.56 (m, 2H), 4.28 - 4.26 m, 2H), 3.75 (d, *J*= 6.0 Hz, 2H), 3.62 - 3.60 (m, 1H), 3.28 - 3.26 (m, 4H), 2.48 - 2.47 (m, 1H), 1.54 (d, *J* = 8.4 Hz, 1H), 1.47 (s, 6H). $D_2O$: $^1$H NMR (600 MHz, DMSO-$d_6$) δ 8.81 (s, 1H), 8.04 (s, 1H), 7.75 (s, 1H), 7.62-7.45 (s, 3H), 6.69 (d, *J* = 8.4 Hz, 2H), 5.70 - 5.63 (m, 1H), 5.03 (d, *J* = 10.2 Hz, 1H), 4.83 (d, *J* = 16.8 Hz, 1H), 4.69 (d, *J* = 3.0 Hz, 2H), 4.60 - 4.58 (m, 2H), 4.32 - 4.29 (m, 2H), 3.76 (d, *J* = 5.4 Hz, 2H), 3.63 - 3.62 (m, 1H), 3.26 - 3.24 (m, 4H), 2.52-2.51 (m, 1H), 1.53 (d, *J* = 8.4 Hz, 1H), 1.48 (s, 6H).

**Example 59: Synthesis of Compound 59:**

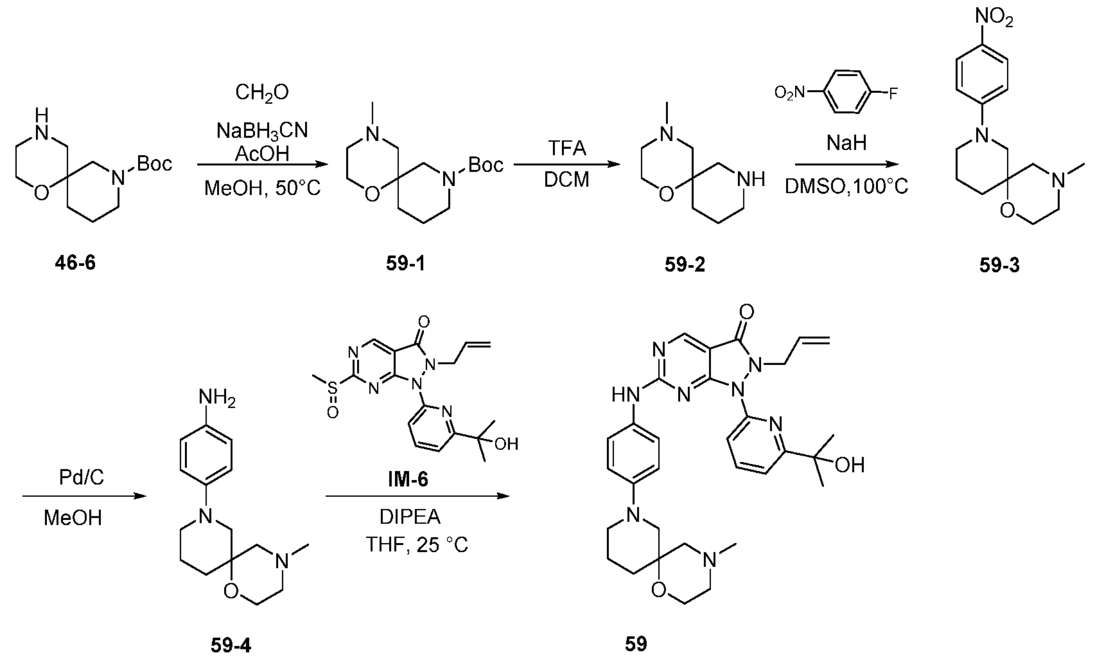


46-6 59-1 59-2 59-3

59-4 59

**Step 1: Synthesis of Compound 59-1:**

The substrate **46-6** (250 mg, 975.27 μmol) was added to a dry single-necked flask and dissolved in methanol (2 mL), added with formaldehyde (16 mg, 469.76 μmol) and acetic acid (0.5 mL), stirred for 30 min at room temperature, then added with Sodium cyanoborohydride (306.43 mg, 4.88 mmol) to react for 2 hours at 50°C and monitored by TLC, then concentrated under reduced pressure after the reaction. The residue was purified by column chromatography to give the compound **59-1** (130 mg, 480.83 μmol). LCMS (ESI) m/z: 262.2[M+H]$^+$.

**Step 2: Synthesis of Compound 59-2:**

The compound **59-1** (54 mg, 199.73 μmol) was added to a dry single-necked flask and dissolved in $CH_2Cl_2$ (2 ml) , added with trifluoroacetic acid (1ml) to react for 30 min at room temperature and monitered by LC-MS. The solvents were removed by distillation under reduced pressure after the disappearance of the raw materials to give the compound **59-2** (30 mg, 176.21 μmol).

**Step 3: Synthesis of Compound 59-3:**

The compound **59-2** (30 mg, 176.21 μmol) was added in a dry single-necked flask and dissolved in dimethyl sulfoxide (2 ml), added sequentially with p-fluoronitrobenzene (24.86 mg, 176.21 μmol) and NaH (21.15 mg, 881.05 μmol). The reaction mixture was stirred for 3 hours at 100°C and monitored by LC-MS, then quenched with water after the reaction and extracted three times with ethyl acetate. The organic phase was dried with anhydrous $Na_2SO_4$, filtered, concentrated under reduced pressure. The residue was purified by column chromatography to give the compound **59-3** (35 mg, 120.13 μmol). LCMS (ESI) m/z: 234.1[M+H]$^+$.

**Step 4: Synthesis of Compound 59-4:**

The compound **59-3** (35 mg, 120.13 μmol) was added to a 50 mL round-bottomed flask and dissolved in methanol (5mL), added with a catalytic amount of Pd/C (15mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **59-4** (30 mg, 114.78 μmol). LCMS (ESI) m/z: 262.2 [M+H]$^+$.

**Step 5: Synthesis of Compound 59:**

The substrate **IM-6** (42.86 mg, 114.78 μmol) and tetrahydrofuran (2mL) were added to a dry single-necked flask, stirred to dissolve, then N,N-diisopropylethylamine (74.17 mg, 573.92 μmol) and 59-4 (30 mg, 114.78 μmol) were added to

react for 2 hours at room temperature and monitored by LC-MS, then the solvents were removed by distillation under reduced pressure after the reaction. The residue was purified by preparative high-performance liquid chromatography (alkaline) to give the compound **59** (3 mg, 4.75 μmol). LCMS (ESI) m/z: 571.2 $[M+H]^+$, HPLC method B: $R_T$ = 7.61 min, purity: 90.3 %. $^1H$ NMR (600 MHz, Chloroform-*d*) δ 8.82 (s, 1H), 7.89 - 7.76 (m, 2H), 7.45 - 7.44 (m, 2H), 7.35 (d, *J*= 7.8 Hz, 1H), 6.97 (s, 2H), 5.73 - 5.67 (m, 1H), 5.04 (d, *J* = 10.2 Hz, 1H), 4.94 (d, *J* = 16.8, 1.2 Hz, 1H), 4.74 (d, *J* = 6.0 Hz, 2H), 3.97 - 3.83 (m, 3H), 3.25 - 3.11 (m, 3H), 2.58 - 2.33 (m, 6H), 1.93 (s, 1H), 1.78 (s, 1H), 1.61 (s, 3H), 1.58 (s, 6H).

**Example 60: Synthesis of Compound 60:**

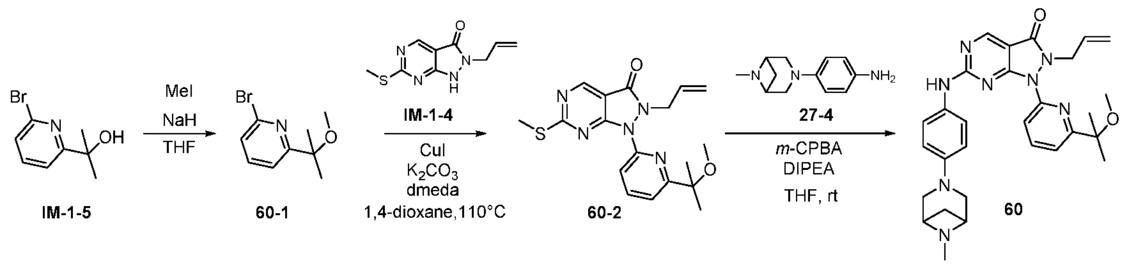


**Step 1: Synthesis of Compound 60-1:**

The substrate **IM-1-5** (250 mg, 1.16 mmol) was dissolved in tetrahydrofuran (2 ml) in a dry single-necked flask, cooled to 0°C and added with NaH (83.30 mg, 3.47 mmol), stirred for 10 min at 0°C, added with $CH_3I$ (492.7 mg, 3.47 mmol), then heated to room temperature, stirred for 6 h and monitored by LC-MS, then quenched with water after the reaction and extracted three times with ethyl acetate. The organic phase was dried with anhydrous $Na_2SO_4$, filtered, concentrated under reduced pressure. The residue was purified by column chromatography to give the compound **60-1** (210 mg, 912.64 μmol). LCMS (ESI) m/z: 230.1$[M+H]^+$.

**Step 2: Synthesis of Compound 60-2:**

The substrate **60-1** (207.05 mg, 899.82 μmol), **IM-1-4** (200 mg, 899.82 μmol), CuI(342.74 mg, 1.80 mmol), $K_2CO_3$ (174.10 mg, 1.26 mmol) and N,N'-Dimethylethylenediamine (705 mg, 2.00 mmol) were added to a dry microwave tube under $N_2$ protection, then added with 1,4-Dioxane (4mL), heated up to 110°C to react for 2 hours under $N_2$ atmosphere and monitored by LC-MS. After the reaction was completed, the reaction system was cooled to room temperature, added with ammonia (2 mL), filtered, extracted three times with ethyl acetate. The organic phase was dried with anhydrous $Na_2SO_4$, filtered, concentrated under reduced pressure. The residue was purified by MPLC to give the compound **60-2** (80 mg, 215.37 μmol). LCMS (ESI) m/z: 372.2$[M+H]^+$.

**Step 3: Synthesis of Compound 60:**

The substrate **60-2** (46 mg, 123.84 μmol) was added to a dry single-necked flask, and added with tetrahydrofuran (0.5mL) to dissolve, then added with M-chloroperoxybenzoic acid (32 mg,185.76 μmol) to react for 10 min at room temperature, then N,N-diisopropylethylamine (80.02 mg, 619.19 μmol) and **27-4** (30.21 mg, 148.60 μmol) were added to the reaction solution to react for 6 hours at room temperature and monitored by LC-MS, then concentrated under reduced pressure after the reaction. The residue was purified by preparative high-performance liquid chromatography (alkaline) to give the compound **60** (14.08 mg, 26.74 μmol). LCMS (ESI) m/z: 527.2 $[M+H]^+$, HPLC method B: $R_T$ =5.86 min, purity:97.6%. $^1H$ NMR (600 MHz, DMSO-$d_6$) δ 10.10 (s, 1H), 8.80 (s, 1H), 8.07 - 8.05 (m, 1H), 7.83 - 7.82 (m, 1H), 7.59 (s, 2H), 7.46 (d, *J* =7.8 Hz, 1H), 6.72 (d, J=7.8 Hz, 2H), 5.70 - 5.63 (m, 1H), 4.99 (d, J=10.2 Hz, 1H), 4.84 - 4.81 (m, 1H), 4.69 (d, *J* =4.2 Hz, 2H), 3.59 (d, *J* =3.6 Hz, 2H), 3.43 (d, J=10.8 Hz, 2H), 3.28 (d, *J* =10.8 Hz, 2H), 3.09 (s, 3H), 2.43 (s, 1H), 1.99 (s, 3H), 1.54 (d, *J* =7.8 Hz, 1H),1.49 (s, 6H).

**Example 61: Synthesis of Compound 61:**

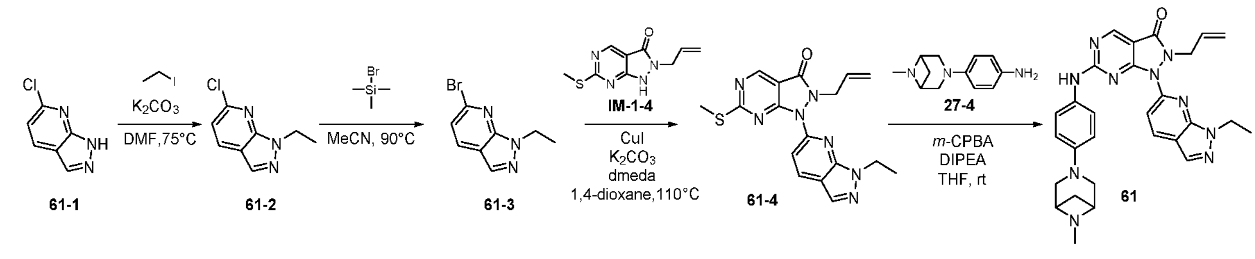


61-1 61-2 61-3 61-4 61

### Step 1: Synthesis of Compound 61-2:

The substrate **61-1** (200 mg, 1.30 mmol) was added in a dry single-necked flask and dissolved in N,N-diisopropylethylamine (2.5 mL), added with $C_2H_5I$ (406.24 mg, 2.60 mmol) and $K_2CO_3$ (5.4 g,3.91 mmol) to react for 1 hour at 75°C and monitored by LC-MS. The reaction solution was cooled to room temperature after the reaction, then filtered, concentrated under reduced pressure. The residue was purified by MPLC to give the compound **61-2** (140 mg, 770.83 μmol). LCMS (ESI) m/z: 266.2 $[M+H]^+$.

### Step 2: Synthesis of Compound 61-3:

The substrate **61-2** (130 mg, 715.77 μmol) was added to a dry single-necked flask and dissolved in acetonitrile (2 mL) , added with Bromotrimethylsilane (109.58 mg, 715.77 μmol) to react for 4 hours at 90°C and monitored by LC-MS. The reaction solution was cooled to room temperature after the reaction, then concentrated under reduced pressure. The residue was purified by MPLC to give the compound **61-3** (60 mg, 265.40 μmol). LCMS (ESI) m/z: 266.2 $[M+H]^+$.

### Step 3: Synthesis of Compound 61-4:

The substrate **61-3** (36.99 mg, 163.60 μmol), **IM-1-4** (40 mg, 179.96 μmol), CuI(62.32 mg, 327.21 μmol), $K_2CO_3$ (31.65 mg, 229.05 μmol) and N,N'-Dimethylethylenediamine (57.69 mg, 654.42 μmol) were added to a dry microwave tube under $N_2$ protection, then added with 1,4-Dioxane (4mL), heated up to 110°C to react for 3 hours under $N_2$ atmosphere and monitored by LC-MS. After the reaction was completed, the reaction system was cooled to room temperature, concentrated under reduced pressure. The residue was purified by MPLC to give the compound **61-4** (19 mg, 51.71 μmol).

### Step 4: Synthesis of Compound 61:

The substrate **61-4** (19 mg, 51.71 μmol) was added to a dry single-necked flask, and added with tetrahydrofuran (0.7mL) to dissolve, then added with M-chloroperoxybenzoic acid (13.39 mg, 77.57 μmol) to react for 30 min at room temperature, then N,N-diisopropylethylamine (33.42 mg, 258.55 μmol) and **27-4** (10.51 mg, 51.71 μmol) were added to the reaction solution to react for 10 hours at room temperature and monitored by LC-MS, then concentrated under reduced pressure after the reaction. The residue was purified by preparative high-performance liquid chromatography (alkaline) to give the compound **61** (3.89 mg, 7.44 μmol). LCMS (ESI) m/z: 523.3 $[M+H]^+$, HPLC method A: $R_T$ = 4.98 min, purity: 98.6%. $^1H$ NMR (600 MHz, CDCl3) δ 8.76 (s, 1H), 8.05 (d, *J* =8.4 Hz, 1H), 7.89 (s, 1H), 7.78 (d, *J* =8.4 Hz, 1H), 7.44 (s, 2H), 6.67 (d, *J* =8.4 Hz, 2H), 5.63 - 5.59(m, 1H), 4.93 - 4.90(m, 3H), 4.87 - 4.84 (m, 1H), 4.46 - 4.42(m, 2H), 3.95 (s, 2H), 3.58 (d, *J* =8.4 Hz, 2H), 3.46 (d, *J* =8.4 Hz, 2H), 2.93 - 2.87(m, 1H), 2.25 (s, 3H), 1.71(d, *J* =6.0 Hz, 1H), 1.61 - 1.59 (m, 3H).

### Example 62: Synthesis of Compound 62:

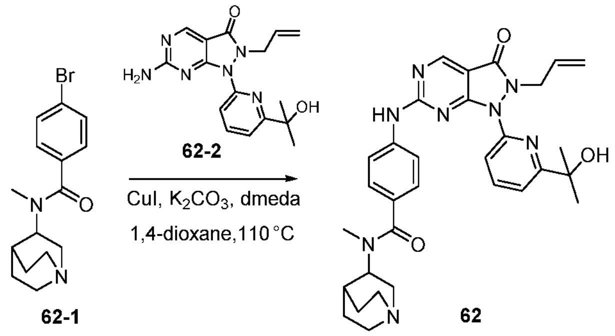


62-1 62

**Step 1: Synthesis of Compound 62:**

The substrate **62-1** (36.99 mg, 163.60 μmol), **62-2** (40.39 mg, 123.75 μmol), CuI(47.14 mg, 247.50 μmol), $K_2CO_3$ (68.41 mg, 495.01 μmol) and N,N'-Dimethylethylenediamine (21.78 mg, 247.50 μmol) were added to a dry microwave tube under $N_2$ protection, then added with 1,4-Dioxane (2mL), heated up to 110°C to react for 16 hours under $N_2$ atmosphere and monitored by LC-MS. After the reaction was completed, the reaction system was cooled to room temperature, concentrated under reduced pressure. The residue was purified by MPLC to give the compound **62** (0.7 mg, 1.23 μmol). LCMS (ESI) m/z: 569.3 $[M+H]^+$, HPLC method A: $R_T$= 6.03 min, purity:73.2%. $^1H$ NMR (600 MHz, DMSO-d6) δ 8.93 - 8.92 (m, 1H), 8.07 - 8.03 (m, 1H), 7.92 - 7.87 (m, 4H), 7.88(d, J = 7.8 Hz, 1H),7.72(d, J = 7.8 Hz, 1H),5.76 - 5.71 (m, 1H), 5.03- 5.01 (m, 1H), 4.92- 4.89 (m, 1H), 4.83- 4.82 (m, 2H), 4.66- 4.63 (m, 1H),4.43- 4.41 (m, 1H),4.29- 4.26 (m, 1H),3.57(d, *J* = 11.4 Hz, 2H),3.04 - 3.00 (m, 2H), 2.95 (s, 3H), 2.19 (d, *J* = 14.4 Hz, 1H),2.16 - 1.51 (m, 4H), 1.58- 1.53 (m, 7H).

**Example 63: Synthesis of Compound 63:**

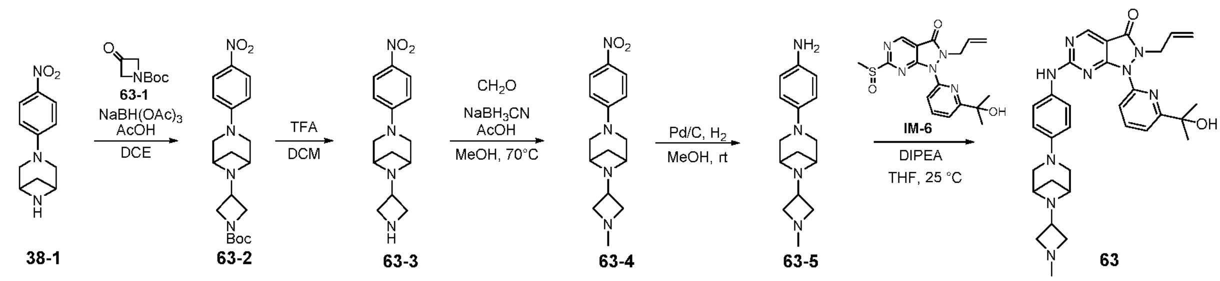


38-1 63-2 63-3 63-4 63-5 63

**Step 1: Synthesis of Compound 63-2:**

The substrate **38-1** (44 mg, 200.69 μmol) was added to a dry single-necked flask and dissolved in 1,2-Dichloroethane (5 mL), added with **63-1** (171.79 mg, 1.00 mmol) and acetic acid (0.5 mL), stirred for 15 min at room temperature, then added with Sodium triacetoxyborohydride (211.94 mg, 1 mmol) to react for 12 hours at room temperature, then concentrated under reduced pressure after the reaction. The residue was purified by MPLC to give the compound **63-2** (350 mg, 934.75 μmol). LCMS (ESI) m/z: 375.2$[M+H]^+$.

**Step 2: Synthesis of Compound 63-3:**

The compound **63-2** (60 mg, 160.24 μmol) was added to a dry single-necked flask and dissolved in $CH_2Cl_2$ (2 ml) , added with trifluoroacetic acid (1ml) to react for 30 min at room temperature and monitered by LC-MS. The solvents were removed by distillation under reduced pressure after the disappearance of the raw materials to give the compound **63-3** (43 mg, 156.75 μmol). LCMS (ESI) m/z: 275.2$[M+H]^+$.

**Step 3: Synthesis of Compound 63-4:**

The substrate **63-3** (43 mg, 156.75 μmol) was added to a dry single-necked flask and dissolved in methanol (3

mL), added with formaldehyde (26.69 mg, 783.76 μmol) and acetic acid (0.2 mL), stirred for 10 min at room temperature, then added with Sodium cyanoborohydride (19.70 mg, 313.51 μmol) to react for 6 hours at 50°C and monitored by LC-MS, then concentrated under reduced pressure after the reaction and extracted three times with ethyl acetate. The organic phase was dried with anhydrous $Na_2SO_4$, filtered, concentrated under reduced pressure to give the compound **63-4** (35 mg, 121.38 μmol). LCMS (ESI) m/z: 289.2[M+H]$^+$.

**Step 4: Synthesis of Compound 63-5:**

The compound **63-4** (35 mg, 120.13 μmol) was added to a 50 mL round-bottomed flask and dissolved in methanol (3mL), added with a catalytic amount of Pd/C (15mg), stirred for 1 hour at room temperature under $H_2$ atmosphere and monitored by LC-MS. After the reaction, the reacton solution was filtered with diatomaceous earth, washed two times with methanol, the filterate was concentrated under reduced pressure to give the compound **63-5** (28 mg, 71.90 μmol). LCMS (ESI) m/z: 259.2 [M+H]$^+$.

**Step 5: Synthesis of Compound 63:**

The substrate **IM-6** (16.89 mg, 65.36 μmol) and tetrahydrofuran (2mL) were added to a dry single-necked flask, stirred to dissolve, then N,N-diisopropylethylamine (42.24 mg, 326.82 μmol) and **63-5** (28 mg, 71.90 μmol) were added to react for 4 hours at room temperature and monitored by LC-MS, then the solvents were removed by distillation under reduced pressure after the reaction. The residue was purified by preparative high-performance liquid chromatography (alkaline) to give the compound **63** (11 mg, 18.49 μmol). LCMS (ESI) m/z: 568.4 [M+H]$^+$, HPLC method B: $R_T$ =5.21 min, purity: 97.5 %. $^1$H NMR (600 MHz, DMSO-$d_6$) δ 8.82 - 8.79 (m, 1H), 7.99 - 7.91 (m, 1H), 7.79 - 7.71 (m, 1H), 7.59 - 7.55 (m, 1H), 7.51 - 7.47 (m, 2H), 6.71 - 6.69 (m, 2H), 5.71 - 5.69(m, 1H), 5.67 - 5.61 (m, 1H), 5.00 - 4.98 (m, 1H), 4.83 - 4.56 (m, 3H), 4.25 - 4.13 (m, 2H), 4.02 - 3.89 (m, 4H), 3.75- 3.57 (m, 5H), 3.28 - 3.21 (m, 3H), , 2.42 - 2.38 (m, 1H), 1.80 - 1.72 (m, 1H), 1.46 - 1.42 (m, 6H). $D_2O$:$^1$H NMR (600 MHz, 333K, DMSO-$d_6$) δ 8.77 (s, 1H), 7.94 (s, 1H), 7.74 (s, 1H), 7.57 (d, *J* = 7.2 Hz, 1H), 7.47 (s, 2H), 6.72 (d, *J* = 9.0 Hz, 2H), 5.68 - 5.62(m, 1H), 5.00 (d, *J*= 10.2 Hz, 1H), 4.85 (d, *J*= 17.4 Hz, 1H), 4.67 (s, 2H), 4.17 - 3.62 (m, 10H), 3.39 - 3.37 (m, 1H), 3.18 (s, 3H), 2.45 (s, 1H), 1.77 (s, 1H), 1.47 (s, 6H).

**Example 64: Synthesis of Compound 64:**

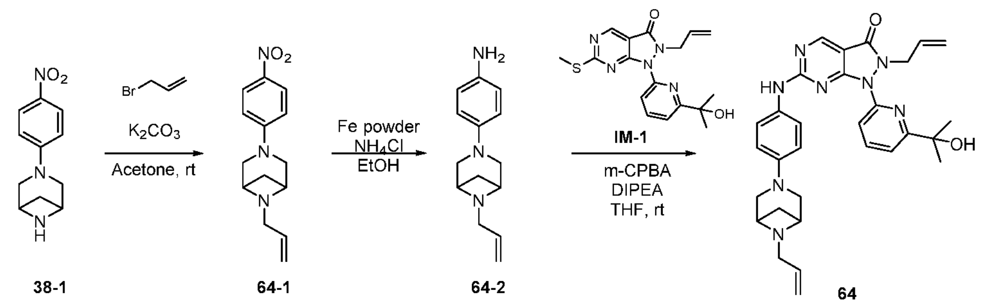


38-1 64-1 64-2 64

**Step 1: Synthesis of Compound 64-1:**

The substrate **38-1** (109 mg, 497.17 μmol) was added to a dry single-necked flask and dissolved in acetone (3 mL), then added with allyl bromide (90.1 mg, 745.76 μmol) and $K_2CO_3$ (207 mg, 745.76 μmol) to react under $N_2$ atmosphere for 2 hours at room temperature and monitored by LC-MS, concentrated under reduced pressure after the reaction. The residue was purified by MPLC to give the compound **64-1** (128 mg, 497.03 μmol). LCMS (ESI) m/z: 260.2 [M+H]$^+$.

**Step 2: Synthesis of Compound 64-2:**

The compound **64-1** (128 mg, 497.03 μmol) was added to a dry single-necked flask and dissolved in ethanol (10 mL), added with iron powder (138.92 mg, 2.49 mmol) and $NH_4Cl$ (399 mg,7.46 mmol), heated up to 80°C to react and stirred for 30 min, monitored by LC-MS, then cooled to room temperature after the reaction, filtered with diatomaceous earth after the reaction, the filterate was concentrated under reduced pressure. The residue was purified by MPLC to give the compound **64-2** (25 mg, 109.02 μmol). LCMS (ESI) m/z: 230.2[M+H]$^+$.

**Step 3: Synthesis of Compound 64:**

The substrate **IM-1** (18 mg, 50.36 μmol) was added to a dry single-necked flask, and added with tetrahydrofuran (1mL) to dissolve, then added with M-chloroperoxybenzoic acid (13 mg, 137.36 μmol) to react for 30 min at room temperature, then N,N-diisopropylethylamine (9.7 mg, 75.05 μmol) and **64-2** (21 mg, 91.58 μmol) were added to the reaction solution to react for 2 hours at room temperature and monitored by LC-MS, then concentrated under reduced pressure after the reaction. The residue was purified by preparative high-performance liquid chromatography (alkaline) to give the compound **64** (6.2 mg, 11.51 μmol). LCMS (ESI) m/z: 539.2 $[M+H]^+$, HPLC method B: $R_T$ = 5.59 min, purity: 92.7 %. $^1H$ NMR (400 MHz, DMSO-$d_6$) δ 8.80 (s, 1H), 8.05 - 8.03 (m, 1H), 7.79 - 7.77 (m, 2H), 7.61 (d, *J*= 7.8 Hz, 3H), 6.72 (d, *J*= 8.4 Hz, 2H), 6.01 - 5.72 (m, 1H), 5.72 - 5.54 (m, 1H), 5.25 - 4.96 (m, 4H), 4.83 (d, *J* = 17.4 Hz, 1H), 4.68 (d, *J* = 6.0 Hz, 2H), 3.44 (d, *J* = 11.2 Hz, 2H), 3.30 (d, *J* = 11.2 Hz, 2H), 3.18 (s, 2H), 2.93 (d, *J* = 6.0 Hz, 2H), 1.55 (d,*J* = 8.4 Hz, 1H), 1.47 (s, 6H).

**Example 65: Synthesis of Compound 65:**

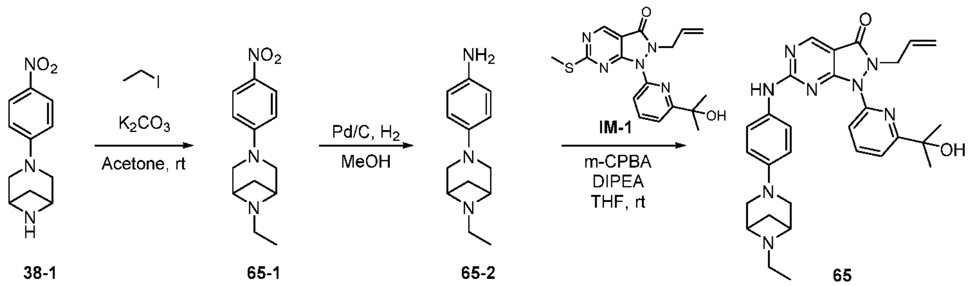


38-1 65-1 65-2 65

**Step 1: Synthesis of Compound 65-1:**

The substrate **38-1** (87 mg, 396.83 μmol) was added in a dry single-necked flask and dissolved in acetone (3 mL), added with $C_2H_5I$ (92.84 mg, 595.25 μmol) and $K_2CO_3$ (83 mg, 1.19 mmol) to react under $N_2$ atmosphere for 2 hours at room temperature and monitored by LC-MS, then concentrated under reduced pressure after the reaction. The residue was purified by MPLC to give the compound 65-1 (87 mg, 351.81 μmol). LCMS (ESI) m/z: 248.1 $[M+H]^+$.

**Step 2: Synthesis of Compound 65-2:**

The compound **65-1** (94 mg, 380.12 μmol) was added to a 50 mL round-bottomed flask and dissolved in methanol (5mL), added with a catalytic amount of Pd/C (10mg), stirred for 2 hours at room temperature under $H_2$ atmosphere and monitored by LC-MS. After the reaction, the reacton solution was filtered with diatomaceous earth, washed two times with methanol, the filterate was concentrated under reduced pressure to give the compound **65-2** (80 mg, 368.14 μmol). LCMS (ESI) m/z: 218.2 $[M+H]^+$.

**Step 3: Synthesis of Compound 65:**

The substrate **IM-1** (18 mg, 50.36 μmol) was added to a dry single-necked flask, and added with tetrahydrofuran (1mL) to dissolve, then added with M-chloroperoxybenzoic acid (13 mg, 137.36 μmol) to react for 30 min at room temperature, then N,N-diisopropylethylamine (9.7 mg, 75.05 μmol) and **65-2** (19.9 mg, 91.58 μmol) were added to the reaction solution to react for 2 hours at room temperature and monitored by LC-MS, then concentrated under reduced pressure after the reaction. The residue was purified by preparative high-performance liquid chromatography (alkaline) to give the compound **65** (6.2 mg, 11.77 μmol). LCMS (ESI) m/z: 527.2$[M+H]^+$, HPLC method B: $R_T$ = 6.27 min, purity:76.2%. $^1H$ NMR (400 MHz, DMSO-$d_6$) δ 10.09 (s, 1H), 8.80 (s, 1H), 8.06 - 8.02 (m, 1H), 7.76 (d, *J* = 8.0 Hz, 1H), 7.60 (d, *J* = 7.6 Hz, 3H), 6.70 (d, *J* = 8.4 Hz, 2H), 5.74 - 5.55 (m, 1H), 5.31 (s, 1H), 5.04 - 4.94 (m, 1H), 4.83 (d, *J*= 17.2 Hz, 1H), 4.68 (d, *J* = 6.0 Hz, 2H), 3.67 (d, *J* = 5.6 Hz, 2H), 3.42 (d, *J* = 11.2 Hz, 2H), 3.26 (d, *J* = 11.2 Hz, 2H), 2.42 (d, *J*= 6.8 Hz, 1H), 2.28 (d, *J* = 7.2 Hz, 2H), 1.55 (s, 1H), 1.46 (s, 6H), 0.93 - 0.91 (m, 3H).

**Example 66: Synthesis of Compound 66:**

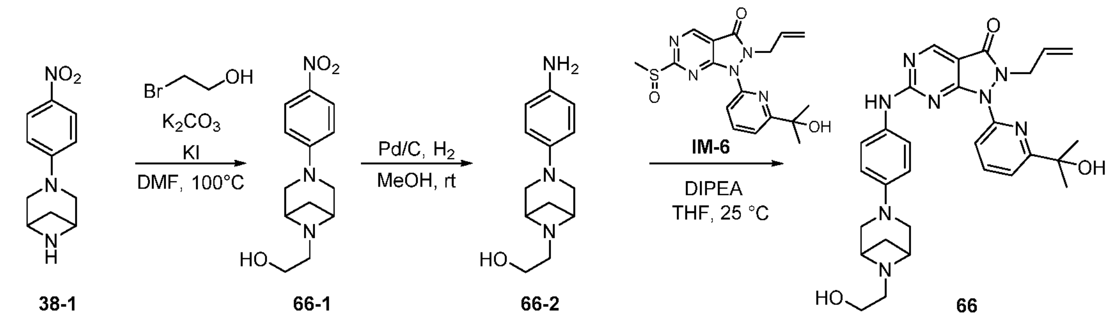


38-1 66-1 66-2 66

**Step 1: Synthesis of Compound 66-1:**

The substrate **38-1** (80 mg, 364.90 μmol) was added to a dry single-necked flask and dissolved in N,N-dimethylformamide (3 mL) , then added with 2-Bromoethanol (68 mg, 544.16 μmol), $K_2CO_3$ (99 mg, 716.35 μmol) and KI (5 mg, 30.12 μmol) to react for 2 hours at 100°C and monitored by LC-MS, then the reaction solution was cooled to room temperature after the reaction, extracted three times with ethyl acetate. The organic phase was dried with anhydrous $Na_2SO_4$, filtered, concentrated under reduced pressure to give the compound **66-1** (70 mg, 265.86 μmol). LCMS (ESI) m/z: 264.1 $[M+H]^+$.

**Step 2: Synthesis of Compound 66-2:**

The compound **66-1** (70 mg, 265.86 μmol) was added to a 50 mL round-bottomed flask and dissolved in methanol (3mL), added with a catalytic amount of Pd/C (15mg), stirred for 3 hours at room temperature under $H_2$ atmosphere and monitored by LC-MS. After the reaction, the reacton solution was filtered with diatomaceous earth, washed two times with methanol, the filterate was concentrated under reduced pressure to give the compound **66-2** (35 mg, 150 μmol). LCMS (ESI) m/z: 234.2 $[M+H]^+$.

**Step 3: Synthesis of Compound 66:**

The substrate **IM-6** (50 mg, 128.39 μmol) and tetrahydrofuran (4mL) were added to a dry single-necked flask, stirred to dissolve, then N,N-diisopropylethylamine (82.97 mg, 641.97 μmol) and 66-2 (32 mg, 57.32 μmol) were added to react for 4 hours at room temperature and monitored by LC-MS, then the solvents were removed by distillation under reduced pressure after the reaction. The residue was purified by preparative high-performance liquid chromatography (alkaline) to give the compound **66** (45 mg, 74.45 μmol). LCMS (ESI) m/z: 543.2 $[M+H]^+$, HPLC method B: $R_T$ = 5.69 min, purity: 97.2 %. $^1$H NMR (600 MHz, Chloroform-*d*) δ 8.81(s, 1H), 7.87 - 7.84(m, 1H), 7.76(d, *J* = 7.8 Hz, 1H), 7.47(s, 2H), 7.34(d, *J* = 7.8 Hz, 1H), 6.72(d, *J* = 9.0 Hz, 2 H), 5.73 - 5.67(m, 1H), 5.04(d, *J* = 10.2 Hz, 1H), 4.94(d, *J* = 16.8 Hz, 1H), 4.73(d, *J* = 6.0 Hz, 2H), 3.97(d, *J* = 5.4Hz, 2H), 3.67 - 3.63(m, 4H), 3.44 (d, *J* = 10.8Hz, 2H), 2.82 - 2.80(m, 1H), 2.64(s, 2H), 1.72 (d, *J* = 8.4 Hz, 1H), 1.59(s, 6H).

**Example 67: Synthesis of Compound 67:**

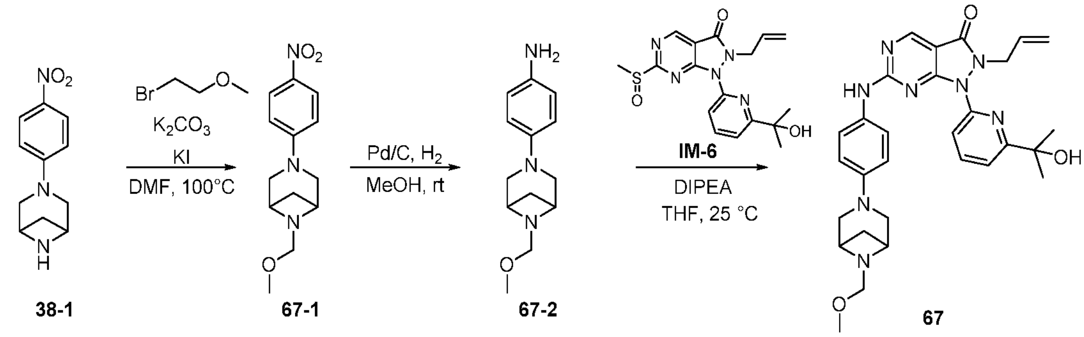


38-1 67-1 67-2 67

**Step 1: Synthesis of Compound 67-1:**

The substrate **38-1** (80 mg, 364.90 μmol) was added to a dry single-necked flask and dissolved in N,N-dimethylformamide (3 mL), then added with 2-Bromoethyl methyl ether (76 mg, 546.80 μmol), $K_2CO_3$ (99 mg, 716.35 μmol) and KI (5 mg, 30.12 μmol) to react for 6 hours at 100°C and monitored by LC-MS, then the reaction solution was cooled to room temperature after the reaction, extracted three times with ethyl acetate. The organic phase was dried with anhydrous $Na_2SO_4$, filtered, concentrated under reduced pressure to give the compound **67-1** (75 mg, 270.45 μmol). LCMS (ESI) m/z: 264.1 $[M+H]^+$.

**Step 2: Synthesis of Compound 67-2:**

The compound **67-1** (75 mg, 270.45 μmol) was added to a 50 mL round-bottomed flask and dissolved in methanol (3mL), added with a catalytic amount of Pd/C (15mg), stirred for 3 hours at room temperature under $H_2$ atmosphere and monitored by LC-MS. After the reaction, the reacton solution was filtered with diatomaceous earth, washed two times with methanol, the filterate was concentrated under reduced pressure to give the compound **67-2** (53 mg, 214.28 μmol). LCMS (ESI) m/z: 234.2 $[M+H]^+$.

**Step 3: Synthesis of Compound 67:**

The substrate **IM-6** (50 mg, 128.39 μmol) and tetrahydrofuran (3mL) were added to a dry single-necked flask, stirred to dissolve, then N,N-diisopropylethylamine (82.97 mg, 641.97 μmol) and 67-2 (53 mg, 214.28 μmol) were added to react for 4 hours at room temperature and monitored by LC-MS, then the solvents were removed by distillation under reduced pressure after the reaction. The residue was purified by preparative high-performance liquid chromatography (alkaline) to give the compound 67 (40 mg, 67.11 μmol). LCMS (ESI) m/z: 557.3 $[M+H]^+$, HPLC method B: $R_T$ = 6.43 min, purity: 93.4 %. $^1$H NMR (600 MHz, Chloroform-*d*) δ 8.82(s, 1H), 7.88 - 7.84(m, 1H), 7.77(d, *J* = 8.4 Hz, 1H), 7.46(s, 2H), 7.34-7.32(m, 1H), 6.72(d, *J* = 8.4 Hz, 2 H), 5.76 - 5.66(m, 1H), 5.06 - 5.03(m, 1H), 4.97 - 4.92(m, 1H), 4.74(d, *J* = 6.0 Hz, 2H), 3.95 - 3.93(m, 2H), 3.65(d, *J* = 10.8Hz, 2H), 3.52 - 3.50 (m, 2H), 3.39(d, *J* = 10.8Hz, 2H), 3.35(s, 3H), 2.83 - 2.81(m, 1H), 2.66 - 2.64(m, 2H), 1.68 (d, *J* = 8.4 Hz, 1H), 1.59(s, 6H).

**Example 68: Synthesis of Compound 68:**

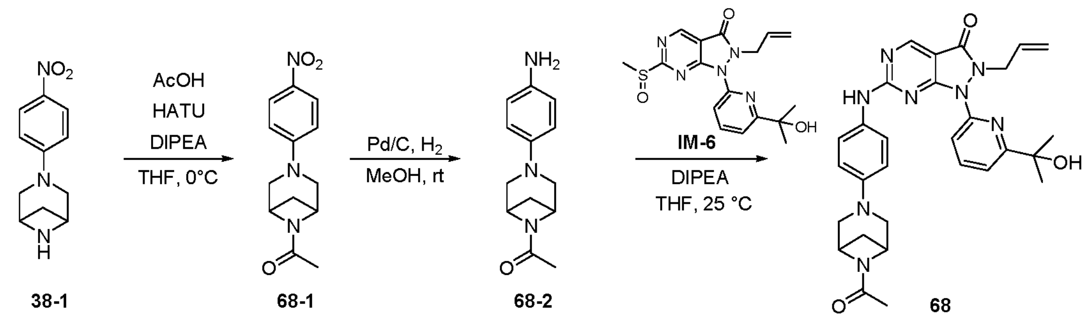


38-1 68-1 68-2 68

**Step 1: Synthesis of Compound 68-1:**

Acetic acid (18 mg, 299.74 μmol) and N,N-Diisopropylethylamine (42.61 mg, 329.72 μmol) were dissolved in tetrahydrofuran (2 mL) in a dry single-necked flask, cooled to 0°C and added with HATU (49.93 mg, 131.39 umol), stirred for 10 min, added with **38-1** (65.72 mg, 299.74 μmol) then stirred for 30 min, quenched with water after the reaction, extracted three times with ethyl acetate. The organic phase was dried with anhydrous $Na_2SO_4$, filtered, concentrated under reduced pressure. The residue was purified by MPLC to give the compound **68-1** (45 mg, 172.23 μmol). LCMS (ESI) m/z: 262.1$[M+H]^+$.

**Step 2: Synthesis of Compound 68-2:**

The compound **68-1** (45 mg, 172.23 μmol) was added to a 50 mL round-bottomed flask and dissolved in methanol (3mL), added with a catalytic amount of Pd/C (20mg), stirred for 1 hour at room temperature under $H_2$ atmosphere and monitored by LC-MS. After the reaction, the reacton solution was filtered with diatomaceous earth,

washed two times with methanol, the filterate was concentrated under reduced pressure to give the compound **68-2** (29 mg, 214.28 μmol). LCMS (ESI) m/z: 236.2 $[M+H]^+$.

**Step 3: Synthesis of Compound 68:**

The substrate **IM-6** (50 mg, 128.39 μmol) and tetrahydrofuran (3mL) were added to a dry single-necked flask, stirred to dissolve, then N,N-diisopropylethylamine (82.97 mg, 641.97 μmol) and 68-2 (29 mg, 125.38 μmol) were added to react for 4 hour at room temperature and monitored by LC-MS, then the solvents were removed by distillation under reduced pressure after the reaction.

The residue was purified by preparative high-performance liquid chromatography (alkaline) to give the compound **68** (51.3 mg, 89.10 μmol). LCMS (ESI) m/z: 541.3$[M+H]^+$, HPLC method B: $R_T$ = 6.04 min, purity:93.9 %. $^1H$ NMR (600 MHz, Chloroform-*d*) δ 8.79(s, 1H),7.87 - 7.83(m, 1H), 7.76(d, *J* = 8.4 Hz, 1H), 7.45(d, *J* = 7.2 Hz, 2H), 7.33(d, *J* = 7.6 Hz, 1H), 6.68(d, *J* = 8.4 Hz, 2 H), 5.75 - 5.65(m, 1H), 5.05 - 5.03(m, 1H), 4.94(d, *J* = 17.4 Hz, 1H), 4.74(d, *J* = 6.0 Hz, 2H), 4.61 - 4.54(m, 2H), 4.09(brs, 1H), 3.97 - 3.95(m, 1H), 3.68 - 3.58(m, 2H), 3.43 (d, *J* = 9.0 Hz, 1H), 2.78 - 2.73(m, 1H), 1.94(s, 3H), 1.79(brs, 1H), 1.71 (d, *J* = 8.4 Hz, 1H), 1.59(s, 6H).

**Example 69: Synthesis of Compound 69:**

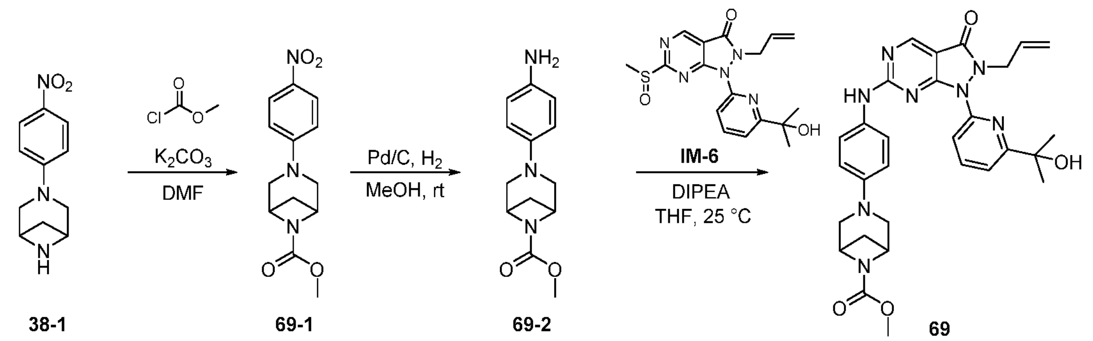


38-1 69-1 69-2 69

**Step 1: Synthesis of Compound 69-1:**

The substrate **38-1** (241.29 mg, 1.10 mmol) was added to a dry single-necked flask and dissolved in N,N-dimethylformamide (5 mL), then added with methyl chloroformate (104 mg, 1.10 mmol), $K_2CO_3$ (194 mg, 1.40 mmol) to react for 5 hours at room temperature and monitored by LC-MS, then extracted three times with ethyl acetate. The organic phase was dried with anhydrous $Na_2SO_4$, filtered, concentrated under reduced pressure to give the compound **69-1** (60 mg, 216.39 μmol). LCMS (ESI) m/z: 278.1 $[M+H]^+$.

**Step 2: Synthesis of Compound 69-2:**

The compound **69-1** (75 mg, 270.45 μmol) was added to a 50 mL round-bottomed flask and dissolved in methanol (3mL), added with a catalytic amount of Pd/C (15mg), stirred for 1 hour at room temperature under $H_2$ atmosphere and monitored by LC-MS. After the reaction, the reacton solution was filtered with diatomaceous earth, washed two times with methanol, the filterate was concentrated under reduced pressure to give the compound **69-2** (38 mg, 153.66 μmol). LCMS (ESI) m/z: 248.1 $[M+H]^+$.

**Step 3: Synthesis of Compound 69:**

The substrate **IM-6** (50 mg, 128.39 μmol) and tetrahydrofuran (3mL) were added to a dry single-necked flask, stirred to dissolve, then N,N-diisopropylethylamine (82.97 mg, 641.97 μmol) and 69-2 (38 mg, 153.66 μmol) were added to react for 4 hours at room temperature and monitored by LC-MS, then the solvents were removed by distillation under reduced pressure after the reaction. The residue was purified by preparative high-performance liquid chromatography (alkaline) to give the compound **69** (35 mg, 62.88 μmol). LCMS (ESI) m/z: 557.2$[M+H]^+$, HPLC method B: $R_T$= 7.13 min, purity: 98.7 %. $^1H$ NMR (600 MHz, Chloroform-*d*) δ 8.79(s, 1H), 7.87 - 7.84 (m, 1H), 7.77 (d, *J* = 8.4 Hz, 1H), 7.44 (s, 2H),7.33 (d, *J*=7.2 Hz, 1H), 6.68 (d, *J*=8.4 Hz, 2 H), 5.75 - 5.65 (m, 1H), 5.05 - 5.02 (m, 1H), 4.96 - 4.91 (m, 1H), 4.74(d, *J* = 6.0 Hz, 2H), 4.39 (d, *J* = 5.4 Hz, 2H), 3.93 - 3.83 (m, 2H), 3.65 (s, 3H), 3.37 (d, *J* = 10.2 Hz, 2H), 2.74 - 2.69 (m, 1H), 1.60 -

1.59 (m, 7H).

**Example 70: Synthesis of Compound 70:**

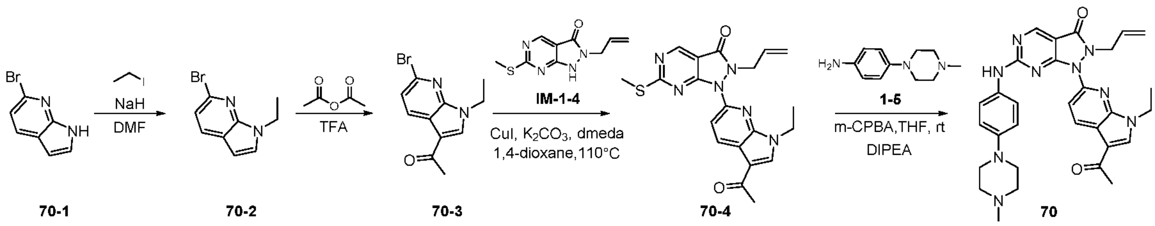


70-1 70-2 70-3 70-4 70

**Step 1: Synthesis of Compound 70-2:**

The compound **70-1** (100 mg, 507.53 μmol) was dissolved in tetrahydrofuran (3 ml) in a dry single-necked flask, cooled to 0°C and added with NaH (14.62 mg, 609.04 μmol), stirred for 10 min at 0°C, added with $C_2H_5I$ (94.99 mg, 609.04 μmol), warmed to room temperature and stirred for 2 hours, monitored by LC-MS, quenched with water after the reaction, extracted three times with ethyl acetate. The organic phase was dried with anhydrous $Na_2SO_4$, filtered, concentrated under reduced pressure. The residue was purified by MPLC to give the compound **70-2** (65 mg, 288.78 μmol). LCMS (ESI) m/z: 225.0[M+H]$^+$.

**Step 2: Synthesis of Compound 70-3:**

The compound **70-2** (65 mg, 288.78 μmol) was dissolved in trifluoroacetic acid (1 ml) in a dry single-necked flask, added with acetic anhydride (30.62 mg, 288.78 μmol, 0.5 mL), warmed to 70°C and stirred for 4 hours, monitored by LC-MS, cooled to room temperature after the reaction, concentrated under reduced pressure. The residue was purified by MPLC to give the compound **70-3** (40 mg, 149.74 μmol). LCMS (ESI) m/z:267.0[M+H]$^+$.

**Step 2: Synthesis of Compound 70-4:**

The substrate **70-3** (32.78 mg, 122.70 μmol), **IM-1-4** (30 mg, 134.97 μmol), CuI(46.74 mg, 245.41 μmol), $K_2CO_3$ (23.74 mg, 171.78 μmol) and N,N'-Dimethylethylenediamine (43.27 mg, 490.81 μmol) were added to a dry microwave tube under $N_2$ protection, then added with 1,4-Dioxane (2mL), heated up to 110°C to react for 3 hours under $N_2$ atmosphere and monitored by LC-MS. After the reaction was completed, the reaction system was cooled to room temperature, concentrated under reduced pressure to remove the solvents. The residue was purified by MPLC to give the compound **70-4** (33 mg, 74.26 μmol). LCMS (ESI) m/z: 409.1[M+H]$^+$.

**Step 3: Synthesis of Compound 70:**

The substrate **70-4** (59.71 mg, 146.17 μmol) was added to a dry single-necked flask, and added with tetrahydrofuran (1.5mL) to dissolve, then added with M-chloroperoxybenzoic acid (45.40 mg, 263.10 μmol) to react for 30 min at room temperature, then N,N-diisopropylethylamine (94.45 mg, 730.83 μmol) and **1-5** (33.55 mg, 175.40 μmol) were added to the reaction solution to react for 12 hours at room temperature and monitored by LC-MS, then concentrated under reduced pressure after the reaction. The residue was purified by preparative high-performance liquid chromatography (alkaline) to give the compound **70** (23 mg, 40.40 μmol). LCMS (ESI) m/z: 552.2 [M+H]$^+$, HPLC method B: $R_T$= 6.81 min, purity: 97.6%. $^1$H NMR (600 MHz, DMSO-$d_6$) δ 10.14 (s, 1H), 8.84 (s, 1H), 8.64 (d, , $J$=10.8 Hz, 2H), 7.74 (d, $J$=8.4 Hz, 1H), 7.56 (s, 2H), 6.91 - 6.89 (m, 2H), 5.71 - 5.66 (m, 1H), 4.50 - 4.99 (m, 1H), 4.85 (d, $J$=17.4 Hz, 1H), 4.66 - 4.59 (m, 2H), 4.35 - 4.32 (m, 2H), 3.09 - 3.08 (m, 4H), 2.49 (s, 3H), 2.48 - 2.46 (m, 4H), 2.23 (s, 3H), 1.47 - 1.45 (m, 3H).

**Example 71: Synthesis of Compound 71:**

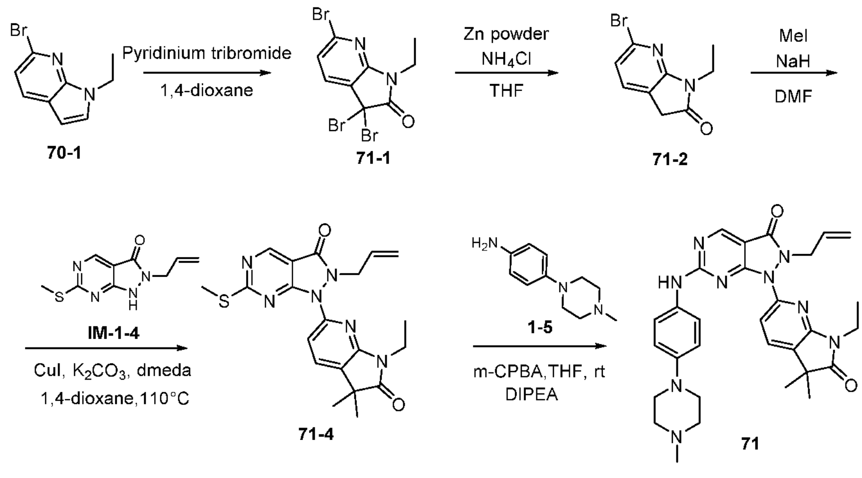


**Step 1: Synthesis of Compound 71-1:**

The compound **70-1** (600 mg, 2.67 mmol) was dissolved in 1,4-Dioxane (1 ml) in a dry single-necked flask, added with Pyridine tribromide (3.41 g, 10.66 mmol), stirred for 16 hours at room temperature and monitored by LC-MS, added with water after the reaction, extracted three times with $CH_2Cl_2$. The organic phase was dried with anhydrous $Na_2SO_4$, filtered, concentrated under reduced pressure. The residue was purified by MPLC to give the compound **71-1** (230 mg, 576.62 μmol). LCMS (ESI) m/z: 398.8[M+H]$^+$.

**Step 2: Synthesis of Compound 71-2:**

The compound **71-1** (230 mg, 576.62 μmol) was dissolved in tetrahydrofuran (1.5 mL) in a dry single-necked flask, added with zinc powder (45.57 mg, 696.93 μmol) and saturated $NH_4Cl$ solution (1.5 mL), stirred for 10 min at room temperature, monitored by LC-MS, filtered with diatomaceous earth after the reaction, the filterate was concentrated under reduced pressure The residue was purified by MPLC to give the compound **71-2** (44 mg, 182.51 μmol). LCMS (ESI) m/z: 241.0[M+H]$^+$.

**Step 3: Synthesis of Compound 71-3:**

The compound **71-2** (250 mg, 1.16 mmol) was dissolved in tetrahydrofuran (2 ml) in a dry single-necked flask, cooled to 0°C and added with NaH (83.30 mg, 3.47 mmol), stirred for 10 min at 0°C, added with $CH_3I$ (492.7 mg, 3.47 mmol), warmed to room temperature and stirred for 6 hours, monitored by LC-MS, quenched with water after the reaction, extracted three times with ethyl acetate. The organic phase was dried with anhydrous $Na_2SO_4$, filtered, concentrated under reduced pressure. The residue was purified by column chromatography to give the compound **71-3** (210 mg, 912.64 μmol). LCMS (ESI) m/z: 269.0[M+H]$^+$.

**Step 4: Synthesis of Compound 71-4:**

The substrate **71-3** (17.61 mg, 65.44 μmol), **IM-1-4** (16 mg, 71.99 μmol), CuI (24.93 mg, 130.88 μmol), $K_2CO_3$ (12.66 mg, 91.62 μmol) and N,N'-Dimethylethylenediamine (23.07 mg, 261.77 μmol) were added to a dry microwave tube under $N_2$ protection, then added with 1,4-Dioxane (1mL), heated up to 110°C to react for 3 hours under $N_2$ atmosphere and monitored by LC-MS. After the reaction was completed, the reaction system was cooled to room temperature, concentrated under reduced pressure. The residue was purified by MPLC to give the compound **71-4** (2.01 mg, 4.91 μmol). LCMS (ESI) m/z: 411.2[M+H]$^+$.

**Step 3: Synthesis of Compound 71:**

The substrate **71-4** (2.01 mg, 4.91 μmol) was added to a dry single-necked flask, and added with tetrahydrofuran (2mL) to dissolve, then added with M-chloroperoxybenzoic acid (1.52 mg, 8.83 μmol) to react for 1 hour at room temperature, then N,N-diisopropylethylamine (3.17 mg, 24.54 μmol, 4.27 μL) and **1-5** (938.70 μg, 4.91 μmol) were added to the reaction solution to react for 8 hours at room temperature and monitored by LC-MS, then concentrated under reduced pressure after the reaction. The residue was purified by preparative high-performance liquid chromatography (alkaline) to give the compound **71** (2.3 mg, 3.45 μmol). LCMS (ESI) m/z: 554.3 [M+H]$^+$, HPLC method B: $R_T$ =5.89 min,

purity:83.2%. $^1$H NMR (600 MHz, CDCL3) δ 8.77 (s, 1H), 7.51 (d, *J* =7.8 Hz, 1H), 7.42 (d, J=1.8 Hz, 2H), 7.36 (d, *J* =8.4 Hz, 1H), 6.87-6.84 (m, 2H), 5.70 - 5.63 (m, 1H), 4.99 (d, *J* = 10.2 Hz, 1H), 4.92 (d, *J* =16.8 Hz, 1H), 4.63 (d, *J* =5.4 Hz, 2H), 3.80 - 3.77 (m, 2H), 3.36 - 3.24 (m, 4H), 2.87 - 2.80 (m, 4H), 2.49 - 2.45 (m, 3H), 1.36 (s, 6H), 1.23 - 1.21 (m, 3H).

**Example 72: Synthesis of Compound 72:**

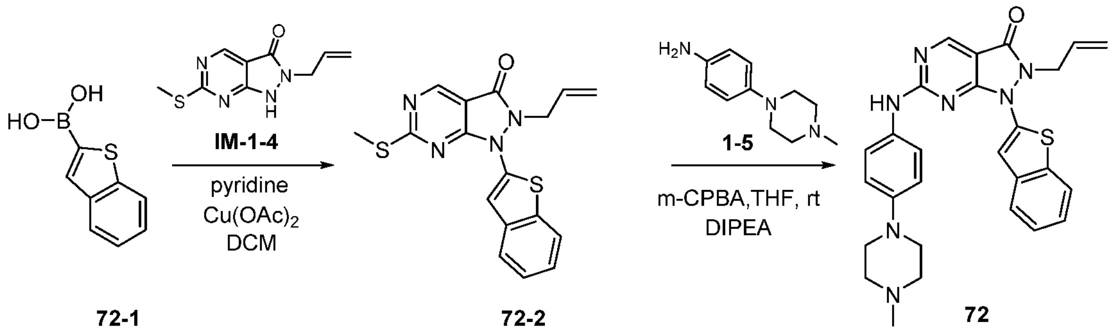


72-1 72-2 72

**Step 1: Synthesis of Compound 72-2:**

The substrate **72-1** (88 mg, 395.92 μmol) was added to a dry single-necked flask, and dissolved in $CH_2Cl_2$ (20mL), then added with **IM-1-4** (170 mg, 764.85 μmol), pyridine (0.4 mL), $Cu(Ac)_2$ (146 mg, 803.83 μmol), stirred for 24 hours at 30°C and monitored by LC-MS, then concentrated under reduced pressure after the reaction. The residue was purified by MPLC to give the compound **72-2** (28 mg, 79.00 μmol). LCMS (ESI) m/z: 354.95 $[M+H]^+$.

**Step 2: Synthesis of Compound 72:**

The substrate **72-2** (28 mg, 79.00 μmol) was added to a dry single-necked flask, and added with tetrahydrofuran (2mL) to dissolve, then added with M-chloroperoxybenzoic acid (27.26 mg, 157.99 μmol) to react for 1 hour at room temperature, then N,N-diisopropylethylamine (16 mg, 83.65 μmol) and **1-5** (938.70 μg, 4.91 μmol) were added to the reaction solution to react for 2 hours at room temperature and monitored by LC-MS, then concentrated under reduced pressure after the reaction. The residue was purified by preparative high-performance liquid chromatography (alkaline) to give the compound **72** (9 mg, 17.72 μmol). LCMS (ESI) m/z: 498. 1$[M+H]^+$, HPLC method B: $R_T$ = 8.01 min, purity: 98%. $^1$H NMR (400 MHz, Chloroform-*d*) δ 8.84 (s, 1H), 7.85 - 7.81 (m, 2H), 7.48 - 7.41 (m, 5H), 6.86 (d, *J* = 8.4 Hz, 2H), 5.79-5.72 (m, 1H), 5.18 - 5.09 (m, 2H), 4.47 (d, *J* = 6.0 Hz, 2H), 3.32 (s, 4H), 2.86 (s, 4H), 2.55 (s, 3H).

**Example 73: Synthesis of Compound 73:**

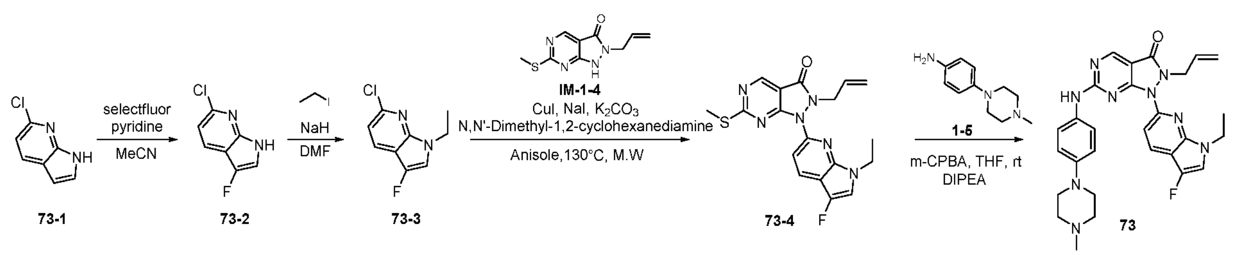


73-1 73-2 73-3 73-4 73

**Step 1: Synthesis of Compound 73-2:**

The compound **73-1** (0.5 g, 3.28 mmol) was dissolved in acetonitrile (5 mL) in a dry single-necked flask, added with pyridine (1.5 mL) and **Selectfluor** reagent (1.05 g, 2.95 mmol), stirred for 16 hours at 15°C and monitored by LC-MS, added with water after the reaction, extracted three times with ethyl acetate. The organic phase was dried with anhydrous $Na_2SO_4$, filtered, concentrated under reduced pressure. The residue was purified by prep-HPLC to give the compound **73-2** (112 mg, 656.62 μmol). LCMS (ESI) m/z: 171.0 $[M+H]^+$.

**Step 2: Synthesis of Compound 73-3:**

The compound **73-2** (0.112 g, 656.62 μmol) was dissolved in N,N-Dimethylformamide (3 ml) in a dry single-necked flask, cooled to 0°C and added with NaH (39.40 mg, 984.93 μmol, 60% purity), stirred for 30 min at 0°C, added with $C_2H_5I$ (153.65 mg, 984.93 μmol, 78.79 μL), warmed to room temperature and stirred for 16 hours, monitored by LC-MS, quenched with saturated $NH_4Cl$ solution after the reaction, extracted three times with ethyl acetate. The organic phase was dried with anhydrous $Na_2SO_4$, filtered, concentrated under reduced pressure. The residue was purified by column chromatography to give the compound **73-3** (120 mg, 604.16 μmol). LCMS (ESI) m/z: 199.2 $[M+H]^+$.

**Step 3: Synthesis of Compound 73-4:**

The substrate **73-3** (0.04 g, 201.39 μmol), **IM-1-4** (44.76 mg, 201.39 μmol), CuI (76.71 mg, 402.77 μmol), NaI (60.37 mg, 402.77 μmol), $K_2CO_3$ (69.58 mg, 503.46 μmol) and N,N'-Dimethylethylenediamine (114.58 mg, 805.54 μmol) were added to a dry microwave tube under $N_2$ protection, then added with anisole (1.5mL), microwave heated up to 130°C to react for 4 hours under $N_2$ atmosphere and monitored by LC-MS. After the reaction was completed, the reaction system was cooled to room temperature, added with water and ammonia (1 mL), extracted three times with ethyl acetate. The organic phase was dried with anhydrous $Na_2SO_4$, filtered, concentrated under reduced pressure. The residue was purified by column chromatography to give the compound **73-4** (20 mg, 52.03 μmol). LCMS (ESI) m/z: 385.0 $[M+H]^+$.

**Step 4: Synthesis of Compound 73:**

The substrate **73-4** (20 mg, 52.03 μmol) was added to a dry single-necked flask, and added with tetrahydrofuran (1.5mL) to dissolve, then added with M-chloroperoxybenzoic acid (19.01 mg, 93.65 μmol, 85% purity) to react for 1 hour at room temperature, then N,N-diisopropylethylamine (67.24 mg, 520.25 μmol, 90.62 μL) and **1-5** (11.94 mg, 62.43 μmol) were added to the reaction solution to react for 5 hours at room temperature and monitored by LC-MS, then concentrated under reduced pressure after the reaction. The residue was purified by preparative high-performance liquid chromatography (alkaline) to give the compound **73** (20.7 mg, 37.39 μmol). LCMS (ESI) m/z: 528.3 $[M+H]^+$, HPLC method B: $R_T$ = 8.09 min, purity:95.3%. $^1H$ NMR (600 MHz, DMSO-$d_6$) δ 10.16 (s, 1H), 8.84 (s, 1H), 8.32 (s, 1H), 7.67 (s, 1H), 7.65 (d, *J* = 8.4 Hz, 1H), 7.61 (s, 2H), 6.92 (d, *J* = 8.4 Hz, 2H), 5.74 - 5.68 (m, 1H), 5.00 (d, *J* = 10.2 Hz, 1H), 4.86 (d, *J* = 17.4 Hz, 1H), 4.65 (s, 2H), 4.26 - 4.22 (m, 2H), 3.12 - 3.09 (m, 4H), 2.47 - 2.45 (m, 4H), 2.22 (s, 3H), 1.39 - 1.36 (m, 3H).

**Example 74: Synthesis of Compound 74:**

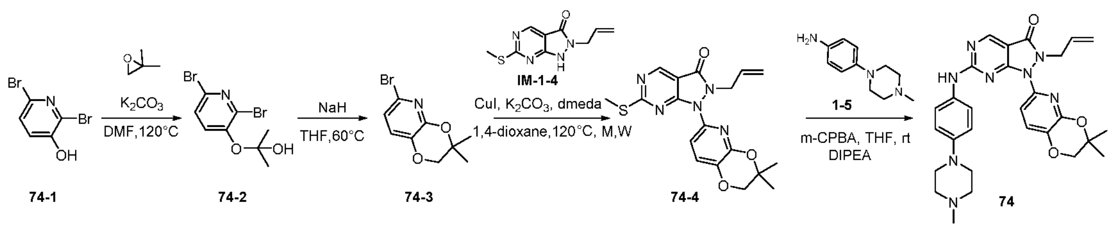


**Step 1: Synthesis of Compound 74-2:**

The compound **74-1** (1.01 g, 3.99 mmol) was dissolved in N,N-Dimethylformamide (30 mL) in a dry single-necked flask, added with isobutylene oxide (345.57 mg, 4.79 mmol) and $K_2CO_3$ (1.66 g, 11.98 mmol), warmed up to 120°C to react for 8 hours and monitored by LC-MS, cooled to room temperature after the reaction, extracted three times with ethyl acetate. The organic phase was dried with anhydrous $Na_2SO_4$, filtered, concentrated under reduced pressure. The residue was purified by column chromatography to give the compound **74-2** (680 mg, 2.09 mmol). LCMS (ESI) m/z: 311.9 $[M+H]^+$.

**Step 2: Synthesis of Compound 74-3:**

The compound **74-2** (98 mg, 301.54 μmol) was dissolved in tetrahydrofuran (4 ml) in a dry single-necked flask, cooled to 0°C and added in batches with NaH (24.12 mg, 603.08 μmol, 60% purity), warmed up to 60°C and stirred for 30 hours, monitored by LC-MS, quenched with saturated $NH_4Cl$ solution after the reaction, extracted three times with ethyl

acetate. The organic phase was dried with anhydrous $Na_2SO_4$, filtered, concentrated under reduced pressure. The residue was purified by column chromatography to give the compound **74-3** (48 mg, 196.65 μmol). LCMS (ESI) m/z: 244.0 $[M+H]^+$.

**Step 3: Synthesis of Compound 74-4:**

The substrate **74-3** (47 mg, 192.56 μmol), **IM-1-4** (31.77 mg, 142.96 μmol), CuI (36.67 mg, 192.56 μmol), $K_2CO_3$ (38.30 mg, 277.14 μmol) and N,N'-Dimethylethylenediamine (33.95 mg, 385.11 μmol) were added to a dry microwave tube under $N_2$ protection, then added with 1,4-Dioxane (3mL), microwave heated up to 120°C to react for 4 hours under $N_2$ atmosphere and monitored by LC-MS. After the reaction was completed, the reaction system was cooled to room temperature, concentrated under reduced pressure. The residue was purified by MPLC to give the compound **74-4** (12 mg, 31.13 μmol). LCMS (ESI) m/z: 386.1$[M+H]^+$.

**Step 4: Synthesis of Compound 74:**

The substrate **74-4** (12 mg, 31.13 μmol) was added to a dry single-necked flask, and added with tetrahydrofuran (1mL) to dissolve, then added with M-chloroperoxybenzoic acid (21.49 mg, 124.53 μmol) to react for 0.5 hour at room temperature, then N,N-diisopropylethylamine (20.22 mg, 156.46 μmol, 27.25 μL) and **1-5** (20 mg, 104.56 μmol) were added to the reaction solution to react for 8 hours at room temperature and monitored by LC-MS, then concentrated under reduced pressure after the reaction. The residue was purified by preparative high-performance liquid chromatography (alkaline) to give the compound **74** (7.2 mg, 12.56 μmol). LCMS (ESI) m/z: 529.2$[M+H]^+$, HPLC method B: $R_T$ = 7.08 min, purity: 92.2%. $^1H$ NMR (600 MHz, Chloroform-*d*) δ 8.80 (s, 1H), 7.47 (d, *J* = 7.8 Hz, 2H), 7.37 - 7.30 (m, 2H), 6.90 (d, *J* = 8.4 Hz, 2H), 5.74 - 5.68 (m, 1H), 5.05 (d, *J* = 10.2 Hz, 1H), 4.98 (d, *J* = 16.8 Hz, 1H), 4.69 (s, 2H), 4.11 (s, 2H), 3.24 (s, 4H), 2.69 (s, 4H), 2.43 (s, 3H), 1.41 (s, 6H).

**Example 75: Synthesis of Compound 75:**

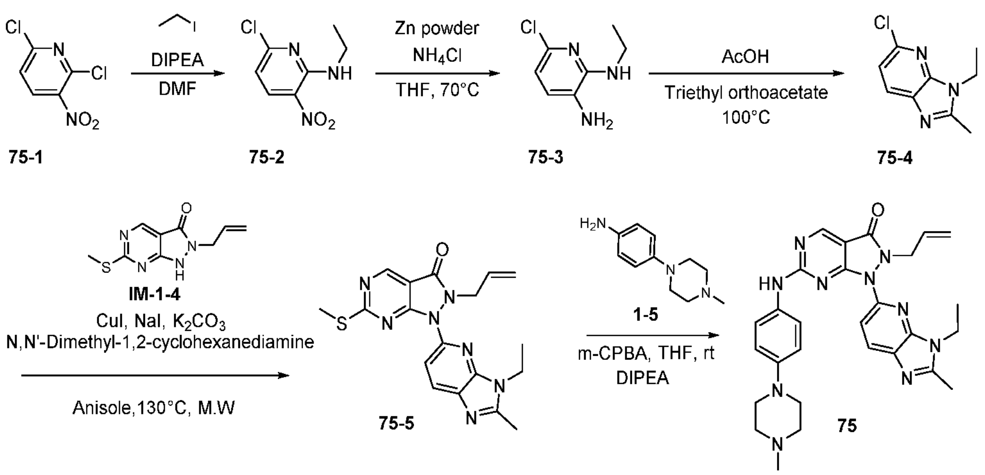

75-1 75-2 75-3 75-4 75-5 75

**Step 1: Synthesis of Compound 75-2:**

The compound **75-1** (965 mg, 5.00 mmol) was dissolved in N,N-Dimethylformamide (20 mL) in a dry single-necked flask, added with $C_2H_5I$ (383 mg, 4.70 mmol) and N,N-Diisopropylethylamine (1.29 g, 10.00 mmol) to 120°C to react for 1 hour at room temperature and monitored by TLC, extracted three times with ethyl acetate after the reaction. The organic phase was dried with anhydrous $Na_2SO_4$, filtered, concentrated under reduced pressure. The residue was purified by column chromatography to give the compound **75-2** (650 mg, 3.22 mmol). LCMS (ESI) m/z:202.0 $[M+H]^+$.

**Step 2: Synthesis of Compound 75-3:**

The compound **75-2** (308 mg, 1.53 mmol) was dissolved in tetrahydrofuran (5 mL) in a dry single-necked flask, added with zinc powder (1.6 g, 24.62 mmol) and $NH_4Cl$ (642 mg, 12.00 mmol), warmed up to 70°C and stirred for 3 hours, monitored by LC-MS, filtered with diatomaceous earth after the reaction, the filterate was concentrated under reduced

pressure The residue was purified by MPLC to give the compound **75-3** (248 mg, 1.44 mmol). LCMS (ESI) m/z: 172.1 $[M+H]^+$.

**Step 3: Synthesis of Compound 75-4:**

The substrate **75-3** (248 mg, 1.44 mmol) was dissolved in triethyl orthoacetate (4 ml) in a dry single-necked flask, stirred for 1 hour at 100°C, added with acetic acid (1.05 g, 17.47 mmol, 1 mL), continue to be stirred for 8 hours, monitored by LC-MS. After the reaction was completed, the reaction system was cooled to room temperature, concentrated under reduced pressure, extracted three times with ethyl acetate after the reaction,. The organic phase was dried with anhydrous $Na_2SO_4$, filtered, concentrated under reduced pressure. The residue was purified by MPLC to give the compound **75-4** (220 mg, 1.12 mmol). LCMS (ESI) m/z: 196.1 $[M+H]^+$.

**Step 4: Synthesis of Compound 75-5:**

The substrate **75-4** (64 mg, 327.12 μmol), **IM-1-4** (72.71 mg, 327.12 μmol), CuI (124.60 mg, 654.23 μmol), NaI (98.06 mg, 654.23 μmol), $K_2CO_3$ (38.30 mg, 277.14 μmol) and N,N'-Dimethylethylenediamine (186.12 mg, 1.31 mmol) were added to a dry microwave tube under $N_2$ protection, then added with anisole (2mL), microwave heated up to 130°C to react for 10 hours under $N_2$ atmosphere and monitored by LC-MS. After the reaction was completed, the reaction system was cooled to room temperature, added with water and ammonia (1 mL) , extracted three times with ethyl acetate. The organic phase was dried with anhydrous $Na_2SO_4$, filtered, concentrated under reduced pressure. The residue was purified by column chromatography to give the compound **75-5** (15 mg, 39.32 μmol). LCMS (ESI) m/z: 382.2 $[M+H]^+$.

**Step 5: Synthesis of Compound 75:**

The substrate **75-5** (15 mg, 39.32 μmol) was added to a dry single-necked flask, and added with tetrahydrofuran (2mL) to dissolve, then added with M-chloroperoxybenzoic acid (20.36 mg, 117.97 μmol) to react for 1 hour at room temperature, then N,N-diisopropylethylamine (25.36 mg, 196.25 μmol, 34.18 μL) and **1-5** (18.77 mg, 98.12 μmol) were added to the reaction solution to react for 8 hours at room temperature and monitored by LC-MS, then concentrated under reduced pressure after the reaction. The residue was purified by preparative high-performance liquid chromatography (alkaline) to give the compound **75** (3.2 mg, 5.12 μmol). LCMS (ESI) m/z: 525.2$[M+H]^+$, HPLC method B: $R_T$ = 6.16 min, purity: 84.0%. $^1H$ NMR (600 MHz, Chloroform-*d*) δ 8.84 (s, 1H), 8.07 (d, *J* = 7.8 Hz, 1H), 7.66 (d, *J* = 7.2 Hz, 1H), 7.46 (d, *J* = 8.4 Hz, 2H), 6.88 (d, *J* = 8.4 Hz, 2H), 5.75 - 5.68 (m, 1H), 5.00 (d, *J* = 10.2 Hz, 1H), 4.89 (d, *J* = 17.4 Hz, 1H), 4.73 (d, *J*= 3.6 Hz, 2H), 4.32 - 4.28 (m, 2H), 3.25 (s, 4H), 2.71 - 2.68 (m, 7H), 2.45 (s, 3H), 1.47 - 1.42 (m, 3H).

**Example 76: Synthesis of Compound 76:**

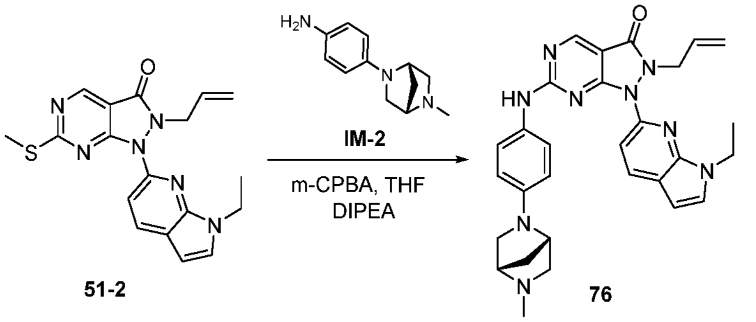

51-2
76

**Step 1: Synthesis of Compound 76:**

The substrate **51-2** (26 mg, 70.95 μmol) was added to a dry single-necked flask, and added with tetrahydrofuran (2mL) to dissolve, then added with M-chloroperoxybenzoic acid (35 mg, 202.82 μmol) to react for 2 hours at 50°C, then N,N-diisopropylethylamine (45.62 mg, 353.00 μmol) and IM-2 (22 mg, 108.22 μmol) were added to the reaction solution to react for 8 hours at room temperature and monitored by LC-MS, then concentrated under reduced pressure after the reaction. The residue was purified by preparative high-performance liquid chromatography (alkaline) to give the compound **76** (15 mg, 28.76 μmol). LCMS (ESI) m/z: 522.3$[M+H]^+$, HPLC method B: $R_T$ = 7.68 min, purity: 94.4%.

$^1$H NMR (600 MHz, Chloroform-*d*) δ 8.83 (s, 1H), 8.03 (d, *J* = 7.8 Hz, 1H), 7.53 - 7.43 (m, 3H), 7.28 (d, *J* = 3.6 Hz, 1H), 6.54 - 6.52 (m, 3H), 5.75 - 5.69 (m, 1H), 5.00 (d, *J* = 10.2 Hz, 1H), 4.90((d, *J* = 17.4 Hz, 1H), 4.76 (s, 2H), 4.34 - 4.28 (m, 3H), 3.78 (s, 1H), 3.51 - 3.45 (m, 2H), 3.21 (s, 1H), 2.84 (s, 1H), 2.53 (s, 3H), 2.21 (s, 1H), 2.06 (d, *J* = 9.6 Hz, 1H), 1.51 - 1.46 (m, 3H).

**Example 77: Synthesis of Compound 77:**

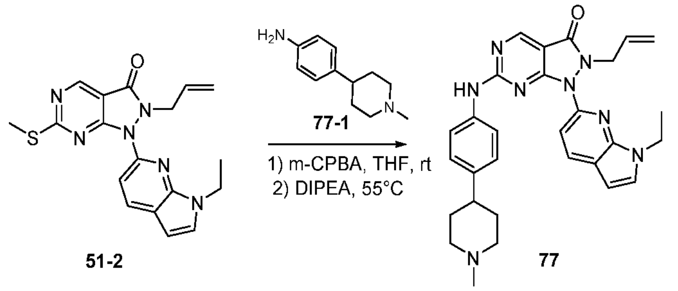


**Step 1: Synthesis of Compound 77:**

The substrate **51-2** (26 mg, 70.95 μmol) was added to a dry single-necked flask, and added with tetrahydrofuran (2mL) to dissolve, then added with M-chloroperoxybenzoic acid (35 mg, 202.82 μmol) to react for 6 hours at 55°C, then N,N-diisopropylethylamine (45.62 mg, 353.00 μmol) and 77-1 (17 mg, 89.34 μmol) were added to the reaction solution to react for 8 hours at room temperature and monitored by LC-MS, then concentrated under reduced pressure after the reaction. The residue was purified by preparative high-performance liquid chromatography (alkaline) to give the compound **77** (5 mg, 9.83 μmol). LCMS (ESI) m/z: 509.6[M+H]$^+$, HPLC method B: $R_T$= 8.50 min, purity: 98.2%. $^1$H NMR (600 MHz, DMSO-$d_6$) δ 10.18 (s, 1H), 8.87 (s, 1H), 8.23 (d, *J* = 7.8 Hz, 1H), 7.67 (d, *J* = 3.0 Hz, 3H), 7.53 (d, *J* = 7.8 Hz, 1H), 7.16 (d, *J* = 8.4 Hz, 2H), 6.58 (d, *J* = 3.6 Hz, 1H), 5.74 - 5.68 (m, 1H), 5.00 (d, *J* = 7.2 Hz, 1H), 4.86 (d, *J* = 16.8 Hz, 1H), 4.61 (s, 2H), 4.30 - 4.26 (m, 2H), 2.86 (d, *J* = 11.4 Hz, 2H), 2.41 - 2.39 (m, 1H), 2.19 (s, 3H), 1.97 - 1.93 (m, 2H), 1.71 - 1.62 (m, 4H), 1.41 - 1.38 (m, 3H).

**Example 78: Synthesis of Compound 78:**

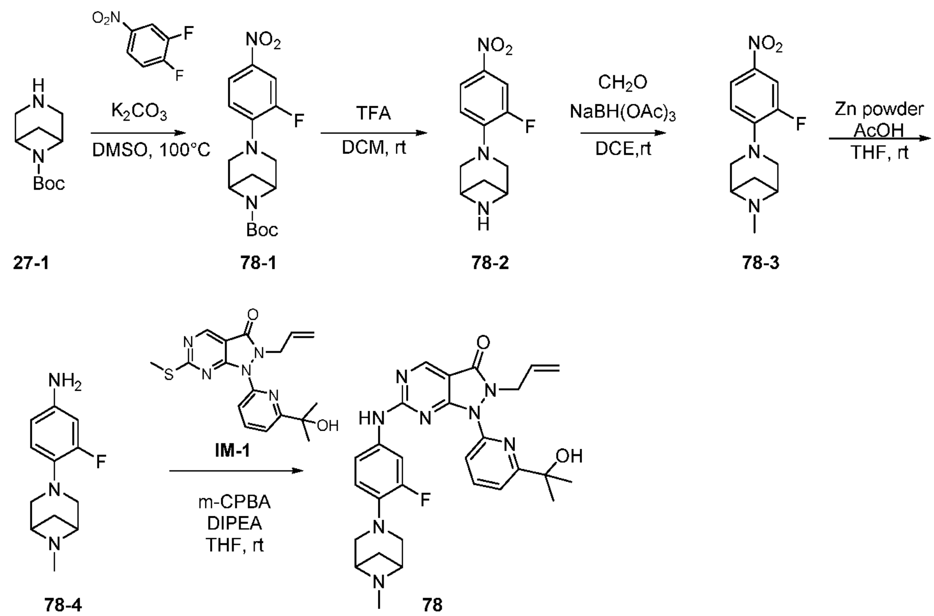

**Step 1: Synthesis of Compound 78-1:**

3,4-Difluoronitrobenzene (159 mg, 999.43 μmol) was dissolved in dimethyl sulfoxide (3 mL) in a dry single-necked flask and, added sequentially with **27-1** (237.78 mg, 1.20 mmol) and $K_2CO_3$ (137.92 mg, 999.43 μmol). The reaction mixture was stirred overnight at 100°Cand monitored by LC-MS, then diluted with water after the reaction and extracted three times with ethyl acetate. The organic phase was dried with anhydrous $Na_2SO_4$, filtered, concentrated under reduced pressure. The residue was purified by column chromatography to give the compound **78-1** (302 mg, 890 μmol). LCMS (ESI) m/z: 338.2[M+H]$^+$.

**Step 2: Synthesis of Compound 78-2:**

The substrate **78-1** (337.35 mg, 1 mmol) was added to a dry single-necked flask, and dissolved in $CH_2Cl_2$ (2mL), then added slowly with trifluoroacetic acid (1.54 g, 13.51 mmol, 1 mL) to react for 0.5 hour at room temperature and monitored by LC-MS, then concentrated by distillation under reduced pressure to remove the solvents to give the compound **78-2** (223 mg, 0.94 mmol). LCMS (ESI) m/z: 238.1[M+H]$^+$.

**Step 3: Synthesis of Compound 78-3:**

The substrate **78-2** (223 mg, 0.94 mmol) was added to a dry single-necked flask, and dissolved in 1,2-Dichloroethane (3 mL), added with formaldehyde (150.13 mg, 5.00 mmol), stirred for 10 min at room temperature, added with sodium triacetoxyborohydride (1.06 g, 5.00 mmol), stirred for 1 hour at room temperature, cooled to room temperature after the reaction, added with water for dilution, extracted three times with ethyl acetate. The organic phase was dried with anhydrous $Na_2SO_4$, filtered, concentrated under reduced pressure. The residue was purified by column chromatography to give the compound **78-3** (198 mg, 0.78 mmol). LCMS (ESI) m/z: 252.1 [M+H]$^+$.

**Step 4: Synthesis of Compound 78-4:**

The compound **78-3** (251.26 mg, 1 mmol) was dissolved in tetrahydrofuran (10 mL) and acetic acid (0.5 mL) in a dry single-necked flask, added with zinc powder (1.95 g, 30.00 mmol), stirred for 30 min at room temperature, monitored by LC-MS, filtered with diatomaceous earth after the reaction, the filterate was concentrated under reduced pressure to give the compound **78-4** (200 mg, 903.86 μmol). LCMS (ESI) m/z: 222.1 [M+H]$^+$.

**Step 5: Synthesis of Compound 78:**

The substrate **IM-1** (42.27 mg, 118.27 μmol) was added to a dry single-necked flask, and added with tetrahydrofuran (1mL) to dissolve, then added with M-chloroperoxybenzoic acid (30.61 mg, 177.40 μmol) to react for 30 min at room temperature, then N,N-diisopropylethylamine (76.43 mg, 591.35 μmol) and **78-4** (27 mg, 118.27 μmol) were added to the reaction solution to react for 4 hours at room temperature and monitored by LC-MS, then concentrated under reduced pressure after the reaction. The residue was purified by preparative high-performance liquid chromatography (alkaline) to give the compound **78** (2 mg, 3.72 μmol). LCMS (ESI) m/z : 531.7 [M+H]$^+$. HPLC: $R_T$= 7.53 min, purity: 85.0%. $^1$H NMR (600 MHz, DMSO-$d_6$) δ 10.28 (s, 1H), 8.85 (s, 1H), 8.002 - 7.99 (m, 1H), 7.76 (d, *J* = 7.8 Hz, 1H), 7.71 - 7.67 (m, 1H), 7.63 (d, *J* = 7.8 Hz, 1H), 7.34 (d, *J* = 8.4 Hz, 1H), 6.95 - 6.91 (m, 1H), 5.70 - 5.64 (m, 1H), 5.34 (s, 1H), 5.00 (d, *J* = 10.2 Hz, 1H), 4.83 (d, *J* = 17.4 Hz, 1H), 4.68 (d, *J* = 6.0 Hz, 2H), 3.63 (d, *J* = 11.1 Hz, 2H), 3.50 (d, *J* = 5.9 Hz, 2H), 3.43 (s, 2H), 3.17 (s, 2H), 2.04 (s, 3H), 1.46 (s, 6H).

**Example 79: Synthesis of Compound 79:**

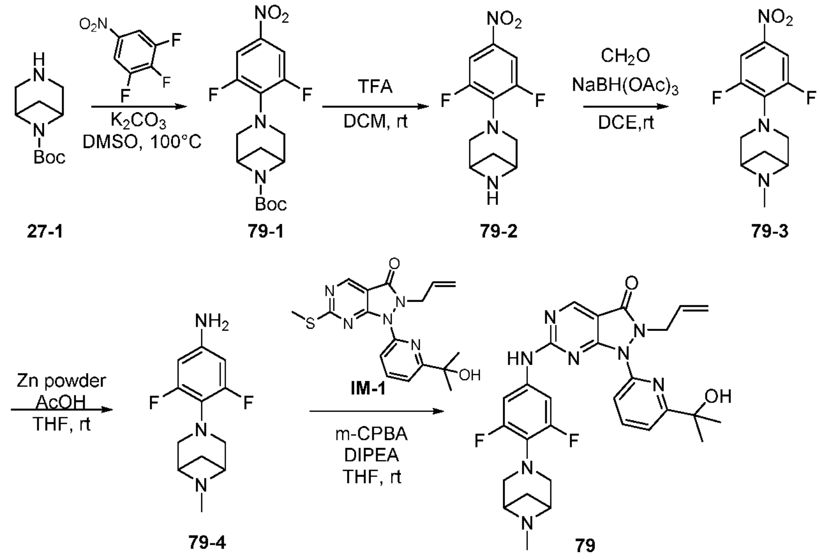


**Step 1: Synthesis of Compound 79-1:**

3,4-Difluoronitrobenzene (354.16 mg, 2 mmol) was dissolved in dimethyl sulfoxide (5 mL) in a dry single-necked flask and, added sequentially with 27-1 (475.83 mg, 2.40 mmol) and $K_2CO_3$ (276.00 mg, 2.00 mmol). The reaction mixture was stirred for 1 hour at 100°C, then diluted with water after the reaction and extracted three times with ethyl acetate. The organic phase was dried with anhydrous $Na_2SO_4$, filtered, concentrated under reduced pressure. The residue was purified by column chromatography to give the compound **79-1** (635 mg, 1.79 mmol). LCMS (ESI) m/z: 356.1$[M+H]^+$.

**Step 2: Synthesis of Compound 79-2:**

The substrate **79-1** (635 mg, 1.79 mmol) was added to a dry single-necked flask, and dissolved in $CH_2Cl_2$ (5mL), then added slowly with trifluoroacetic acid (2 mL) to react for 0.5 hour at room temperature and monitored by LC-MS, then concentrated by distillation under reduced pressure to remove the solvents to give the compound **79-2** (300 mg, 1.18 mmol). LCMS (ESI) m/z: 256.1$[M+H]^+$.

**Step 3: Synthesis of Compound 79-3:**

The substrate **79-2** (150 mg, 587.73 μmol) was added to a dry single-necked flask, and dissolved in 1,2-Dichloroethane (2 mL), added with formaldehyde (88.24 mg, 2.94 mmol), stirred for 10 min at room temperature, added with sodium triacetoxyborohydride (622.81 mg, 2.94 mmol), stirred for 1 hour at room temperature, cooled to room temperature after the reaction, added with water for dilution, extracted three times with $CH_2Cl_2$. The organic phase was dried with anhydrous $Na_2SO_4$, filtered, concentrated under reduced pressure to give the compound **79-3** (131 mg, 487 μmol). LCMS (ESI) m/z: 270.1$[M+H]^+$.

**Step 4: Synthesis of Compound 79-4:**

The compound **79-3** (131 mg, 487 μmol) was dissolved in tetrahydrofuran (3 mL) and acetic acid (0.2 mL) in a dry single-necked flask, added with zinc powder (0.96 g, 14.61 mmol), stirred for 30 min at room temperature, monitored by LC-MS, filtered with diatomaceous earth after the reaction, the filterate was concentrated under reduced pressure to give the compound **79-4** (123 mg, 515 μmol). LCMS (ESI) m/z: 240.1$[M+H]^+$.

**Step 5: Synthesis of Compound 79:**

The substrate **IM-1** (15 mg, 42.20 μmol) was added to a dry single-necked flask, and added with tetrahydrofuran (1mL) to dissolve, then added with M-chloroperoxybenzoic acid (14.09 mg, 84.40 μmol) to react for 30 min at room temperature, then N,N-diisopropylethylamine (27.27 mg, 211.00 μmol) and **79-4** (10.10 mg, 42.20 μmol) were added to the reaction solution to react for 3 hours at room temperature and monitored by LC-MS, then concentrated under reduced pressure after the reaction. The residue was purified by preparative high-performance liquid chromatography (alkaline) to give the compound **79** (1.5 mg, 2.48 μmol). LCMS (ESI) m/z : 549.3 $[M+H]^+$. HPLC: $R_T$ = 7.95 min, purity: 90.6%. $^1H$ NMR

(600 MHz, DMSO-$d_6$) δ 10.51 (s, 1H), 8.95 (s, 1H), 8.03 - 7.99 (m, 1H), 7.75 (d, *J* = 12.0 Hz, 1H), 7.67 (d, *J* = 12.0 Hz, 1H), 7.55 (d, *J* = 7.8 Hz, 1H), 5.71 - 5.64 (m, 1H), 5.70 - 5.64 (m, 1H), 5.35 (s, 1H), 5.01 - 4.98 (m, 1H), 4.84 (d, *J* = 1.8 Hz, 1H), 4.80 (d, *J* = 1.8 Hz, 1H), 4.69 (d, *J* = 8.0 Hz, 1H), 3.62 (d, *J* = 15.6 Hz, 2H), 3.17 (d, *J* = 10.2 Hz, 2H), 3.05 (d, *J* = 10.2 Hz, 2H), 2.67 (d, *J* = 2.4 Hz, 1H), 2.33 (d, *J* = 3.0 Hz, 1H), 2.21 (s, 3H), 1.80 (d, *J* = 12.0 Hz, 1H), 1.46 (s, 6H).

**Example 80: Synthesis of Compound 80:**

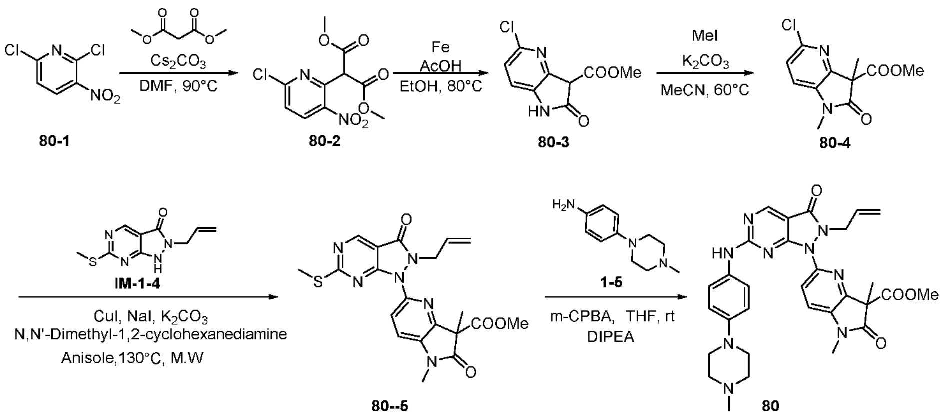


**Step 1: Synthesis of Compound 80-2:**

The substrate **80-1** (581.98 mg, 3.02 mmol) was added to a dry single-necked flask and dissolved in N,N-Dimethylformamide (10 mL), added with dimethyl malonate (436 mg, 3.30 mmol) and $Cs_2CO_3$ (3 g, 9.05 mmol), warmed up to 90°C to react for 2 hours and monitored by LC-MS, then cooled to room temperature after the reaction, extracted three times with ethyl acetate. The organic phase was dried with anhydrous $Na_2SO_4$, filtered, concentrated under reduced pressure. The residue was purified by column chromatography to give the compound **80-2** (753 mg, 2.61 mmol). LCMS (ESI) m/z: 289.1 $[M+H]^+$.

**Step 2: Synthesis of Compound 80-3:**

The compound **80-2** (753 mg, 2.61 mmol) was dissolved in ethanol (10 mL) and acetic acid (2 mL) in a dry single-necked flask, added with iron powder (582.80 mg, 10.44 mmol), warmed up to 80°C to react for 2 hours and monitored by LC-MS, filtered with diatomaceous earth after the reaction, the filterate was concentrated under reduced pressure. The residue was purified by MPLC to give the compound **80-3** (417 mg, 1.84 mmol). LCMS (ESI) m/z: 227.1 $[M+H]^+$.

**Step 3: Synthesis of Compound 80-4:**

The substrate **80-3** (45 mg, 198.57 μmol) was added to a dry single-necked flask and dissolved in acetonitrile (10 mL), added with $CH_3I$ (0.22 g, 1.99 mmol) and $K_2CO_3$ (111 mg, 794.29 μmol) to react for 8 hours at 60°C and monitored by LC-MS. The reaction solution was cooled to room temperature after the reaction, added with water and extracted three times with ethyl acetate. The organic phase was dried with anhydrous $Na_2SO_4$, filtered, concentrated under reduced pressure. The residue was purified by column chromatography to give the compound **80-4** (15 mg, 58.90 μmol). LCMS (ESI) m/z: 255.1$[M+H]^+$.

**Step 4: Synthesis of Compound 80-5:**

The substrate **80-4** (15 mg, 58.90 μmol), **IM-1-4** (13.09 mg, 58.90 μmol), CuI (22.43 mg, 117.80 μmol), NaI (17.66 mg, 117.80 μmol), $K_2CO_3$ (25 mg, 176.70 μmol) and N,N'-Dimethyl-1,2-cyclohexanediamine (32.2 mg, 235.6 μmol) were added to a dry microwave tube under $N_2$ protection, then added with anisole (1.5mL), microwave heated up to 130°C to react for 4 hours under $N_2$ atmosphere and monitored by LC-MS. The reaction system was cooled to room temperature after the reaction, added with water and ammonia (1 mL), extracted three times with ethyl acetate. The organic phase was dried with anhydrous $Na_2SO_4$, filtered, concentrated under reduced pressure. The residue was purified by column chromatography to give the compound **80-5** (10.5 mg, 23.84 μmol). LCMS (ESI) m/z: 441.1$[M+H]^+$.

**Step 5: Synthesis of Compound 80:**

The substrate **80-5** (30 mg, 68.11 μmol) was added to a dry single-necked flask, and added with tetrahydrofuran (1.5mL) to dissolve, then added with M-chloroperoxybenzoic acid (26.39 mg, 152.92 μmol) to react for 1 hour at room temperature, then N,N-diisopropylethylamine (19.76 mg, 152.92 μmol) and **1-5** (19.5 mg, 101.95 μmol) were added to the reaction solution to react for 3 hours at room temperature and monitored by LC-MS, then concentrated under reduced pressure after the reaction. The residue was purified by preparative high-performance liquid chromatography (alkaline) to give the compound **80** (5.7 mg, 9.77 μmol). LCMS (ESI) m/z : 584.2 [M+H]$^+$. HPLC: $R_T$ = 6.37 min, purity: 98.8%. $^1$H NMR (600 MHz, DMSO-$d_6$) δ 10.14 (s, 1H), 8.82 (s, 1H), 7.80 (d, $J$ = 7.8 Hz, 2H), 7.67 - 7.39 (m, 2H), 6.92 (d, $J$ = 8.4 Hz, 2H), 5.66 - 5.63 (m, 1H), 4.99 (d, $J$ = 10.2 Hz, 1H), 4.81 (d, $J$ = 17.4 Hz, 1H), 4.46 (s, 2H), 3.59 (s, 3H), 3.28 (s, 3H), 3.10 (s, 4H), 2.48 - 2.43 (m, 4H), 2.23 (s, 3H), 1.58 (s, 3H).

**Biological evaluation assay:**

Unless otherwise specified, some biological evaluation experiments in this part of Example were compared with compound AZD-1775 as control. The structural information of AZD-1775(CAS No.: 955365-80-7) is as follows:

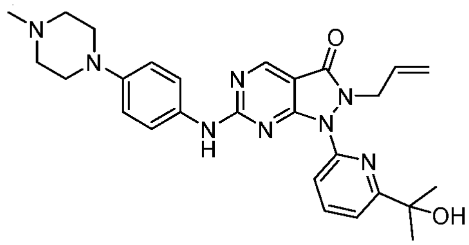

Chemical Formula: $C_{27}H_{32}N_8O_2$
Molecular Weight: 500.61
**AZD1775**

**Test Example 1:** The binding of compound to Wee1 protein and Tracer 178 was evaluated by TR-FRET method.

First, solutions of the compounds in different concentration gradients were prepared. The compounds were dissolved in DMSO and the compounds were diluted 4 folds with a total of 10 dose points and 2 parallel replicates for each concentration. DMSO was added as a positive control (maximal signal control) and a negative control (minimal signal control) and a final level of 0.25% DMSO was ensured in each reaction well.

The compound was added to 384-well plates with ECHO665, then added with WEE1 (Thermo Fisher, Cat # PR7373A) protein in buffer (50 mM Hepes pH 7.5, 10 mM $MgCl_2$, 1 mM EGTA, 0.01% Brij-35) with a final protein concentration of 15 nM, and The substrate Tracer 178 (Invitrogen, PV5593) and MAb Anti-GST-Eu crypate (Cisbio, 61GSTKLA) were added to 384-well plates (Corning, cat#3574), centrifuged at 1000 rpm for 1 min and the 384-well plates were incubated in a constant temperature shaker for 60 min at 25°C and 300 rpm, wherein Tracer 178 and MAb Anti-GST-Eu crypate were prepared in buffer (50 mM HEPES pH 7.5, 10 mM $MgCl_2$, 1 mM EGTA, 0.01% Brij-35) with a final reaction concentration of 50 nM for Tracer 178 and a final concentration of 2 nM for MAb Anti-GST-Eu crypate, where the negative control (minimal signal control) used an equal amount of buffer in place of the protein solution.

After incubation, readings were performed using BMG PHERAStar (excitation light at 337 nm and emission light at wavelength values of 620 nm and 665 nm to read the fluorescence signal values). The ratio of the fluorescence signal was calculated: 665/620*1000 was the final signal value of the enzyme activity, and the TR-FRET signal of the reads obtained from the positive control (maximum signal control) and the negative control (minimum signal control) was normalized to give the inhibition rate for different concentrations of the compound. The IC50 for inhibition of enzyme activity by the compounds was then calculated using GraphPad Prism 6 and fitted with a log (inhibitor) vs. response-Variable slope mode. The fitting equation was: Y=Bottom + (Top-Bottom)/(1+10^((LogIC50-X)*HillSlope)), wherein Y represents the percentage of residual enzyme activity known and X represents the known concentration of compound after the logarithm. The Wee1 inhibitory activity of the compounds in the Examples was tested according to the method described above and the results are shown in Table 1, where the $IC_{50}$ of each compound is categorized as follows:

"-" represents $IC_{50}$ measured value of more than 10 μM;
"+" represents $IC_{50}$ measured value of less than or equal to 10 μM and more than 1 μM;
"++" represents $IC_{50}$ measured value of less than or equal to 1 μM and more than 100 nM;
"+++" represents $IC_{50}$ measured value of less than or equal to 100 nM and more than 10 nM;
"++++" represents $IC_{50}$ measured value of less than or equal to 10 nM and more than 5 nM;

"+++++" represents $IC_{50}$ measured value of less than or equal to 5 nM.

Table 1 The inhibitory activity against Wee1 kinase of the compounds of the present invention

| Compound No. | $IC_{50}$/nM | Compound No. | $IC_{50}$/nM | Compound No. | $IC_{50}$/nM |
|---|---|---|---|---|---|
| AZD1775 | ++++ | Compound1 | ++++ | Compound3 | +++++ |
| Compound4 | +++++ | Compound5 | +++++ | Compound6 | +++++ |
| Compound7 | +++++ | Compound8 | ++++ | Compound9 | +++++ |
| Compound10 | +++++ | Compound11 | ++++ | Compound12 | ++++ |
| Compound13 | ++++ | Compound14 | ++++ | Compound15 | ++++ |
| Compound16 | +++ | Compound17 | ++++ | Compound18 | ++++ |
| Compound19 | +++++ | Compound20 | ++++ | Compound21 | +++ |
| Compound22 | +++ | Compound23 | +++ | Compound24 | +++ |
| Compound25 | ++++ | Compound27 | ++++ | Compound28 | ++++ |
| Compound29 | ++++ | Compound30 | +++++ | Compound31 | +++ |
| Compound32 | +++ | Compound33 | +++ | Compound34 | +++ |
| Compound35 | +++ | Compound36 | +++ | Compound37 | +++ |
| Compound38 | +++ | Compound39 | +++++ | Compound41 | +++ |
| Compound42 | +++ | Compound43 | +++ | Compound44 | ++++ |
| Compound45 | +++ | Compound46 | +++ | Compound47 | +++++ |
| Compound47a | +++ | Compound47b | +++ | Compound48 | +++++ |
| Compound49 | +++++ | Compound50 | ++ | Compound51 | +++++ |
| Compound52 | +++ | Compound53 | ++++ | Compound54 | ++++ |
| Compound55 | +++ | Compound56 | +++ | Compound57 | +++ |
| Compound58 | ++++ | Compound60 | ++++ | Compound61 | +++++ |
| Compound62 | ++++ | Compound63 | + | Compound64 | +++ |
| Compound65 | ++++ | Compound66 | ++++ | Compound67 | ++++ |
| Compound68 | +++ | Compound69 | +++ | Compound70 | ++++ |
| Compound71 | +++ | Compound72 | +++ | Compound73 | +++ |
| Compound74 | +++ | Compound75 | +++ | Compound76 | +++ |
| Compound77 | ++++ | Compound78 | +++ | Compound79 | +++ |

The above experiments showed that the disclosed compounds of the present invention have significant inhibitory activity against Wee1 kinase.

**Test Example 2:** Evaluation of cell proliferation inhibitory activity

1. Evaluation of the anti-proliferative effect of compounds on H1299 cells by the Cell Titer-Glo method

H1299 cells (ATCC, cat#HTB-96) in culture medium (90%1640 (Hyclone, cat#16600082) +10% FBS (Corning, Cat#35-081-CV )+100ug/ml Normocin (InvivoGen, cat#Ant-nr-2)) at 750 cells/well were inoculated in 384-well plates, then incubated overnight at 37°C with 5% $CO_2$ for cell adhesion.

Compound solutions of different concentration gradients were prepared. DMSO was dissolved to a concentration of 10 mM test compound and 10 mM reference compound AZD1775, and the compounds were serially diluted in culture medium for a total of 9 dose points, with 2 parallel replicates set at each concentration. The cell growth group without compound was used as a positive control (maximum signal control) and the medium was used as a negative

control (minimum signal control), while ensuring that the final level of DMSO in each reaction well was 0.2%. After removing the medium from the 384-well plate, 25ul of the configured compound at different concentrations was transferred into the well plate and the compound and cells were incubated in the cell incubator at 37°C with 5% $CO_2$ for 3 days.

The 384-well plates were removed from the cell incubator and allowed to equilibrate for 1h to room temperature. 25ul of Cell Titer-Glo assay was added to each well, lysed on a shaker for 2min and then read out (Luminescence) using a BMG PHERAStar after 10min incubation. Calculate the inhibition rate from the luminescence signal: first calculate the average of the positive control (maximum signal control) and the negative control (minimum signal control).

$$\text{Inhibition\%} = 1 - \left(\frac{\text{S(Compound signal value)} - \text{S(Negative Control signal value)}}{\text{S(Positive Control signal value)} - \text{S(Negative Control signal value)}}\right) \times 100\%$$

was used to calculate the rate of inhibition of the cells by different concentrations of the compounds. The IC50 of the compound on cell activity inhibition was calculated by fitting a log(inhibitor) vs. response-Variable slope model to GraphPad Prism 6. The fitted equation was: Y=Bottom + (Top-Bottom)/(1+10^(( LogIC50-X)*HillSlope)), where Y represents the rate of inhibition and X represents the concentration of the known compound after Log.

2. Evaluation of the anti-proliferative effect of compounds on MIA PaCa2 cells by the Cell Titer-Glo method

MIA PaCa2 cells (ATCC, cat#CRL-1420) in culture medium (90%1640 (Hyclone, cat#16600082) +10% FBS (Corning, Cat#35-081-CV )+100ug/ml Normocin (InvivoGen, cat#Ant-nr-2)) at 750 cells/well were inoculated in 384-well plates (Corning, 3707), then incubated overnight at 37°C with 5% $CO_2$ for cell adhesion.

Compound solutions of different concentration gradients were prepared. DMSO was dissolved to a concentration of 10 mM test compound and 10 mM reference compound AZD1775, and the compounds were serially diluted in culture medium for a total of 9 dose points, with 2 parallel replicates set at each concentration. The cell growth group without compound was used as a positive control (maximum signal control) and the medium was used as a negative control (minimum signal control), while ensuring that the final level of DMSO in each reaction well was 0.2%. After removing the medium from the 384-well plate, 25ul of the configured compound at different concentrations was transferred into the well plate and the compound and cells were incubated in the cell incubator at 37°C with 5% $CO_2$ for 3 days.

The 384-well plates were removed from the cell incubator and allowed to equilibrate for 1h to room temperature. 25ul of Cell Titer-Glo assay was added to each well, lysed on a shaker for 2min and then read out (Luminescence) using a BMG PHERAStar after 10min incubation. Calculate the inhibition rate from the luminescence signal: first calculate the average of the positive control (maximum signal control) and the negative control (minimum signal control).

$$\text{Inhibition\%} = 1 - \left(\frac{\text{S(Compound signal value)} - \text{S(Negative Control signal value)}}{\text{S(Positive Control signal value)} - \text{S(Negative Control signal value)}}\right) \times 100\%$$

was used to calculate the rate of inhibition of the cells by different concentrations of the compounds. The $IC_{50}$ of the compound on cell activity inhibition was calculated by fitting a log(inhibitor) vs. response-Variable slope model to GraphPad Prism 6. The fitted equation was: Y=Bottom + (Top-Bottom)/(1+10^(( Log$IC_{50}$-X)*HillSlope)), wherein Y represents the rate of inhibition and X represents the concentration of the known compound after Log.

The results of the in vitro anti-cell proliferation assays of H1299 and MIA Paca-2 for the example compounds according to the method described above are shown in Table 2, wherein the IC50 of each compound was determined and classified according to the description as follows:

"-" represents $IC_{50}$ measured value of more than 10 μM;
"+" represents $IC_{50}$ measured value of less than or equal to 10 μM and more than 5 μM;
"++" represents $IC_{50}$ measured value of less than or equal to 5 μM and more than 2 μM;
"+++" represents $IC_{50}$ measured value of less than or equal to 2 μM and more than 1 μM;
"++++" represents $IC_{50}$ measured value of less than or equal to 1 μM and more than 0.1 μM;
"+++++" represents $IC_{50}$ measured value of less than or equal to 0.1 μM.

Table 2 Inhibitory activity of compounds of the present invention on the proliferation of H1299 and MIA Paca-2 cells in vitro

| Compound No. | H1299 | MIA PaCa 2 | Compound No. | H1299 | MIA PaCa 2 |
|---|---|---|---|---|---|
| AZD1775 | ++++ | ++++ | Compound1 | ++ | ND |
| Compound3 | ++++ | ++++ | Compound4 | +++ | ND |
| Compound5 | ++++ | ND | Compound6 | ++++ | ND |
| Compound7 | ++++ | ND | Compound8 | + | ND |
| Compound9 | +++ | ND | Compound10 | ++++ | ND |
| Compound11 | ++++ | ++++ | Compound12 | ++++ | ++++ |
| Compound13 | + | + | Compound14 | + | ++ |
| Compound15 | - | ++ | Compound16 | - | - |
| Compound17 | ++++ | +++ | Compound18 | ++ | ++++ |
| Compound19 | - | - | Compound20 | ++++ | +++ |
| Compound21 | +++ | ++++ | Compound22 | ++++ | +++ |
| Compound23 | - | ++ | Compound24 | + | ++++ |
| Compound25 | +++ | +++ | Compound27 | ++++ | ++++ |
| Compound28 | ++++ | ++++ | Compound29 | - | - |
| Compound30 | +++ | +++ | Compound31 | + | + |
| Compound32 | ++ | ++ | Compound33 | +++ | ++ |
| Compound34 | + | ++ | Compound35 | ++ | +++ |
| Compound36 | +++ | +++ | Compound37 | ++ | ++++ |
| Compound38 | ++ | ++++ | Compound39 | ++++ | +++++ |
| Compound41 | ++++ | ++++ | Compound42 | ++++ | ++++ |
| Compound43 | ++ | ++ | Compound44 | ++++ | ++++ |
| Compound45 | - | + | Compound46 | ++++ | ++++ |
| Compound47 | ++++ | ++++ | Compound47a | ++++ | +++ |
| Compound47b | ++ | ++ | Compound48 | ++++ | ++++ |
| Compound49 | ++++ | ++++ | Compound50 | + | - |
| Compound51 | ++++ | ++++ | Compound52 | - | - |
| Compound53 | ++++ | ++ | Compound54 | ++ | ++ |
| Compound55 | +++ | +++ | Compound56 | + | + |
| Compound57 | - | - | Compound58 | ++++ | ++++ |
| Compound60 | ++++ | ++++ | Compound61 | ++ | ++ |
| Compound62 | +++ | ++++ | Compound63 | - | - |
| Compound64 | ++++ | ++++ | Compound65 | ++++ | ++++ |
| Compound66 | ++++ | ++++ | Compound67 | ++++ | +++++ |
| Compound68 | +++ | ++++ | Compound69 | ++++ | ND |
| Compound70 | ++++ | ND | Compound71 | ++ | ND |
| Compound72 | + | ND | Compound73 | +++ | ND |
| Compound74 | - | - | Compound75 | +++ | ND |
| Compound76 | ++++ | +++ | Compound77 | +++ | +++ |

(continued)

| Compound No. | H1299 | MIA PaCa 2 | Compound No. | H1299 | MIA PaCa 2 |
|---|---|---|---|---|---|
| Compound78 | - | +++ | Compound79 | - | ND |

As shown in Table 2, most of the compounds of the present invention exhibited strong cell proliferation inhibitory activity against H1299 and MIA Paca-2, some compounds were better than the control compound AZD 1775 on cell proliferation inhibitory activity.

**Test Example 3:** Evaluation of liver microsomes (mouse and human) in vitro

1. Preparation of working solution:

Microsomes were taken out from the -80 °C refrigerator, rapidly melted in a 37 °C water bath and placed on ice until ready to use. The test article was diluted with DMSO to prepare a 10 mM stock solution, and then diluted with acetonitrile to a 0.5 mM secondary stock solution. The microsomes were diluted to 0.75 mg/ml using Buffer C; the secondary stock solution was then added to a final concentration of 1.5 μM of compound as working solution, based on n=2, 5 time points, 350 μL of each compound was prepared and placed on ice prior to use. NADPH was diluted with Buffer C to a working solution of 6 mM for the starter solution. An acetonitrile solution containing an internal standard was prepared as the precipitant, and Verapamil-HCl was chosen as the internal standard at a concentration of 4 ng/ml.

2. Experimental procedure:

A round-bottom well plate was taken, noted as the reaction plate, and the prepared working solution for each compound was dispensed into the well plate according to the number of replicates and time points (0 h samples were also added to the reaction plate), 30 μL/well; the plate was incubated at 37 °C for 10 min. A separate plate with pointed bottom wells, noted as a precipitation plate, was added with 135 μL precipitant per well; 0 h samples were transferred to the plate after 10 min incubation and 15 μL of starter solution was added; the plate was placed on ice before centrifugation.

The diluted starter solution was added in sufficient quantity to the dispensing plate to facilitate the multichannel pipette aspiration operation.

The reaction was carried out on a warm incubation shaker and 15 μL of starter solution/sample is added to the plate using a multichannel pipette. The reaction was mixed with a slight shake to initiate the reaction, which was accurately timed and recorded using a timer. In general, samples with shorter reaction times are initiated late and vice versa early, allowing sufficient time for multiple plate manipulation to terminate the reaction. For example, as for the four experiments of 5, 15, 30, and 60 min, the 60 min experiment was started first, and the 5 min experiment was started later, so as to leave sufficient time for the 5 min group.

After the reaction time had elapsed, all the solution in the plate was aspirated using the multichannel pipette and added to the precipitation plate to terminate the reaction at that point in time. After all reactions had been terminated, the plates were shaken for ten minutes on a plate shaker at 600 rpm to precipitate the protein. The plate was centrifuged at 4 °C for 15 minutes at maximum rpm. 80 μL of supernatant was taken, 320 μL of pure water was added and mixed for LC-MS analysis.

3. Test results : as shown in Table 3

Table 3 Test results of liver microsomes (mouse and human in vitro

| **Compound** | **$T_{1/2}$ /min (M)** | **$T_{1/2}$ /min (H)** | **Compound** | **$T_{1/2}$ /min (M)** | **$T_{1/2}$ /min (H)** |
|---|---|---|---|---|---|
| **AZD1775** | 25.99 | 38.12 | **Compound**3 | 96.25 | >120 |
| **Compound4** | 10.08 | 22.21 | **Compound**6 | 5.72 | 5.16 |
| **Compound8** | >120 | untested | **Compound**9 | 0.78 | 2.82 |
| **Compound10** | 1.34 | 8.31 | **Compound**11 | 110.0 | untested |
| **Compound12** | 31.50 | untested | **Compound**15 | > 120 | untested |
| **Compound18** | > 120 | untested | **Compound**19 | 48.12 | untested |

(continued)

| Compound | $T_{1/2}$ /min (M) | $T_{1/2}$ /min (H) | Compound | $T_{1/2}$ /min (M) | $T_{1/2}$ /min (H) |
|---|---|---|---|---|---|
| **Compound23** | 38.72 | untested | **Compound**24 | >120 | untested |
| **Compound25** | 14.09 | untested | **Compound**27 | 33 | 105 |
| **Compound28** | 15.03 | untested | **Compound**37 | 21.72 | untested |
| **Compound39** | > 120 | untested | **Compound**46 | 13.64 | 16.82 |
| **Compound51** | 14.65 | 19.04 | **Compound**54 | 77 | untested |
| **Compound57** | 43.86 | untested | **Compound**61 | > 120 | untested |
| **Compound62** | 25.11 | untested | / | / | / |

As shown in Table 3, the compounds of the present invention have good metabolic stability as evaluated in the model of liver microsomes in vitro.

**Test Example 4: Membrane Permeability study (Caco-2)**

Caco-2 cells were purchased from the American Model Tissue Cell Collection (Rockville, MD). The cell culture medium was modified Eagle's medium (MEM) containing 10% inactivated fetal bovine serum and 1% non-essential amino acids. Cells were inoculated on polycarbonate filter membranes (Cat no. 3396) and incubated at 37°C in a 5% $CO_2$ incubator.

The cells were incubated for 21-28 days after inoculation for transport experiments and the apparent permeability (Papp) of Lucifer Yellow was used to characterize and verify the compactness of the cell monolayer. A stock solution of 10 mM was prepared by dissolving the compound in DMSO and diluted using Hanks Balanced Salt Solution (HBSS, Invitrogen, Cat# 14025-092) containing 25 mM HEPES (pH 7.4) to obtain the working solution. A 10 μM working solution of the compound to be tested was added to the apical side and basolateral side of Caco-2 and incubated at 37°C for 90 min. After the incubation, dilute the samples on the apical side and basolateral side, and the concentrations of compounds on the apical and basolateral sides were detected by LC-MS/MS, and the concentrations of the compounds were quantified by standard curve.

**Table 4** Membrane Permeability (Caco-2) data of compounds

| Compound | A-B ($10^{-6}$ cm·s$^{-1}$) | B-A ($10^{-6}$ cm·s$^{-1}$) | Efflux Ratio |
|---|---|---|---|
| **AZD1775** | 15.63 | 39.83 | 2.55 |
| **Compound3** | 2.31 | 28.92 | 12.52 |
| **Compound12** | 2.84 | 33.55 | 11.81 |
| **Compound27** | 3.07 | 53.53 | 17.45 |
| **Compound33** | 9.12 | 7.56 | 0.83 |
| **Compound42** | 6.22 | 49.70 | 7.99 |
| **Compound48** | 13.32 | 35.26 | 2.65 |

According to the test results of Table 4, the compounds of the present invention have the same membrane permeability (Caco-2) as the control compound.

**Test Example 5:** Evaluation of plasma protein binding (PPB)

1. Experimental procedure

Sample preparation: The compound was dissolved in DMSO to a stock solution of 10 mM, then the compound was diluted with PBS to a secondary stock solution of 0.02 mM, and then the above 0.02 mM was diluted to 1 μM using blank plasma, which was the sample to be incubated.

Dialysis set-up preparation: 400 μL of blank PBS was first added to the white wells of the equilibrated dialysis plate and 200 μL of the configured plasma sample was added to the red wells, and the dialysis plate was sealed with a

sealing film.

Recovery plate preparation: Two 96-well deep-well plates, labelled T0 and T5, were prepared and all plasma samples were added at n=2. 300 μL of acetonitrile (Verapamil-HCl, 4 ng/mL) was added directly to the T0 plate, followed by 50 μL of blank PBS mix well for 5 min and left to stand in a 4 °C refrigerator until the end of the incubation.

Experimental Operation: The dialysis device and the T5 plate were incubated together for 5 h in a microplate thermostatic shaker (37 °C, using 300 rpm or minimum speed). At the end of the incubation, 300 μL of acetonitrile (Verapamil-HCl, 4 ng/mL) was added and 50 μL of PBS solution was added. At the end of the dialysis incubation, a new 96-well deep well plate was taken. Add 50 μL of plasma well sample to the corresponding position of the 96-well plate, 300 μL of acetonitrile and 50 μL of blank PBS; take 50 μL of buffer well sample to the corresponding position of the 96-well plate, then add 300 μL of acetonitrile and 50 μL of blank plasma. Add 300 μL of acetonitrile (Verapamil-HCl, 4ng/mL) to the plasma-containing wells of the T5 plate, and then 50μL of PBS solution was added. Shake for 5 min to fully precipitate the proteins and centrifuge at 20,000 g for 10 min at 4°C. Add 200μL of supernatant to 200μL of pure water, mix well and perform LC-MS/MS analysis.

2. Data processing and parameter calculation

$$\text{Plasma protein binding rate} = [(\text{Rpe-Rb})/ \text{Rpe}] \times 100\ \%$$

$$\text{Recovery} = [(\text{Rpe+ Rb})/ \text{R5h}] \times 100\ \%$$

$$\text{Stability} = (\text{R5/R0}) \times 100\ \%$$

Wherein

$R_{pe}$ = ratio of plasma-side testing sample peak area to internal standard
$R_b$ = ratio of buffer side testing sample peak area to internal standard
$R_5$ = ratio of incubator stability sample peak area to internal standard
$R_0$= ratio of refrigerator stability sample peak area to internal standard

3. Test results: as shown in Table 5

Table 5 Plasma protein binding of representative compounds (mouse)

| Compound | Mouse plasma PPB(%) |
|---|---|
| AZD1775 | 91.3 |
| Compound3 | 85.8 |
| Compound27 | 85.9 |
| Compound46 | 82.1 |
| Compound48 | 96.9 |
| Compound49 | 95.8 |
| Compound51 | 97.0 |
| Compound53 | 96.5 |

According to the test results of Table 5, the compounds of the present invention have a good plasma protein binding.

**Test Example 6: Evaluation of enzymatic inhibitory activity of CYP450 isoforms**

Enzymatic experiments were performed to quantify the inhibition of CYP450 enzyme activity of each isoform of

CYP450 by small molecule inhibitors through fluorescence generated by the oxidation of the The substrate by cytochrome P450. The experiments were performed in 384-well plates (Corning, Cat# 3575) using a reaction buffer of 142.86 mM Potassium Phosphate, pH 7.4. The Solution A components used in the experiments were: 26.13 mM NADP+ (Sigma-aldrich, Cat# N0505) 65.77 mM G6P (J&K, Cat#968161) and 65.42 mM MgCl2 (Sigma-aldrich, Cat#M2670). The Solution B composition used for the experiment was: 40 U/mL G6PDH (Sigma-aldrich, Cat# G6378). The The substrate mix was 0.05 X Solution A, 0.01 X Solution B, 50 mM Potassium Phosphate, 0.01 mM BOMCC/ 0.01 mM EOMCC/ 0.001 mM DBOMF. For CYP3A4 and CYP2C9, the reaction system was 50 μL or 20 μL, respectively, including 3 nM CYP3A4 or 120 nM CYP2C9, BOMCC The substrate mixed solution and different concentrations of compounds to be tested. For CYP2C19, CYP2D6 and CYP1A2, the reaction system was 20 μL and included 12.5 nM CYP2C19, 80 nM CYP2D6 or 1 nM CYP1A2, EOMCC The substrate mix and various concentrations of the compounds to be tested. For CYP2C8, the reaction system is 50 μL and includes 1.5 nM CYP2C8, DBOMF The substrate mix and various concentrations of compound to be tested. After preincubation with the enzyme for 10 minutes, the The substrate was added and the fluorescence signal was read at different wavelengths (BOMCC/EOMCC Ex430 nm/Em480 nm, DBOMF Ex490 nm/Em520 nm) using BMG PHERAStar depending on the The substrate, with reaction intervals of 30 seconds or more (depending on the actual number of wells) and reaction times of 30 minutes. The data were analyzed and processed using GraphPad Prism 6 software to obtain IC50 values. The results were shown as Table 6.

Table 6 Compound inhibition test results on different isoforms of CYPs

| Compound | | CYP inhibiton/% | | | | | | |
|---|---|---|---|---|---|---|---|---|
| | | 1A2 | 2C8 | 2C9 | 2C19 | 2D6 | 3A4 | 2B6 |
| **AZD-1775** | 1uM | 20.2 | 7.1 | 15.4 | 25.4 | 3.4 | -14.0 | ND |
| | 10uM | 17.5 | 17.3 | 6.4 | 71.9 | 1.5 | -32.9 | 17.39 |
| **Compound 3** | 1uM | 4.5 | -9.3 | -1.5 | 1.0 | 2.6 | -19.4 | ND |
| | 10uM | 7.8 | -16.9 | -7.0 | 16.6 | -0.1 | -35.3 | 1.38 |

According to the test results of Table 6, Compound 3 of the present invention had no significant cytochrome P450 enzymatic inhibitory activity.

**Test Example 7:** hERG potassium channel inhibition assay

Experimental Aim: To detect the effect of compounds to be tested on hERG potassium ion channels by manual membrane clamp method.

Experimental procedure:

(I) Experimental materials:

A. CHO (Chinese hamster ovary cells) stably transfected cell line culture

The cell line used for the patch clamp assay was a 10th generation CHO cell overexpressing hERG potassium channel cDNA.

CHO hERG cells were cultured in Petri dishes or flasks at 37°C in a 5 % $CO_2$ incubator. Cells were dropped onto circular slides 24-48 hours prior to electrophysiological experiments and cultured in cell culture medium and used for experiments after the cells had been adhered.

Cell culture medium (purchased from Invitrogen) Composition:

- Ham's F12 medium
- 10 % (v/v) heat inactivated FBS
- 100 μg/ml Hygromycin B (thaumatin)
- 100 μg/ml Geneticin (Genomycin, G418)

B. Compound preparation

Compound powders are dissolved in the extracellular solution and are subjected to a routine 5 to 10 minute sonication and shaking to ensure complete dissolution of the compound.

The final concentrations of compounds used for electrophysiological assays were 5, 20 μM and the final concentration of DMSO was 0.1%.

(II) Experimental protocol:

A. Experimental procedure for electrophysiological recordings

Cell membrane currents were recorded using a HEKA EPC-10 USB patch-clamp amplifier (HEKA Elektronik, Germany).

1) A coverslip with a large number of uniformly growing individual CHO hERG cells on its surface was taken. Place in a continuous recording cell on an inverted microscope, perfused with extracellular fluid (approximately 1 ml per minute) and recorded continuously, waiting for the current to stabilize.
2) Record HERG channel currents for individual cells using standard whole cell recording mode. The membrane voltage is first clamped at -80 mV and the cell is given a +20 mV stimulus for 5 s to activate the hERG potassium channel, then repolarized to -50 mV for 5 s to generate an outward tail current, which is continuously perfused until the current is stable, at which point the peak tail current is the control current value.
3) The extracellular solution containing the drug to be tested was then perfused and recorded until the inhibitory effect of the drug on the hERG current reached a steady state, at which point the peak tail current was the post-drug current value.
4) The cells are again perfused with the extracellular solution until the hERG current returns to or approaches the level prior to the addition of the drug, then the perfusion can be continued to test other concentrations or drugs. One or more compound or drug concentrations may be tested on each cell.
5) Cisapride (C4740-10mg, Sigma) is used as a positive control in the experiment to ensure that the cells used respond properly.

(III) Quality control

The following criteria need to be met for the reported experimental data:

Electrophysiological recording parameters

a) Sealing resistance > 500MΩ
b) contact resistance (Ra) < 10MΩ
c) Initial tail current amplitude > 200pA
d) Current rundown (spontaneous reduction) < 2%/min
e) Leakage current < 200pA or 10% of peak hERG current (within 90% of recording time)

The test results were shown as Table 7.

Table 7 Test results of compound hERG inhibition

| Compound | hERG inhibition/% | |
|---|---|---|
| | 5μM | 20μM |
| **AZD1775** | 21.45 | 49.98 |
| **Compound 3** | 3.84 | 21.56 |

According to the test results of Table 7, Compound 3 of the present invention had no significant hERG inhibitory activity. It is clear from the results of the tests of the present invention that the compounds of the present invention, especially, the preferred compounds, are superior to the prior art in terms of pharmaceutical activity and toxic side effects.

**Test Example 8:** Evaluation of compound pharmacokinetics on single dose in mice

The present experiment aimed to study the pharmacokinetics in the plasma of male ICR mice after the administration.

1.Experimental Aim:

The present experiment aimed to obtain the pharmacokinetic profile of the subject compounds in ICR mice (both

intravenous and oral)

2. Standard compliance

In this experiment (non-GLP study), the test articles-testing, DMPK animal test, and DMPK analysis were done in Chengdu Hitgen, and all the tests followed the present test protocol, and the relevant SOPs of the related organizations.

3. Test materials, instruments and equipment

3.1 Test materials

3.1.1 test articles

The following test articles were provided by Chengdu Hitgen and their quality was ensured to meet the requirements.

| Name/Code | Test compound |
|---|---|
| Nature | solution |
| Solvent/Dosage form | 5% DMSO - 10%Solutol - 85% HPBCD (20%, W/V) |
| preservation condition | room temperature |
| Concentration of test solution | 0.2 mg/ml (intravenous), 1 mg/ml (oral) |
| Volume of test solution | 3 ml,3 ml |

3.1.2 Test system

Species: SPF grade male ICR mice
Body weight/weekly age: about 30g
Amount: 6
Experimental animal source: Charles River

4. Experimental procedure:

4.1 Solvent Preparation

Intravenous solvents: 5% DMSO - 10% Solutol-85% HPBCD (20%,W/V)
"Gavage solvent/dosage form: 5% DMSO - 10% Solutol-85% HPBCD (20%,W/V) "

4.2 Subjects given (route)

Intravenous: 1 mg/kg in a volume of 5 mL/kg
Gavage: 10 mg/kg in a volume of 10 mL/kg
Ultrasound for 5 min before administration

4.3 Subject Preparation (concentration)

Intravenous: 0.2 mg/mL
Gavage: 1 mg/mL

Fast overnight before administration and feed four hours after administration.

4.4 Sample collection

Blood samples were collected by orbital venous plexus puncture (40-50 μL) into anticoagulant tubes containing pre-sprayed EDTA-K2 at 5 min (IV only), 15 min, 30 min, 1 h, 2 h, 4 h, 8 h, and 24 h, respectively, and the supernatant, i.e., the plasma, was centrifuged at 10,000 rpm for 20 min within 1 h. The blood samples were stored in a refrigerator at or below -20°C for LC-MS/MS analysis.

4.5 Biopharmaceutical analytical methods and assays: LC-MS/MS Analytical Detection used

5. Data processing

The samples were detected for drug concentration at each time point by LC-MS/MS. Pharmacokinetic parameters terminal elimination half-life ($t_{1/2}$), area under the curve (AUC), apparent volume of distribution (Vd), clearance (CL), mean residence time (MRT), and Cmax were calculated using the non-compartmental model of Phoenix WinNonlin 5.2. Bioavailability (F%) was directly from serum concentration results. Mean ± standard deviation (X ± SD) was used for blood concentration and pharmacokinetic parameters, etc. Specific testing and analytical methods were specified in the form of protocol revisions.

6. The test results were shown as Table 8

Table 8 Pharmacokinetic parameters in mice

| Compound | Forms of administration | Dosages | Parameters | | | | |
|---|---|---|---|---|---|---|---|
| | | | $C_{max}$(ng/mL) | $AUC_{last}$ (h*ng/mL) | $T_{1/2}$ (h) | MRT(h) | F/% |
| **AZD1775** | Intravenous | 1mg/kg | 250 | 116 | 0.5 | / | 32 |
| | Oral | 10mg/kg | 398 | 396 | 0.87 | / | |
| Compound **3** | Intravenous | 1mg/kg | 124 | 145 | 1.17 | 0.97 | 22.4 |
| | Oral | 10mg/kg | 247 | 324 | 1.07 | 1.10 | |
| Compound **27** | Intravenous | 1 mg/kg | 170 | 134 | 0.78 | 0.66 | 28.6 |
| | Oral | 10 mg/kg | 392 | 285 | 1.03 | 1.10 | |
| Compound **48** | Oral | 10 mg/kg | 470 | 482 | 0.64 | 0.9 | / |
| Compound **49** | Oral | 10 mg/kg | 388 | 534 | 1.03 | 1.14 | / |
| Compound **51** | Oral | 10 mg/kg | 290 | 430 | 0.95 | 1.18 | / |
| Compound **53** | Oral | 10 mg/kg | 800 | 1638 | 1.88 | 1.92 | / |

As shown in Table 8, pharmacokinetic assay analysis in mice in vivo indicated that compounds 48 and 49 of the present invention had comparable pharmacokinetic properties to the control compound AZD1775, and compounds 51, 53 had better pharmacokinetic properties than the control compound AZD1775.

## Claims

1. A compound represented by formula I, or a stereoisomer thereof, or a pharmaceutically acceptable salt thereof:

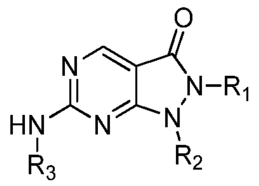

I

wherein,

$R_1$ is selected from a group consisting of $-C_{1\sim6}$ alkyl, $-C_{2\sim6}$ alkenyl, $-C_{2\sim6}$ alkynyl, $-C_{0\sim2}$ alkylene-CN, $-C_{0\sim2}$ alkylene-(3-10-membered cycloalkyl), $-C_{0\sim2}$ alkylene-(3-10-membered heterocycloalkyl); wherein said $-C_{0\sim2}$ alkylene, $-C_{1\sim6}$ alkyl, $-C_{2\sim6}$ alkenyl, $-C_{2\sim6}$ alkynyl, 3~10-membered cycloalkyl, 3~10-membered heterocycloalkyl are optionally substituted by one, two, three or four independent $R^{11}$;

$R^{11}$ is selected from a group consisting of H, $-C_{1\sim6}$ alkyl, halogen substituted $-C_{1\sim6}$ alkyl, halogen;

$R_2$ is selected from a group consisting of

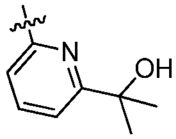 , 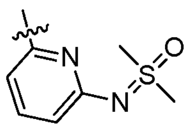 , 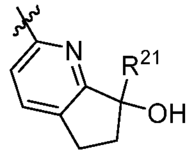 ,

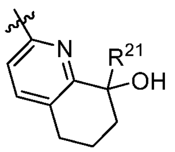 , 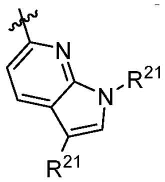 , 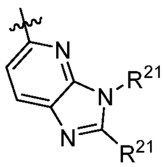 , 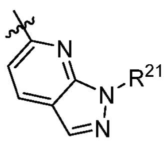 , 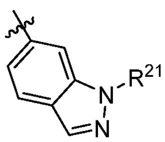 , 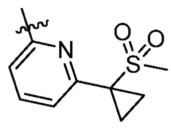 ,

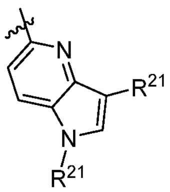 , 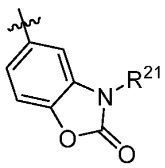 , 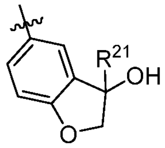 , 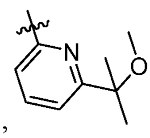 , 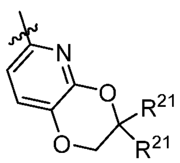 , 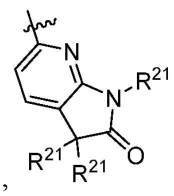 , 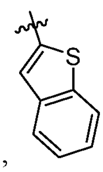 ,

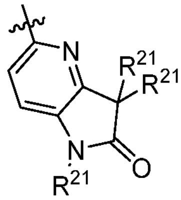 ;

wherein said $R^{21}$ is selected from a group consisting of H, $-C_{1\sim6}$ alkyl, halogen substituted $-C_{1\sim6}$ alkyl, $-C_{0\sim2}$ alkylene-$C(O)O(C_{1\sim6}$ alkyl), $-C_{0\sim2}$ alkylene-COOH, $-C(O)C_{1\sim6}$ alkyl, halogen;

$R_3$ is selected from a group consisting of

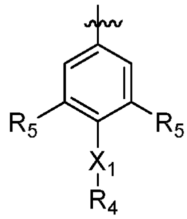, 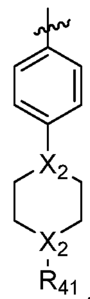, 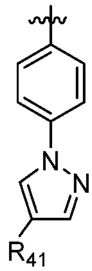, 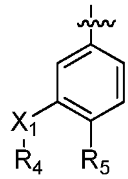, 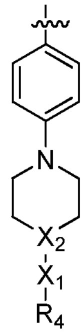,

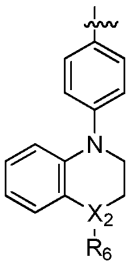, 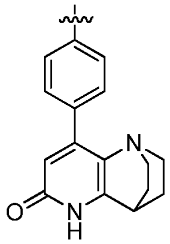, 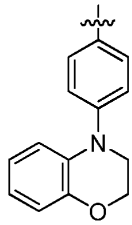, 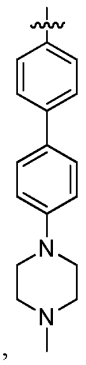, 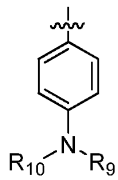, 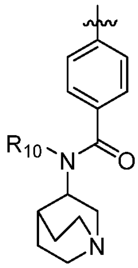, 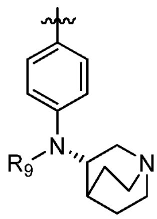, 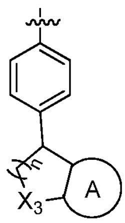,

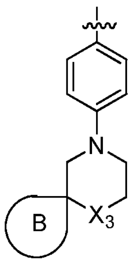, 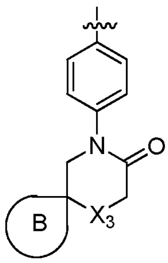, 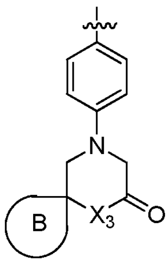, 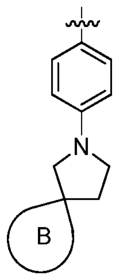, 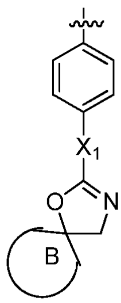, 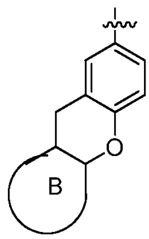, 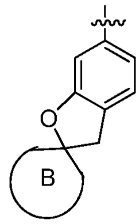,

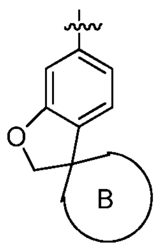, 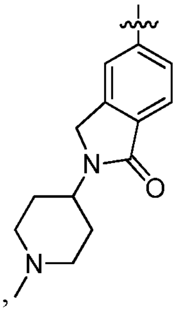, 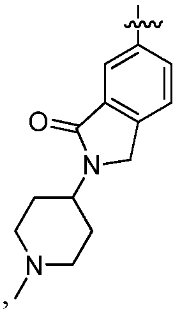, 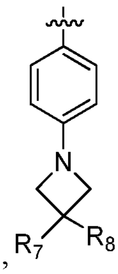, 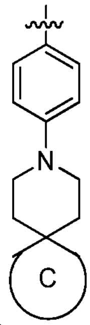, 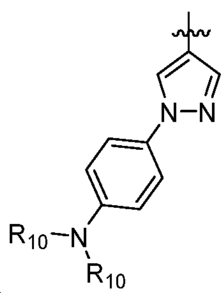;

$X_1$ is selected from a group consisting of chemical bond, O, $NR^{N1}$ or $CR^{C1}R^{C2}$; $X_2$ is selected from N or $CR^{C1}$; $X_3$ is selected from a group consisting of O, $NR^{N1}$ or $CR^{C1}R^{C2}$;

n is selected from 1, 2 or 3;

$R^{N1}$ is selected from a group consisting of H, halogen, -OH, $-C_{1\sim6}$ alkyl, halogen substituted $-C_{1\sim6}$ alkyl, $-O(C_{1\sim6}$ alkyl), -O(halogen substituted $-C_{1\sim6}$ alkyl), $-NH_2$, $-NH(C_{1\sim6}$ alkyl), $-N(C_{1\sim6}$ alkyl)( $C_{1\sim6}$ alkyl), $-C(O)(C_{1\sim6}$ alkyl);

$R^{C1}$, $R^{C2}$ is independently selected from a group consisting of H, halogen, cyano, nitro, -OH, $-C_{1\sim6}$ alkyl, halogen substituted $-C_{1\sim6}$ alkyl, $-O(C_{1\sim6}$ alkyl), -O(halogen substituted $-C_{1\sim6}$ alkyl), $-NH_2$, $-C_{0\sim2}$ alkylene-$NH(C_{1\sim6}$ alkyl), $-C_{0\sim2}$ alkylene-$N(C_{1\sim6}$ alkyl)( $C_{1\sim6}$ alkyl);

$R_4$ is selected from -(5-12-membered bridged heterocycloalkyl); wherein said bridged heterocycloalkyl is optionally substituted by one, two, three or four independent $R^{41}$,

$R_{41}$ is selected from a group consisting of H, oxo, -$C_{1\sim6}$ alkyl, -$C_{2\sim6}$ alkenyl, halogen substituted -$C_{1\sim6}$ alkyl, hydroxy-substituted -$C_{1\sim6}$ alkyl, halogen, cyano, nitro, -OH, -$C_{0\sim2}$ alkylene-O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl), -$C_{0\sim2}$ alkylene-C(O)$R^{42}$, -$C_{0\sim2}$ alkylene-C(O)$NR^{42}R^{43}$, -$C_{0\sim2}$ alkylene-C(O)O$R^{42}$, -$C_{0\sim2}$ alkylene-S(O)$R^{42}$, -$C_{0\sim2}$ alkylene-S(O)$NR^{42}R^{43}$, -$C_{0\sim2}$ alkylene-S(O)O$R^{42}$, -$C_{0\sim2}$ alkylene-S$(O)_2R^{42}$, -$C_{0\sim2}$ alkylene-S$(O)_2NR^{42}R^{43}$, -$C_{0\sim2}$ alkylene-S$(O)_2OR^{42}$, -$C_{0\sim2}$ alkylene-(3-10-membered cycloalkyl), -$C_{0\sim2}$ alkylene-(3-10-membered heterocycloalkyl), -$C_{0\sim2}$ alkylene-(5~ 10-membered aromatic cyclyl), -$C_{0\sim2}$ alkylene-(5~ 10-membered aromatic heterocyclyl); wherein said alkyl, alkylene, cycloalkyl, heterocycloalkyl, aromatic cyclyl, aromatic heterocyclyl are optionally substituted by one, two, three or four independent $R^{44}$;

$R^{42}$, $R^{43}$ are independently selected from a group consisting of H, halogen, cyano, nitro, -OH, -$C_{1\sim6}$ alkyl, halogen substituted -$C_{1\sim6}$ alkyl, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl), -$C_{0\sim2}$ alkylene-(3-10-membered cycloalkyl), -$C_{0\sim2}$ alkylene-(3-10-membered heterocycloalkyl), -$C_{0\sim2}$ alkylene-(5-10-membered aromatic cyclyl), -$C_{0\sim2}$ alkylene-(5-10-membered aromatic heterocyclyl);

$R^{44}$ is selected from a group consisting of H, oxo, halogen, cyano, nitro, -OH, -$C_{1\sim6}$ alkyl, halogen substituted -$C_{1\sim6}$ alkyl, -O($C_{1\sim6}$ alkyl), -O(halogen substituted -$C_{1\sim6}$ alkyl), -$NH_2$, -$C_{0\sim2}$ alkylene-NH($C_{1\sim6}$ alkyl), -N($C_{1\sim6}$ alkyl)( $C_{1\sim6}$ alkyl);

or, said $R^{41}$and $R^{C1}$ , or $R^{C2}$ together with the atom adjacent therewith form 3~10-membered carbocyclyl, 3~10-membered heterocyclyl;

$R_5$ is selected from a group consisting of H, oxo, halogen, cyano, nitro, -OH, -$NH_2$, -$C_{1\sim6}$ alkyl, halogen substituted -$C_{1\sim6}$ alkyl, hydroxy-substituted -$C_{1\sim6}$ alkyl, -O($C_{1\sim6}$ alkyl), -O(halogen substituted -$C_{1\sim6}$ alkyl), -$C_{0\sim2}$ alkylene-NH($C_{1\sim6}$ alkyl), -O-$C_{0\sim2}$ alkylene-N($C_{1\sim6}$ alkyl)( $C_{1\sim6}$ alkyl), -$C_{0\sim2}$ alkylene-N($C_{1\sim6}$ alkyl)( $C_{1\sim6}$ alkyl), -$C_{0\sim2}$ alkylene-(3-10-membered cycloalkyl), -$C_{0\sim2}$ alkylene-(3-10-membered heterocycloalkyl), -O-(3~10-membered cycloalkyl), -O-$C_{0\sim2}$ alkylene-(3~10-membered heterocycloalkyl), -$C_{0\sim2}$ alkylene-(5-10-membered aromatic cyclyl), -$C_{0\sim2}$ alkylene-(5-10-membered aromatic heterocyclyl); wherein said alkyl, alkylene, cycloalkyl, heterocycloalkyl, aromatic cyclyl, aromatic heterocyclyl are optionally substituted by one, two, three or four independent $R^{51}$;

$R^{51}$ is selected from a group consisting of H, -$C_{1\sim6}$ alkyl, halogen substituted -$C_{1\sim6}$ alkyl, oxo, halogen, cyano, nitro, -OH, -O($C_{1\sim6}$ alkyl), -O(halogen substituted -$C_{1\sim6}$ alkyl), -$NH_2$, -$C_{0\sim2}$ alkylene-NH($C_{1\sim6}$ alkyl), -N($C_{1\sim6}$ alkyl)( $C_{1\sim6}$ alkyl);

each $R_6$ is independently selected from a group consisting of H, -$C_{1\sim6}$ alkyl, halogen substituted -$C_{1\sim6}$ alkyl, halogen, cyano, nitro, -OH, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl), -$C_{0\sim2}$ alkylene-(3-10-membered cycloalkyl), -$C_{0\sim2}$ alkylene-(3-10-membered heterocycloalkyl), -$C_{0\sim2}$ alkylene-(5-10-membered aromatic cyclyl), -$C_{0\sim2}$ alkylene-(5~10-membered aromatic heterocyclyl), -$C_{0\sim2}$ alkylene-(5~ 12-membered spirocyclyl), -$C_{0\sim2}$ alkylene-(5~ 12-membered spiro heterocyclyl), -$C_{0\sim2}$ alkylene-(5~ 12-membered bridged cyclyl), -$C_{0\sim2}$ alkylene-(5~ 12-membered bridged heterocyclyl);

wherein,

A ring is selected from a group consisting of 5~12-membered spirocyclyl, 5~12-membered spiro heterocyclyl, 5~12-membered bridged cyclyl, 5~12-membered bridged heterocyclyl; wherein said spirocyclyl, spiro heterocyclyl, bridged cyclyl, bridged heterocyclyl are optionally substituted by one, two, three or four independent $R^{A1}$;

each $R^{A1}$ is independently selected from a group consisting of H, halogen, cyano, nitro, -OH, -$C_{1\sim6}$ alkyl, halogen substituted -$C_{1\sim6}$ alkyl, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl), -$C_{0\sim2}$ alkylene-(3-10-membered cycloalkyl), -$C_{0\sim2}$ alkylene-(3-10-membered heterocycloalkyl), -$C_{0\sim2}$ alkylene-(5-10-membered aromatic cyclyl), -$C_{0\sim2}$ alkylene-(5-10-membered aromatic heterocyclyl), -$C_{0\sim2}$ alkylene-(5-12-membered spirocyclyl), -$C_{0\sim2}$ alkylene-(5-12-membered spiro heterocyclyl), -$C_{0\sim2}$ alkylene-(5-12-membered bridged cyclyl), -$C_{0\sim2}$ alkylene-(5-12-membered bridged heterocyclyl);

wherein,

B ring is selected from a group consisting of 3~10-membered carbocyclyl, 3~10-membered heterocyclyl, 5~10-membered aromatic cyclyl, 5~10-membered aromatic heterocyclyl, 5~12-membered spirocyclyl, 5~12-membered spiro heterocyclyl, 5~12-membered bridged cyclyl, 5~12-membered bridged heterocyclyl, 3~10-membered fused cyclyl, 3~10-membered fused heterocyclyl; wherein said carbocyclyl, heterocyclyl, aromatic cyclyl, aromatic heterocyclyl, spirocyclyl, spiro heterocyclyl, bridged cyclyl, bridged heterocyclyl, fused cyclyl, fused heterocyclyl are optionally substituted by one, two, three or four independent $R^{B1}$;

each $R^{B1}$ is independently selected from a group consisting of H, -$C_{1\sim6}$ alkyl, halogen substituted -$C_{1\sim6}$ alkyl, halogen, cyano, nitro, -OH, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl), -$C_{0\sim2}$ alkylene-(3-10-membered cycloalkyl), -$C_{0\sim2}$ alkylene-(3-10-membered heterocycloalkyl), -$C_{0\sim2}$ alkylene-(5-10-membered aromatic cyclyl), -$C_{0\sim2}$ alkylene-(5~ 10-membered aromatic heterocyclyl);

$R_7$, $R_8$ are independently selected from a group consisting of H, halogen, cyano, nitro, -OH, -$C_{1\sim6}$ alkyl, halogen substituted -$C_{1\sim6}$ alkyl, -O($C_{1\sim6}$ alkyl), -O(halogen substituted -$C_{1\sim6}$ alkyl), -$NH_2$, -NH($C_{1\sim6}$ alkyl), -N($C_{1\sim6}$

alkyl)( $C_{1\sim6}$ alkyl), -$C_{0\sim2}$ alkylene-(3-10-membered cycloalkyl), -$C_{0\sim2}$ alkylene-(3-10-membered heterocycloalkyl), -$C_{0\sim2}$ alkylene-(5-10-membered aromatic cyclyl), -$C_{0\sim2}$ alkylene-(5-10-membered aromatic heterocyclyl); or $R_7$, $R_8$ together with the atom adjacent therewith form 3~10-membered fused cyclyl, 3~10-membered fused heterocyclyl, 3~10-membered spirocyclyl, 3~10-membered spiro heterocyclyl; wherein said fused cyclyl, fused heterocyclyl, spirocyclyl, spiro heterocyclyl are optionally substituted by one, two, three or four independent $R^{71}$; each $R^{71}$ is independently selected from a group consisting of H, -$C_{1\sim6}$ alkyl, halogen substituted -$C_{1\sim6}$ alkyl, halogen, cyano, nitro, -OH, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl);

wherein,

C ring is selected from a group consisting of 5~12-membered spirocyclyl, 5~12-membered spiro heterocyclyl, 5~12-membered bridged cyclyl, 5~12-membered bridged heterocyclyl, 3~10-membered fused cyclyl, 3~10-membered fused heterocyclyl; wherein said spirocyclyl, spiro heterocyclyl, bridged cyclyl, bridged heterocyclyl, fused cyclyl, fused heterocyclyl are optionally substituted by one, two, three or four independent $R^{C1}$; each $R^{C1}$ is independently selected from a group consisting of H, halogen, cyano, nitro, -OH, -$C_{1\sim6}$ alkyl, halogen substituted -$C_{1\sim6}$ alkyl, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl), -$C_{0\sim2}$ alkylene-(3-10-membered cycloalkyl), -$C_{0\sim2}$ alkylene-(3-10-membered heterocycloalkyl), -$C_{0\sim2}$ alkylene-(5-10-membered aromatic cyclyl), -$C_{0\sim2}$ alkylene-(5-10-membered aromatic heterocyclyl), -$C_{0\sim2}$ alkylene-(5-12-membered spirocyclyl), -$C_{0\sim2}$ alkylene-(5-12-membered spiro heterocyclyl), -$C_{0\sim2}$ alkylene-(5-12-membered bridged cyclyl), -$C_{0\sim2}$ alkylene-(5-12-membered bridged heterocyclyl);

$R_{10}$ is selected from a group consisting of H, -$C_{1\sim6}$ alkyl, halogen substituted -$C_{1\sim6}$ alkyl;

$R_9$ is selected from -$C_{0\sim2}$ alkylene-C(O)N$R^{91}R^{92}$, -C(O)($C_{1\sim6}$ alkyl); each $R^{91}$, $R^{92}$ is independently selected from a group consisting of H, -$C_{1\sim6}$ alkyl, halogen substituted -$C_{1\sim6}$ alkyl;

or $R^{91}$, $R^{92}$ together with the atom adjacent therewith form 3~10-membered heterocyclyl; wherein said heterocyclyl is optionally substituted by one, two, three or four independent $R^{93}$;

each $R^{93}$ is independently selected from a group consisting of H, -$C_{1\sim6}$ alkyl, halogen substituted -$C_{1\sim6}$ alkyl, halogen, cyano, nitro, -OH, -O($C_{1\sim6}$ alkyl), -O(halogen substituted -$C_{1\sim6}$ alkyl), -$NH_2$, -NH($C_{1\sim6}$ alkyl), -N($C_{1\sim6}$ alkyl)( $C_{1\sim6}$ alkyl);

when $R_3$ is

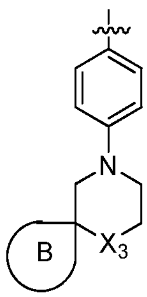

,

B ring is 3-membered carbocyclyl and $R_1$ is

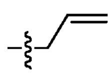

, $R_2$ is not

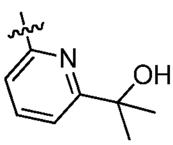

;

when $R_3$ is

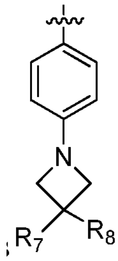

, $R_7$, $R_8$ are both H and $R_1$ is

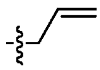
,

$R_2$ is not

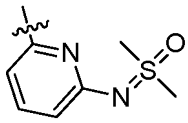
;

when $R_3$ is

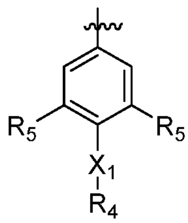
,

$X_1$ is bond, $R_4$ is

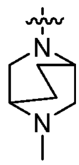

and $R_1$ is

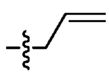
,

$R_2$ is not

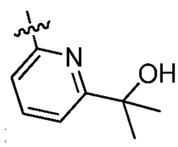
;

when $R_3$ is

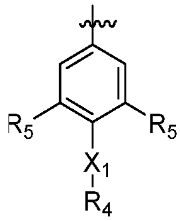,

$X_1$ is bond, $R_4$ is

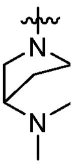

and $R_1$ is

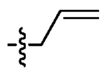,

$R_2$ is not

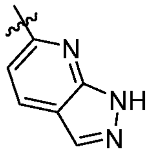;

when $R_3$ is

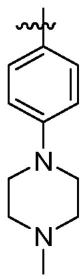

and $R_1$ is

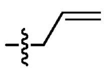,

$R_2$ is selected from a group consisting of

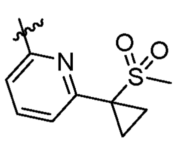, 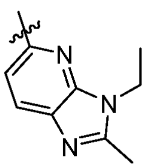, 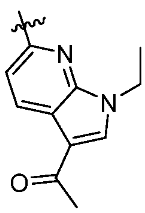, 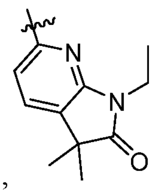, 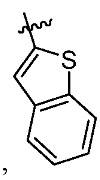, 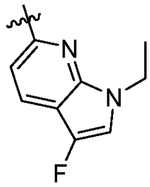 or 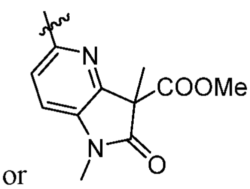,

when $R_3$ is

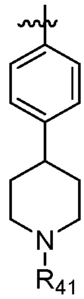

and $R_1$ is

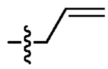

,

$R_2$ is not selected from

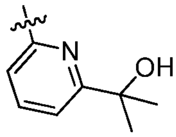

,

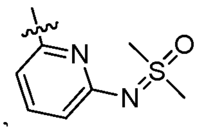

or

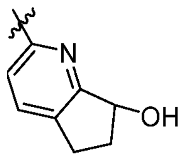

,

when $R_2$ is

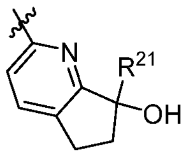

,

, $R_{21}$ is methyl and $R_1$ is

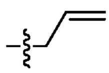

,

$R_3$ is not

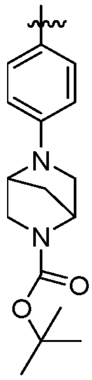 or 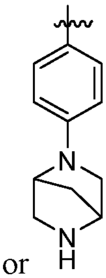 ;

when $R_2$ is

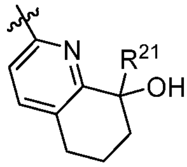 ,

$R_{21}$ is H and $R_1$ is

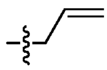 ,

$R_3$ is not

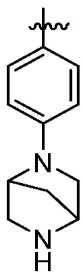 ;

when $R_2$ is

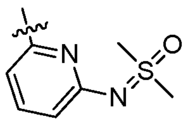 ,

$R_{21}$ is H and $R_1$ is

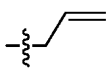 ,

$R_3$ is not selected from

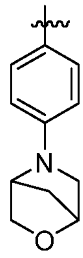,

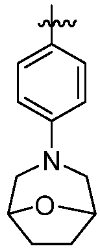 or 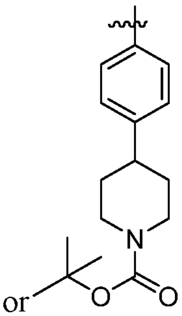;

when $R_2$ is

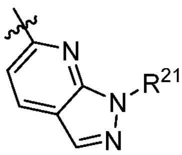,

$R_{21}$ is H and $R_1$ is

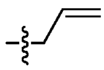,

$R_3$ is not

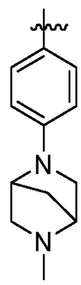;

when $R_2$ is

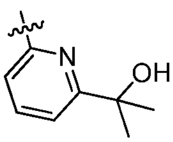;

and $R_1$ is

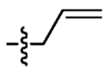,

R$_3$ is not selected from

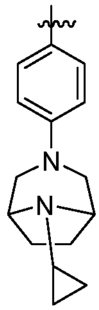, 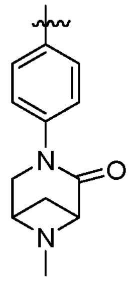,

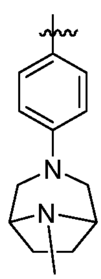 or 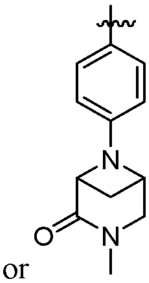

2. The compound, or a stereoisomer thereof, or a pharmaceutically acceptable salt thereof according to claim 1, wherein:
R$_1$ is selected from a group consisting of methyl, ethyl, propyl, isopropyl, cyclopropyl,

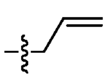, 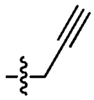, 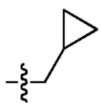, 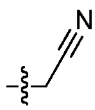, 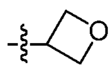, 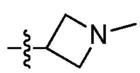, 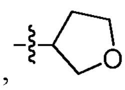

3. The compound, or a stereoisomer thereof, or a pharmaceutically acceptable salt thereof according to claim 1, wherein:
R$_2$ is selected from a group consisting of

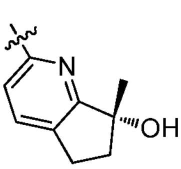, 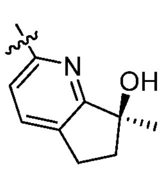, 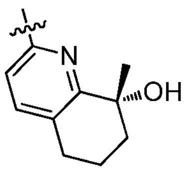,

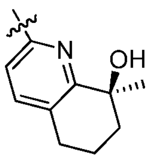, 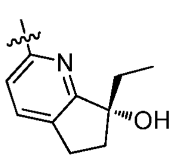, 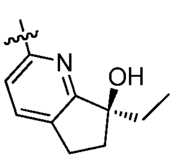, 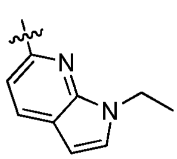, 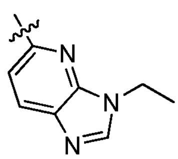, 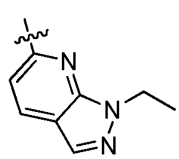,

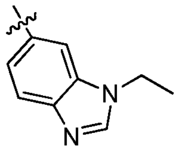, 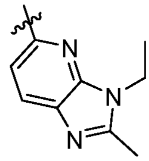, 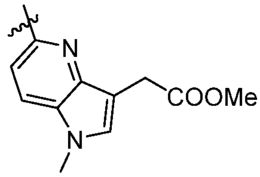, 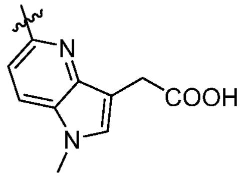, 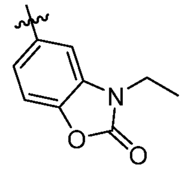,

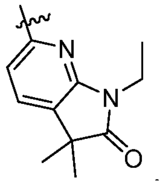, 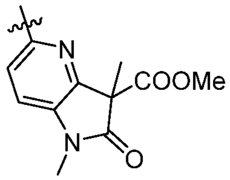, 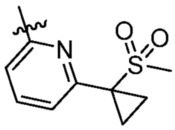, 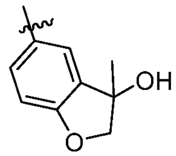, 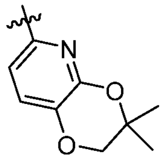, 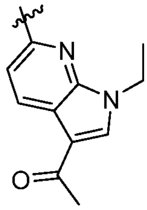,

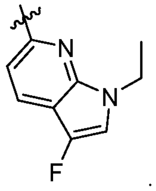.

4. The compound, or a stereoisomer thereof, or a pharmaceutically acceptable salt thereof according to claim 1, wherein:

$X_1$ is selected from a group consisting of chemical bond, O, $NR^{N1}$ or $CR^{C1}R^{C2}$;
$R^{C1}$, $R^{C2}$ are independently selected from a group consisting of H, -$C_{1\sim3}$ alkyl, halogen, halogen substituted -$C_{1\sim3}$ alkyl, -$C_{1\sim2}$ alkylene-N($C_{1\sim3}$ alkyl)( $C_{1\sim3}$ alkyl);
$R^{N1}$ is selected from a group consisting of H, -$C_{1\sim3}$ alkyl, halogen substituted -$C_{1\sim3}$ alkyl, -(3~10-membered heterocycloalkyl).

5. The compound, or a stereoisomer thereof, or a pharmaceutically acceptable salt thereof according to claim 4, wherein:

$R_4$ is selected from a group consisting of

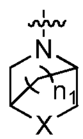, 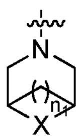, 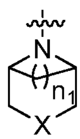, 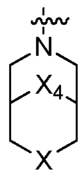, 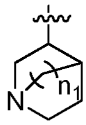, 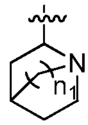,

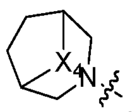,

wherein X is selected from O or $NR^{41}$; $X_4$ is selected from O, NH or $CH_2$; $n_1$ is selected from 1 or 2; said $R_4$ is optionally substituted by one, two, three or four independent $R^{41}$,
$R^{41}$ is selected from a group consisting of H, oxo, -$C_{1\sim3}$ alkyl, -$C_{2\sim6}$ alkenyl, halogen substituted -$C_{1\sim3}$ alkyl, hydroxy-substituted -$C_{1\sim6}$ alkyl, -$C_{0\sim2}$ alkylene-O($C_{1\sim3}$ alkyl), -$NH_2$, -NH($C_{1\sim3}$ alkyl), -N($C_{1\sim3}$ alkyl)( $C_{1\sim3}$ alkyl), -C(O)$R^{42}$, -C(O)$NR^{42}R^{43}$, -C(O)O$R^{42}$, -(3~10-membered cycloalkyl), - (3-10-membered heterocycloalkyl), -$C_{1\sim2}$ alkylene-(5-10-membered aromatic cyclyl); wherein said alkyl, alkylene, cycloalkyl, heterocycloalkyl, aromatic cyclyl are optionally substituted by one, two, three or four independent $R^{44}$.
$R^{42}$, $R^{43}$ are independently selected from a group consisting of H, halogen, -OH, -$C_{1\sim3}$ alkyl, halogen substituted

$-C_{1\sim3}$ alkyl;

$R^{44}$ is selected from a group consisting of H, $-C_{1\sim3}$ alkyl, halogen substituted $-C_{1\sim3}$ alkyl, $-O(C_{1\sim3}$ alkyl).

**6.** The compound, or a stereoisomer thereof, or a pharmaceutically acceptable salt thereof according to claim 5, wherein: $R_4$ is specifically selected from a group consisting of

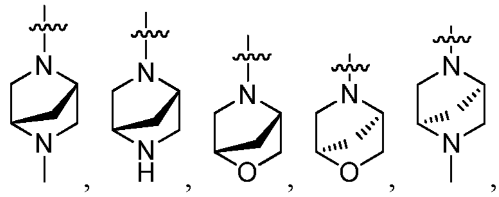

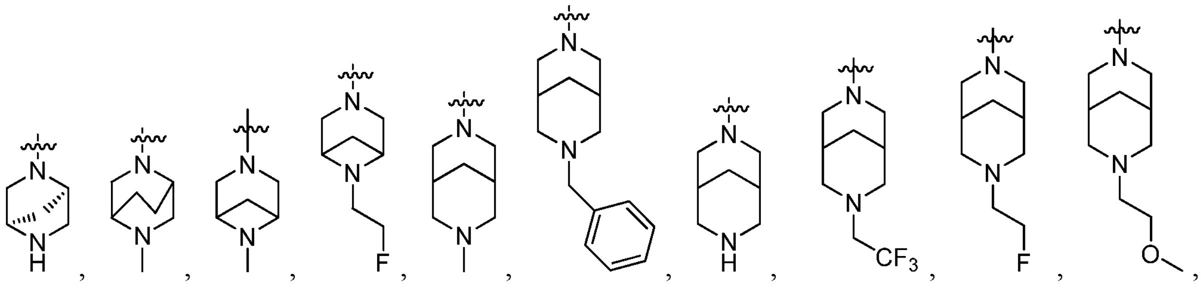

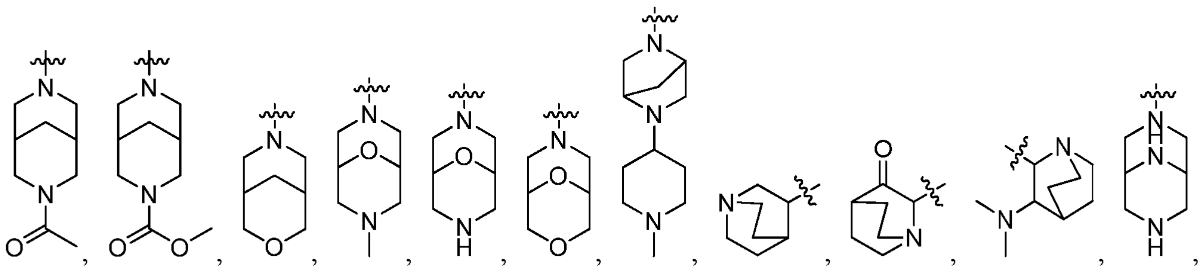

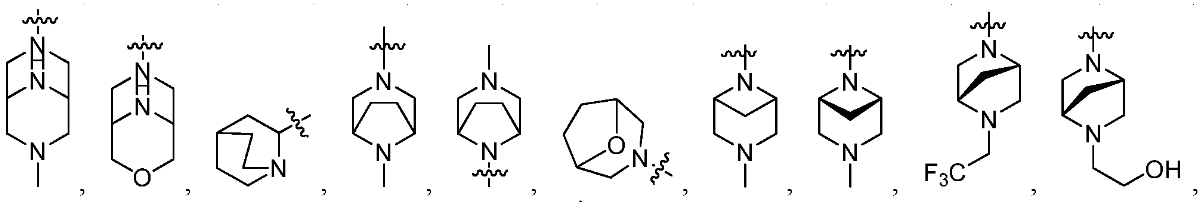

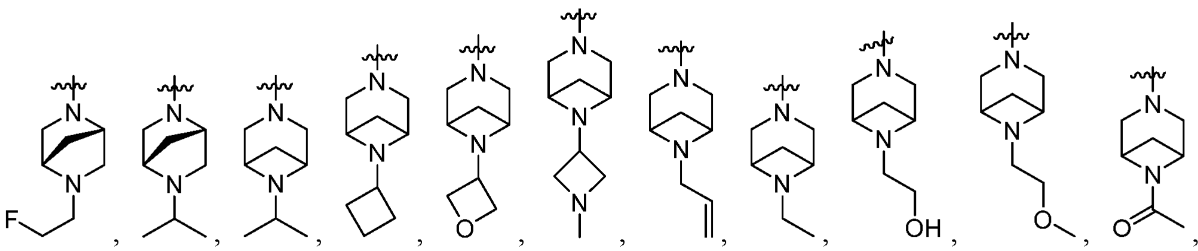

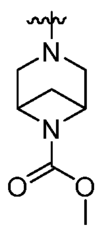

**7.** The compound, or a stereoisomer thereof, or a pharmaceutically acceptable salt thereof according to claim 1, wherein:

$R_5$ is selected from a group consisting of H, halogen, -$C_{1\sim3}$ alkyl, halogen substituted -$C_{1\sim3}$ alkyl, hydroxy-substituted -$C_{1\sim3}$ alkyl, -O($C_{1\sim3}$ alkyl), -NH($C_{1\sim3}$ alkyl), -O-$C_2$alkylene-N($C_{1\sim3}$ alkyl)( $C_{1\sim3}$ alkyl), -O-(3~10-membered cycloalkyl), -O-(3~10-membered heterocycloalkyl); wherein said alkyl, alkylene, cycloalkyl, heterocycloalkyl are optionally substituted by one, two, three or four independent $R^{51}$,
$R^{51}$ is selected from a group consisting of H, -$C_{1\sim3}$ alkyl, halogen substituted -$C_{1\sim3}$ alkyl.

**8.** The compound, or a stereoisomer thereof, or a pharmaceutically acceptable salt thereof according to claim 7, wherein:
$R^5$ is selected from a group consisting of methyl, ethyl, ethoxyl, F, hydroxy-substituted methyl,

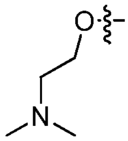 , 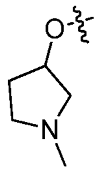

**9.** The compound, or a stereoisomer thereof, or a pharmaceutically acceptable salt thereof according to claim 1, wherein:
$R_6$ is selected from a group consisting of H, -$C_{1\sim3}$ alkyl, -NH($C_{1\sim3}$ alkyl), -N($C_{1\sim3}$ alkyl)( $C_{1\sim3}$ alkyl).

**10.** The compound, or a stereoisomer thereof, or a pharmaceutically acceptable salt thereof according to claim 1, wherein:
A ring is selected from

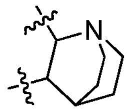

**11.** The compound, or a stereoisomer thereof, or a pharmaceutically acceptable salt thereof according to claim 1, wherein:

B ring is selected from a group consisting of 3~10-membered heterocyclyl, 5~12-membered bridged heterocyclyl, 3~10-membered fused heterocyclyl; wherein said heterocyclyl, bridged heterocyclyl, fused heterocyclyl are optionally substituted by one, two, three or four independent $R^{B1}$;
each $R^{B1}$ is independently selected from a group consisting of H, -$C_{1\sim3}$ alkyl, halogen substituted -$C_{1\sim3}$ alkyl.

**12.** The compound, or a stereoisomer thereof, or a pharmaceutically acceptable salt thereof according to claim 1, wherein: B ring is selected from a group consisting of

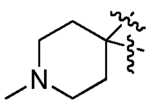 , 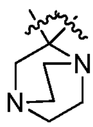 , 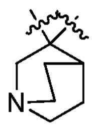 , 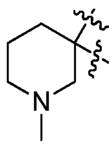 ,

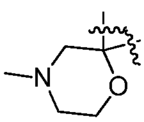 , 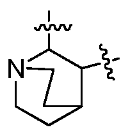 , 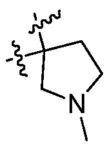 , 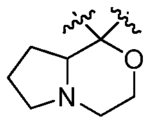 , 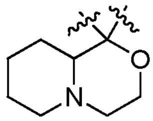

**13.** The compound, or a stereoisomer thereof, or a pharmaceutically acceptable salt thereof according to claim 1, wherein:

C ring is selected from a group consisting of

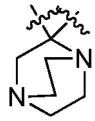, 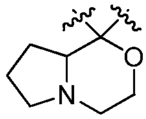, 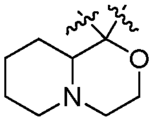

14. The compound, or a stereoisomer thereof, or a pharmaceutically acceptable salt thereof according to claim 1, wherein:

$R_7$, $R_8$ are independently selected from a group consisting of -OH,

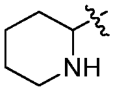, 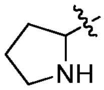;

or $R_7$, $R_8$ together with the atom adjacent therewith form

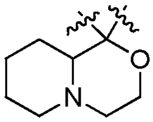, 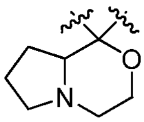

15. The compound, or a stereoisomer thereof, or a pharmaceutically acceptable salt thereof according to claim 1 wherein: $R_3$ is selected from a group consisting of

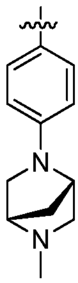, 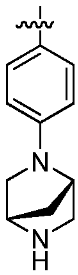, 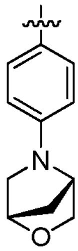, 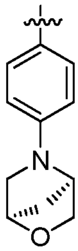, 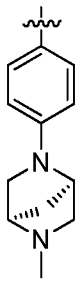, 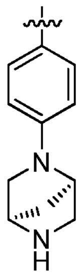,

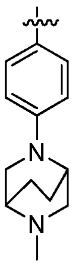, 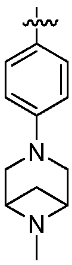, 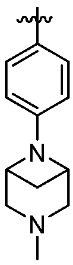, 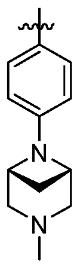, 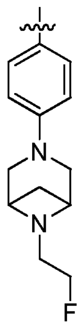, 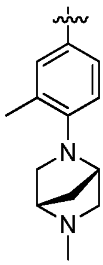, 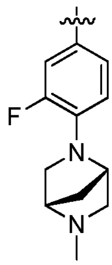, 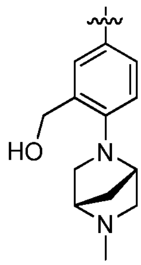, 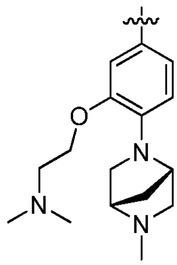, 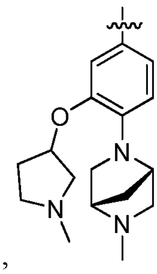,

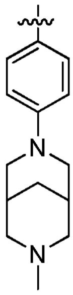, 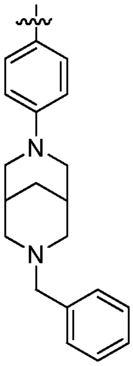, 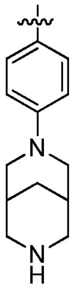, 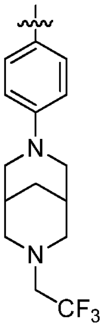, 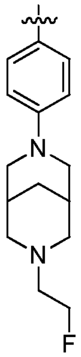, 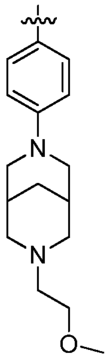, 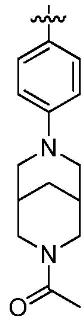, 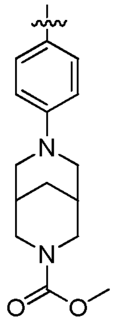, 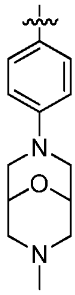, 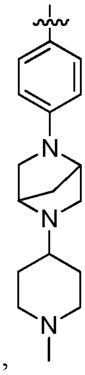, 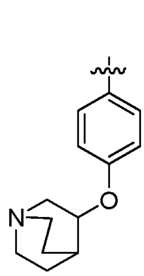,

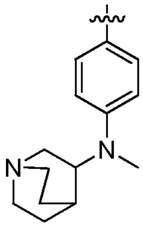, 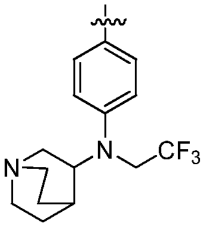, 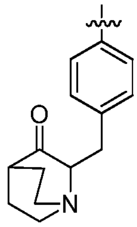, 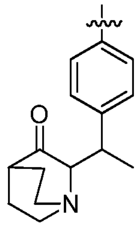, 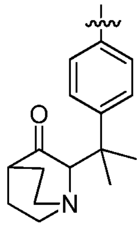, 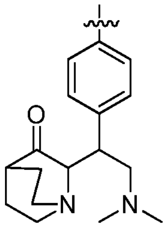, 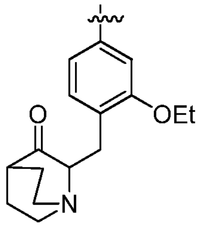,

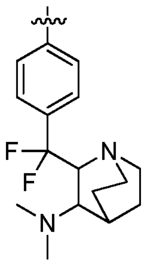, 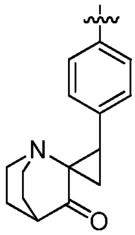, 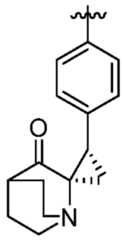, 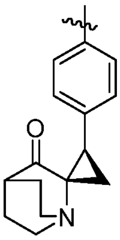, 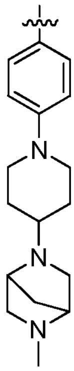, 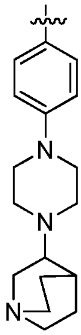, 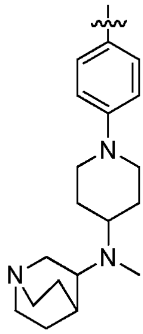, 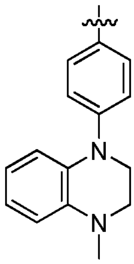, 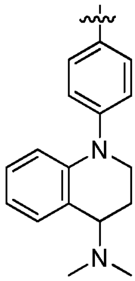,

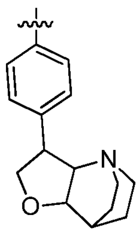, 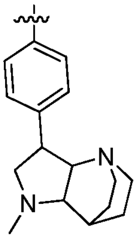, 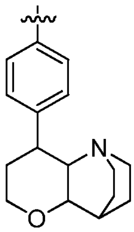, 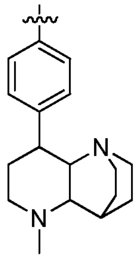, 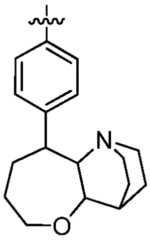, 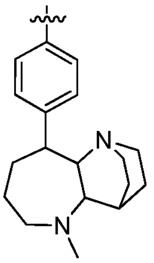, 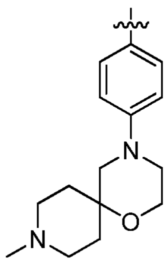,

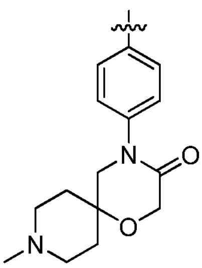, 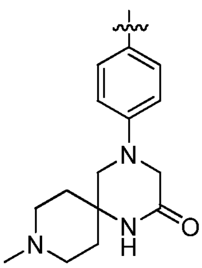, 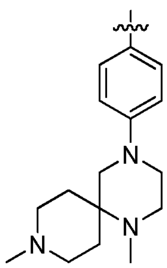, 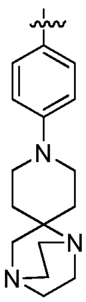, 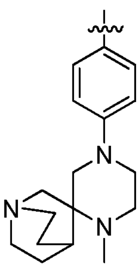, 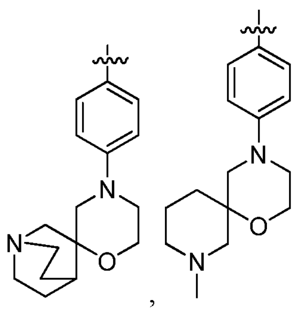, ,

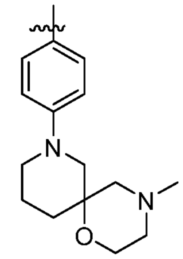, 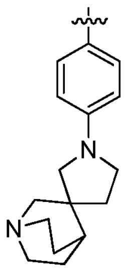, 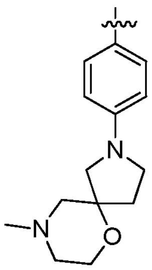, 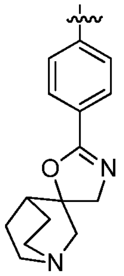, 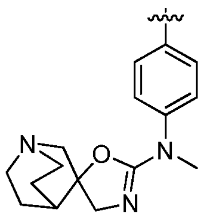, 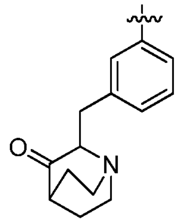, 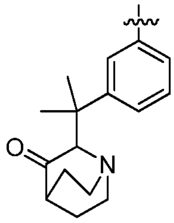,

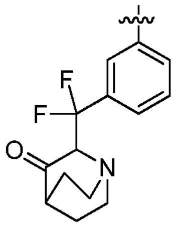, 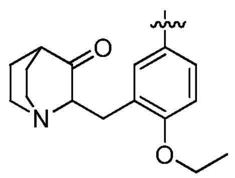, 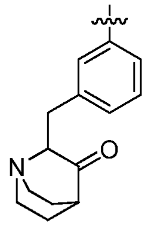, 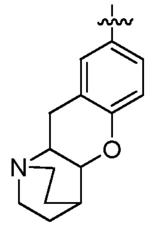, 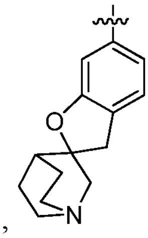, 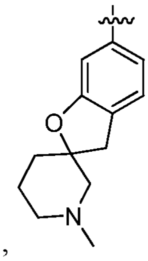, 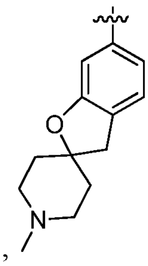,

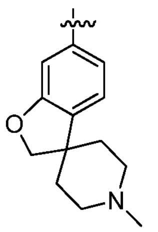, 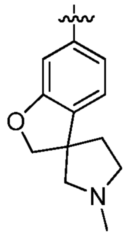, 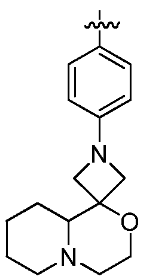, 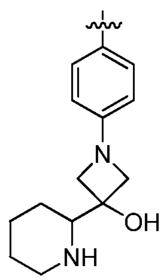, 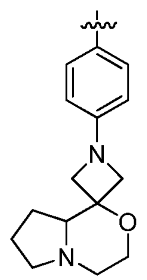, 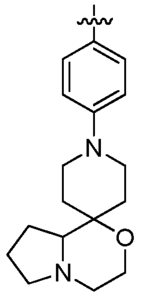, 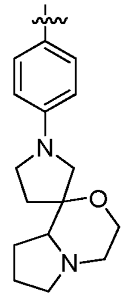,

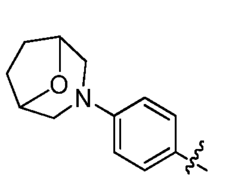, 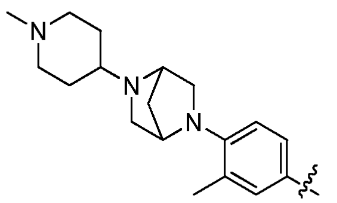, 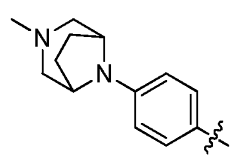, 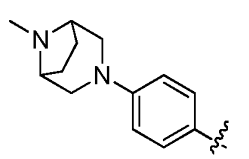,

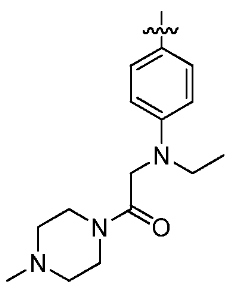, 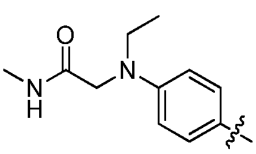, 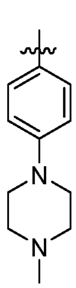, 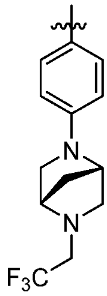, 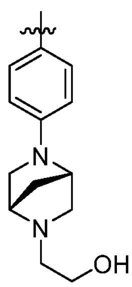, 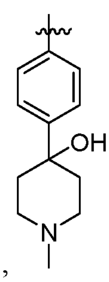, 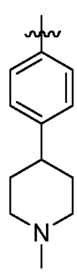, 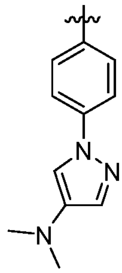,

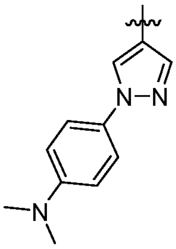, 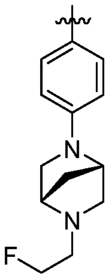, 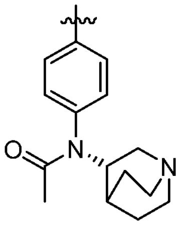, 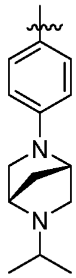, 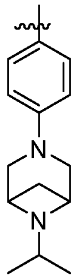, 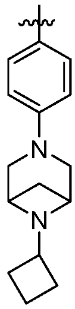, 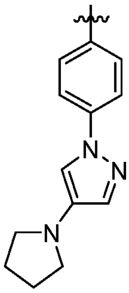, 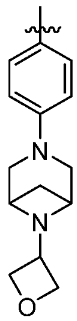, 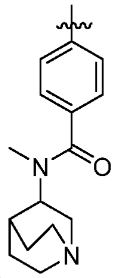, 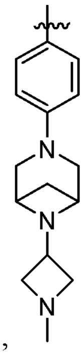,

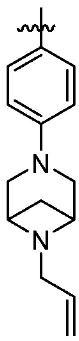, 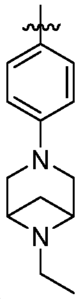, 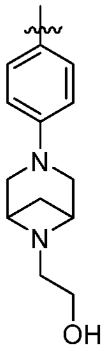, 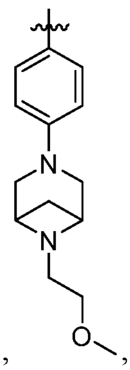, 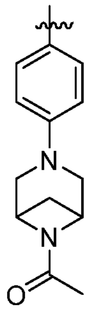, 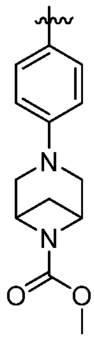, 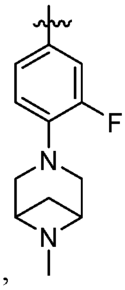, 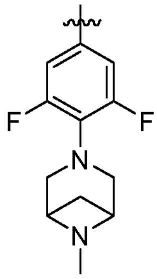

**16.** The compound, or a stereoisomer thereof, or a pharmaceutically acceptable salt thereof according to claim 1, wherein:
the specific compound represented by formula I is

| No. of the compound | Chemical structure | No. of the compound | Chemical structure |
|---|---|---|---|
| WEE1-001 | 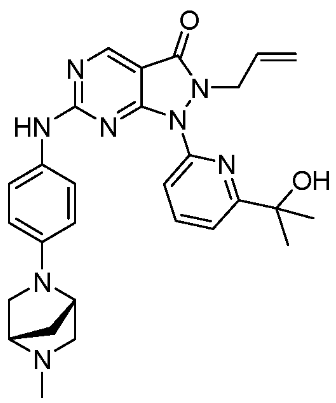 | WEE1-002 | 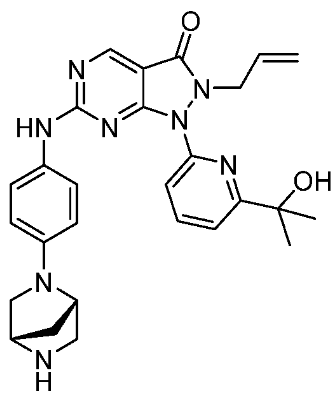 |
| WEE1-003 | 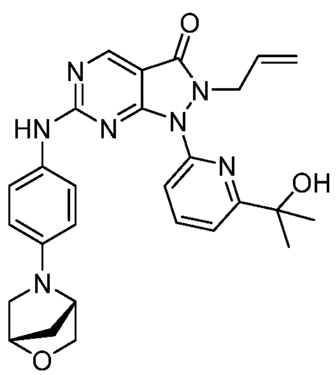 | WEE1-004 | 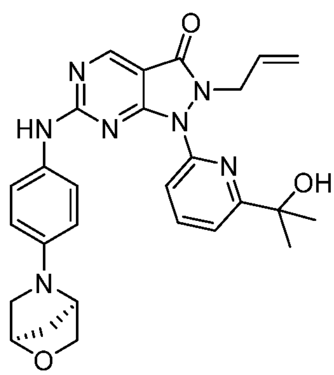 |

(continued)

| No. of the compound | Chemical structure | No. of the compound | Chemical structure |
|---|---|---|---|
| WEE1-005 | 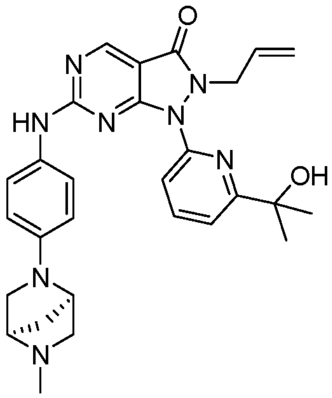 | WEE1-006 | 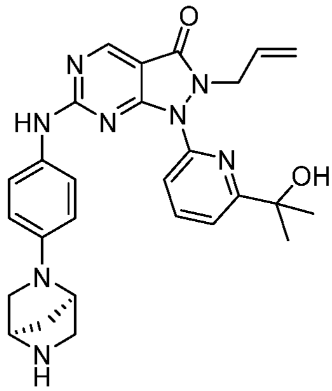 |
| WEE1-007 | 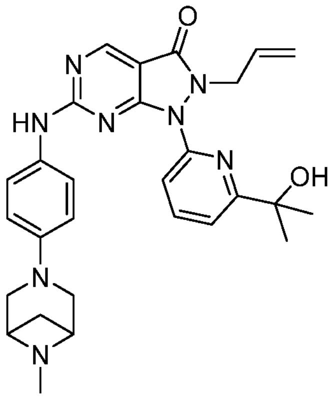 | WEE1-008 | 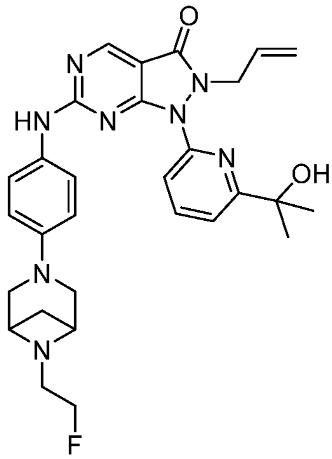 |
| WEE1-009 | 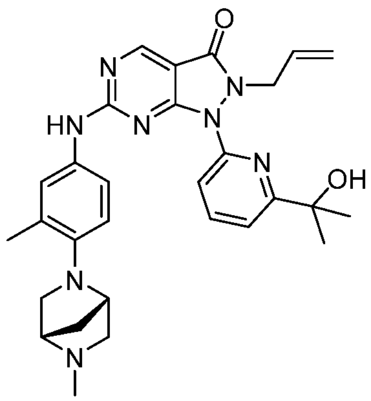 | WEE1-010 | 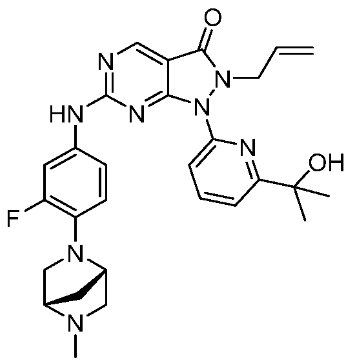 |
| WEE1-011 | 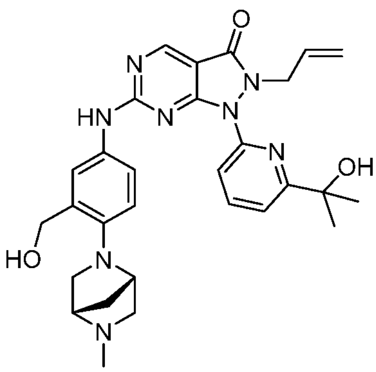 | WEE1-012 | 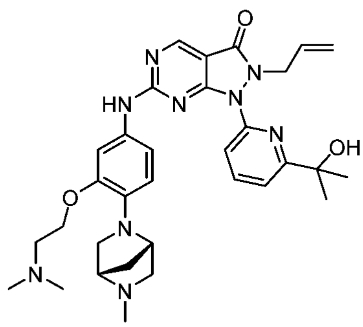 |

(continued)

| No. of the compound | Chemical structure | No. of the compound | Chemical structure |
|---|---|---|---|
| WEE1-013 | 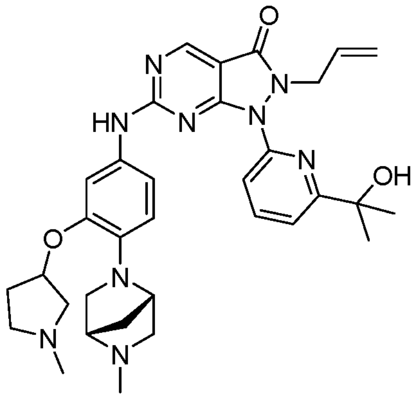 | WEE1-014 | 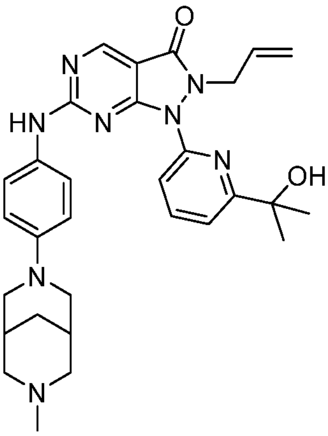 |
| WEE1-015 | 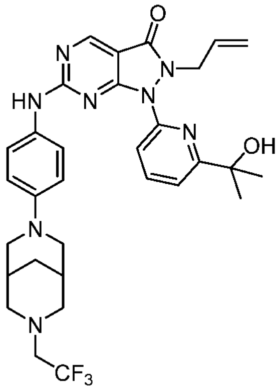 | WEE1-016 | 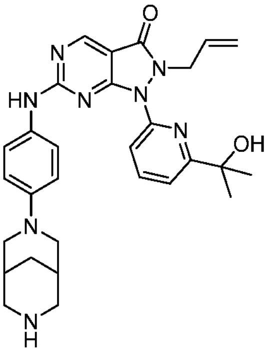 |
| WEE1-017 | 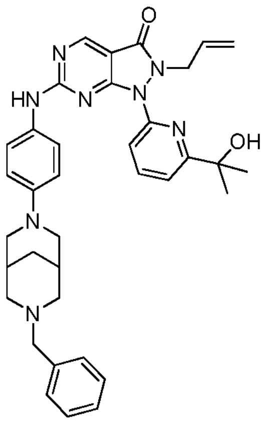 | WEE1-018 | 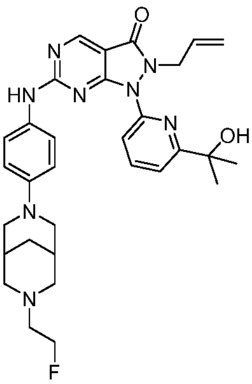 |
| WEE1-019 | 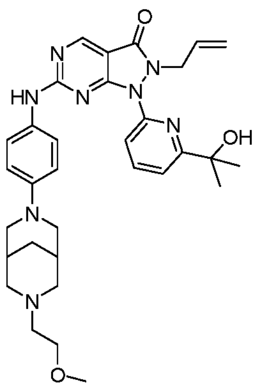 | WEE1-020 | 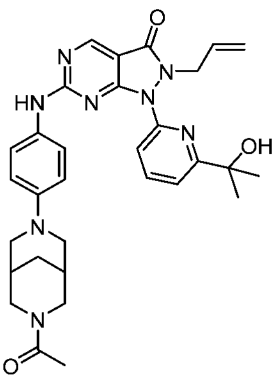 |

(continued)

| No. of the compound | Chemical structure | No. of the compound | Chemical structure |
|---|---|---|---|
| WEE1-021 | 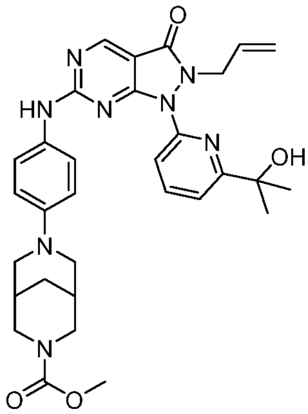 | WEE1-022 | 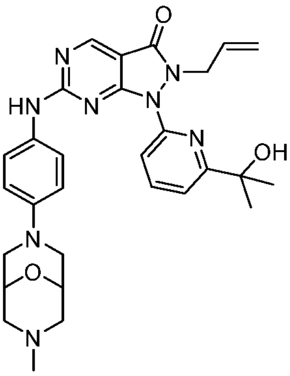 |
| WEE1-023 | 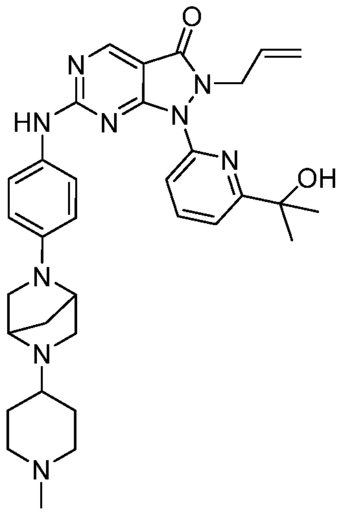 | WEE 1-024 | 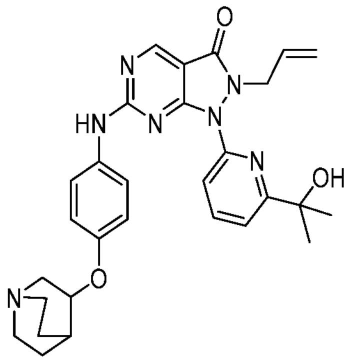 |
| WEE1-025 | 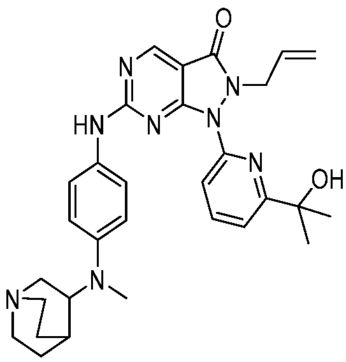 | WEE1-026 | 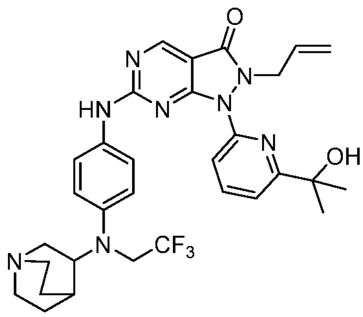 |

(continued)

| No. of the compound | Chemical structure | No. of the compound | Chemical structure |
|---|---|---|---|
| WEE1-027 | 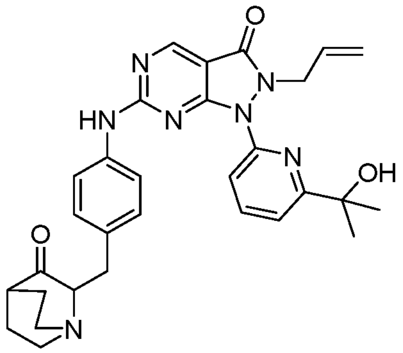 | WEE1-028 | 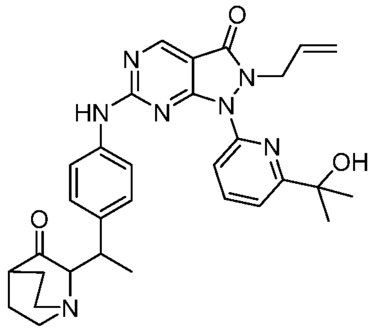 |
| WEE1-029 | 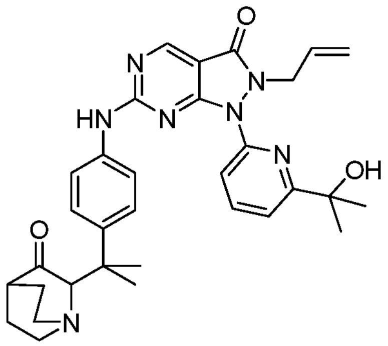 | WEE1-030 | 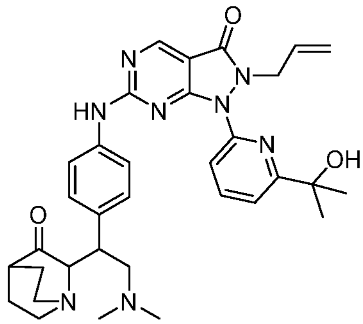 |
| WEE1-031 | 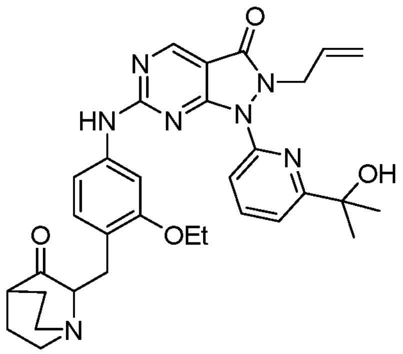 | WEE1-032 | 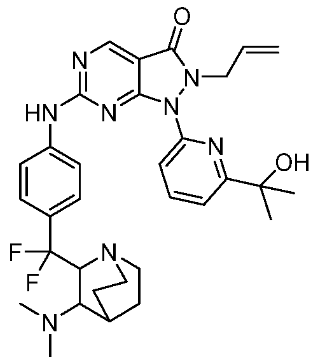 |
| WEE1-033 | 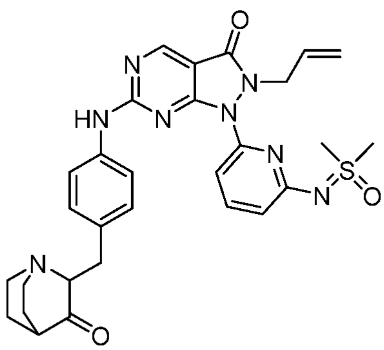 | WEE1-034 | 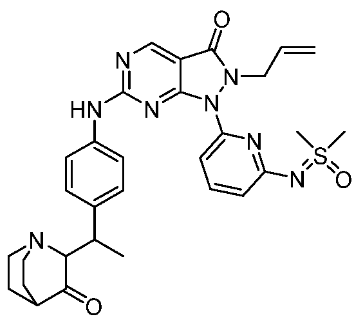 |

(continued)

| No. of the compound | Chemical structure | No. of the compound | Chemical structure |
|---|---|---|---|
| WEE1-035 | 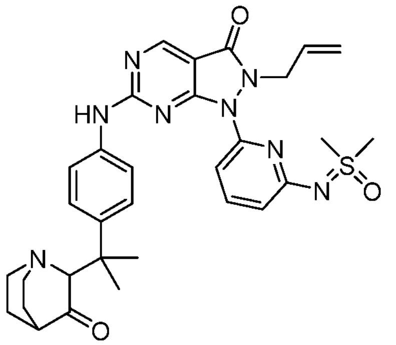 | WEE1-036 | 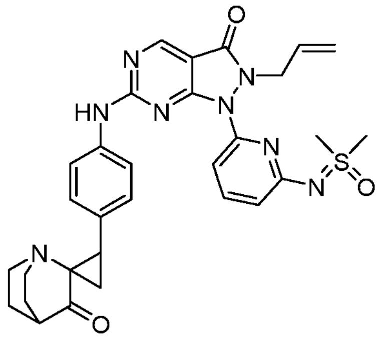 |
| WEE1-037 | 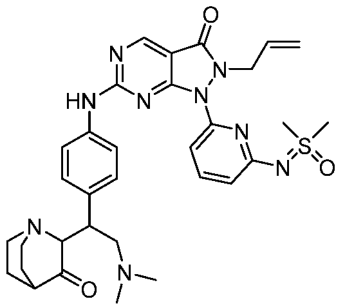 | WEE1-038 | 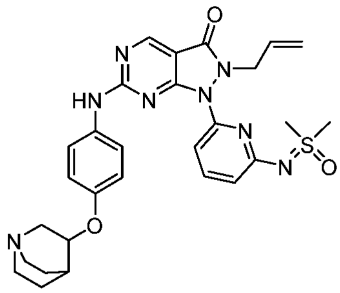 |
| WEE1-039 | 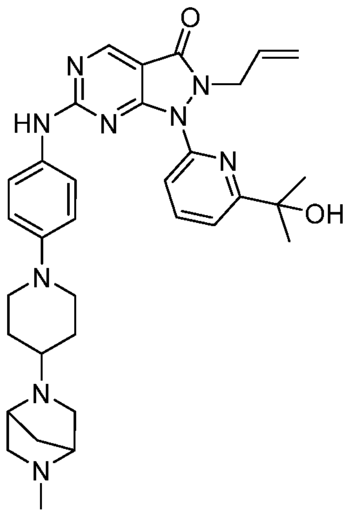 | WEE1-040 | 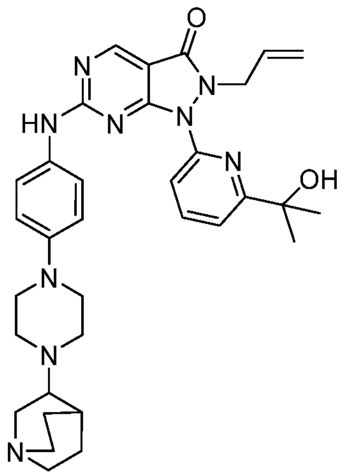 |

(continued)

| No. of the compound | Chemical structure | No. of the compound | Chemical structure |
|---|---|---|---|
| WEE1-041 | 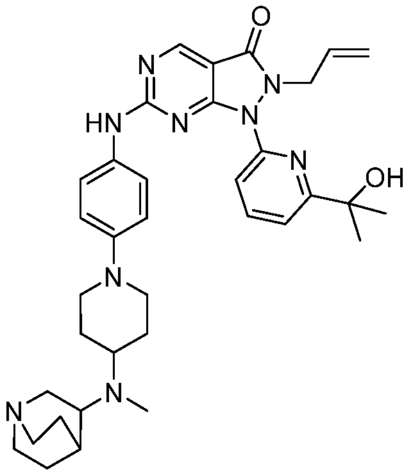 | WEE1-042 | 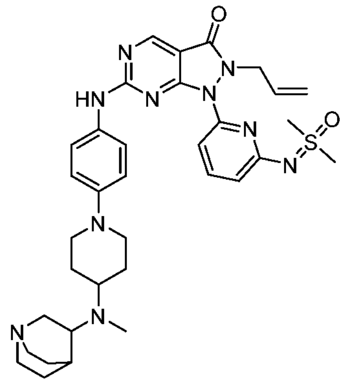 |
| WEE1-043 | 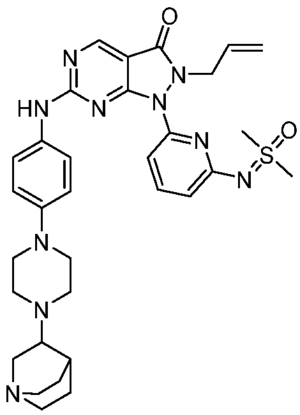 | WEE1-044 | 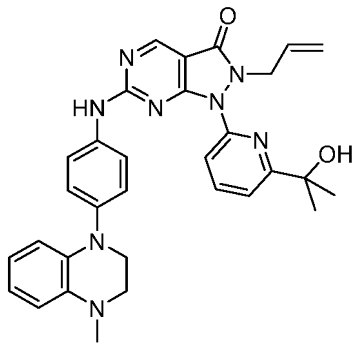 |
| WEE1-045 | 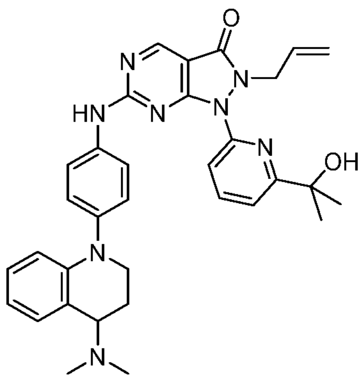 | WEE1-046 | 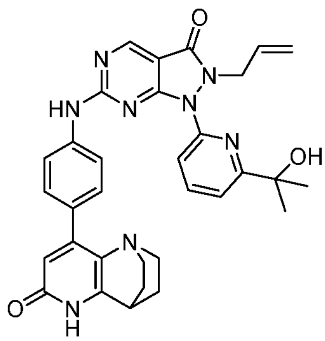 |

(continued)

| No. of the compound | Chemical structure | No. of the compound | Chemical structure |
|---|---|---|---|
| WEE1-047 | 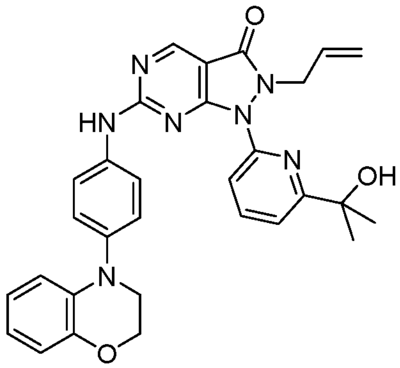 | WEE1-048 | 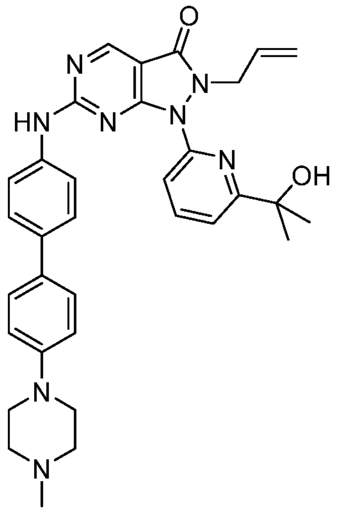 |
| WEE1-049 | 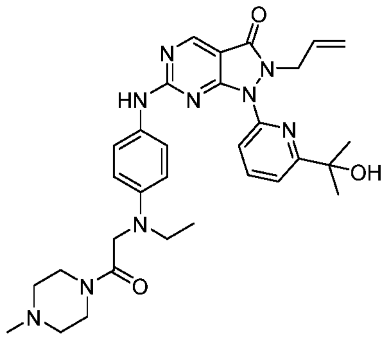 | WEE1-050 | 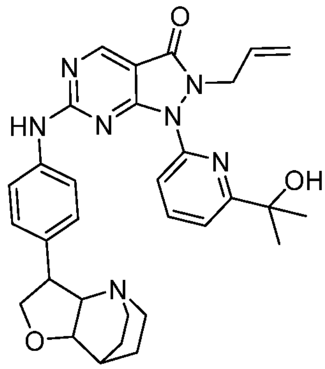 |
| WEE1-051 | 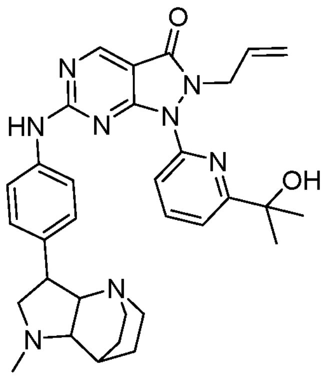 | WEE1-052 | 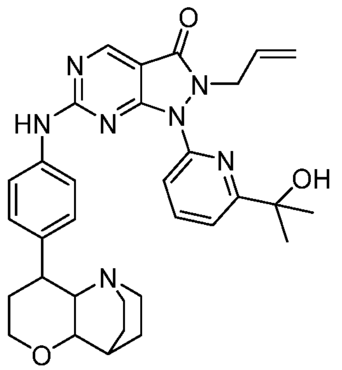 |

(continued)

| No. of the compound | Chemical structure | No. of the compound | Chemical structure |
|---|---|---|---|
| WEE1-053 | 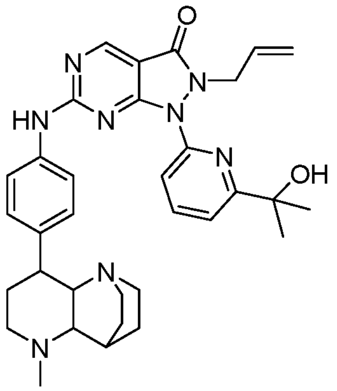 | WEE1-054 | 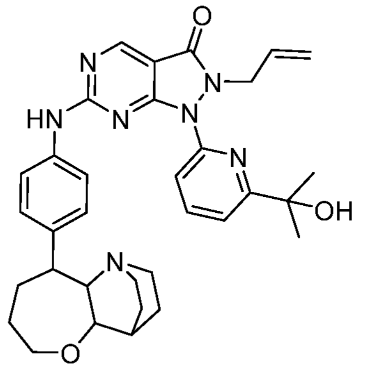 |
| WEE1-055 | 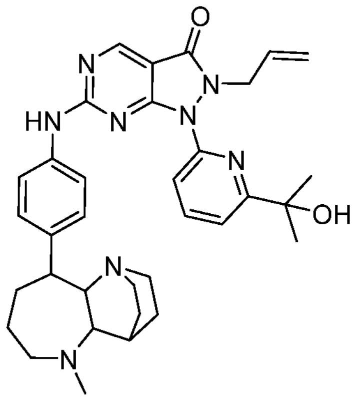 | WEE1-056 | 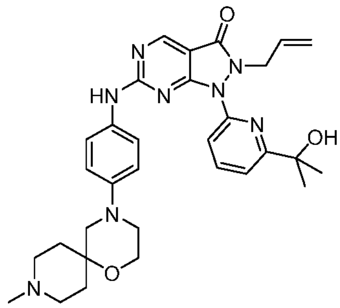 |
| WEE1-057 | 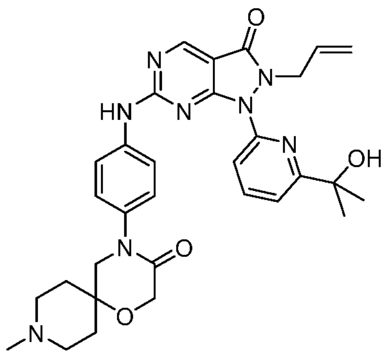 | WEE1-058 | 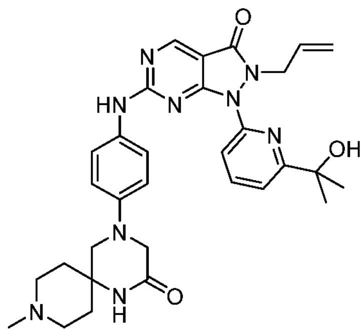 |
| WEE1-059 | 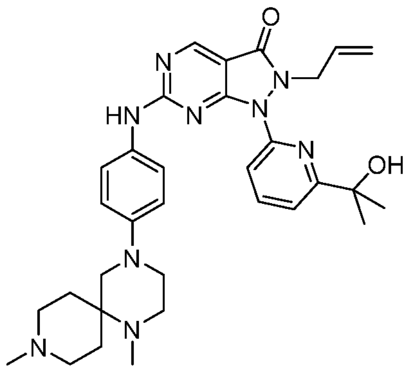 | WEE1-060 | 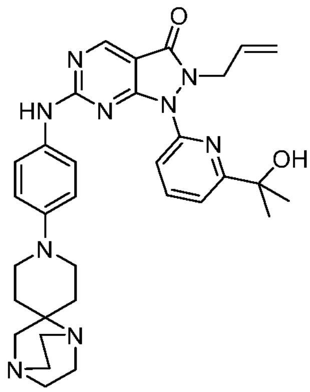 |

(continued)

| No. of the compound | Chemical structure | No. of the compound | Chemical structure |
|---|---|---|---|
| WEE1-061 | 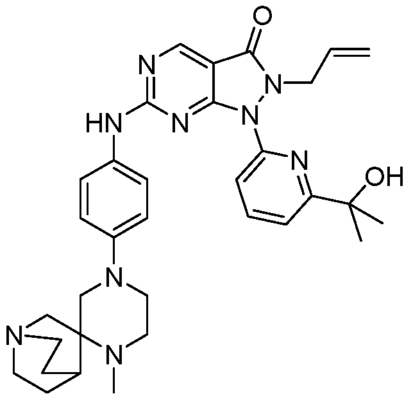 | WEE1-062 | 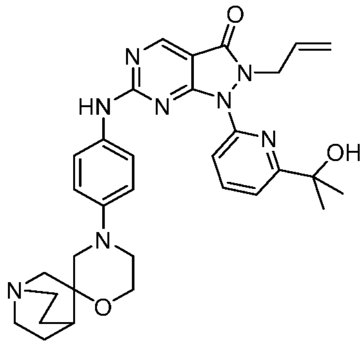 |
| WEE1-063 | 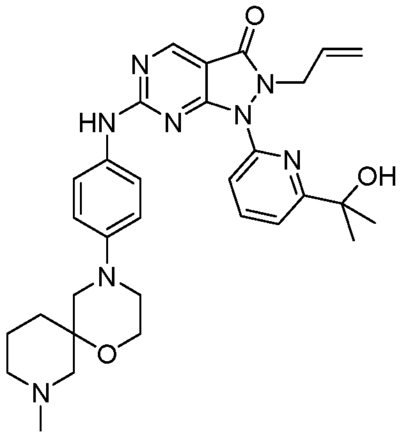 | WEE1-064 | 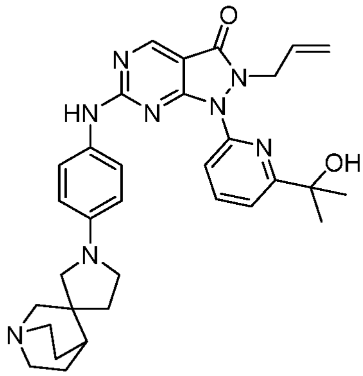 |
| WEE1-065 | 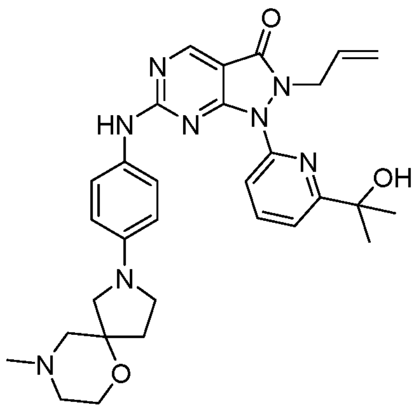 | WEE1-066 | 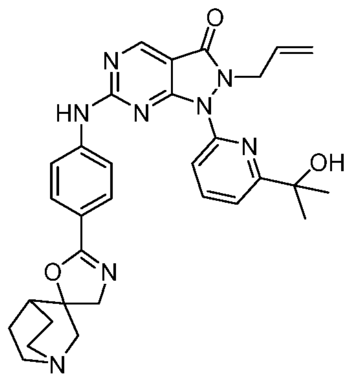 |
| WEE1-067 | 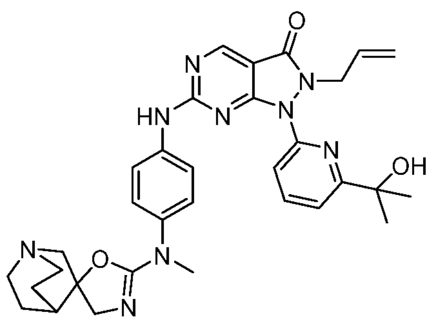 | WEE1-068 | 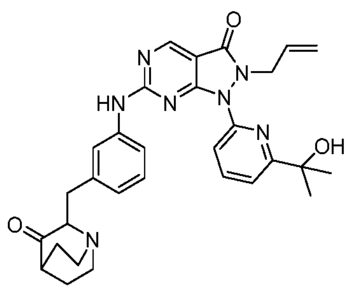 |

(continued)

| No. of the compound | Chemical structure | No. of the compound | Chemical structure |
|---|---|---|---|
| WEE1-069 | 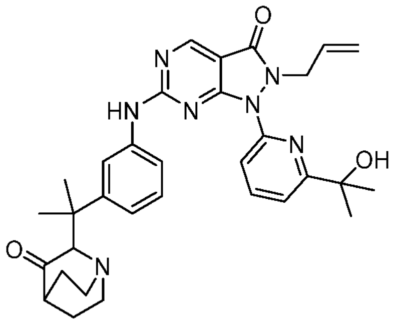 | WEE1-070 | 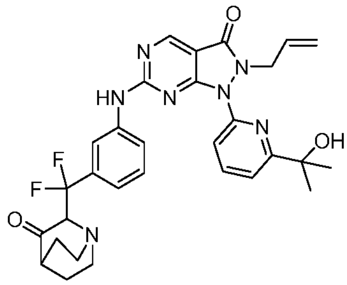 |
| WEE1-071 | 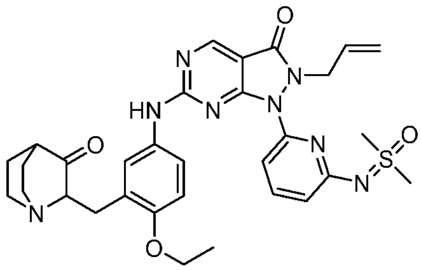 | WEE1-072 | 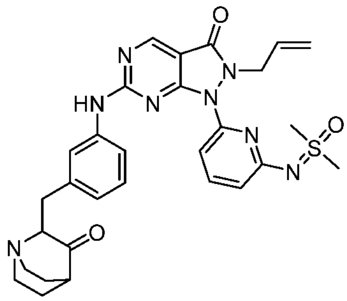 |
| WEE1-073 | 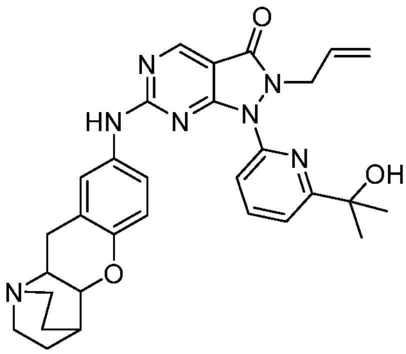 | WEE1-074 | 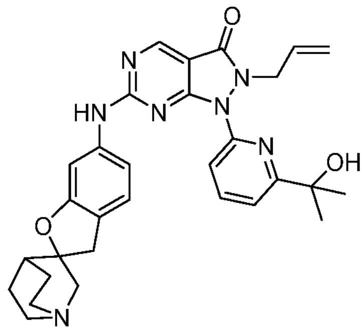 |
| WEE1-075 | 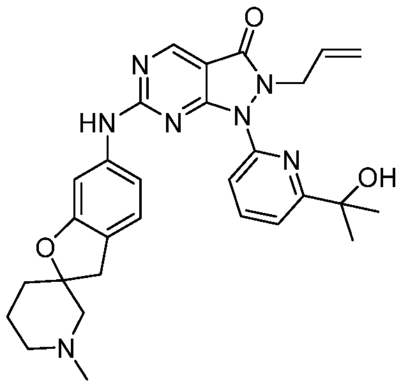 | WEE1-076 | 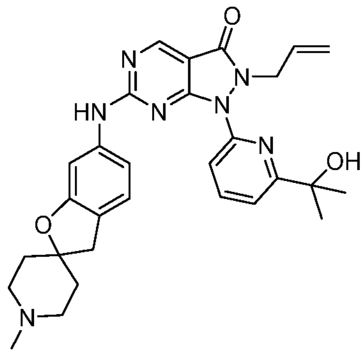 |

(continued)

| No. of the compound | Chemical structure | No. of the compound | Chemical structure |
|---|---|---|---|
| WEE1-077 | 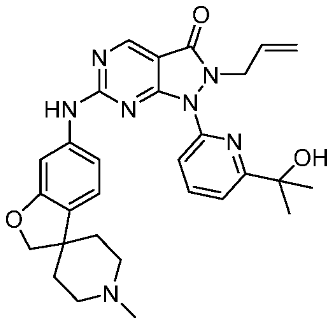 | WEE1-078 | 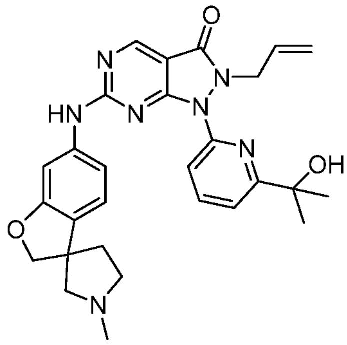 |
| WEE1-079 | 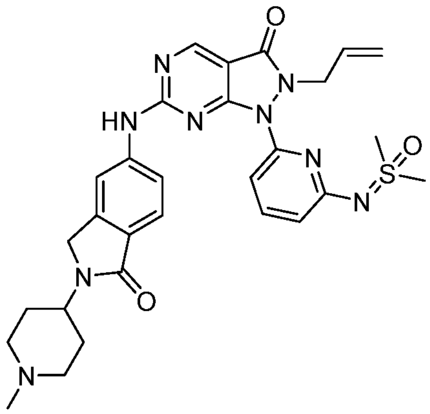 | WEE1-080 | 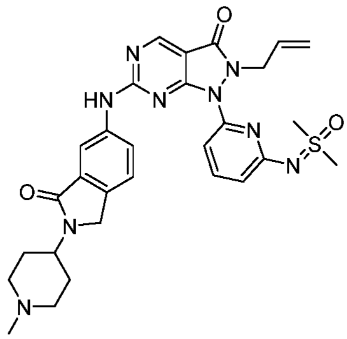 |
| WEE1-081 | 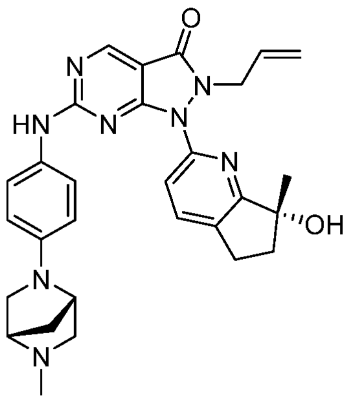 | WEE1-082 | 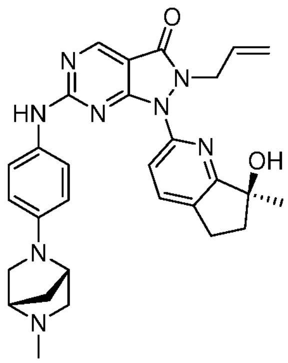 |
| WEE1-083 | 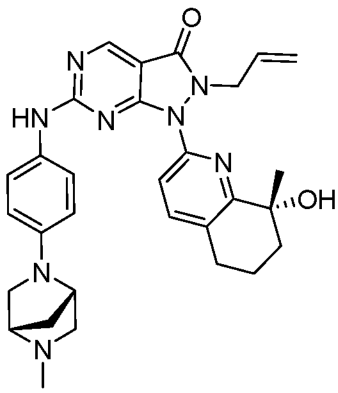 | WEE1-084 | 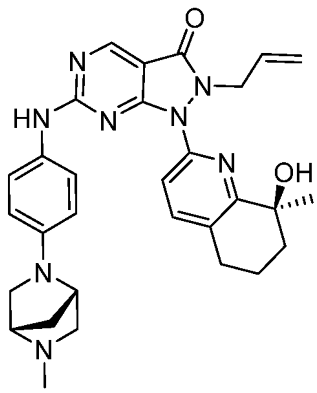 |

(continued)

| No. of the compound | Chemical structure | No. of the compound | Chemical structure |
|---|---|---|---|
| WEE1-085 | 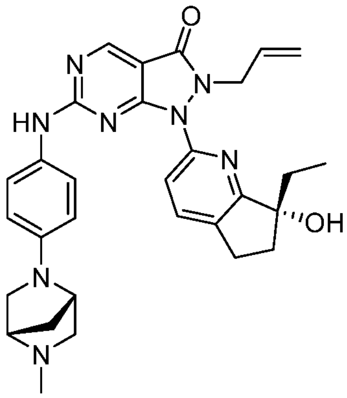 | WEE1-086 | 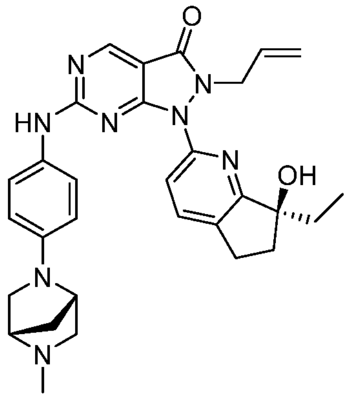 |
| WEE1-087 | 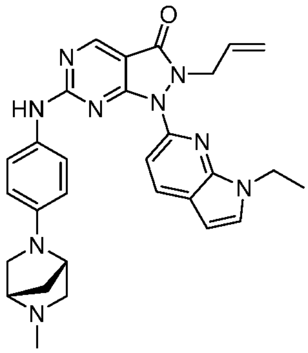 | WEE1-088 | 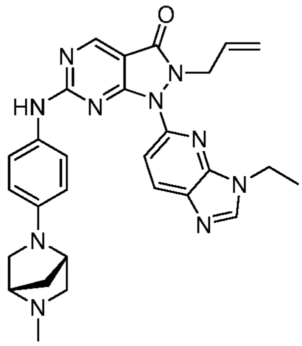 |
| WEE1-089 | 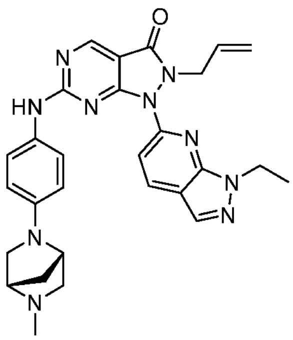 | WEE1-090 | 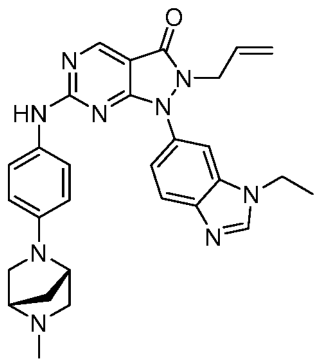 |
| WEE1-091 | 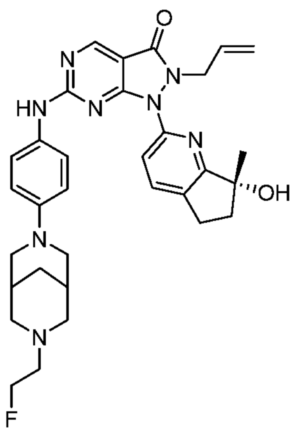 | WEE1-092 | 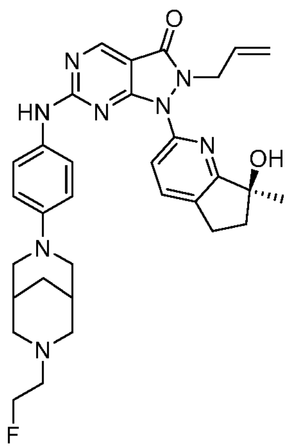 |

(continued)

| No. of the compound | Chemical structure | No. of the compound | Chemical structure |
|---|---|---|---|
| WEE1-093 | 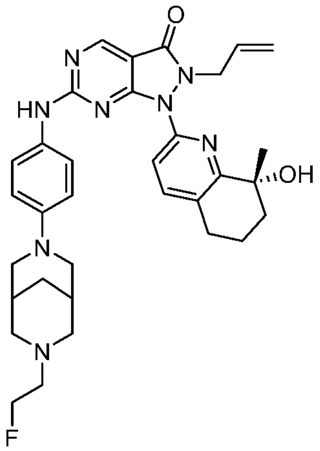 | WEE1-094 | 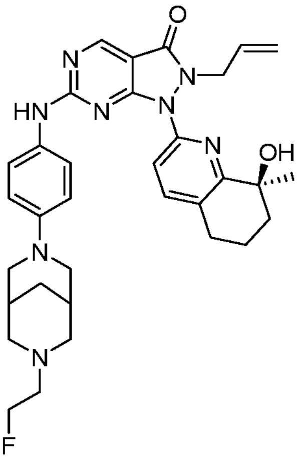 |
| WEE1-095 | 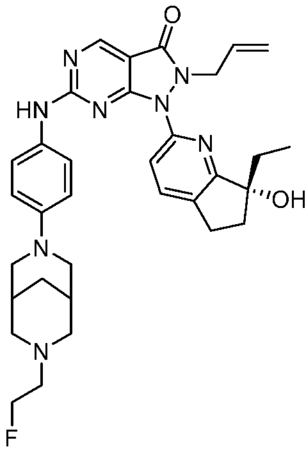 | WEE1-096 | 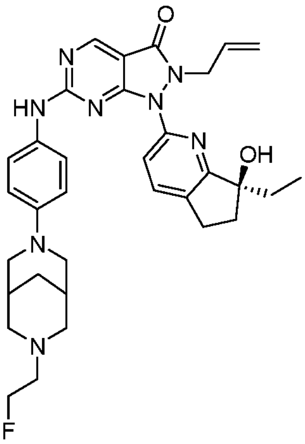 |
| WEE1-097 | 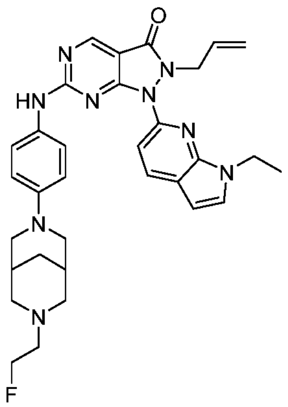 | WEE1-098 | 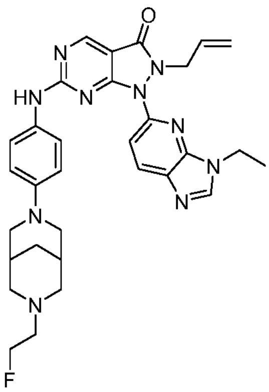 |

(continued)

| No. of the compound | Chemical structure | No. of the compound | Chemical structure |
|---|---|---|---|
| WEE1-099 | 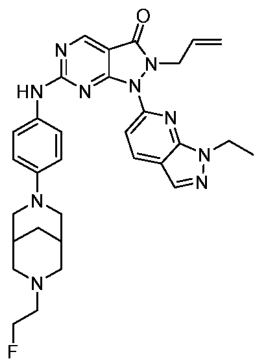 | WEE1-100 | 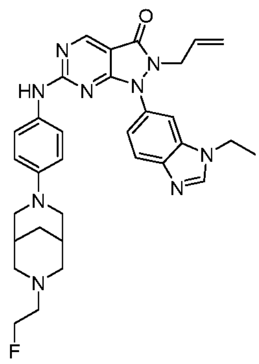 |
| WEE1-101 | 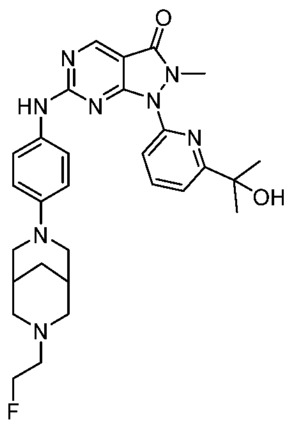 | WEE1-102 | 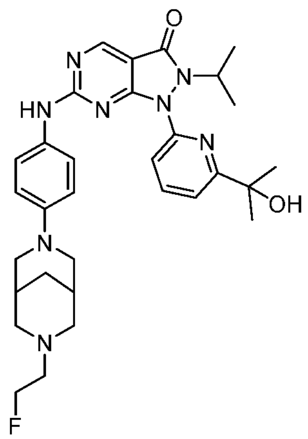 |
| WEE1-103 | 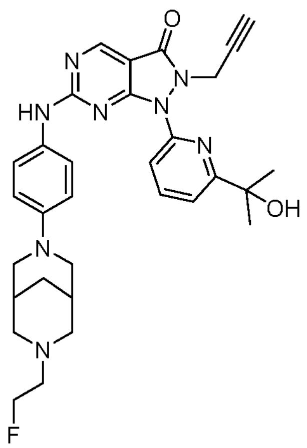 | WEE1-104 | 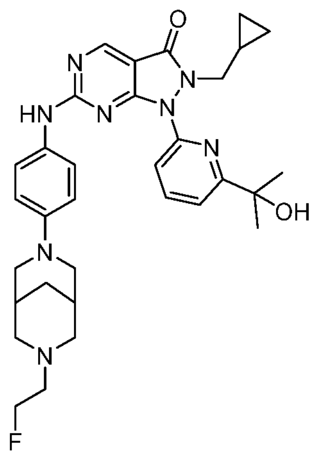 |

(continued)

| No. of the compound | Chemical structure | No. of the compound | Chemical structure |
|---|---|---|---|
| WEE1-105 | 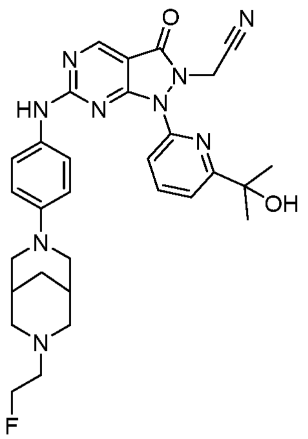 | WEE1-106 | 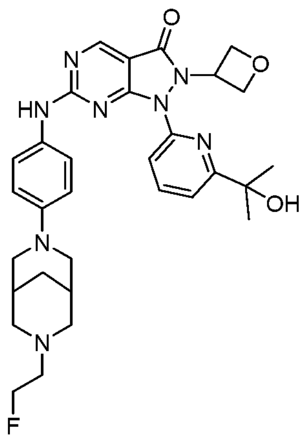 |
| WEE1-107 | 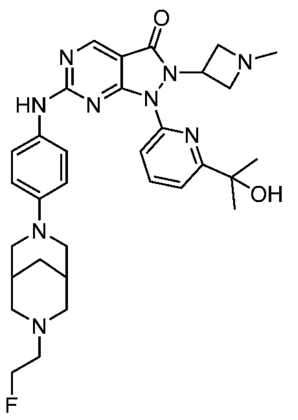 | WEE1-108 | 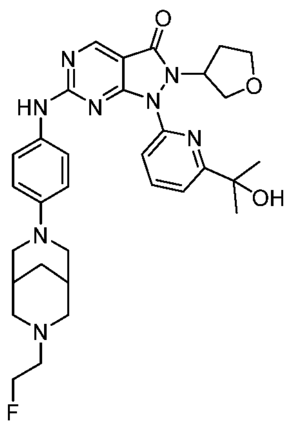 |
| WEE1-109 | 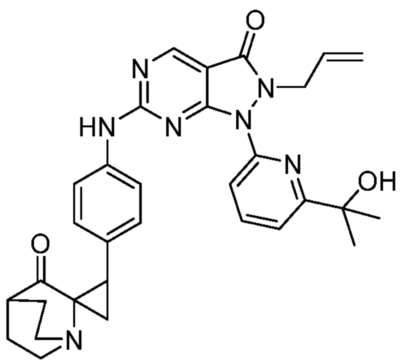 | WEE1-110 | 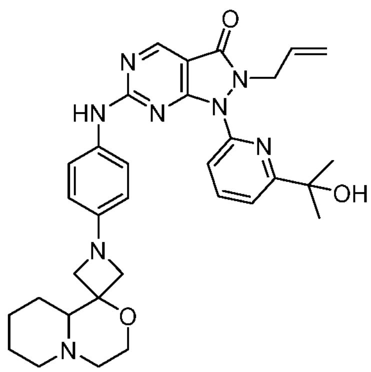 |
| WEE1-111 | 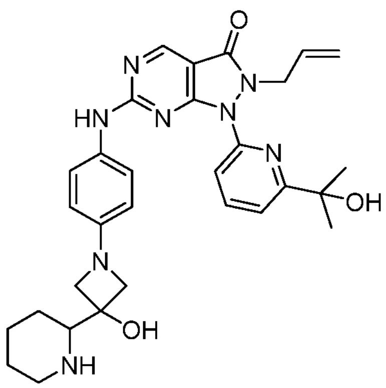 | WEE1-112 | 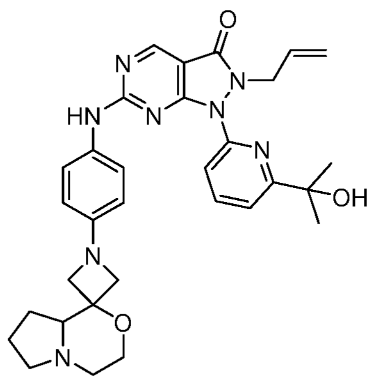 |

(continued)

| No. of the compound | Chemical structure | No. of the compound | Chemical structure |
|---|---|---|---|
| WEE1-113 | 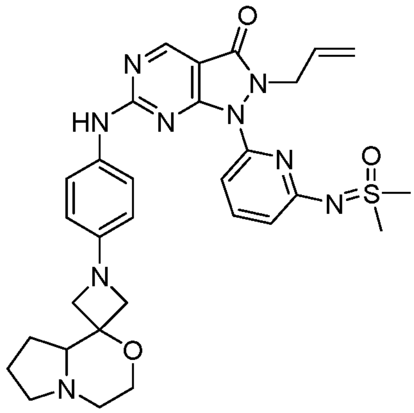 | WEE1-114 | 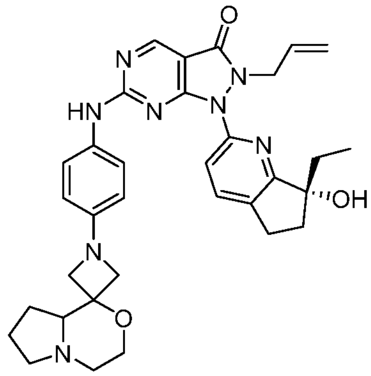 |
| WEE1-115 | 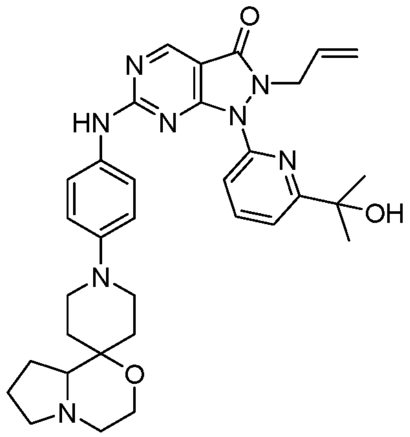 | WEE1-116 | 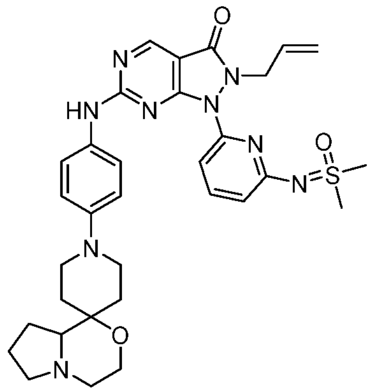 |
| WEE1-117 | 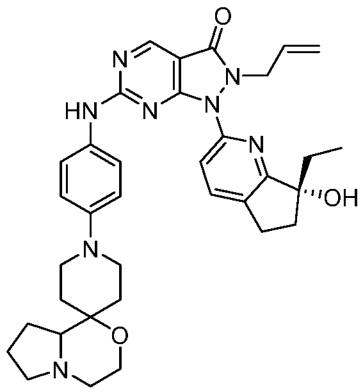 | WEE1-118 | 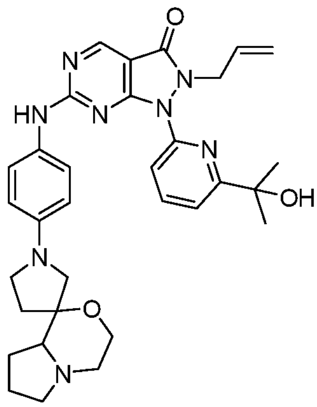 |

(continued)

| No. of the compound | Chemical structure | No. of the compound | Chemical structure |
|---|---|---|---|
| WEE1-119 | 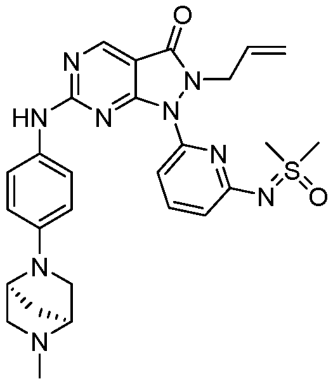 | WEE1-120 | 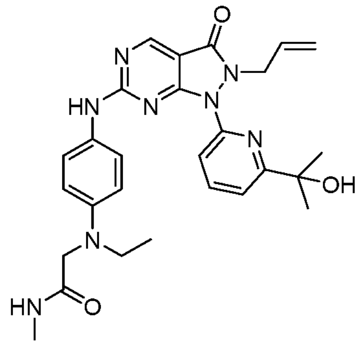 |
| WEE1-121 | 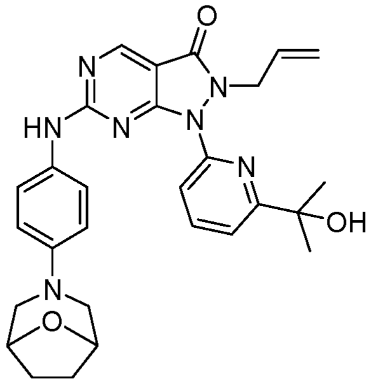 | WEE1-122 | 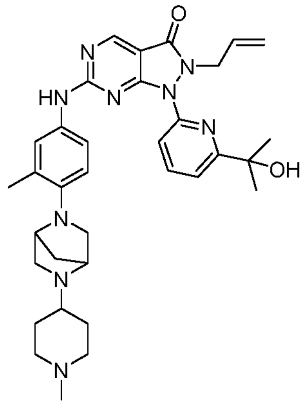 |
| WEE1-123 | 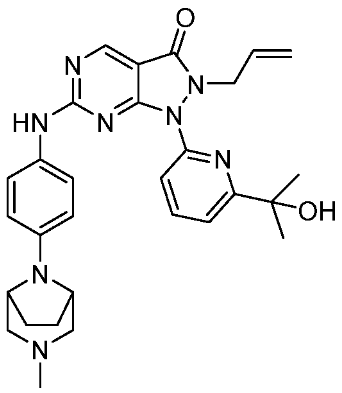 | WEE1-124 | 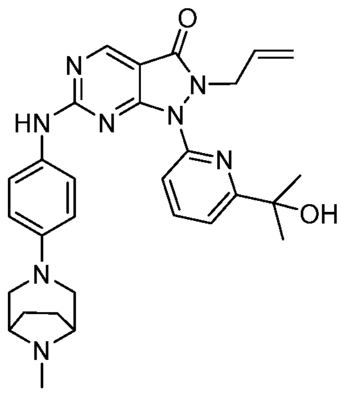 |
| WEE1-125 | 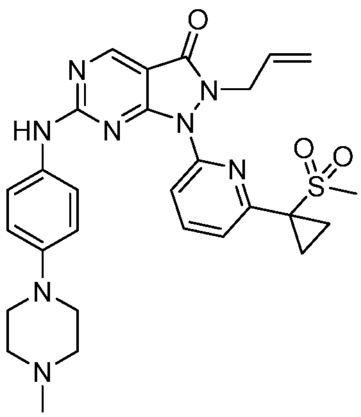 | WEE1-126 | 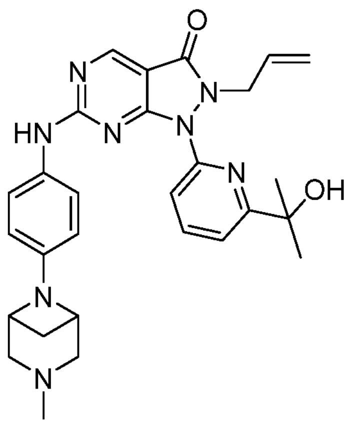 |

(continued)

| No. of the compound | Chemical structure | No. of the compound | Chemical structure |
|---|---|---|---|
| WEE1-127 | 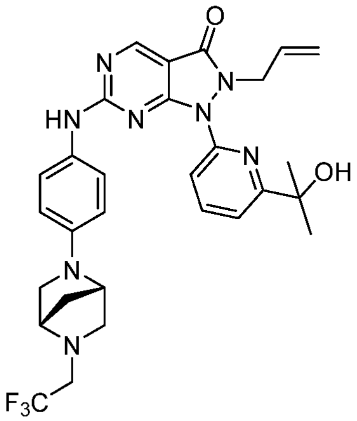 | WEE1-128 | 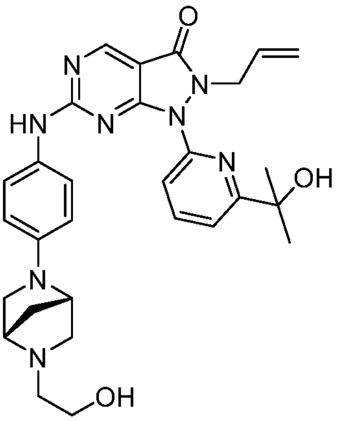 |
| WEE1-129 | 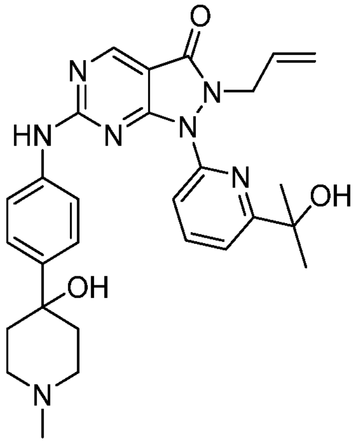 | WEE1-130 | 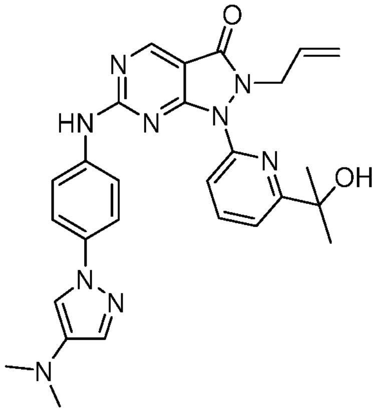 |
| WEE1-131 | 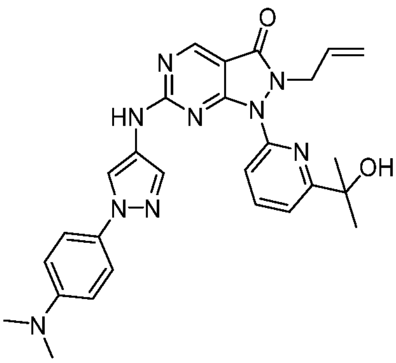 | WEE1-132 | 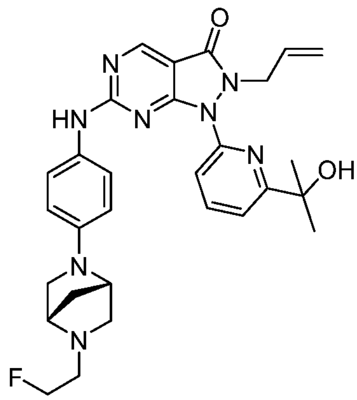 |

(continued)

| No. of the compound | Chemical structure | No. of the compound | Chemical structure |
|---|---|---|---|
| WEE1-133 | 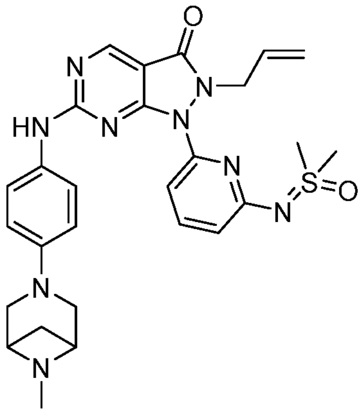 | WEE1-134 | 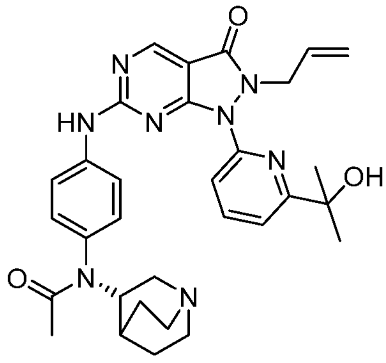 |
| WEE1-135 | 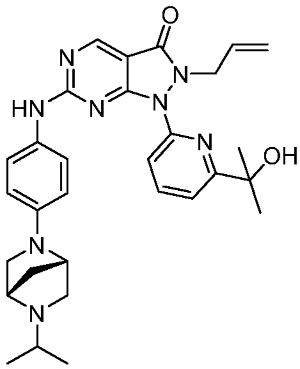 | WEE1-136 | 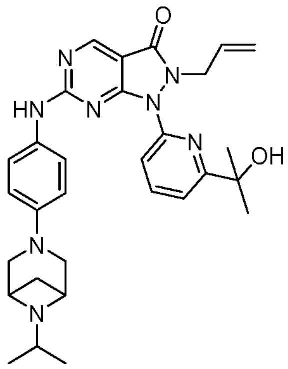 |
| WEE1-137 | 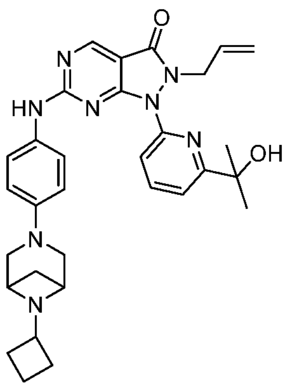 | WEE1-138 | 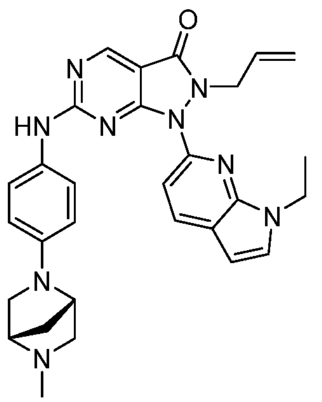 |
| WEE1-139 | 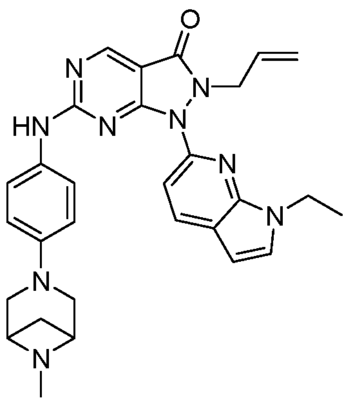 | WEE1-140 | 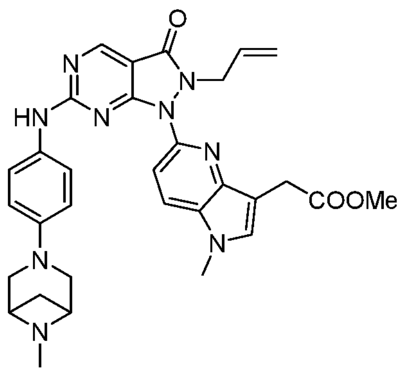 |

(continued)

| No. of the compound | Chemical structure | No. of the compound | Chemical structure |
|---|---|---|---|
| WEE1-141 | 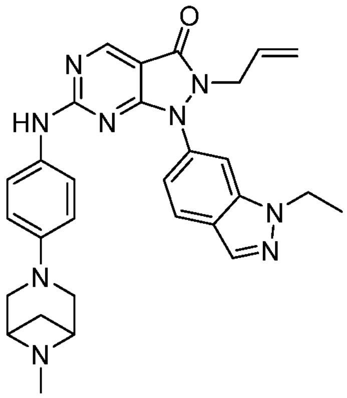 | WEE1-142 | 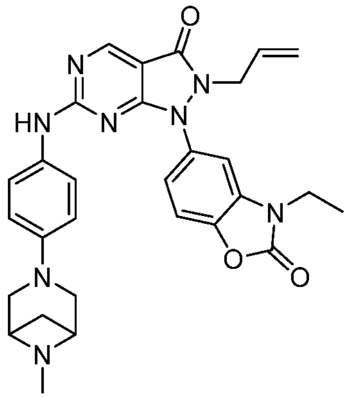 |
| WEE1-143 | 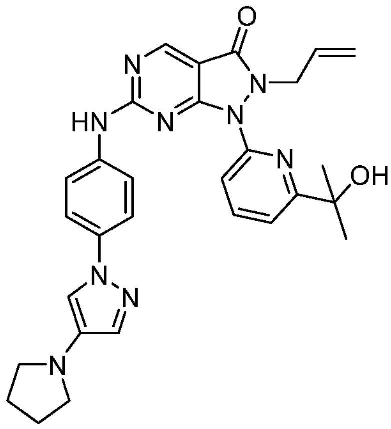 | WEE1-144 | 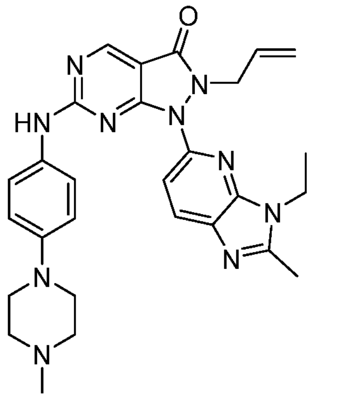 |
| WEE1-145 | 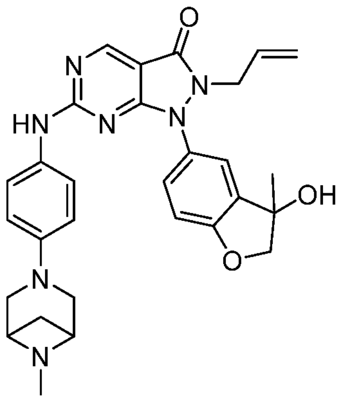 | WEE1-146 | 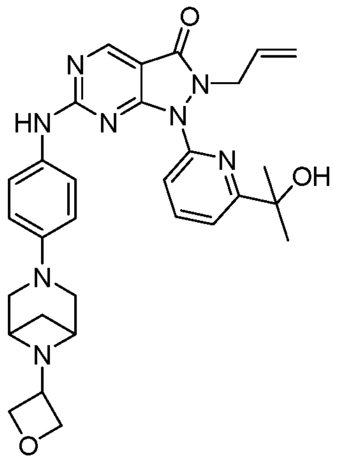 |

(continued)

| No. of the compound | Chemical structure | No. of the compound | Chemical structure |
|---|---|---|---|
| WEE1-147 | 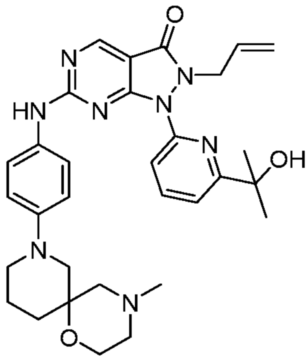 | WEE1-148 | 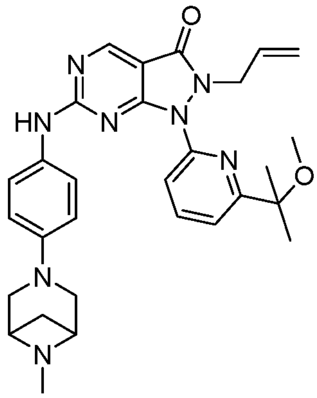 |
| WEE1-149 | 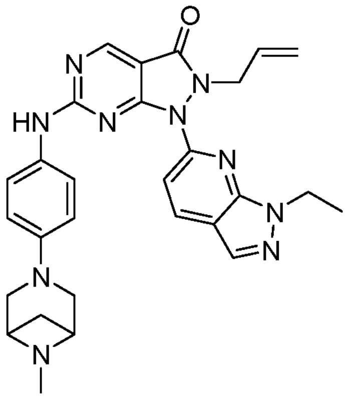 | WEE1-150 | 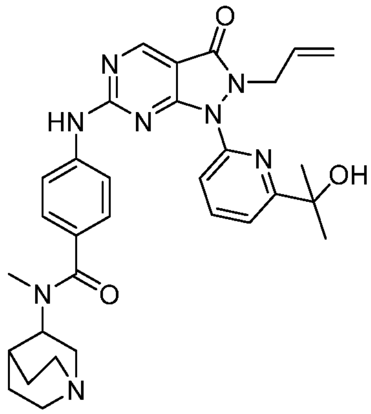 |
| WEE1-151 | 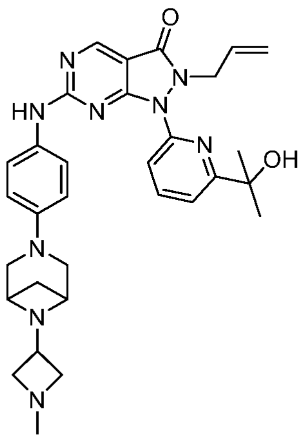 | WEE1-152 | 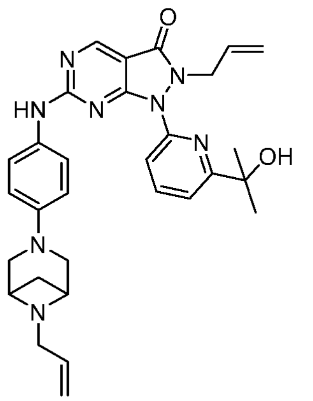 |
| WEE1-153 | 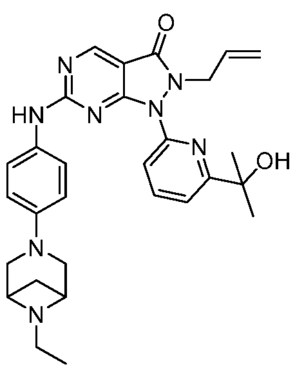 | WEE1-154 | 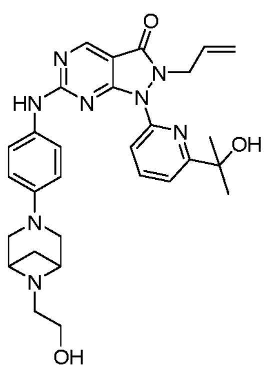 |

(continued)

| No. of the compound | Chemical structure | No. of the compound | Chemical structure |
|---|---|---|---|
| WEE1-155 | 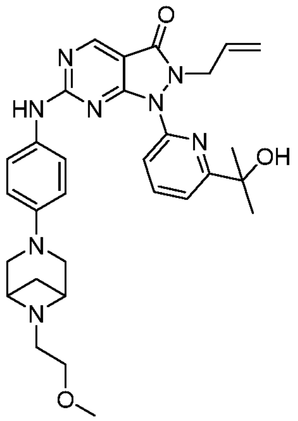 | WEE1-156 | 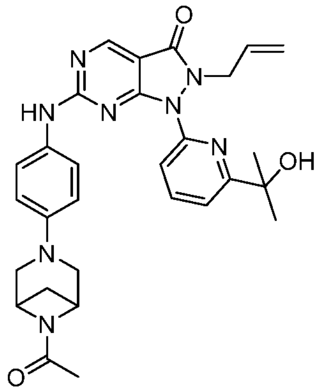 |
| WEE1-157 | 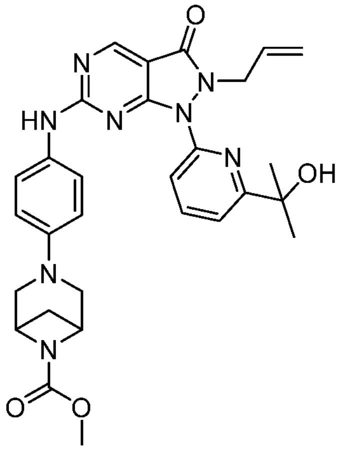 | WEE1-158 | 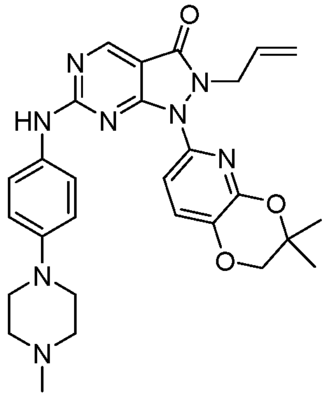 |
| WEE1-159 | 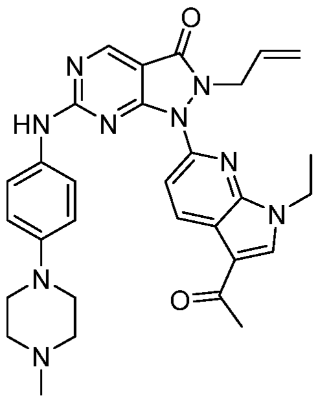 | WEE1-160 | 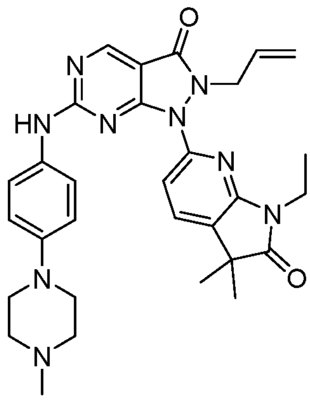 |

(continued)

| No. of the compound | Chemical structure | No. of the compound | Chemical structure |
|---|---|---|---|
| WEE1-161 | 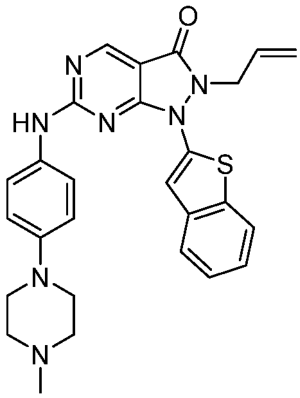 | WEE1-162 | 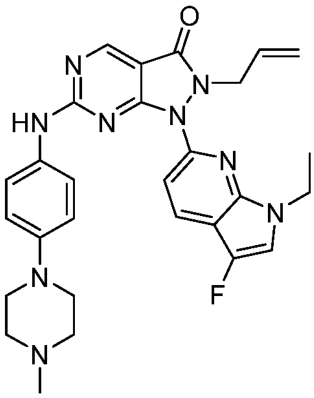 |
| WEE1-163 | 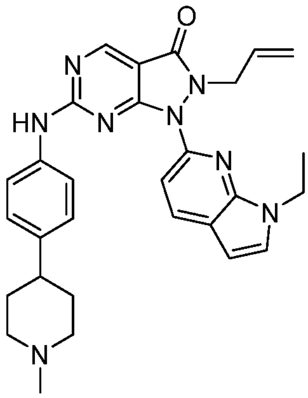 | WEE1-164 | 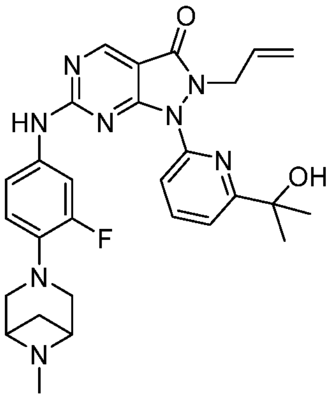 |
| WEE1-165 | 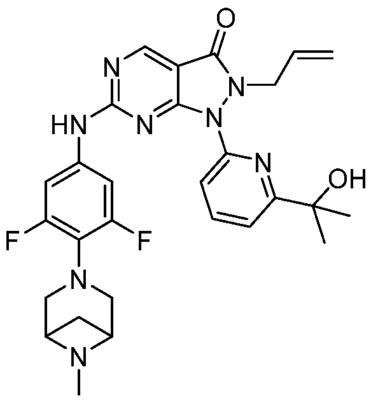 | WEE1-166 | 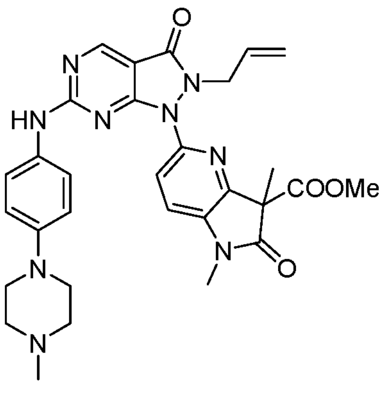 |
| WEE1-167 | 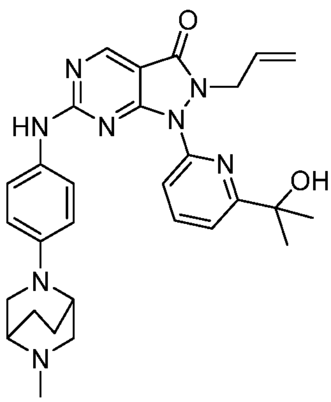 | WEE1-168 | 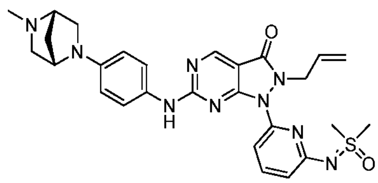 |

**17.** The compound, or a stereoisomer thereof, or a pharmaceutically acceptable salt thereof according to claim 1, wherein:
the specific compound represented by formula I is

| | | |
|---|---|---|
| 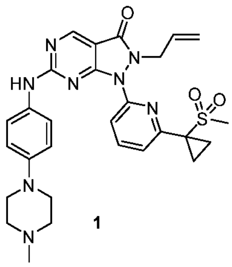 1 | 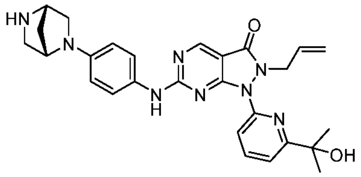 2 | 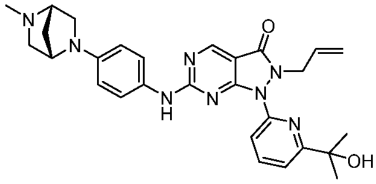 3 |
| 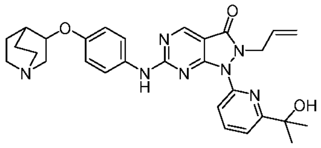 4 | 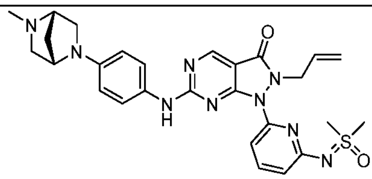 5 | 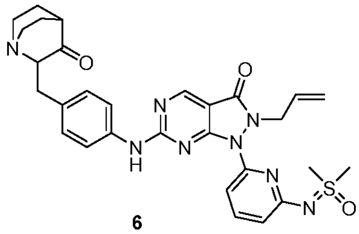 6 |

| | | |
|---|---|---|
| 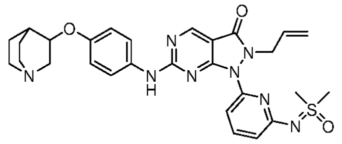 7 | 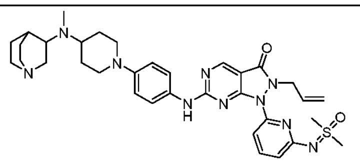 8 | 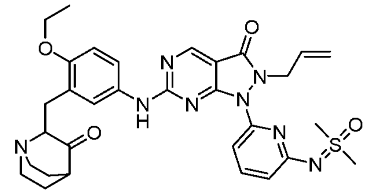 9 |
| 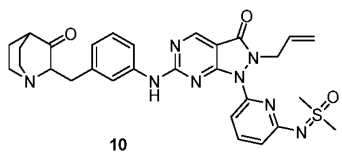 10 | 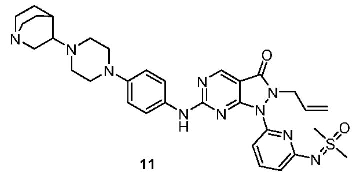 11 | 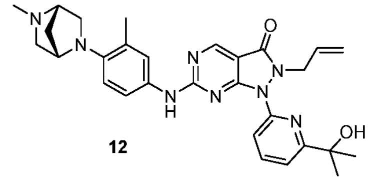 12 |
| 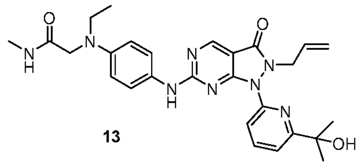 13 | 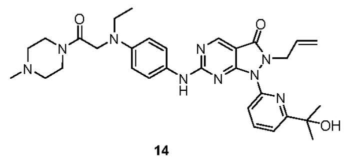 14 | 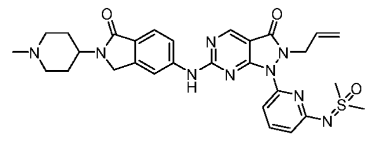 15 |
| 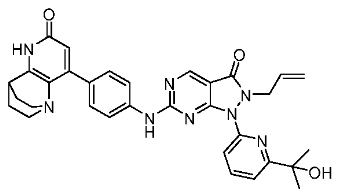 16 | 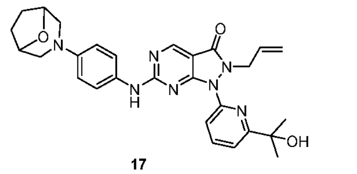 17 | 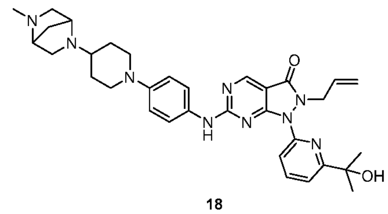 18 |
| 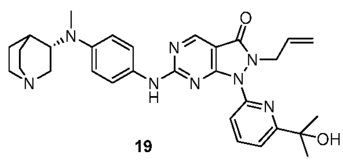 19 | 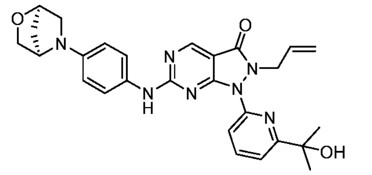 20 | 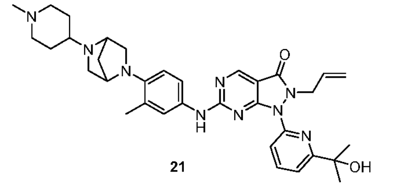 21 |
| 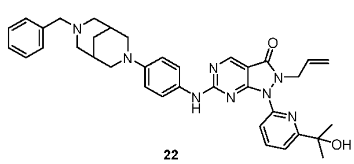 22 | 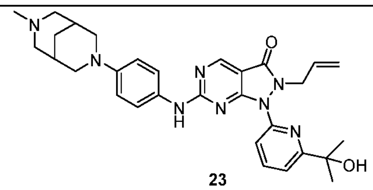 23 | 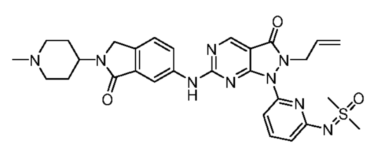 24 |
| 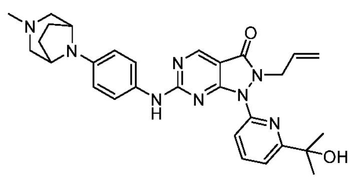 25 | 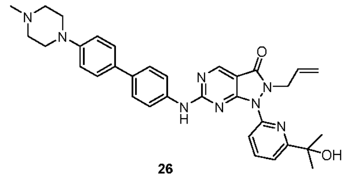 26 | 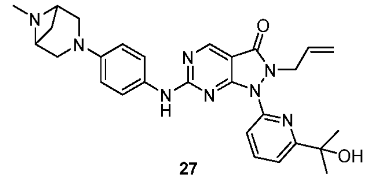 27 |

| | | |
|---|---|---|
| 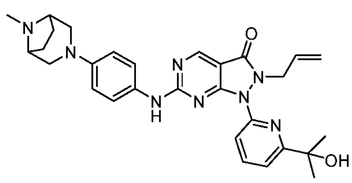 28 | 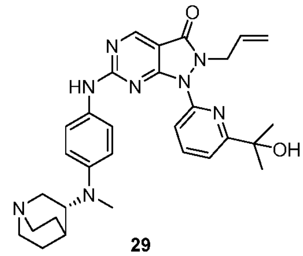 29 | 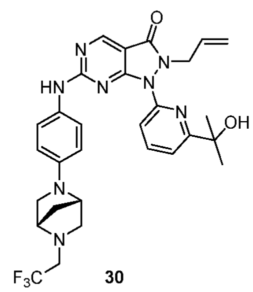 30 |
| 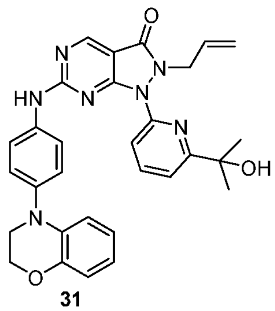 31 | 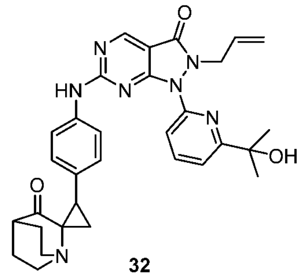 32 | 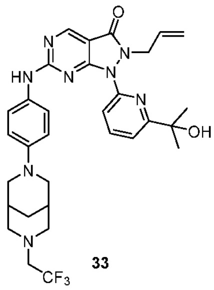 33 |
| 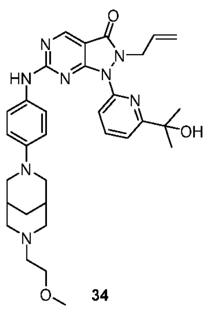 34 | 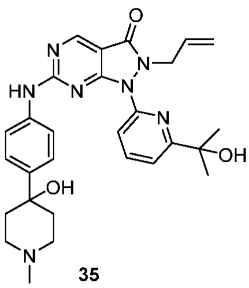 35 | 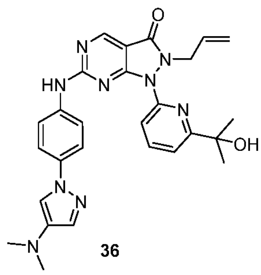 36 |
| 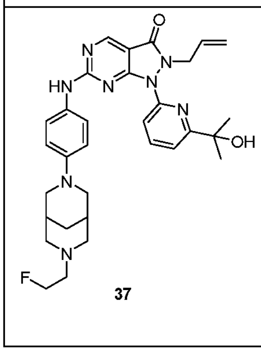 37 | 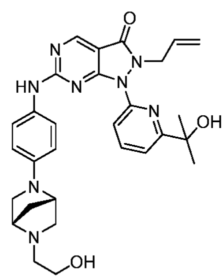 38 | 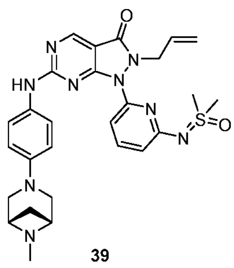 39 |

| | | |
|---|---|---|
| 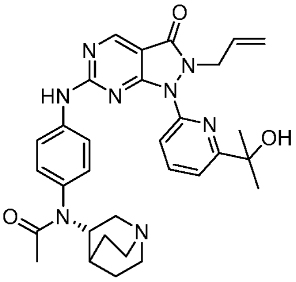 40 | 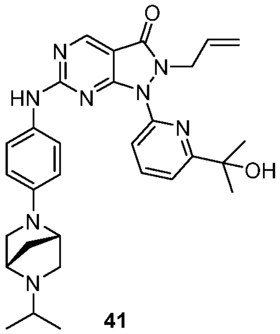 41 | 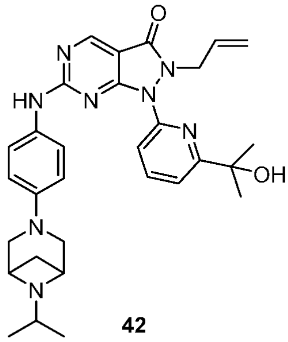 42 |
| 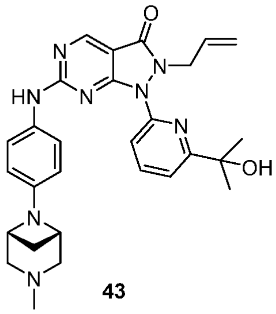 43 | 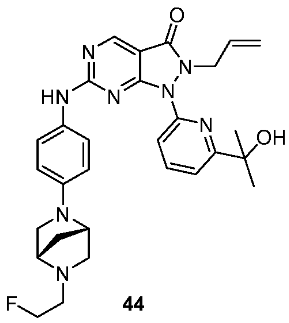 44 | 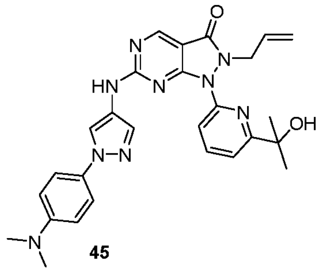 45 |
| 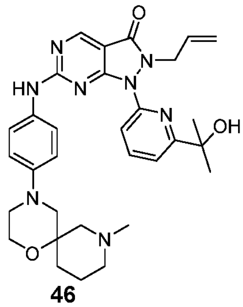 46 | 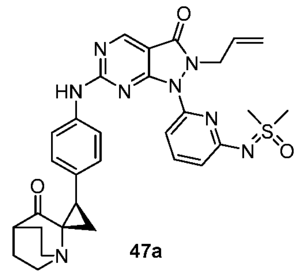 47a | 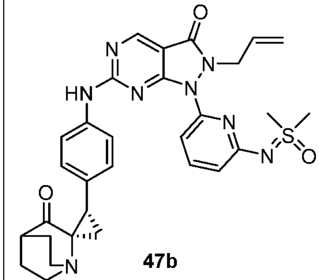 47b |
| 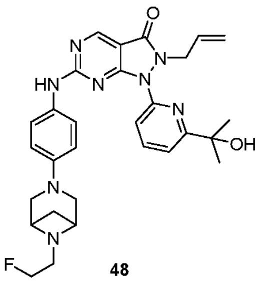 48 | 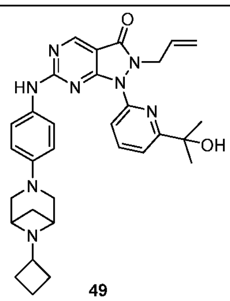 49 | 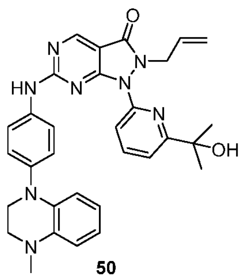 50 |

| | | |
|---|---|---|
| 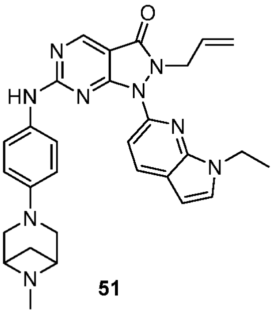 51 | 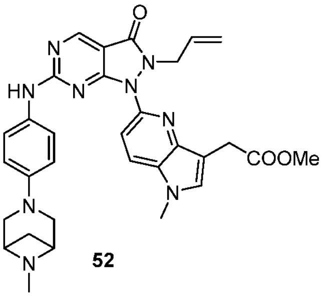 52 | 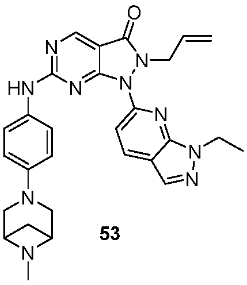 53 |
| 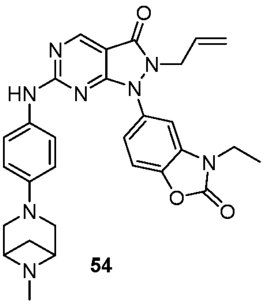 54 | 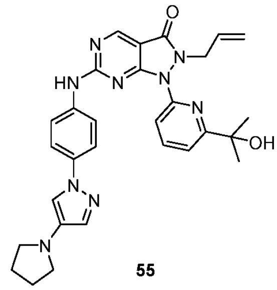 55 | 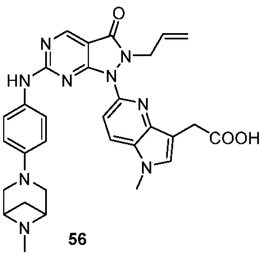 56 |
| 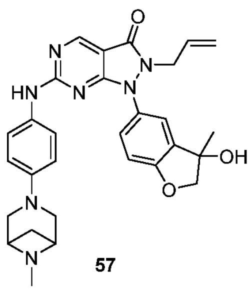 57 | 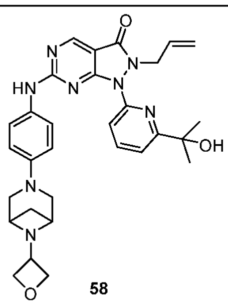 58 | 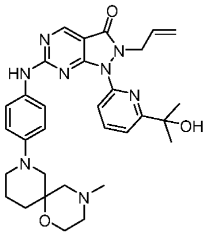 59 |
| 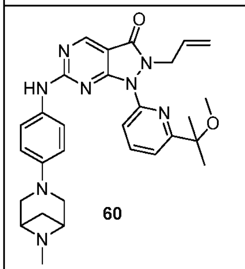 60 | 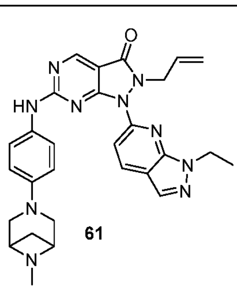 61 | 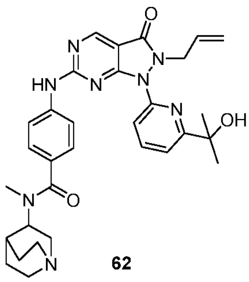 62 |
| 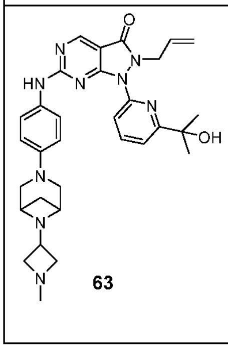 63 | 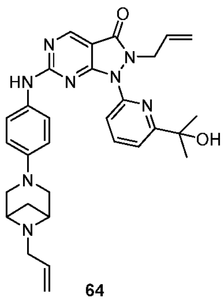 64 | 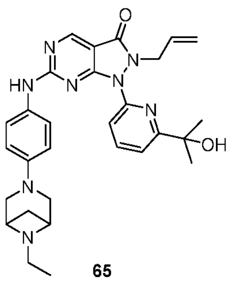 65 |

| | | |
|---|---|---|
| 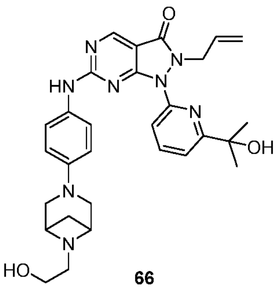 66 | 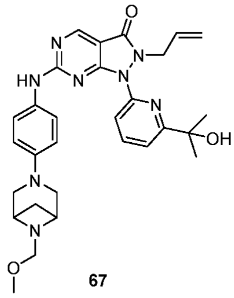 67 | 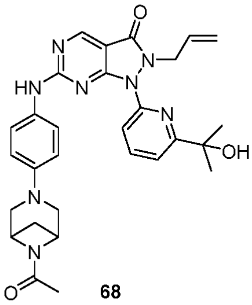 68 |
| 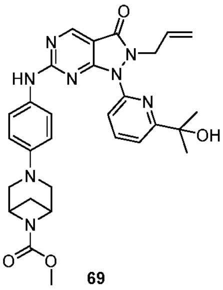 69 | 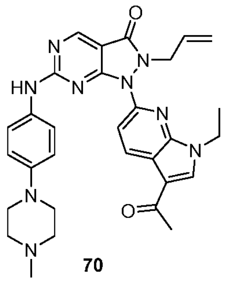 70 | 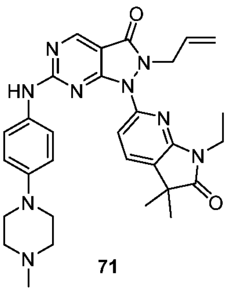 71 |
| 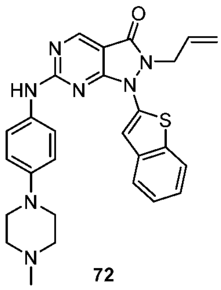 72 | 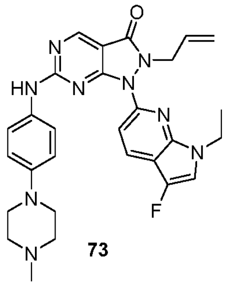 73 | 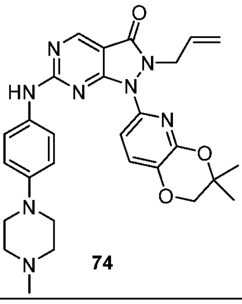 74 |
| 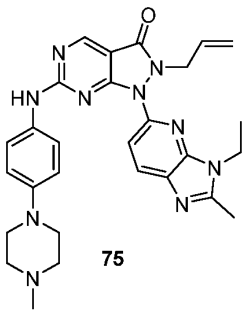 75 | 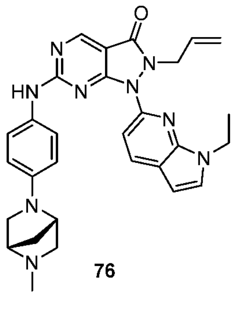 76 | 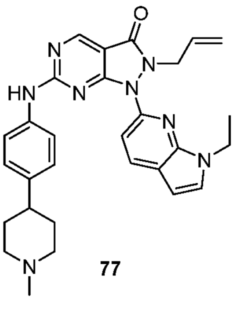 77 |
| 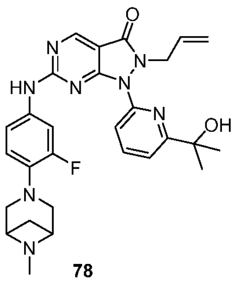 78 | 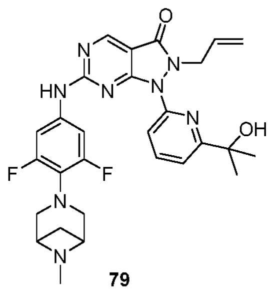 79 | 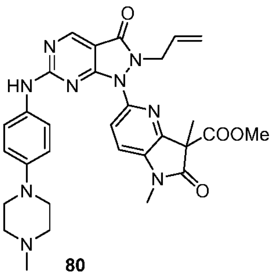 80 |

**18.** The compound, or a stereoisomer thereof, or a pharmaceutically acceptable salt thereof according to claim 1, wherein:

the specific compound represented by formula I is

| | | |
|---|---|---|
| 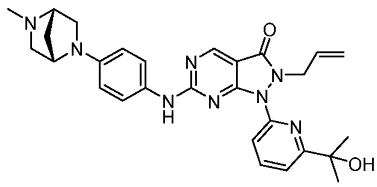 3 | 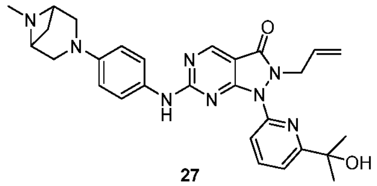 27 | 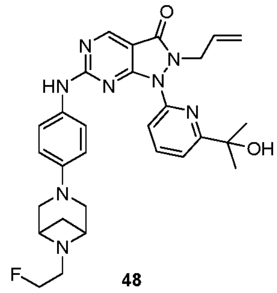 48 |
| 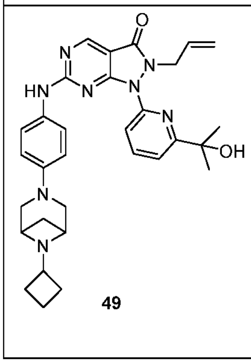 49 | 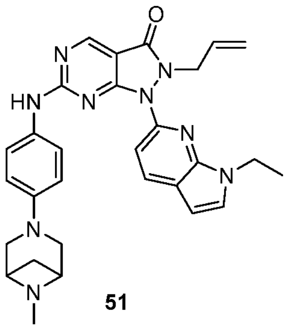 51 | 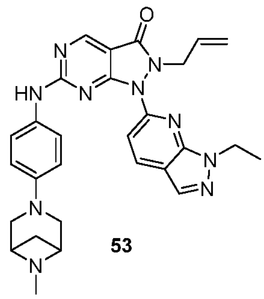 53 |

**19.** Use of the compound according to any one of claims 1 to 18, or a stereoisomer thereof, or a pharmaceutically acceptable salt thereof, in the preparation of the drug for the treatment of WEE1-mediated disease.

**20.** The use according to claim 19, wherein the WEE1-mediated disease is one or more of the diseases associated with inflammation, autoimmune disease, infectious disease, cancer, precancer syndrome.

**21.** A pharmaceutical composition, **characterized in that** it is a formulation prepared with the compound of any one of claims 1 to 18, or a stereoisomer thereof, or a pharmaceutically acceptable salt thereof, as the pharmaceutically active ingredient, together with pharmaceutically acceptable excipients.

| **INTERNATIONAL SEARCH REPORT** | International application No. **PCT/CN2023/070285** |
|---|---|

**A. CLASSIFICATION OF SUBJECT MATTER**

C07D487/04(2006.01)i;C07D487/08(2006.01)i;C07D491/04(2006.01)i;A61K31/519(2006.01)i;A61P29/02(2006.01)i;A61P35/00(2006.01)i;A61P37/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNTXT, WPABS, WPABSC, ENTXT, ENTXTC, DWPI, VEN, CNKI, STN CaPlus, STN Reg, STN CasReact: 嘧啶并吡唑; WEE1激酶抑制剂; 炎症; 抗炎; 自身免疫疾病; 自身免疫性疾病; 癌症; 肿瘤; anti-inflammatory; antiinflammatory; inflammation; inflammatory; autoimmune; immune; cancer?; tumor?

**C. DOCUMENTS CONSIDERED TO BE RELEVANT**

| Category* | Citation of document, with indication, where appropriate, of the relevant passages | Relevant to claim No. |
|---|---|---|
| X | CN 101432284 A (BANYU PHARMACEUTICAL CO., LTD.) 13 May 2009 (2009-05-13)<br>page 1, paragraph 1; compounds 1a -189 on pages 163-189 | 1-21 |
| X | CN 113402520 A (SHANGHAI INSTITUTE OF MATERIA MEDICA, CHINESE ACADEMY OF SCIENCES) 17 September 2021 (2021-09-17)<br>description, paragraphs 0003, 0005-0011, 0159-0161, and 0259-0268 | 1-21 |
| X | US 2010221211 A1 (Hidetomo Furuyama et al.) 02 September 2010 (2010-09-02)<br>description, paragraph 0001, and embodiments 1-42 | 1-21 |
| X | WO 2022011391 A1 (RECURIUM IP HOLDINGS, LLC) 13 January 2022 (2022-01-13)<br>claims 1 and 33-40, and description, paragraph 0002 | 1-21 |
| X | WO 2021252667 A1 (RECURIUM IP HOLDINGS, LLC) 16 December 2021 (2021-12-16)<br>description, paragraphs 0002, 0004, and 0008, and embodiment 1 | 1-21 |
| E | CN 115707708 A (XUANZHU BIOTECHNOLOGY CO., LTD.) 21 February 2023 (2023-02-21)<br>claims 8-11 | 1-21 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 March 2023** | **28 March 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer |
| Facsimile No. **(86-10)62019451** | Telephone No. |

Form PCT/ISA/210 (second sheet) (July 2022)

**INTERNATIONAL SEARCH REPORT**
**Information on patent family members**

International application No.
**PCT/CN2023/070285**

| Patent document cited in search report | Publication date (day/month/year) | Patent family member(s) | Publication date (day/month/year) |
|---|---|---|---|
| CN 101432284 A | 13 May 2009 | NO 20084968 L | 26 November 2008 |
| | | NO 341617 B1 | 11 December 2017 |
| | | HK 1132498 A1 | 26 February 2010 |
| | | PT 2016080 E | 14 October 2010 |
| | | RU 2008146759 A | 10 June 2010 |
| | | RU 2437885 C2 | 27 December 2011 |
| | | CY 1118526 T1 | 12 July 2017 |
| | | NZ 571196 A | 31 March 2011 |
| | | AT 475662 T | 15 August 2010 |
| | | PT 2017278 T | 03 January 2017 |
| | | TW 200811176 A | 01 March 2008 |
| | | TWI 409262 B | 21 September 2013 |
| | | BRPI 0710081 A2 | 02 August 2011 |
| | | WO 2007126122 A1 | 08 November 2007 |
| | | US 2007254892 A1 | 01 November 2007 |
| | | US 7834019 B2 | 16 November 2010 |
| | | DE 602007008085 D1 | 09 September 2010 |
| | | SI 2017278 T1 | 31 May 2017 |
| | | AU 2007244185 A1 | 08 November 2007 |
| | | AU 2007244185 B2 | 04 October 2012 |
| | | US 2014303178 A1 | 09 October 2014 |
| | | MX 2008013063 A | 16 December 2008 |
| | | PL 2016080 T3 | 31 March 2011 |
| | | DK 2016080 T3 | 22 November 2010 |
| | | CR 10359 A | 19 February 2009 |
| | | MY 145408 A | 15 February 2012 |
| | | AR 060635 A1 | 02 July 2008 |
| | | EP 2017278 A1 | 21 January 2009 |
| | | EP 2017278 A4 | 22 April 2009 |
| | | EP 2017278 B1 | 02 November 2016 |
| | | US 2011189130 A1 | 04 August 2011 |
| | | US 8791125 B2 | 29 July 2014 |
| | | US 2010063024 A1 | 11 March 2010 |
| | | US 7935708 B2 | 03 May 2011 |
| | | ES 2609087 T3 | 18 April 2017 |
| | | DK 2017278 T3 | 13 February 2017 |
| | | MA 30428 B1 | 04 May 2009 |
| | | PL 2017278 T3 | 31 October 2017 |
| | | WO 2007126128 A1 | 08 November 2007 |
| | | IL 194367 A0 | 03 August 2009 |
| | | IL 194367 A | 31 December 2014 |
| | | SI 2016080 T1 | 31 March 2011 |
| | | JPWO 2007126128 A1 | 17 September 2009 |
| | | JP 4513919 B2 | 28 July 2010 |
| | | HRP 20161763 T1 | 24 February 2017 |
| | | HUE 032987 T2 | 28 November 2017 |
| | | ZA 200807748 B | 28 October 2009 |
| | | KR 20090017491 A | 18 February 2009 |
| | | KR 101409161 B1 | 19 June 2014 |
| | | EP 2016080 A1 | 21 January 2009 |

Form PCT/ISA/210 (patent family annex) (July 2022)

**INTERNATIONAL SEARCH REPORT**
**Information on patent family members**

International application No.
**PCT/CN2023/070285**

| Patent document cited in search report | | | Publication date (day/month/year) | Patent family member(s) | | | Publication date (day/month/year) |
|---|---|---|---|---|---|---|---|
| | | | | EP | 2016080 | A4 | 13 May 2009 |
| | | | | EP | 2016080 | B1 | 28 July 2010 |
| | | | | ES | 2348751 | T3 | 13 December 2010 |
| | | | | SV | 2009003060 | A | 28 April 2009 |
| | | | | HN | 2008001532 | A | 10 June 2009 |
| | | | | GT | 200800211 | A | 07 April 2009 |
| | | | | DOP | 2007000084 | A | 31 December 2007 |
| | | | | UA | 96152 | C2 | 10 October 2011 |
| | | | | JP | 2010132689 | A | 17 June 2010 |
| | | | | JP | 5167291 | B2 | 21 March 2013 |
| | | | | LT | 2017278 | T | 25 January 2017 |
| | | | | HRP | 20100563 | T1 | 30 November 2010 |
| | | | | PE | 20080695 | A1 | 28 June 2008 |
| | | | | ECSP | 088812 | A | 27 November 2008 |
| | | | | CA | 2650119 | A1 | 08 November 2007 |
| | | | | CA | 2650119 | C | 14 March 2017 |
| CN | 113402520 | A | 17 September 2021 | None | | | |
| US | 2010221211 | A1 | 02 September 2010 | WO | 2009054332 | A1 | 30 April 2009 |
| | | | | EP | 2213673 | A1 | 04 August 2010 |
| | | | | EP | 2213673 | A4 | 07 September 2011 |
| | | | | EP | 2213673 | B1 | 05 June 2013 |
| | | | | AU | 2008315048 | A1 | 30 April 2009 |
| | | | | US | 8329711 | B2 | 11 December 2012 |
| | | | | JPWO | 2009054332 | A1 | 03 March 2011 |
| | | | | CA | 2703489 | A1 | 30 April 2009 |
| WO | 2022011391 | A1 | 13 January 2022 | AU | 2021305347 | A1 | 16 February 2023 |
| | | | | CA | 3188737 | A1 | 13 January 2022 |
| | | | | TW | 202216716 | A | 01 May 2022 |
| WO | 2021252667 | A1 | 16 December 2021 | TW | 202214638 | A | 16 April 2022 |
| | | | | AU | 2021287910 | A1 | 02 February 2023 |
| | | | | CA | 3186632 | A1 | 16 December 2021 |
| CN | 115707708 | A | 21 February 2023 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007126122 A
- WO 2008133866 A
- WO 2013012681 A
- WO 2013126656 A
- WO 2014167347 A
- WO 2015092431 A
- WO 2018011569 A
- WO 2018011570 A
- WO 2018090939 A
- WO 2018133829 A
- WO 2018171633 A


### Non-patent literature cited in the description


- *Clinical Cancer Research*, 2011, vol. 17 (13), 4200-4207
- *Molecular&CellularBiology*, 2012, vol. 32 (20), 4226
- *Proceedings of the National Academy of Sciences of the United States of America*, 2007, vol. 104 (10), 3753-3758
- *Oncotarget*, 2016, vol. 7 (31), 49902-49916
- *MolecularCancer*, 2014, vol. 13 (1), 72
- *Drug News&Perspectives*, 2010, vol. 23 (7), 425
- *Molecular Cancer Therapeutics*, 2013, vol. 12 (12), 2675-2684
- *Cancer Biology&Therapy*, 2010, vol. 9 (7), 523-525
- *CHEMICAL ABSTRACTS*, 955365-80-7